# EUROPEAN PATENT APPLICATION

(11) **EP 4 306 520 A1**
(43) Date of publication of application: **17.01.2024**
(21) Application number: 22766234.3
(22) Date of filing: 04.03.2022
(51) Int. Cl.: C07D 409/04, C07D 409/06, A61K 31/381, A61P 35/00

(54) **THIOPHENE COMPOUND AND APPLICATION THEREOF**

(30) Priority: 11.03.2021 CN 202110268287; 25.05.2021 CN 202110573989; 19.10.2021 CN 202111217911; 24.02.2022 CN 202210172993
(71) Applicant: Medshine Discovery Inc., Nanjing, Jiangsu 210032 (CN)
(72) Inventor: WU, Lingyun, Shanghai 200131 (CN); ZHAN, Zhen, Shanghai 200131 (CN); ZHAO, Lele, Shanghai 200131 (CN); SUN, Jianjun, Shanghai 200131 (CN); QIAN, Yi, Shanghai 200131 (CN); CHEN, Shuhui, Shanghai 200131 (CN)
(74) Representative: Dehns
(86) International application number: PCT/CN2022/079277
(87) International publication number: WO 2022/188709

(57) **Abstract**

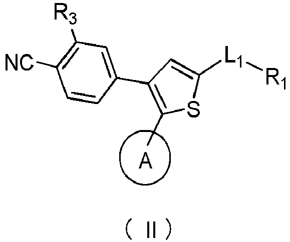

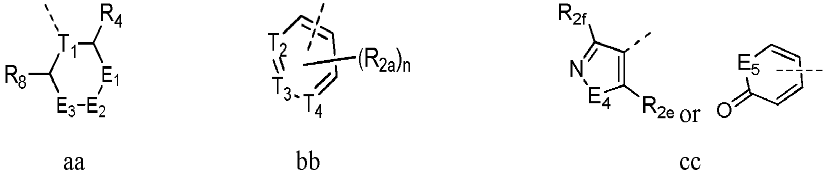

The present invention relates to a thiophene compound and an application thereof. The present invention specifically relates to a compound represented by formula (II) and a pharmaceutically acceptable salt thereof, wherein L₁ is -C(=O)- or -C(=O)-NH-; R₁ is R₁ₐ or aa; ring A is bb or cc; R₁ₐ is C₁₋₃ alkyl substituted with 1, 2 or 3 Rₐ; and R₃ is independently H, halogen, or C₁₋₃ alkoxy. The compound represented by formula (II) and the pharmaceutically acceptable salt thereof can better inhibit the activity of LSD1, further shows significant inhibitory activity against NCI-H1417 cell proliferation, and has good pharmacokinetic properties (including good oral bioavailability, oral exposure, half-life, and clearance rate, etc.).

## Description

The present application claims the right of the following priorities:
CN202110268287.6, March 11, 2021;
CN202110573989.5, May 25, 2021;
CN202111217911.6, October 19, 2021;
CN202210172993.5, February 24, 2022.

### TECHNICAL FIELD

The present disclosure relates to a class of thiophene compounds and a use thereof. The present disclosure specifically relates to a compound of formula (I) and a pharmaceutically acceptable salt thereof.

### BACKGROUND

Post-translational modifications of histones, which comprise processes such as methylation, acetylation, phosphorylation, and ubiquitination, are important regulatory mechanisms in epigenetics, affecting gene expression by altering chromatin structure. [Xueshun Wang, Boshi Huang, Takayoshi Suzuki et al. Epigenomics, 2015, 1379-1396]. Although these modifications do not alter the basic DNA sequence, such epigenetic alteration may persist through cell division throughout the cell life cycle or iteration process [Adrian Bird, Nature, 2007, 396-398]. Therefore, abnormalities in epigenetic function are closely associated with the pathological processes of various diseases [James T Lynch, William J Harris & Tim C P Somervaille, Expert Opin. Ther. Targets, 2012, 1239-1249], such as a variety of solid tumors, hematological tumors, viral infections, and neurological abnormalities. As a result, epigenetics has now become a research hotspot in the field of drug research and development. The methylation state of histones is jointly regulated by histone methyltransferases and histone demethylases. Lysine specific demethylase 1 (LSD1, also known as KDM1A) is the first reported histone lysine demethylase, which is widely involved in transcriptional regulation and affects many physiological processes such as cell proliferation and differentiation and embryonic stem cell pluripotency, by regulating the methylation state of histone lysine. [Yujiang Shi, Fei Lan, Caitlin Matson et al., Cell, 2004, 941-953] [Daniel P. Mould, Alison E. McGonagle, Daniel H. Wiseman et al., Medicinal Research Reviews, 2015, 586-618]. The LSD1 structure comprises three main parts: the N-terminal SWIRM domain, the C-terminal amine oxidase-like (AOL) domain, and the central Tower domain [Ruchi Anand, Ronen Marmorstein, Journal of Biological Chemistry, 2007, 35425-35429]. The C-terminal amine oxidase-like domain comprises two active pockets, one is the FAD binding site and the other is a site used to recognize and bind to substrates [Pete Stavropoulos, Günter Blobel, André Hoelz, Nature Structral & Molecular Biology, 2006, 626-632]. The function of the SWIRM domain is not yet clear. It does not directly participate in FAD or substrate binding, but mutations or deletions in this domain will reduce LSD1 activity. Therefore, it is speculated that this domain may affect the active region by adjusting the conformation [Yong Chen, Yuting Yang, Feng Wang et al., Proceedings of the National Academy of Sciences, 2006, 13956-13961]. The Tower domain is the binding domain of LSD1 with other protein factors. LSD1 binds to different protein factors and acts on different substrates, thereby exerting different regulatory effects on histones as well as gene expression. For example, when LSD1 binds to CoREST, it acts preferentially on histone H3K4, removing activation-related histone marks and repressing gene transcription through demethylation; whereas, when bound to androgen receptor proteins, recombinant LSD1 acts preferentially on H3K9, activating androgen receptor-related gene transcription through demethylation [Ruchi Anand, Ronen Marmorstein, Journal of Biological Chemistry, 2007, 35425-35429; Eric Metzger, Melanie Wissmann, Na Yin et al. Nature, 2005, 436-439]. In addition, LSD1 also regulates the methylation state of some non-histone substrates, including oncogene p53, DNA methyltransferase 1 (DNMT1), etc. [Yi Chao Zheng, Jinlian Ma, Zhiru Wang, Medicinal Research Reviews, 2015, 1032-1071].

LSD1 is a FAD-dependent amine oxidase in which proton transfer is considered as its most likely oxidation mechanism [Zheng Y C, Yu B, Chen Z S, et al. Epigenomics, 2016, 8, 651-666]. First, the N-CH3 bond of the substrate is converted to an imine bond by proton transfer, and this imine ion intermediate undergoes a hydrolysis reaction to produce a demethylated amine on one side and formaldehyde on the other. During this catalytic cycle process, FAD is reduced to FADH2, which is subsequently oxidized back to FAD by a molecule of oxygen while generating a molecule of H2O2 [Yujiang Shi, Fei Lan, Caitlin Matson, Cell, 2004, 941-953].

LSD1 is abnormally expressed in various types of tumors. LSD1 is highly expressed in acute myeloid leukemia (AML) subtypes and is an important factor in maintaining the potential of leukemia stem cell (LSC). LSD1 is highly expressed in a variety of solid tumors such as lung cancer, breast cancer, prostate cancer, liver cancer, and pancreatic cancer, and is closely associated with poor tumor prognosis. LSD1 inhibits cadherin expression and is closely associated with tumor invasion and epithelial-mesenchymal transition (EMT). [Hosseini A, Minucci S. Epigenomics, 2017, 9, 1123-1142].

Currently, no drugs for LSD1 inhibitors are approved in the market. There are 8 drugs in clinical research, mainly used for the treatment of diseases such as hematological tumors, small cell lung cancer, and Ewing's sarcoma. However, in the face of a huge unmet market, candidate compounds with better activity and pharmacokinetic parameters are still needed in the field for advancing clinical trials to meet treatment needs.

### CONTENT OF THE PRESENT INVENTION

The present disclosure provides a compound of formula (II) or a pharmaceutically acceptable salt thereof, 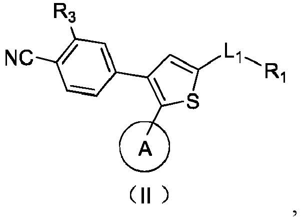
wherein L₁ is -C(=O)- or -C(=O)-NH-;
R₁ is R₁ₐ or 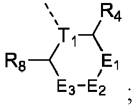
ring A is 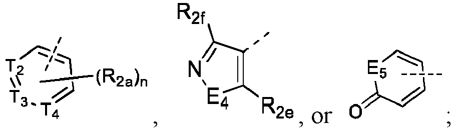
T₁ is CH or N;
T₂ is CR_{2c} or N;
T₃ is CR₂ or N;
T₄ is CR_{2b} or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
E₄ is O, S, CH₂, or NR_{2g};
E₅ is O, CH₂, or NR_{2g};
R₁ₐ is C₁₋₃ alkyl substituted by 1, 2, or 3 Rₐ;
R₂ₐ is H or halogen;
R₂ and R_{2b} are each independently H, halogen, NH₂, C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, or -NHC₁₋₃ alkyl-C₃₋₆ cycloalkyl, wherein the C₁₋₃ alkyl, C₁₋₃ alkoxy, - S(=O)₂C₁₋₃ alkyl, -NHC₁₋₃ alkyl, 4- to 6-membered heterocycloalkyl, -C(=O)-4- to 6-membered heterocycloalkyl, and -NHC₁₋₃ alkyl-C₃₋₆ cycloalkyl are each independently and optionally substituted by 1, 2, or 3 R_{b};
R_{2c} is H;
or, R₂ and R_{2c}, together with the C atom to which they are attached, make the structural moiety 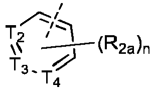 into 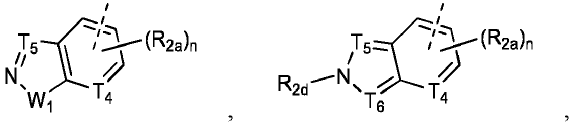 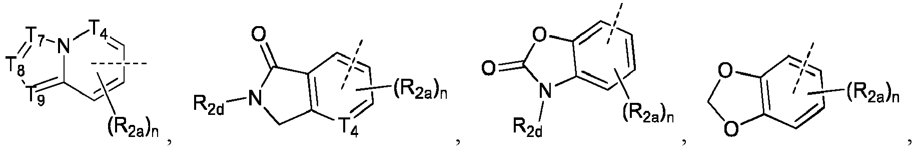 or 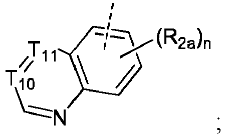
T₅, T₆, T₇, T₈, T₉, T₁₀, and T₁₁ are each independently N or CRt;
W₁ isO or NR_{2d};
R_{2d} is H, C₁₋₄ alkyl, C₃₋₆ cycloalkyl, 4- to 6-membered heterocycloalkyl, -C₁₋₃ alkyl-C₃₋₆ cycloalkyl, or -C₁₋₃ alkyl-4- to 6-membered heterocycloalkyl, wherein the C₁₋₄ alkyl, C₃₋₆ cycloalkyl, 4- to 6-membered heterocycloalkyl, -C₁₋₃ alkyl-C₃₋₆ cycloalkyl, and -C₁₋₃ alkyl-4-to 6-membered heterocycloalkyl are each independently and optionally substituted by 1, 2, or 3 R_{c};
R₂ₑ, R_{2f}, and R_{2g} are each independently H or C₁₋₃ alkyl;
each R₃ is independently H, halogen, or C₁₋₃ alkoxy;
R₄ and R₈ are each independently H or C₁₋₃ alkyl;
each R₅ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{d};
R₆ is H or C₁₋₃ alkyl, wherein the C₁₋₃ alkyl is optionally substituted by 1, 2, or 3 Rₑ;
each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 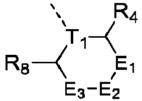 into 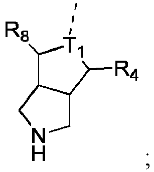
or, when E₂ is -C(R₆₁)₂-, two R₆₁, together with the C atom to which they are attached, make the structural moiety 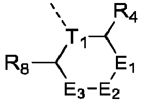 into 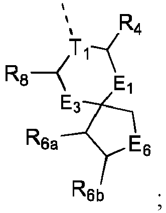
or, when E₃ is a single bond, R₄ and R₈, together with the C atom to which they are attached, make the structural moiety 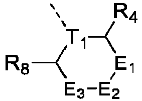 into 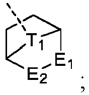
or, when T₁ is CH and E₁ is -C(R₅)₂-, R₅ and R₈, together with the C atom to which they are attached, make the structural moiety 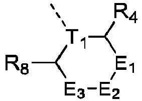 into 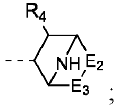
E₆ is NR_{6c} or O;
R₆ₐ and R_{6b} are each independently H, halogen, OH, =O, NH₂, or C₁₋₃ alkyl;
R_{6c} is H or C₁₋₃ alkyl;
Rₐ, R_{b}, R_{c}, R_{d}, Rₑ, R_{f}, and R_{g} are each independently F, Cl, Br, OH, CN, COOH, NH₂, C₁₋₃ alkyl, or C₁₋₃ alkoxy;
each Rₜ is independently H, F, Cl, Br, OH, CN, COOH, NH₂, C₁₋₃ alkyl, or C₁₋₃ alkoxy;
n is 1 or 2;
the 4- to 6-membered heterocycloalkyl contains 1, 2, 3, or 4 heteroatoms or heteroatom groups independently selected from -O-, -NH-, -S-, and N.

The present disclosure provides a compound of formula (II) or a pharmaceutically acceptable salt thereof, 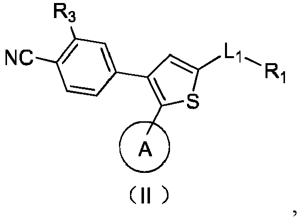
wherein L₁ is -C(=O)- or -C(=O)-NH-;
R₁ is R₁ₐ or 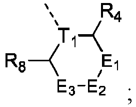
ring A is 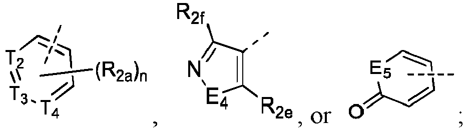
T₁ is CH or N;
T₂ is CR_{2c} or N;
T₃ is CR₂ or N;
T₄ is CR_{2b} or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
E₄ is O, S, CH₂, or NR_{2g};
E₅ is O, CH₂, or NR_{2g};
R₁ₐ is C₁₋₃ alkyl substituted by 1, 2, or 3 Rₐ;
R₂ₐ is H or halogen;
R₂ and R_{2b} are each independently H, halogen, NH₂, C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, or -NHC₁₋₃ alkyl-C₃₋₆ cycloalkyl, wherein the C₁₋₃ alkyl, C₁₋₃ alkoxy, - S(=O)₂C₁₋₃ alkyl, -NHC₁₋₃ alkyl, 4- to 6-membered heterocycloalkyl, -C(=O)-4- to 6-membered heterocycloalkyl, and -NHC₁₋₃ alkyl-C₃₋₆ cycloalkyl are each independently and optionally substituted by 1, 2, or 3 R_{b};
R_{2c} is H;
or, R₂ and R_{2c}, together with the C atom to which they are attached, make the structural moiety 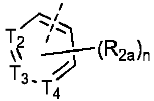 into 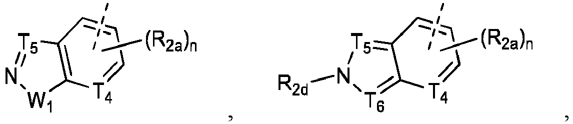 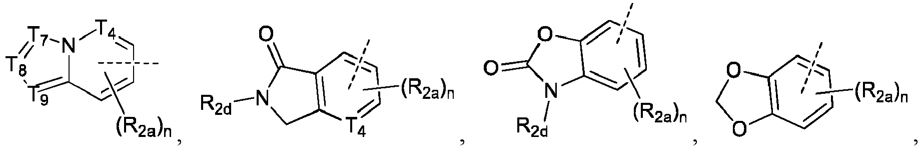 or 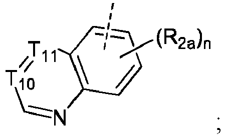
T₅, T₆, T₇, T₈, T₉, T₁₀, and T₁₁ are each independently N or CRt;
W₁ isO or NR_{2d};
R_{2d} is H, C₁₋₄ alkyl, C₃₋₆ cycloalkyl, 4- to 6-membered heterocycloalkyl, -C₁₋₃ alkyl-C₃₋₆ cycloalkyl, or -C₁₋₃ alkyl-4- to 6-membered heterocycloalkyl, wherein the C₁₋₄ alkyl, C₃₋₆ cycloalkyl, 4- to 6-membered heterocycloalkyl, -C₁₋₃ alkyl-C₃₋₆ cycloalkyl, and -C₁₋₃ alkyl-4-to 6-membered heterocycloalkyl are each independently and optionally substituted by 1, 2, or 3 R_{c};
R₂ₑ, R_{2f}, and R_{2g} are each independently H or C₁₋₃ alkyl;
each R₃ is independently H, halogen, or C₁₋₃ alkoxy;
R₄ and R₈ are each independently H or C₁₋₃ alkyl;
each R₅ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{d};
R₆ is H or C₁₋₃ alkyl, wherein the C₁₋₃ alkyl is optionally substituted by 1, 2, or 3 Rₑ;
each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 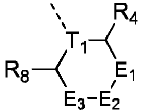 into 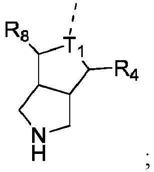
or, when E₂ is -C(R₆₁)₂-, two R₆₁, together with the C atom to which they are attached, make the structural moiety 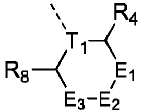 into 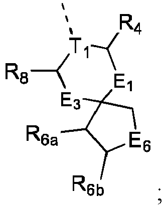
or, when E₃ is a single bond, R₄ and R₈, together with the C atom to which they are attached, make the structural moiety 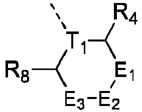 into 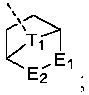
or, when T₁ is CH and E₁ is -C(R₅)₂-, R₅ and R₈, together with the C atom to which they are attached, make the structural moiety 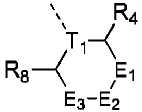 into 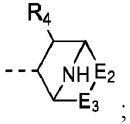
E₆ is NR_{6c} or O;
R₆ₐ and R_{6b} are each independently H, halogen, OH, =O, NH₂, or C₁₋₃ alkyl;
R_{6c} is H or C₁₋₃ alkyl;
Rₐ, R_{b}, R_{c}, R_{d}, Rₑ, R_{f}, R_{g}, and Rₜ are each independently F, Cl, Br, OH, CN, COOH, NH₂, C₁₋₃ alkyl, or C₁₋₃ alkoxy;
n is 1 or 2;
the 4- to 6-membered heterocycloalkyl contains 1, 2, 3, or 4 heteroatoms or heteroatom groups independently selected from -O-, -NH-, -S-, and N.

The present disclosure provides a compound of formula (II) or a pharmaceutically acceptable salt thereof, 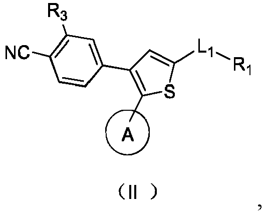
wherein L₁ is -C(=O)- or -C(=O)-NH-;
R₁ is R₁ₐ or 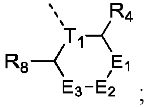
ring A is 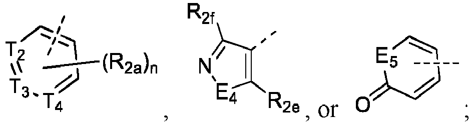
T₁ is CH or N;
T₂ is CR_{2c} or N;
T₃ is CR₂ or N;
T₄ is CR_{2b} or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
E₄ is O, S, CH₂, or NR_{2g};
E₅ is O, CH₂, or NR_{2g};
R₁ₐ is C₁₋₃ alkyl substituted by 1, 2, or 3 Rₐ;
R₂ₐ is H or halogen;
R₂ and R_{2b} are each independently H, halogen, NH₂, C₁₋₃ alkyl, C₁₋₃ alkoxy, -S(=O)₂C₁₋₃ alkyl, 4- to 6-membered heterocycloalkyl, or -C(=O)-4- to 6-membered heterocycloalkyl, wherein the C₁₋₃ alkyl, C₁₋₃ alkoxy, -S(=O)₂C₁₋₃ alkyl, 4- to 6-membered heterocycloalkyl, and -C(=O)-4- to 6-membered heterocycloalkyl are each independently and optionally substituted by 1, 2, or 3 R_{b};
R_{2c} is H;
or, R₂ and R_{2c}, together with the C atom to which they are attached, make the structural moiety 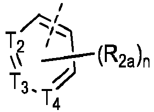 into 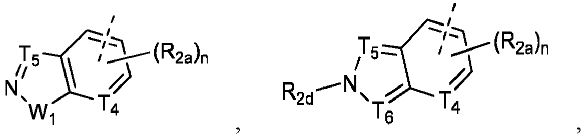 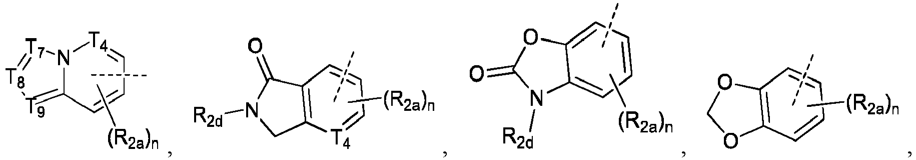 or 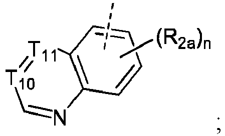
T₅, T₆, T₇, T₈, T₉, T₁₀, and T₁₁ are each independently N or CRt;
W₁ isO or NR_{2d};
R_{2d} is H, C₁₋₄ alkyl, C₃₋₆ cycloalkyl, 4- to 6-membered heterocycloalkyl, -C₁₋₃ alkyl-C₃₋₆ cycloalkyl, or -C₁₋₃ alkyl-4- to 6-membered heterocycloalkyl, wherein the C₁₋₄ alkyl, C₃₋₆ cycloalkyl, 4- to 6-membered heterocycloalkyl, -C₁₋₃ alkyl-C₃₋₆ cycloalkyl, and -C₁₋₃ alkyl-4-to 6-membered heterocycloalkyl are optionally substituted by 1, 2, or 3 R_{c};
R₂ₑ, R_{2f}, and R_{2g} are each independently H or C₁₋₃ alkyl;
each R₃ is independently H, halogen, or C₁₋₃ alkoxy;
R₄ and R₈ are each independently H or C₁₋₃ alkyl;
each R₅ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{d};
R₆ is H or C₁₋₃ alkyl, wherein the C₁₋₃ alkyl is optionally substituted by 1, 2, or 3 Rₑ;
each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 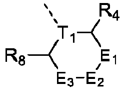 into 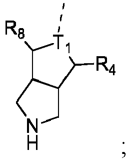
or, when E₂ is -C(R₆₁)₂-, two R₆₁, together with the C atom to which they are attached, make the structural moiety 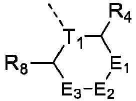 into 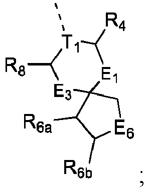
or, when E₃ is a single bond, R₄ and R₈, together with the C atom to which they are attached, make the structural moiety 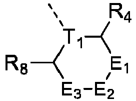 into 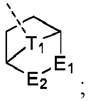
or, when T₁ is CH and E₁ is -C(R₅)₂-, R₅ and R₈, together with the C atom to which they are attached, make the structural moiety 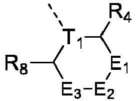 into 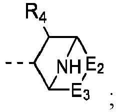
E₆ isNR_{6c} orO;
R₆ₐ and R_{6b} are each independently H, halogen, OH, =O, NH₂, or C₁₋₃ alkyl;
R_{6c} is H or C₁₋₃ alkyl;
Rₐ, R_{b}, R_{c}, R_{d}, Rₑ, R_{f}, R_{g}, and Rₜ are each independently F, Cl, Br, OH, CN, COOH, NH₂, C₁₋₃ alkyl, or C₁₋₃ alkoxy;
n is 1 or 2;
the 4- to 6-membered heterocycloalkyl contains 1, 2, 3, or 4 heteroatoms or heteroatom groups independently selected from -O-, -NH-, -S-, and N.

The present disclosure provides a compound of formula (II) or a pharmaceutically acceptable salt thereof, 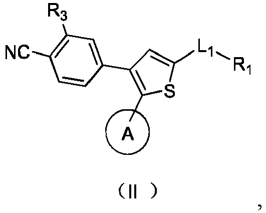
wherein L₁ is -C(=O)- or -C(=O)-NH-;
R₁ is R₁ₐ or 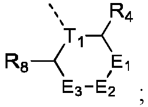
ring A is 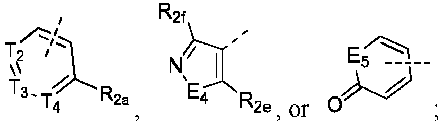
T₁ is CH or N;
T₂ is CR_{2c} or N;
T₃ is CR₂ or N;
T₄ is CR_{2b} or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
E₄ is O, S, CH₂, or NR_{2g};
E₅ is O, CH₂, or NR_{2g};
R₁ₐ is C₁₋₃ alkyl substituted by 1, 2, or 3 Rₐ;
R₂ₐ is H or halogen;
R₂ and R_{2b} are each independently H, halogen, NH₂, C₁₋₃ alkyl, C₁₋₃ alkoxy, or - S(=O)₂C₁₋₃ alkyl, wherein the C₁₋₃ alkyl, C₁₋₃ alkoxy, and -S(=O)₂C₁₋₃ alkyl are each independently and optionally substituted by 1, 2, or 3 R_{b};
R_{2c} is H;
or, R₂ and R_{2c}, together with the C atom to which they are attached, make the structural moiety 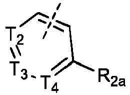 into 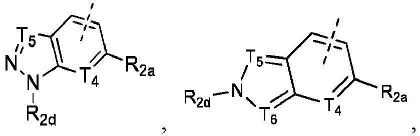 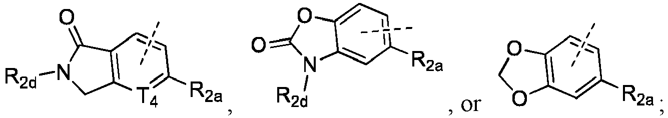
R_{2d} is H or C₁₋₄ alkyl, wherein the C₁₋₄ alkyl is optionally substituted by 1, 2, or 3 R_{c};
R₂ₑ, R_{2f}, and R_{2g} are each independently H or C₁₋₃ alkyl;
T₅ and T₆ are each independently N or CH;
each R₃ is independently H, halogen, or C₁₋₃ alkoxy;
R₄ and R₈ are each independently H or C₁₋₃ alkyl;
each R₅ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{d};
R₆ is H or C₁₋₃ alkyl, wherein the C₁₋₃ alkyl is optionally substituted by 1, 2, or 3 Rₑ;
each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 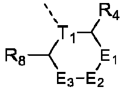 into 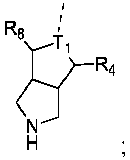
or, when E₂ is -C(R₆₁)₂-, two R₆₁, together with the C atom to which they are attached, make the structural moiety 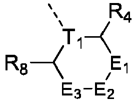 into 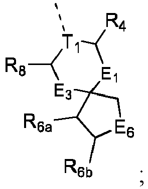
or, when E₃ is a single bond, R₄ and R₈, together with the C atom to which they are attached, make the structural moiety 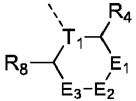 into 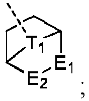
or, when T₁ is CH and E₁ is -C(R₅)₂-, R₅ and R₈, together with the C atom to which they are attached, make the structural moiety 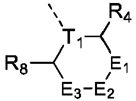 into 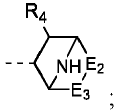
E₆ is NR_{6c} or O;
R₆ₐ and R_{6b} are each independently H, halogen, OH, =O, NH₂, or C₁₋₃ alkyl;
R_{6c} is H or C₁₋₃ alkyl;
Rₐ, R_{b}, R_{c}, R_{d}, Rₑ, R_{f}, and R_{g} are each independently F, Cl, Br, OH, CN, COOH, or NH₂.

The present disclosure provides a compound of formula (I) or a pharmaceutically acceptable salt thereof, 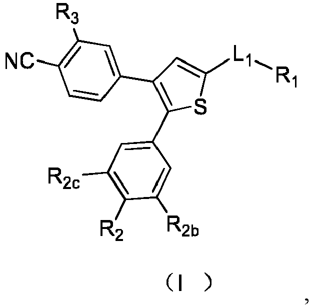 wherein
L₁ is -C(=O)- or -C(=O)-NH-;
R₁ is R₁ₐ or 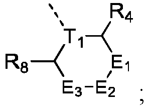
T₁ is CH or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
R₁ₐ is C₁₋₃ alkyl substituted by 1, 2, or 3 Rₐ;
R₂ and R_{2b} are each independently H, halogen, C₁₋₃ alkyl, or C₁₋₃ alkoxy, wherein the C₁₋₃ alkyl and C₁₋₃ alkoxy are optionally substituted by 1, 2, or 3 R_{b};
R_{2c} is H;
or, R₂ and R_{2c}, together with the C atom to which they are attached, make the structural moiety 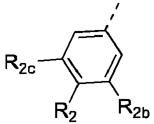 into 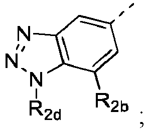
R_{2d} is H or C₁₋₄ alkyl, wherein the C₁₋₄ alkyl is optionally substituted by 1, 2, or 3 R_{c};
each R₃ is independently H, halogen, or C₁₋₃ alkoxy;
R₄ and R₈ are each H;
each R₅ is independently H, halogen, OH, NH₂, or C₁₋₃ alkyl, wherein the C₁₋₃ alkyl is optionally substituted by 1, 2, or 3 R_{d};
R₆ is H or C₁₋₃ alkyl, wherein the C₁₋₃ alkyl is optionally substituted by 1, 2, or 3 Rₑ;
each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, or C₁₋₃ alkyl, wherein the C₁₋₃ alkyl is optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 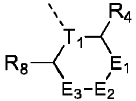 into 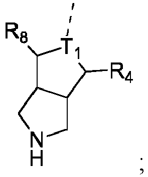
or, when E₂ is -C(R₆₁)₂-, two R₆₁, together with the C atom to which they are attached, make the structural moiety 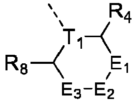 into 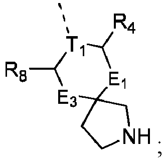
or, when E₃ is a single bond, R₄ and R₈, together with the C atom to which they are attached, make the structural moiety 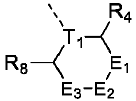 into 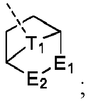
or, when T₁ is CH and E₁ is -C(R₅)₂-, R₅ and R₈, together with the C atom to which they are attached, make the structural moiety 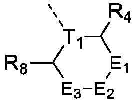 into 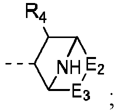
Rₐ, R_{b}, R_{c}, R_{d}, Rₑ, R_{f}, and R_{g} are each independently F, Cl, Br, OH, or NH₂.

In some embodiments of the present disclosure, the compound or the pharmaceutically acceptable salt thereof, wherein the compound is 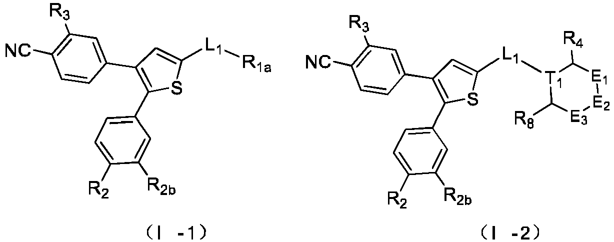 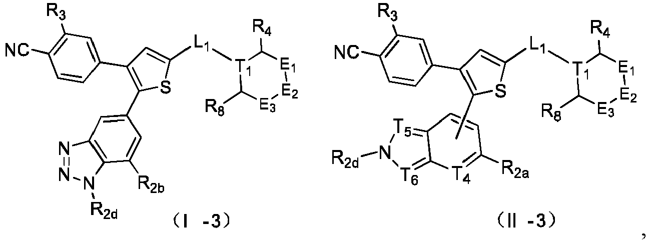 wherein
R₂ is H, halogen, NH₂, C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, or -NHC₁₋₃ alkyl-C₃₋₆ cycloalkyl, wherein the C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, and -NHC₁₋₃ alkyl-C₃₋₆ cycloalkyl are each independently and optionally substituted by 1, 2, or 3 R_{b};
R_{b}, R₁ₐ, R₂ₐ, R_{2b}, R_{2d}, R₃, R₄, R₈, L₁, T₁, T₄, T₅, T₆, E₁, E₂, and E₃ are as defined in the present disclosure.

In some embodiments of the present disclosure, the compound or the pharmaceutically acceptable salt thereof, wherein the compound is 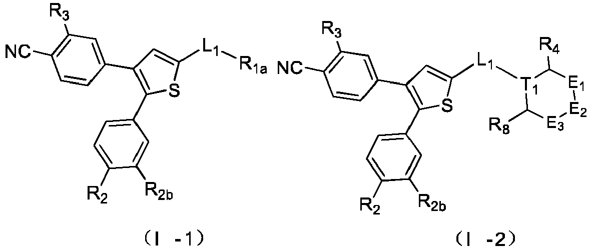 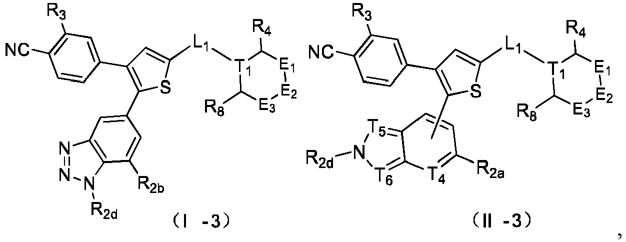 wherein
R₂ and R_{2b} are each independently H, halogen, NH₂, C₁₋₃ alkyl, C₁₋₃ alkoxy, - S(=O)₂C₁₋₃ alkyl, 4- to 6-membered heterocycloalkyl, or -C(=O)-4- to 6-membered heterocycloalkyl, wherein the C₁₋₃ alkyl, C₁₋₃ alkoxy, -S(=O)₂C₁₋₃ alkyl, 4- to 6-membered heterocycloalkyl, and -C(=O)-4- to 6-membered heterocycloalkyl are each independently and optionally substituted by 1, 2, or 3 R_{b};
R_{b}, R₁ₐ, R₂ₐ, R_{2d}, R₃, R₄, R₈, L₁, T₁, T₄, T₅, T₆, E₁, E₂, and E₃ are as defined in the present disclosure.

In some embodiments of the present disclosure, the compound or the pharmaceutically acceptable salt thereof, wherein the compound is 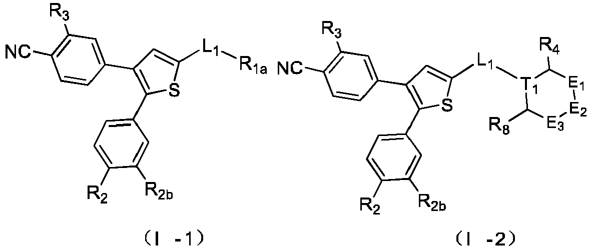 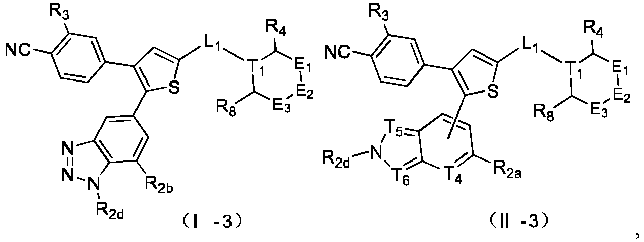 wherein
R₂ and R_{2b} are each independently H, halogen, NH₂, C₁₋₃ alkyl, C₁₋₃ alkoxy, or - S(=O)₂C₁₋₃ alkyl, wherein the C₁₋₃ alkyl, C₁₋₃ alkoxy, and -S(=O)₂C₁₋₃ alkyl are each independently and optionally substituted by 1, 2, or 3 R_{b};
R_{b}, R₁ₐ, R₂ₐ, R_{2d}, R₃, R₄, R₈, L₁, T₁, T₄, T₅, T₆, E₁, E₂, and E₃ are as defined in the present disclosure.

In some embodiments of the present disclosure, the compound or the pharmaceutically acceptable salt thereof, wherein the compound is 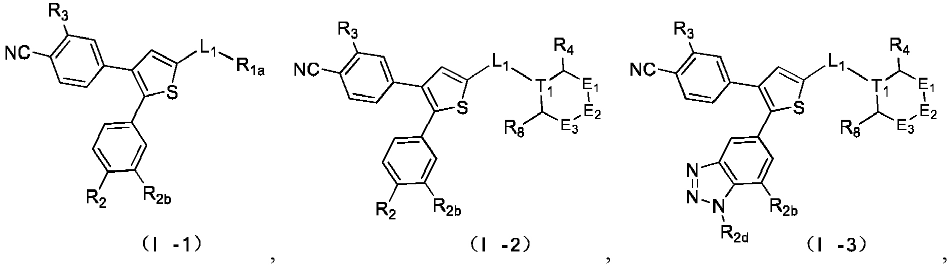 wherein
R₂ and R_{2b} are each independently H, halogen, C₁₋₃ alkyl, or C₁₋₃ alkoxy, wherein the C₁₋₃ alkyl and C₁₋₃ alkoxy are optionally substituted by 1, 2, or 3 R_{b};
R_{b}, R₁ₐ, R_{2d}, R₃, R₄, R₈, L₁, T₁, E₁, E₂, and E₃ are as defined in the present disclosure.

In some embodiments of the present disclosure, the Rₐ, R_{b}, R_{c}, R_{d}, Rₑ, R_{f}, R_{g}, and Rₜ are each independently F, Cl, Br, OH, CN, COOH, NH₂, CH₃, or OCH₃, and other variables are as defined in the present disclosure.

In some embodiments of the present disclosure, the Rₐ, R_{b}, R_{c}, R_{d}, Rₑ, R_{f}, and R_{g} are each independently F, Cl, Br, OH, CN, COOH, NH₂, CH₃, or OCH₃, and other variables are as defined in the present disclosure.

In some embodiments of the present disclosure, each Rₜ is independently H, F, Cl, or CH₃, and other variables are as defined in the present disclosure.

In some embodiments of the present disclosure, the R₁ₐ is 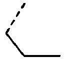 substituted by 1, 2, or 3 Rₐ, and Rₐ and other variables are as defined in the present disclosure.

In some embodiments of the present disclosure, the R₁ₐ is 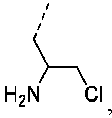 and other variables are as defined in the present disclosure.

In some embodiments of the present disclosure, the R₂ₐ is H or F, and other variables are as defined in the present disclosure.

In some embodiments of the present disclosure, the R₂ and R_{2b} are each independently H, F, Cl, NH₂, -OCH₃, 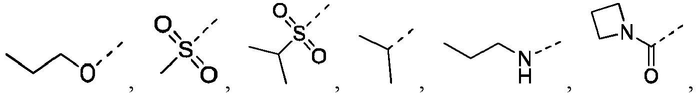 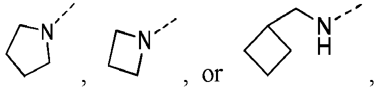 wherein the -OCH₃, 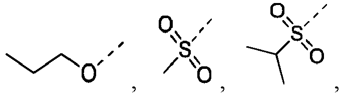 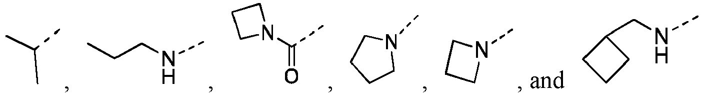 are each independently and optionally substituted by 1, 2, or 3 R_{b}, and R_{b} and other variables are as defined in the present disclosure.

In some embodiments of the present disclosure, the R₂ and R_{2b} are each independently H, F, Cl, NH₂, -OCH₃, 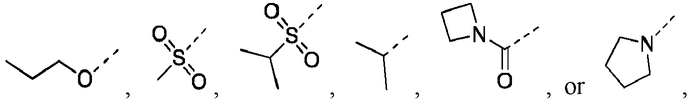 wherein the -OCH₃, 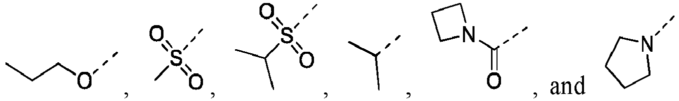 are each independently and optionally substituted by 1, 2, or 3 R_{b}, and R_{b} and other variables are as defined in the present disclosure.

In some embodiments of the present disclosure, the R₂ and R_{2b} are each independently H, F, Cl, NH₂, -OCH₃, 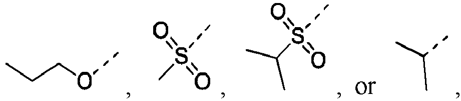 wherein the -OCH₃, 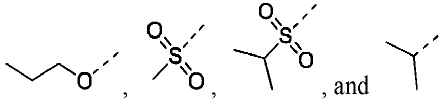 are each independently and optionally substituted by 1, 2, or 3 R_{b}, and R_{b} and other variables are as defined in the present disclosure.

In some embodiments of the present disclosure, the R₂ and R_{2b} are each independently H, F, Cl, or -OCH₃, wherein the -OCH₃ is optionally substituted by 1, 2, or 3 R_{b}, and R_{b} and other variables are as defined in the present disclosure.

In some embodiments of the present disclosure, the R₂ and R_{2b} are each independently H, F, Cl, NH₂, -OCH₃, 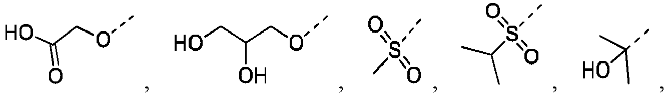 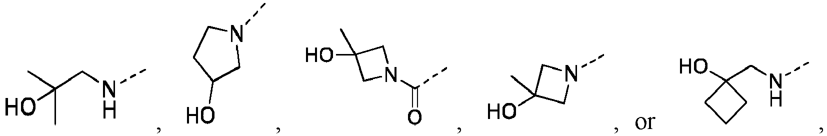 and other variables are as defined in the present disclosure.

In some embodiments of the present disclosure, the R₂ and R_{2b} are each independently H, F, Cl, NH₂, -OCH₃, 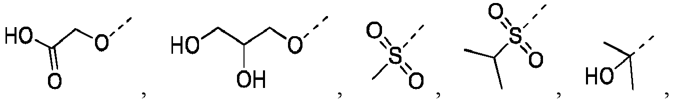 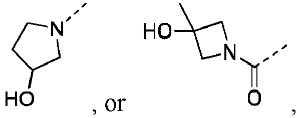 and other variables are as defined in the present disclosure.

In some embodiments of the present disclosure, the R₂ and R_{2b} are each independently H, F, Cl, NH₂, -OCH₃, 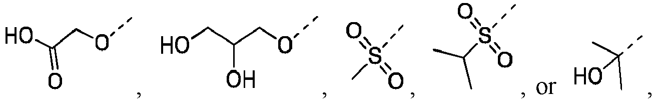 and other variables are as defined in the present disclosure.

In some embodiments of the present disclosure, the R₂ and R_{2b} are each independently F, Cl, or -OCH₃, and other variables are as defined in the present disclosure.

In some embodiments of the present disclosure, the R_{2d} is H, CH₃, 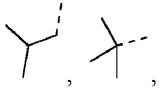 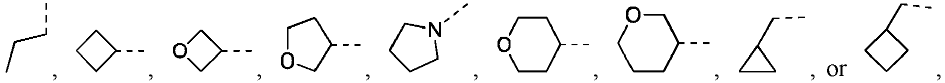 wherein the -CH₃, 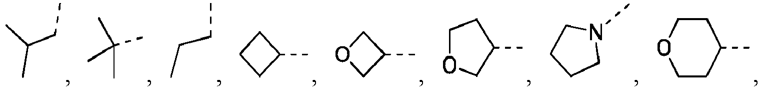 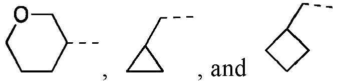 are optionally substituted by 1, 2, or 3 R_{c}, and R_{c} and other variables are as defined in the present disclosure.

In some embodiments of the present disclosure, the R_{2d} is H, CH₃, 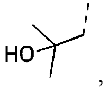 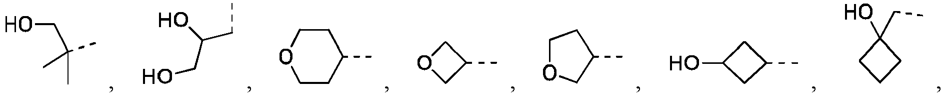 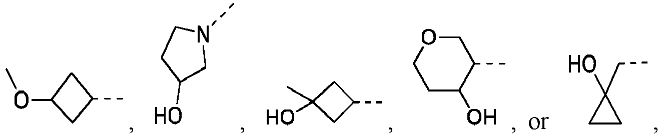 and other variables are as defined in the present disclosure.

In some embodiments of the present disclosure, the R_{2d} is H, CH₃, or 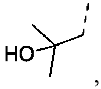 and other variables are as defined in the present disclosure.

In some embodiments of the present disclosure, the R_{2d} is CH₃ or 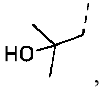 and other variables are as defined in the present disclosure.

In some embodiments of the present disclosure, the R₂ₑ, R_{2f}, and R_{2g} are each independently H or CH₃, and other variables are as defined in the present disclosure.

In some embodiments of the present disclosure, each R₃ is independently F or OCH₃, and other variables are as defined in the present disclosure.

In some embodiments of the present disclosure, each R₃ is independently F, and other variables are as defined in the present disclosure.

In some embodiments of the present disclosure, the R₄ and R₈ are each independently H or CH₃, and other variables are as defined in the present disclosure.

In some embodiments of the present disclosure, each R₅ is independently H, F, Cl, Br, I, OH, NH₂, CH₃, -NH-CH₃, or 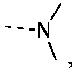 wherein the CH₃, -NH-CH₃, and 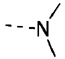 are each independently and optionally substituted by 1, 2, or 3 R_{d}, and R_{d} and other variables are as defined in the present disclosure.

In some embodiments of the present disclosure, each R₅ is independently H, F, Cl, Br, I, OH, NH₂, or CH₃, wherein the CH₃ is optionally substituted by 1, 2, or 3 R_{d}, and R_{d} and other variables are as defined in the present disclosure.

In some embodiments of the present disclosure, each R₅ is independently H, OH, NH₂, CH₃, or 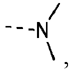 and other variables are as defined in the present disclosure.

In some embodiments of the present disclosure, each R₅ is independently H or NH₂, and other variables are as defined in the present disclosure.

In some embodiments of the present disclosure, the R₆ is H, CH₃, or CH₂-CH₃, wherein the CH₃ and CH₂-CH₃ are each independently and optionally substituted by 1, 2, or 3 Rₑ, and Rₑ and other variables are as defined in the present disclosure.

In some embodiments of the present disclosure, the R₆ is H or CH₃, wherein the CH₃ is optionally substituted by 1, 2, or 3 Rₑ, and other variables are as defined in the present disclosure.

In some embodiments of the present disclosure, the R₆ is H, CH₂-CN, or CH₂-CF₃, and other variables are as defined in the present disclosure.

In some embodiments of the present disclosure, the R₆ is H, and other variables are as defined in the present disclosure.

In some embodiments of the present disclosure, each R₆₁ is independently H, F, Cl, Br, I, OH, NH₂, or CH₃, wherein the CH₃ is optionally substituted by 1, 2, or 3 R_{f}, and R_{f} and other variables are as defined in the present disclosure.

In some embodiments of the present disclosure, each R₆₁ is independently H, F, OH, or NH₂, and other variables are as defined in the present disclosure.

In some embodiments of the present disclosure, each R₆₁ is independently H or NH₂, and other variables are as defined in the present disclosure.

In some embodiments of the present disclosure, the R₆ₐ and R_{6b} are each independently H, OH, =O, NH₂, or CH₃, and other variables are as defined in the present disclosure.

In some embodiments of the present disclosure, each R₇ is independently H, F, Cl, Br, I, OH, NH₂, CH₃, -NH-CH₃, or 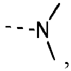 wherein the CH₃, -NH-CH₃, and 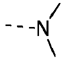 are optionally substituted by 1, 2, or 3 R_{g}, and R_{g} and other variables are as defined in the present disclosure.

In some embodiments of the present disclosure, each R₇ is independently H, F, Cl, Br, I, OH, NH₂, or CH₃, wherein the -CH₃ is optionally substituted by 1, 2, or 3 R_{g}, and R_{g} and other variables are as defined in the present disclosure.

In some embodiments of the present disclosure, each R₇ is independently H, OH, NH₂, CH₃, or 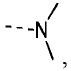 and other variables are as defined in the present disclosure.

In some embodiments of the present disclosure, each R₇ is independently H or NH₂, and other variables are as defined in the present disclosure.

In some embodiments of the present disclosure, the structural moiety 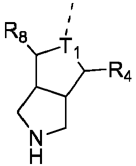 is 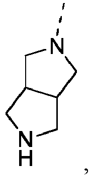 and other variables are as defined in the present disclosure.

In some embodiments of the present disclosure, the structural moiety 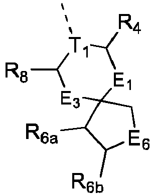 is 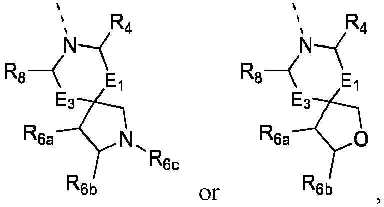 and other variables are as defined in the present disclosure.

In some embodiments of the present disclosure, the structural moiety 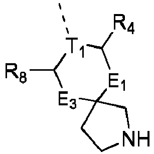 is 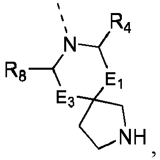 and other variables are as defined in the present disclosure.

In some embodiments of the present disclosure, the structural moiety 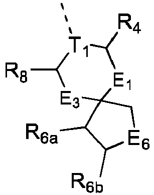 is 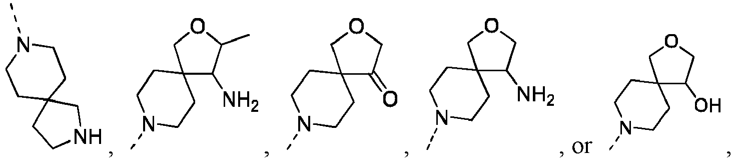 and other variables are as defined in the present disclosure.

In some embodiments of the present disclosure, the structural moiety 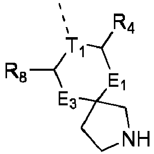 is 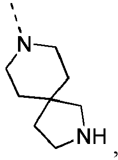 and other variables are as defined in the present disclosure.

In some embodiments of the present disclosure, the structural moiety 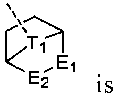 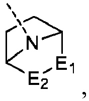 and other variables are as defined in the present disclosure.

In some embodiments of the present disclosure, the structural moiety 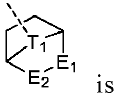 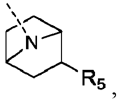 and other variables are as defined in the present disclosure.

In some embodiments of the present disclosure, the structural moiety 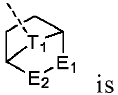 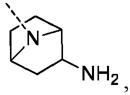 and other variables are as defined in the present disclosure.

In some embodiments of the present disclosure, the structural moiety 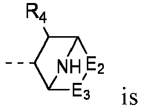 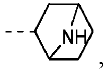 and other variables are as defined in the present disclosure.

In some embodiments of the present disclosure, the R₁ is 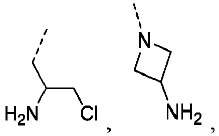 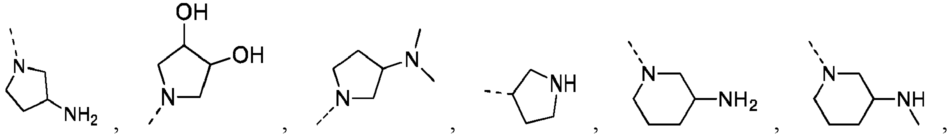 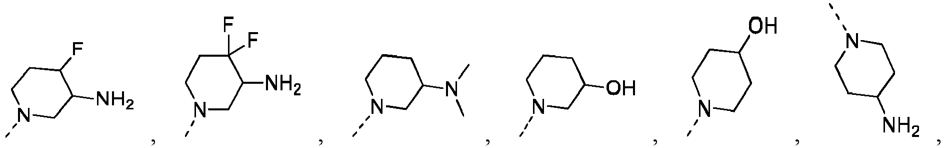 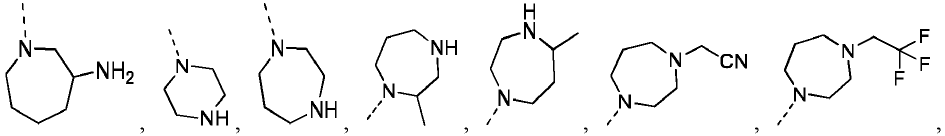 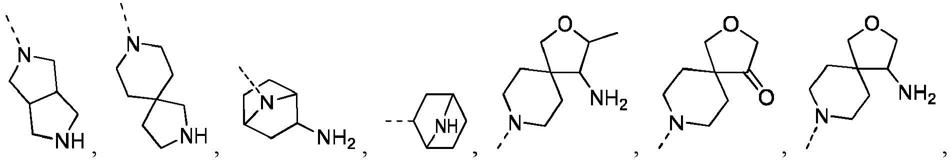 or 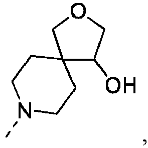 and other variables are as defined in the present disclosure.

In some embodiments of the present disclosure, the R₁ is 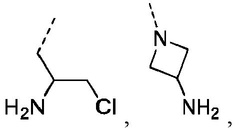 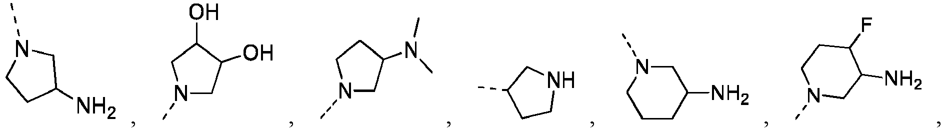 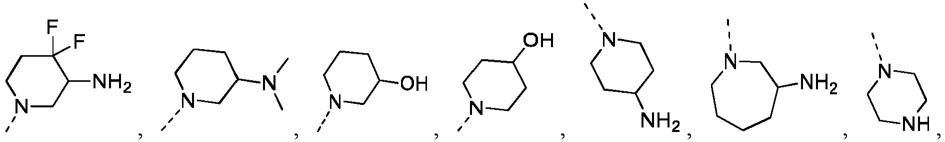 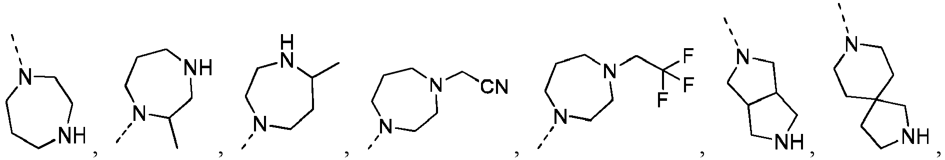 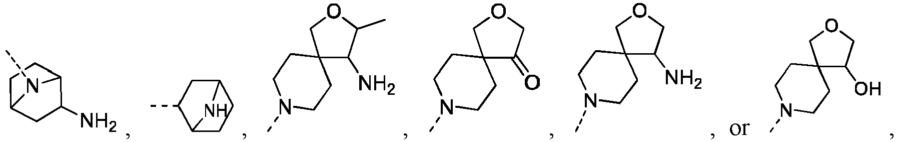 and other variables are as defined in the present disclosure.

In some embodiments of the present disclosure, the R₁ is 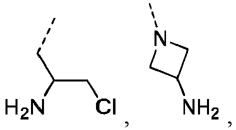 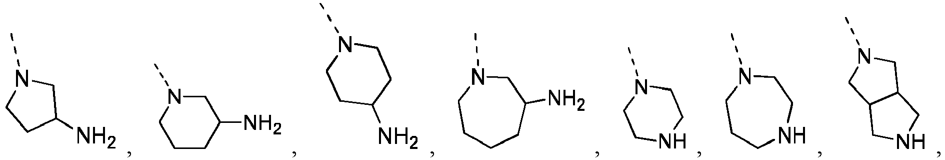 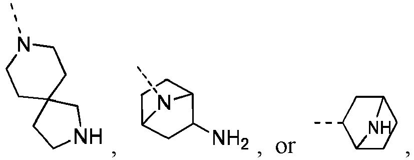 and other variables are as defined in the present disclosure.

In some embodiments of the present disclosure, the ring A is 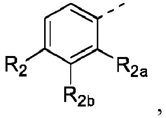 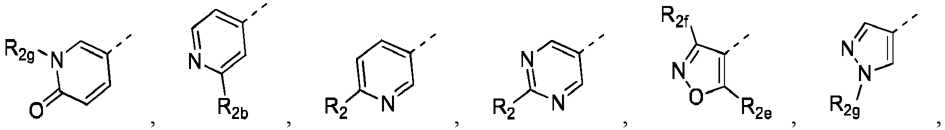 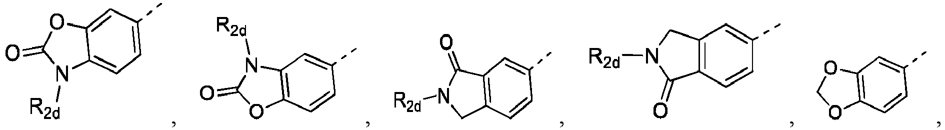 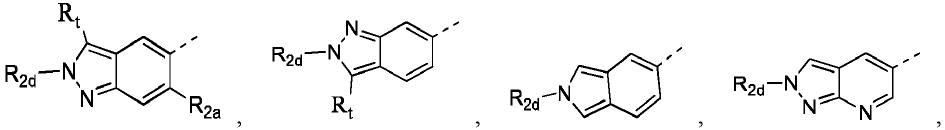 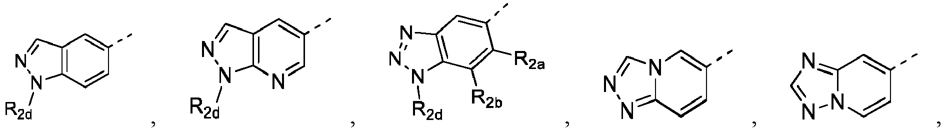 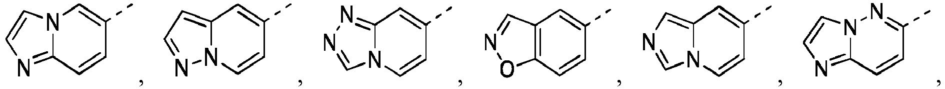 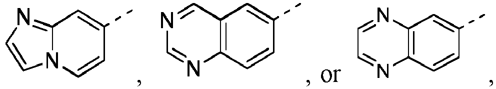 and R₂, R₂ₐ, R_{2b}, R_{2d}, R₂ₑ, R_{2f}, R_{2g}, Rₜ, and other variables are as defined in the present disclosure.

In some embodiments of the present disclosure, the ring A is 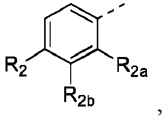 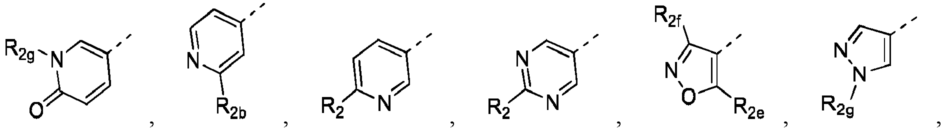 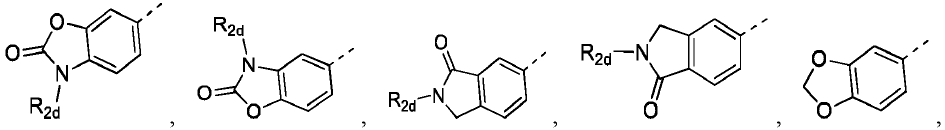 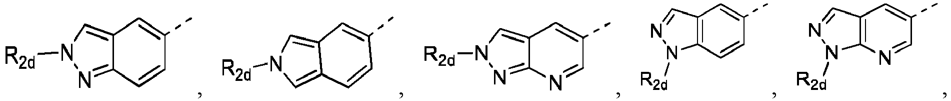 or 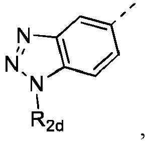 and R₂, R₂ₐ, R_{2b}, R_{2d}, R₂ₑ, R_{2f}, R_{2g}, Rₜ, and other variables are as defined in the present disclosure.

In some embodiments of the present disclosure, the ring A is 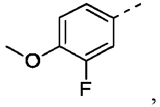 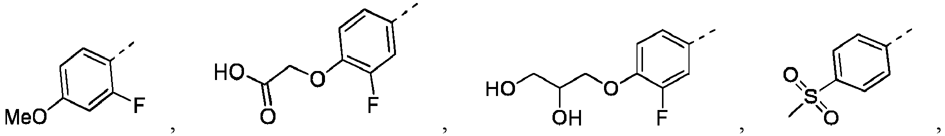 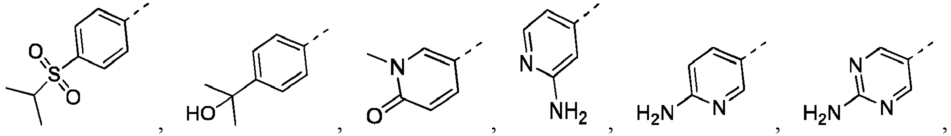 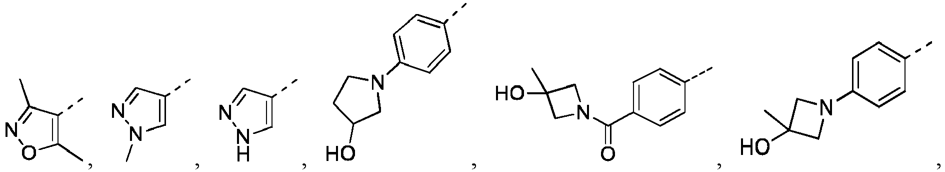 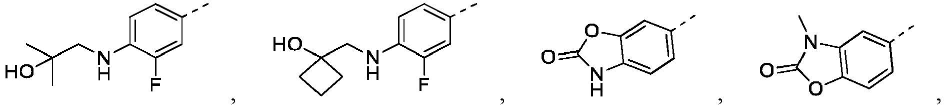 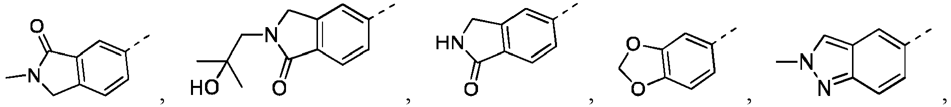 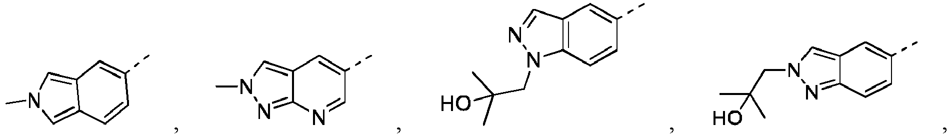 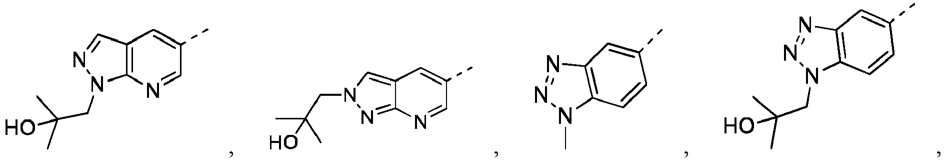 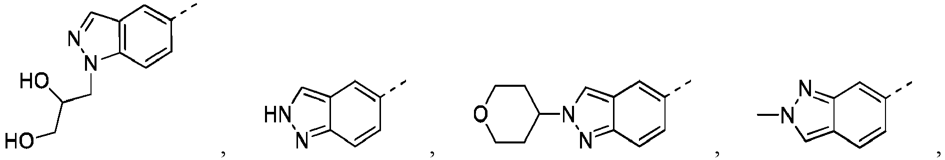 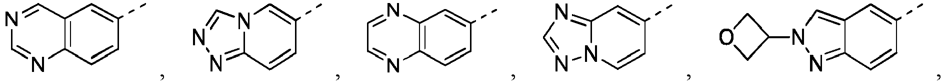 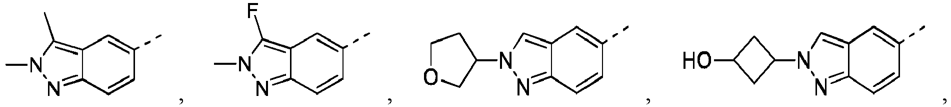 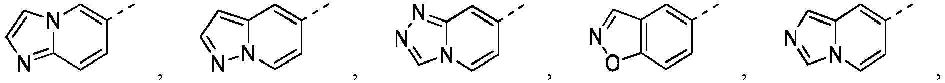 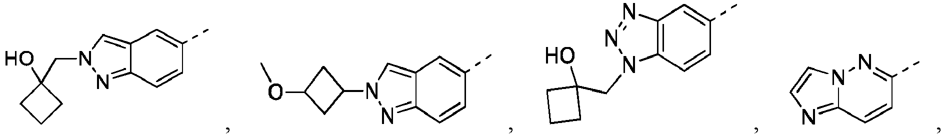 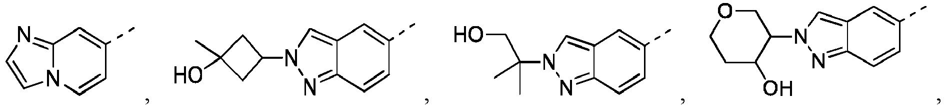 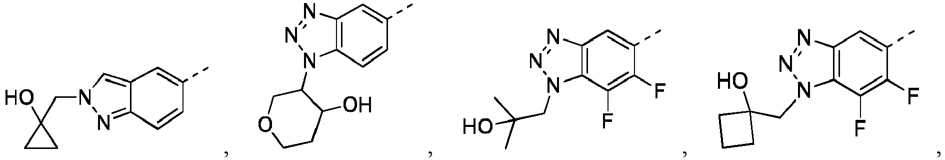 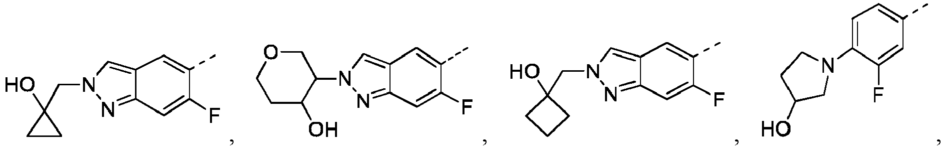 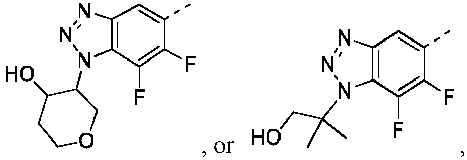 and other variables are as defined in the present disclosure.

In some embodiments of the present disclosure, the ring A is 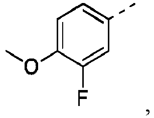 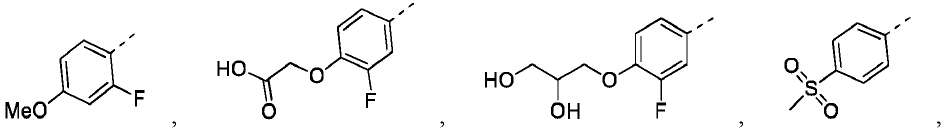 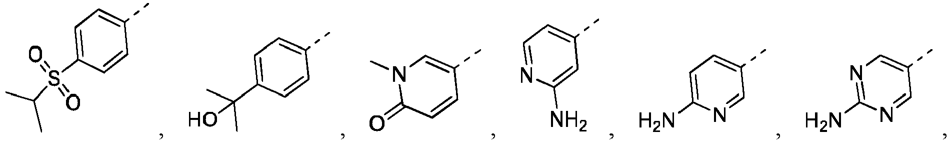 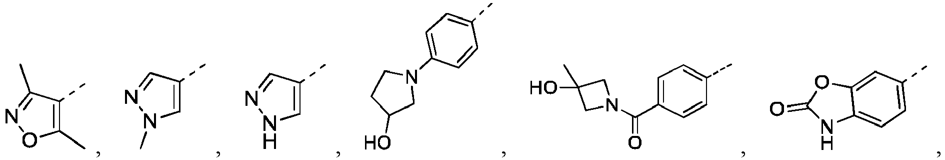 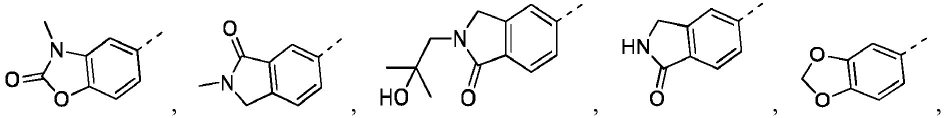 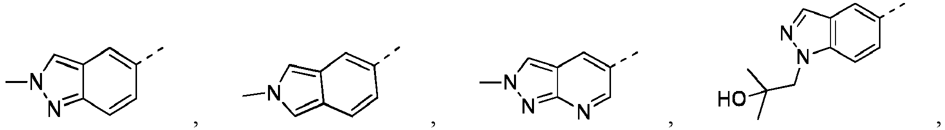 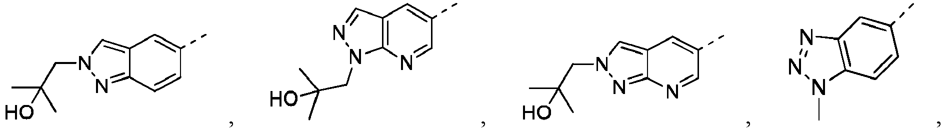 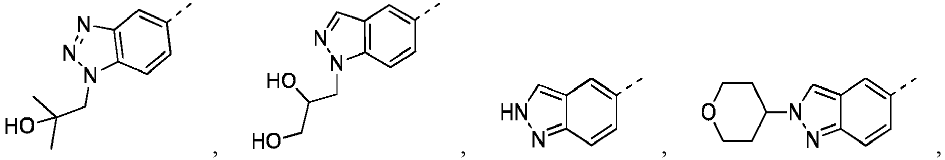 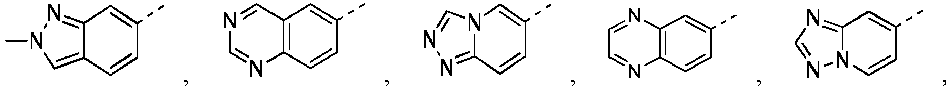 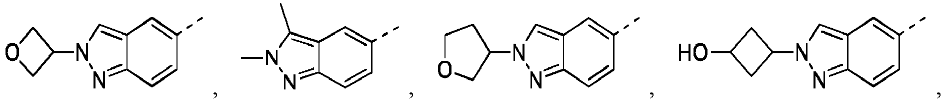 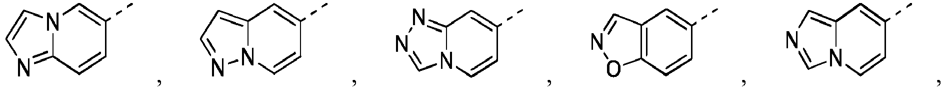 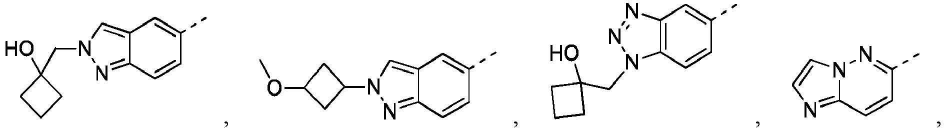 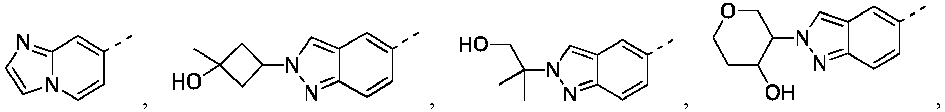 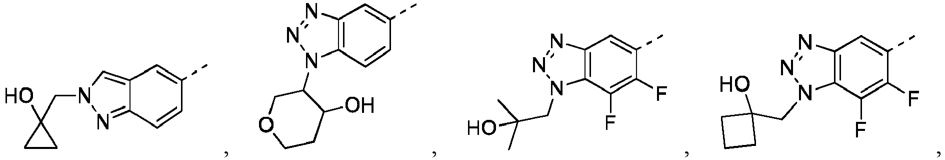 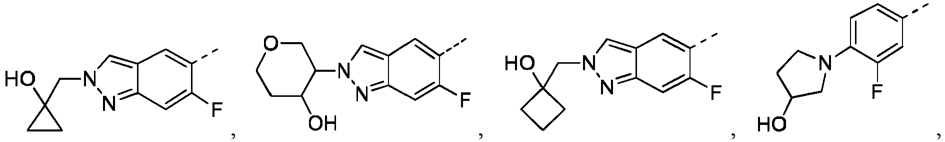 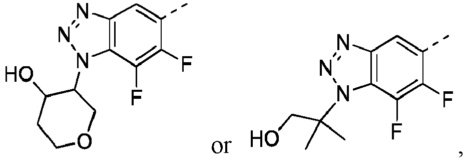 and other variables are as defined in the present disclosure.

In some embodiments of the present disclosure, the ring A is 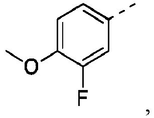 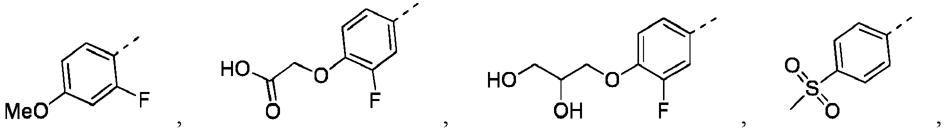 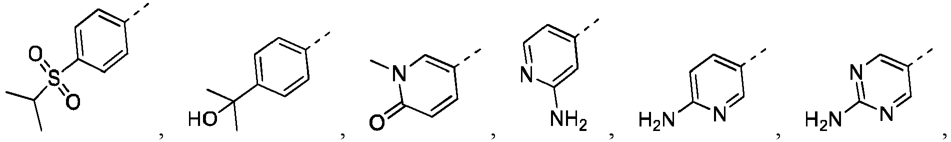 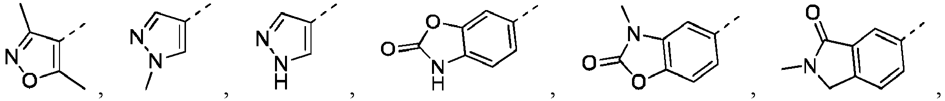 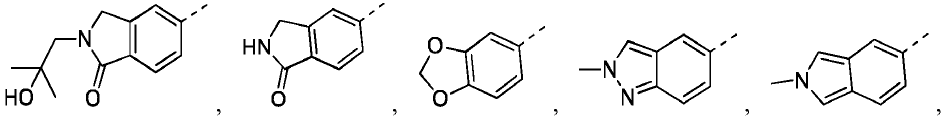 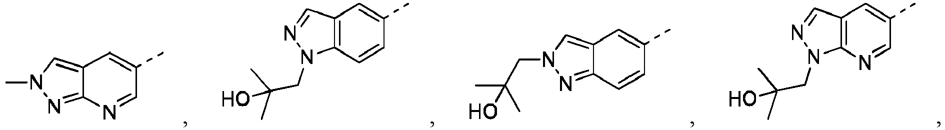 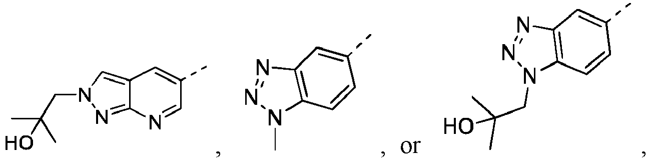 and other variables are as defined in the present disclosure.

In some embodiments of the present disclosure, the compound or the pharmaceutically acceptable salt thereof, wherein the compound is 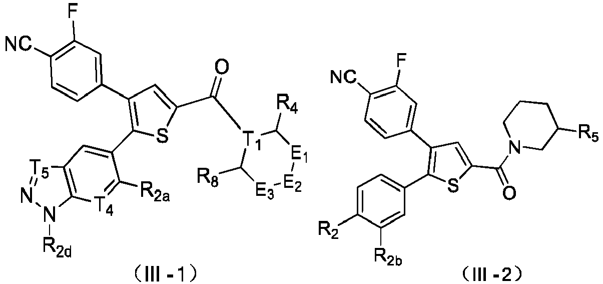 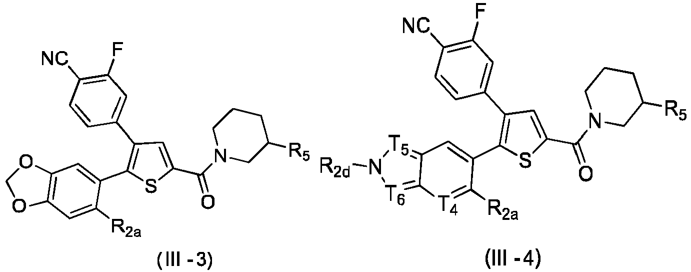 wherein
R₂ and R_{2b} are each independently H, halogen, NH₂, C₁₋₃ alkyl, C₁₋₃ alkoxy, -S(=O)₂C₁₋₃ alkyl, 4- to 6-membered heterocycloalkyl, or -C(=O)-4- to 6-membered heterocycloalkyl, wherein the C₁₋₃ alkyl, C₁₋₃ alkoxy, -S(=O)₂C₁₋₃ alkyl, 4- to 6-membered heterocycloalkyl, and -C(=O)-4- to 6-membered heterocycloalkyl are each independently and optionally substituted by 1, 2, or 3 R_{b};
R_{b}, R₂ₐ, R_{2d}, R₄, R₅, R₈, T₁, T₄, T₅, T₆, E₁, E₂, and E₃ are as defined in the present disclosure.

In some embodiments of the present disclosure, the compound or the pharmaceutically acceptable salt thereof, wherein the compound is 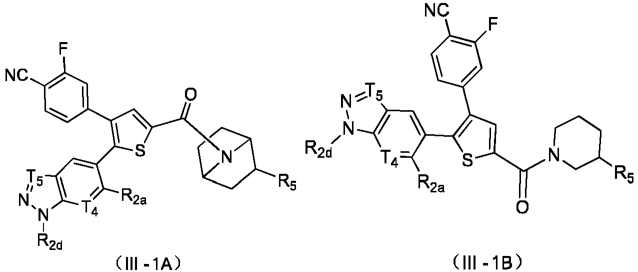 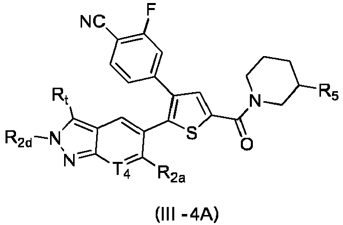 wherein R₂ₐ, R_{2d}, Rₜ, R₅, T₄, and T₅ are as defined in the present disclosure.

In some embodiments of the present disclosure, the compound or the pharmaceutically acceptable salt thereof, wherein the compound is 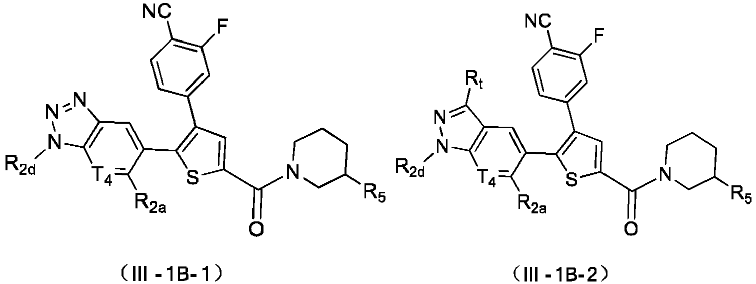 wherein R₂ₐ, R_{2d}, Rₜ, T₄, and R₅ are as defined in the present disclosure.

There are still some embodiments of the present disclosure which are obtained by any combination of the above variables.

The present disclosure also provides a compound of the following formula or a pharmaceutically acceptable salt thereof,

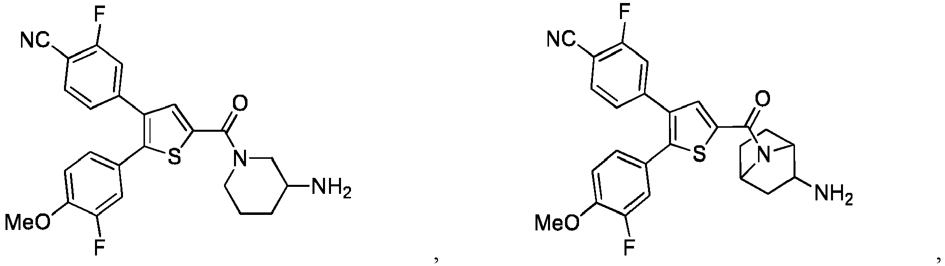

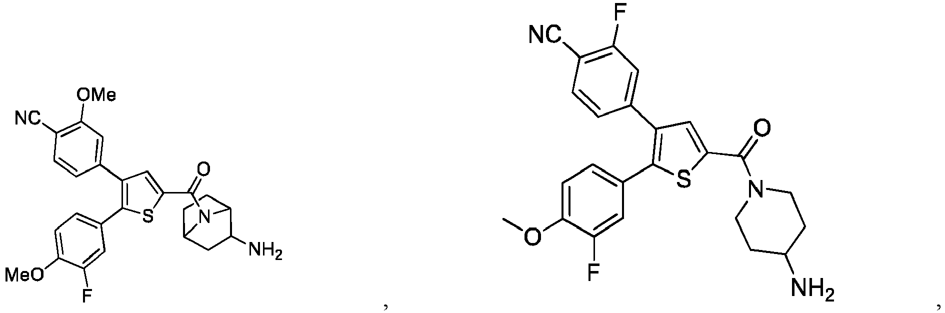

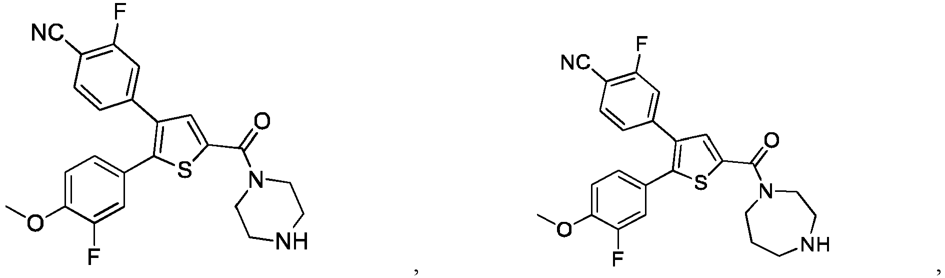

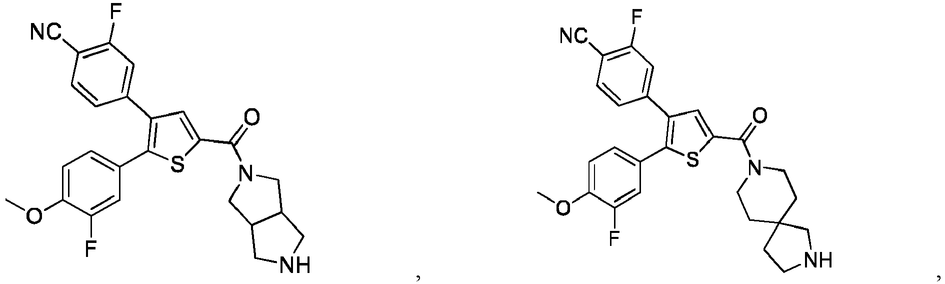

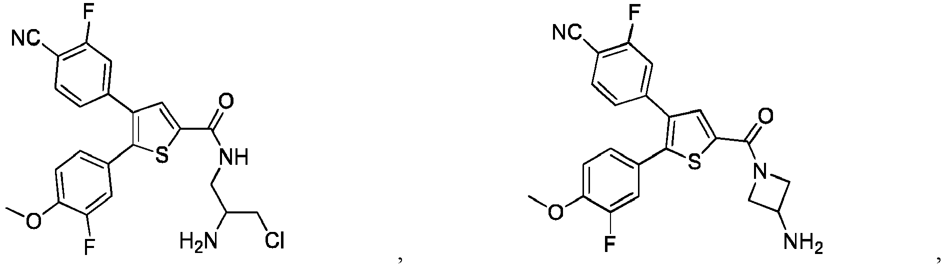

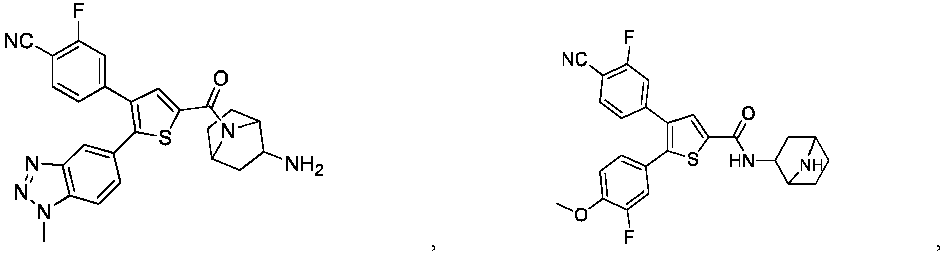

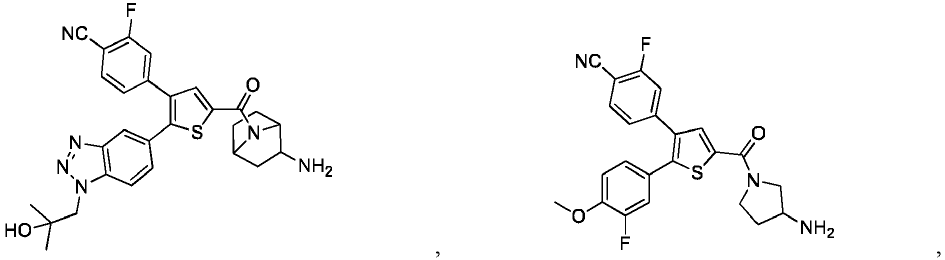

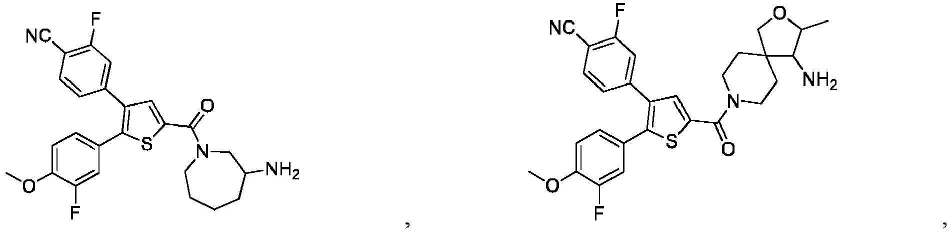

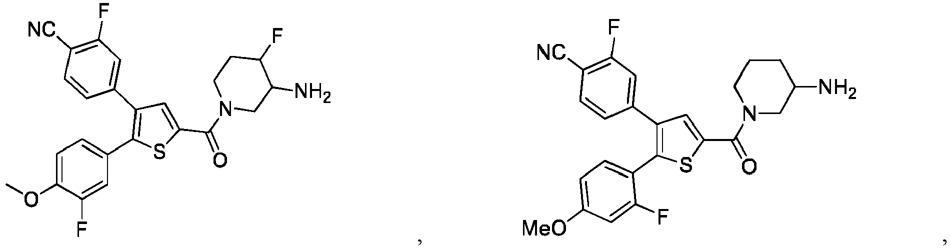

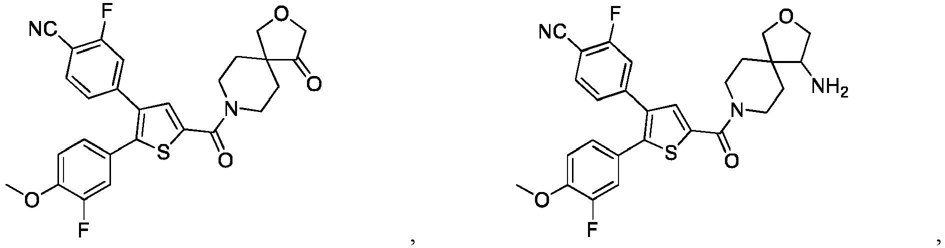

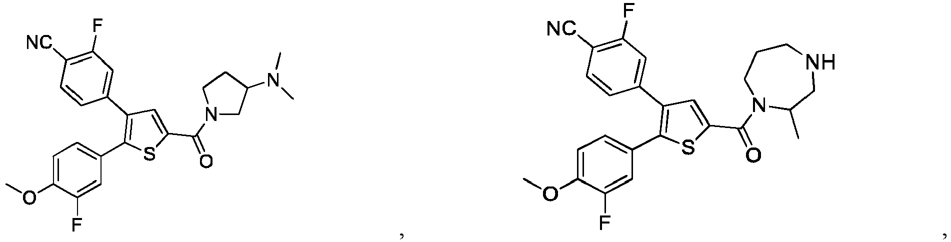

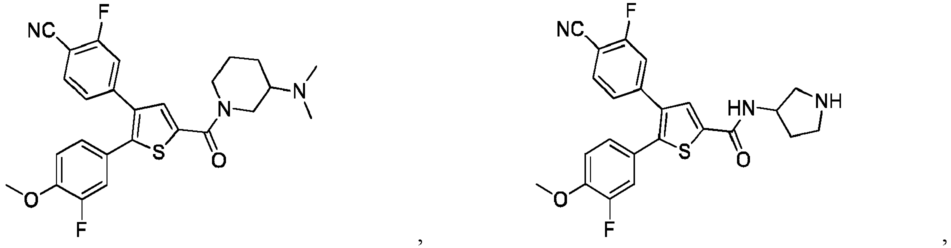

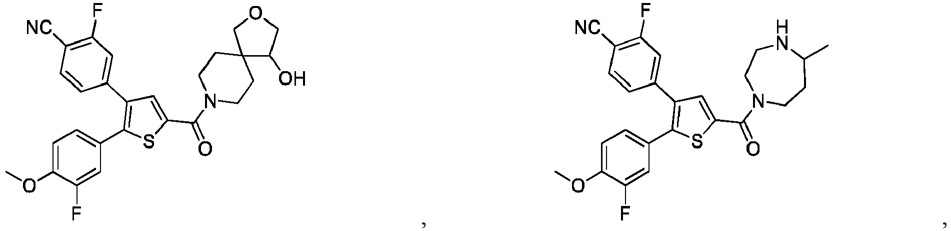

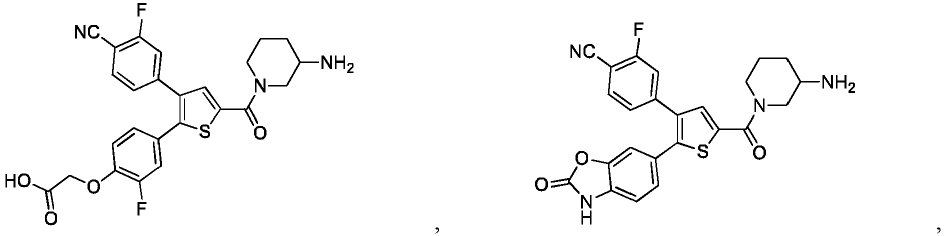

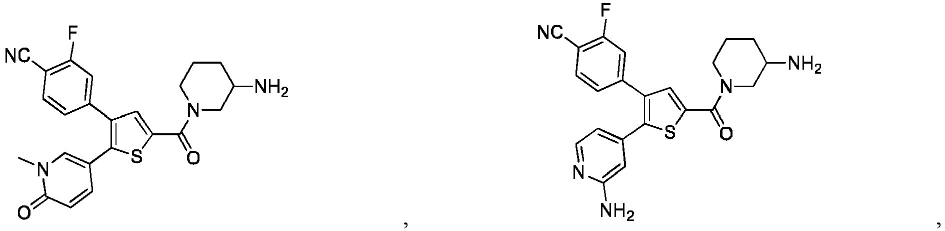

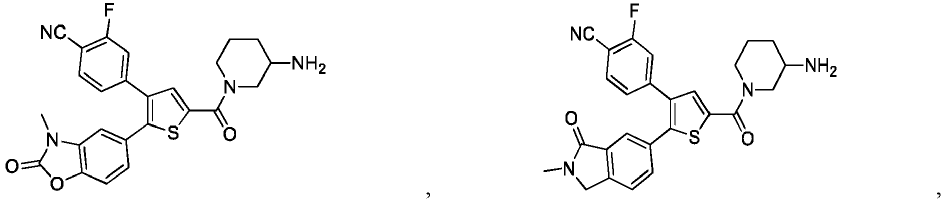

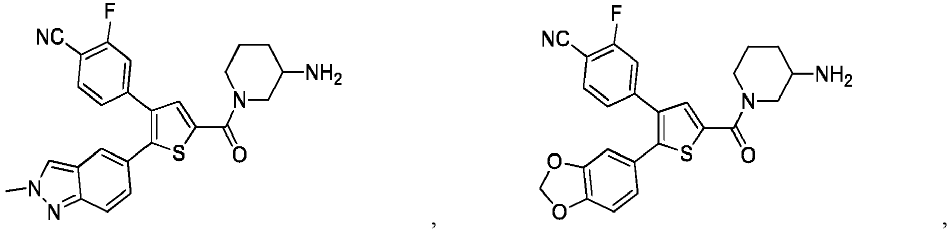

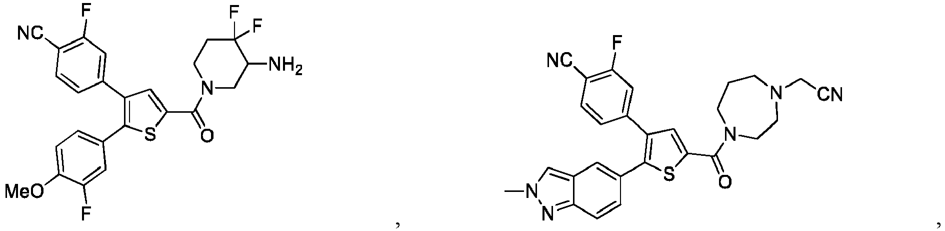

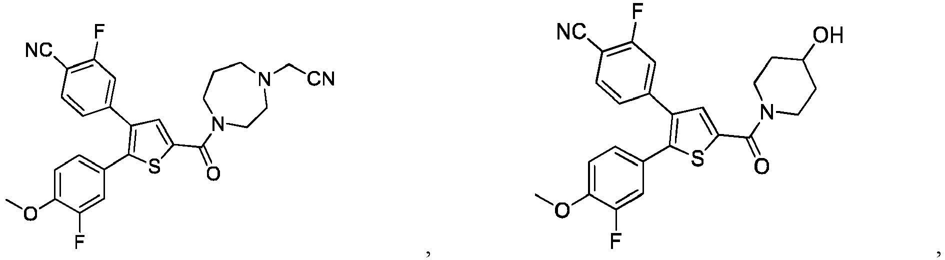

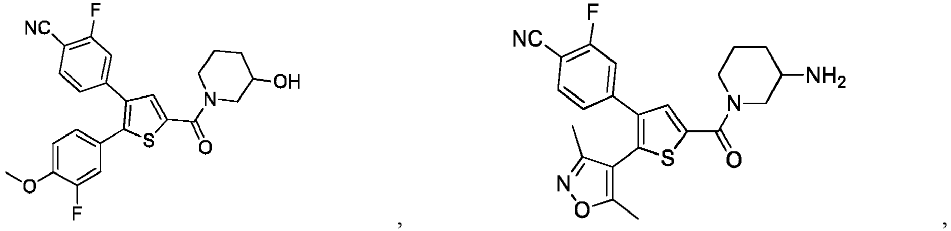

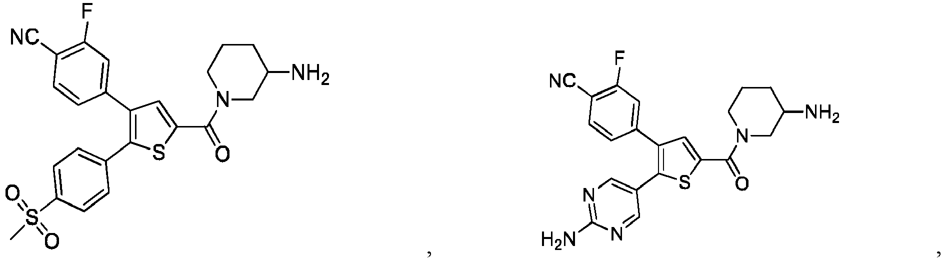

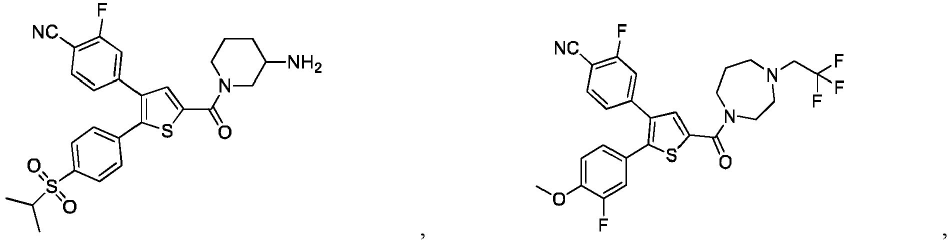

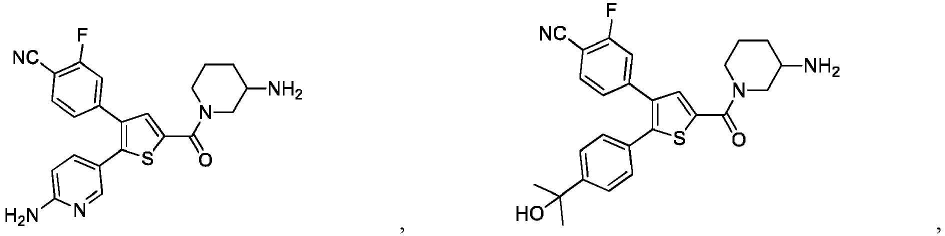

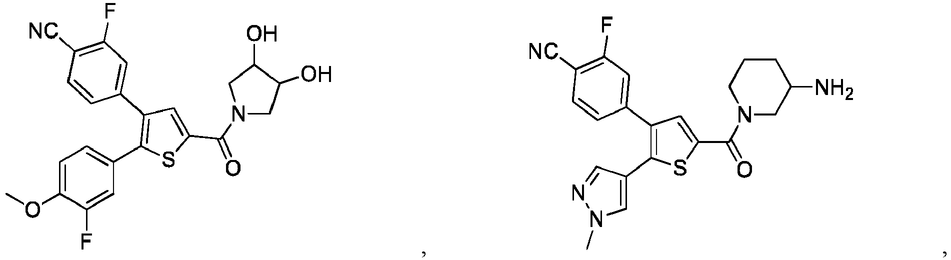

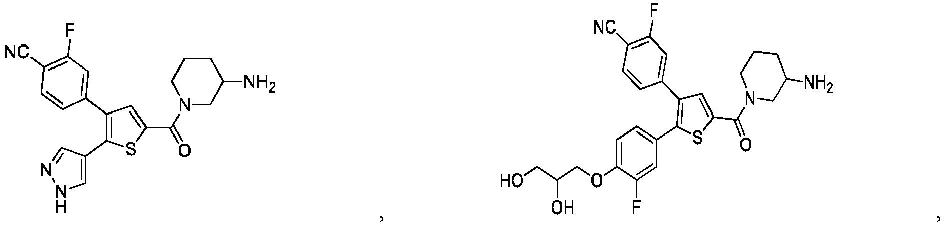

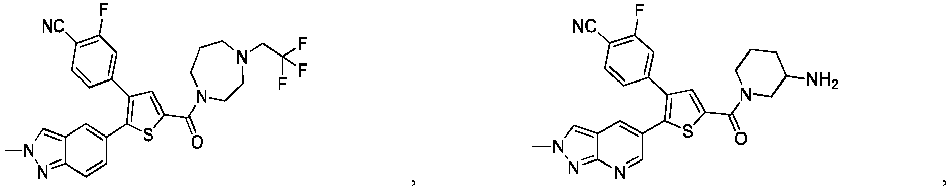

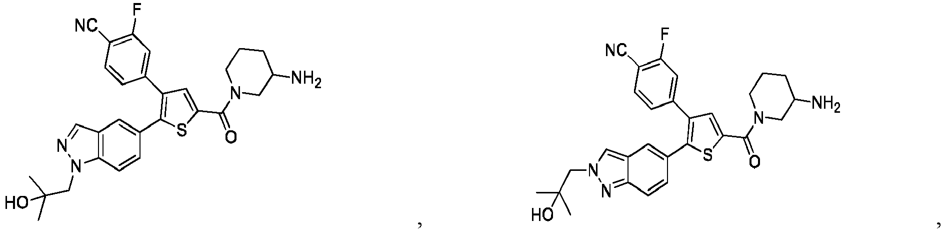

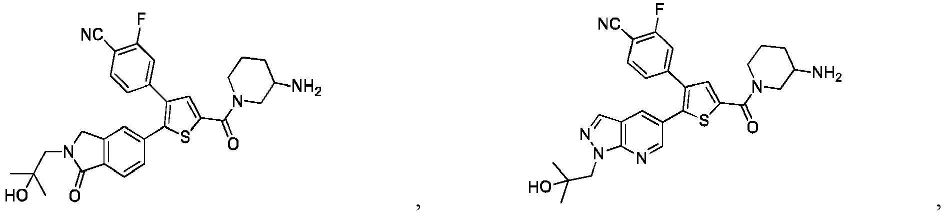

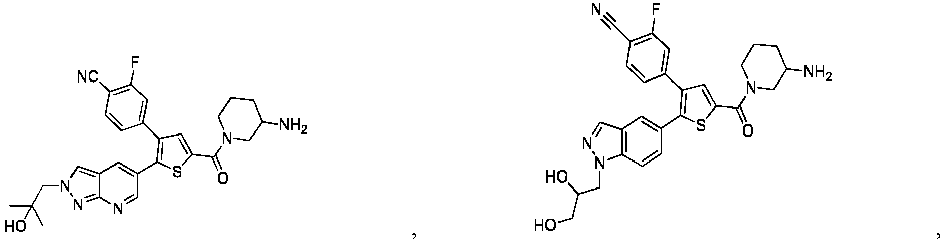

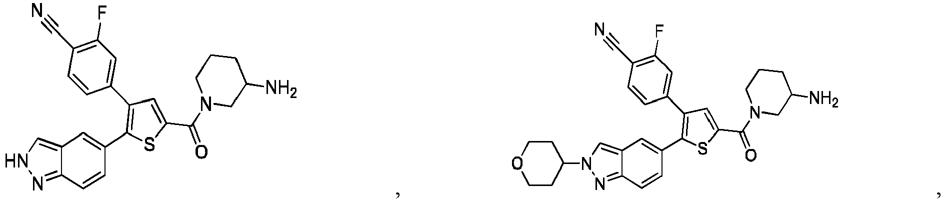

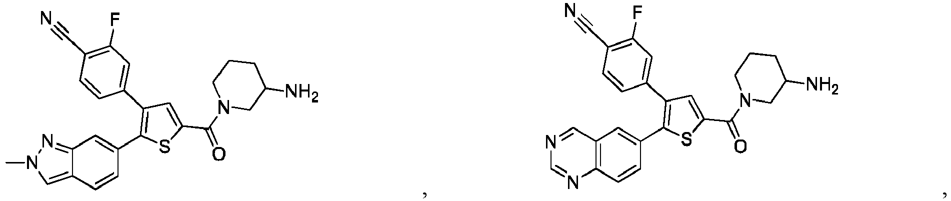

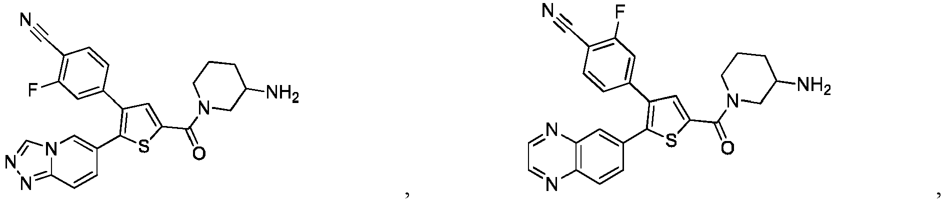

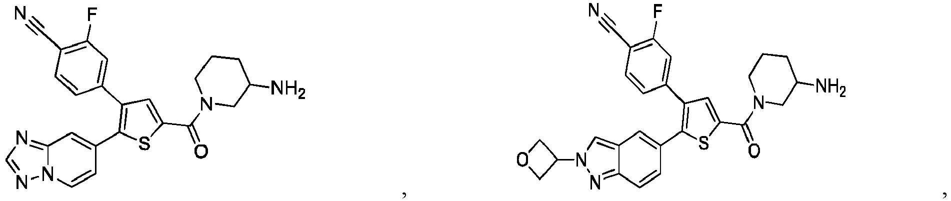

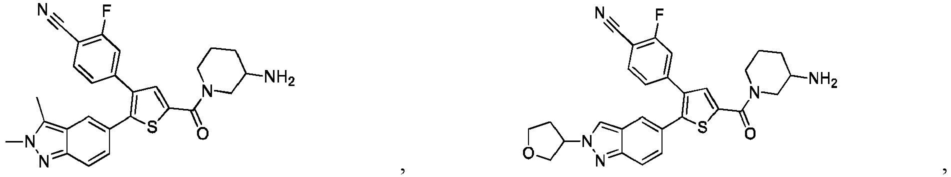

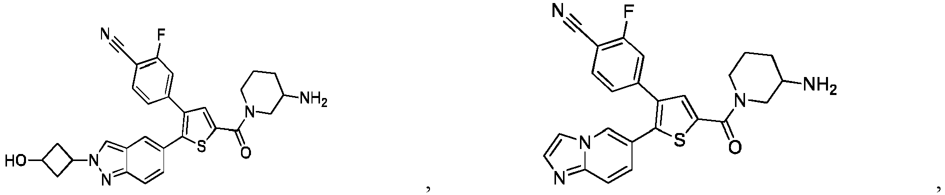

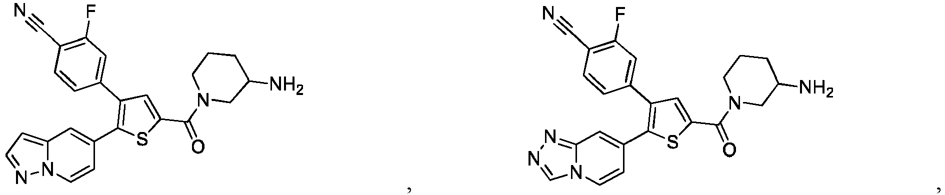

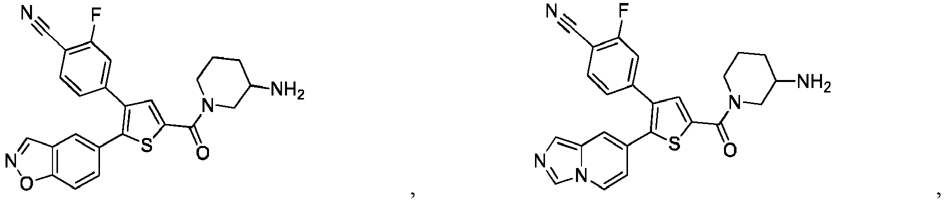

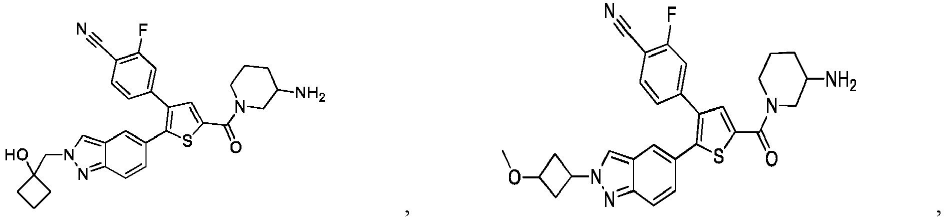

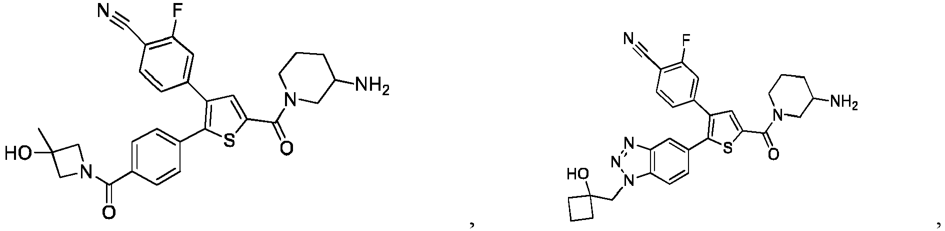

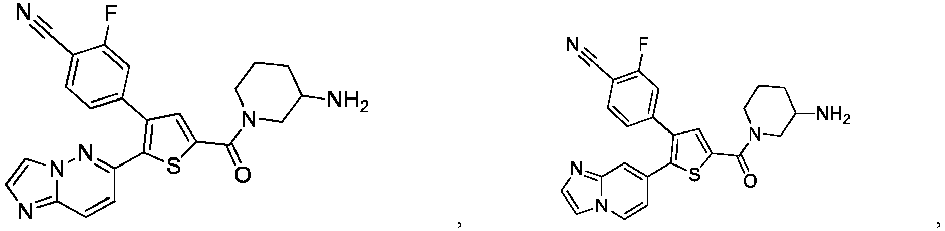

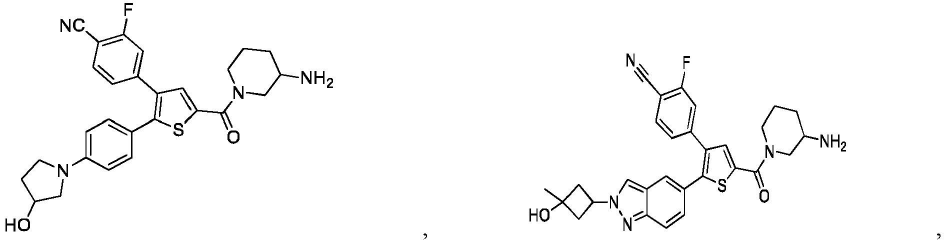

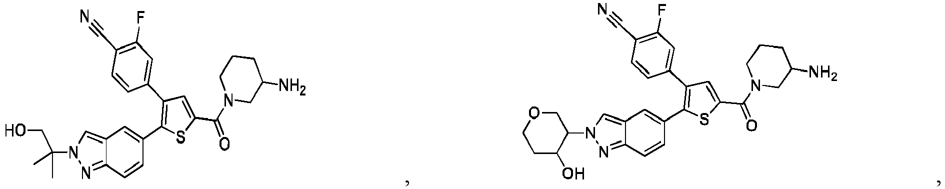

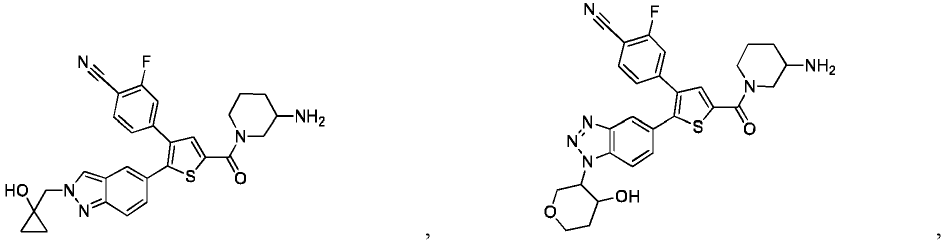

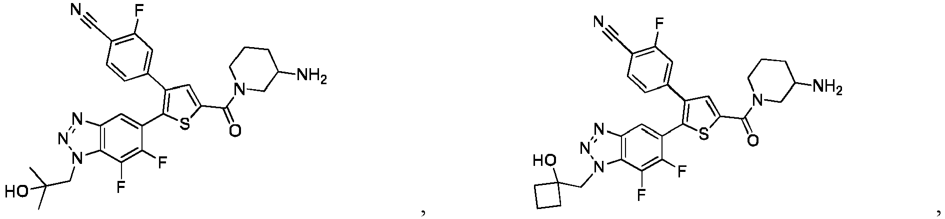

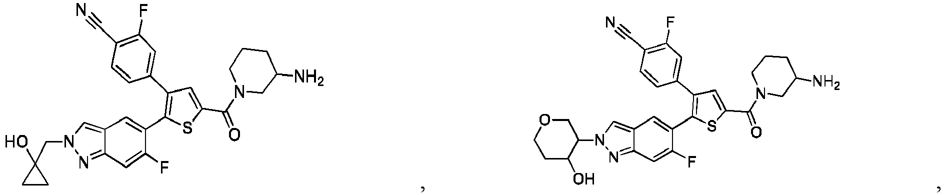

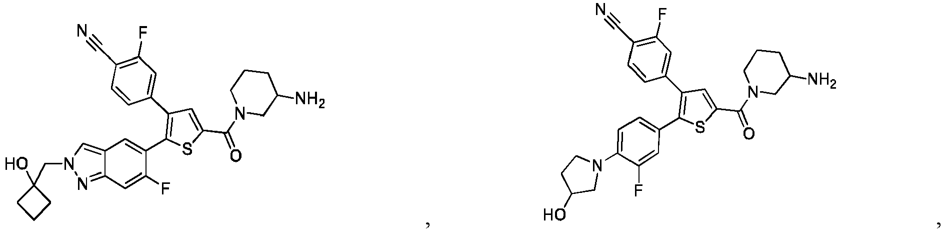

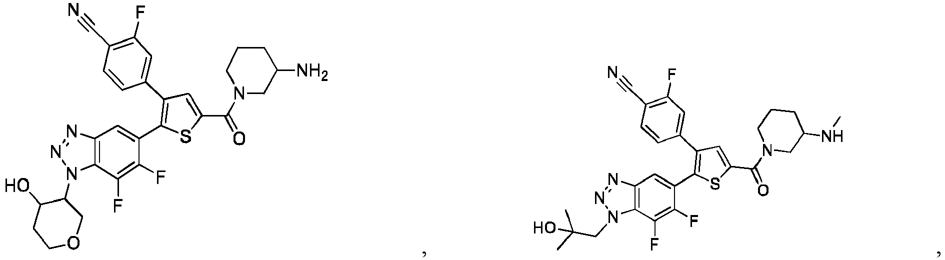

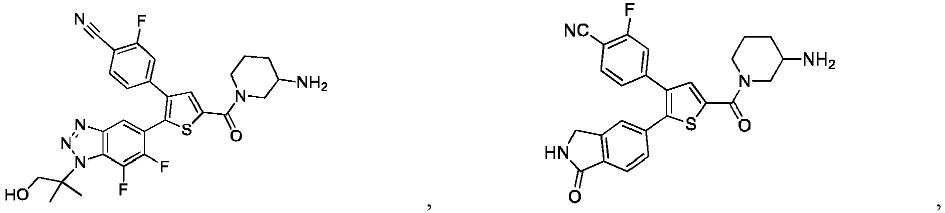

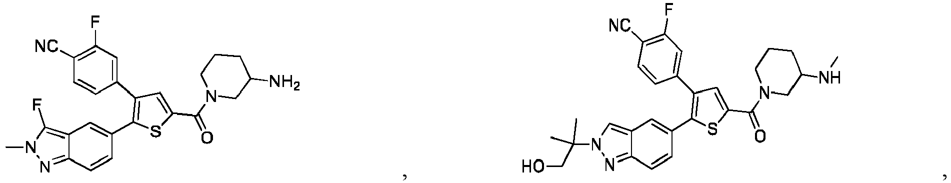

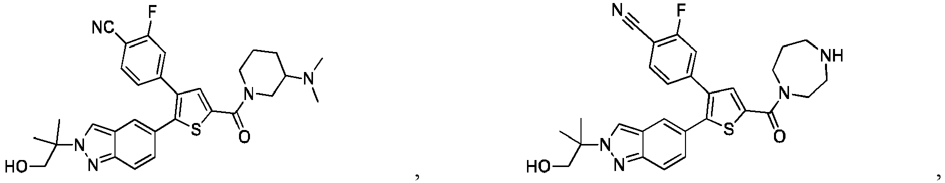

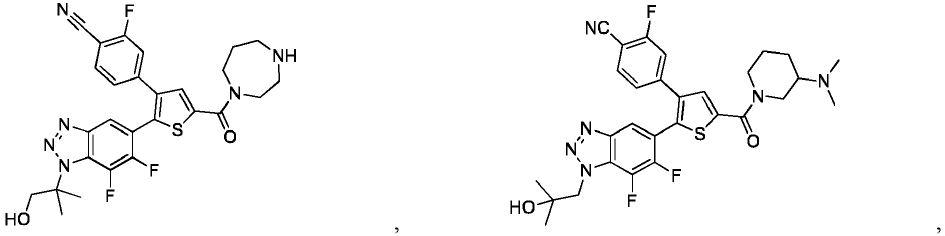

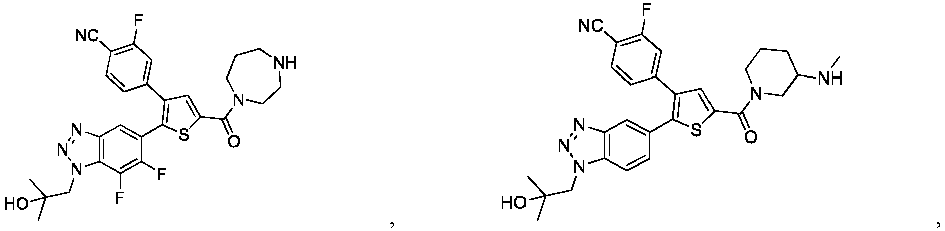

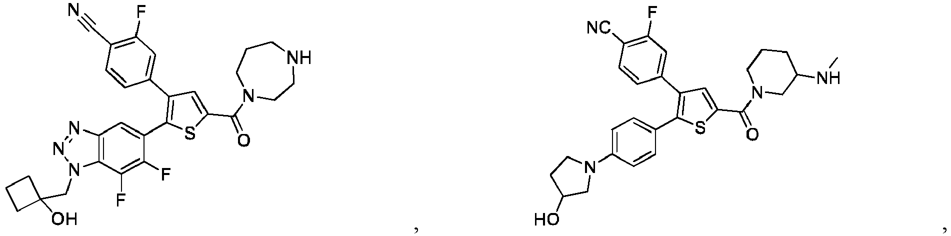

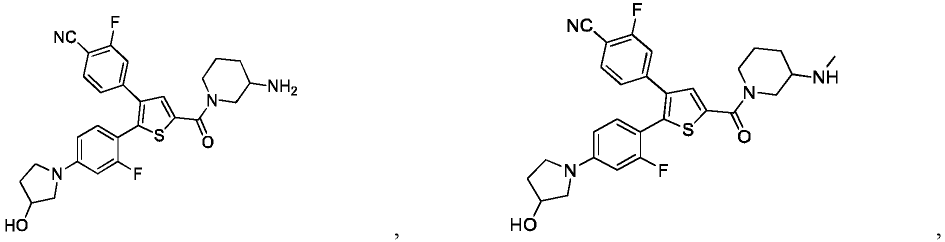

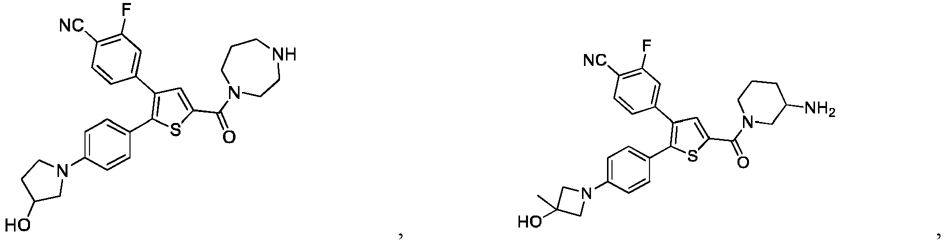

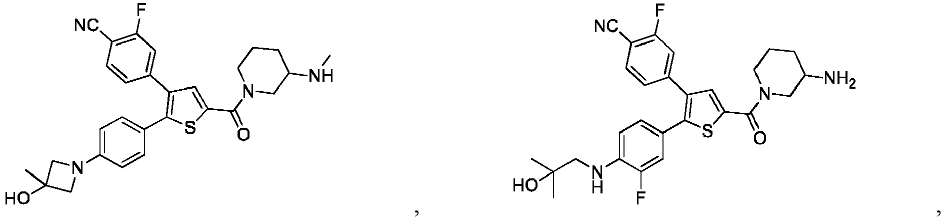

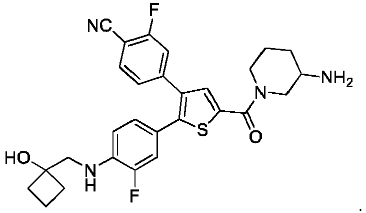

In some embodiments of the present disclosure, the compound or the pharmaceutically acceptable salt thereof is selected from:

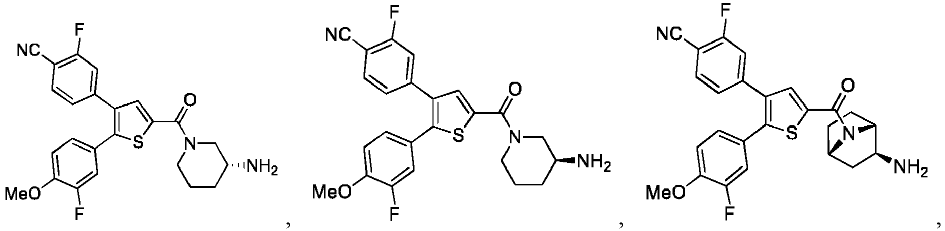

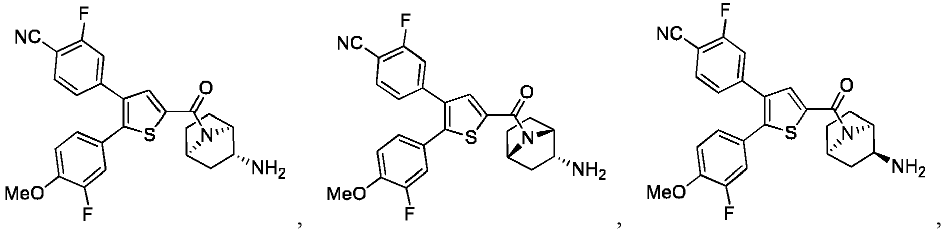

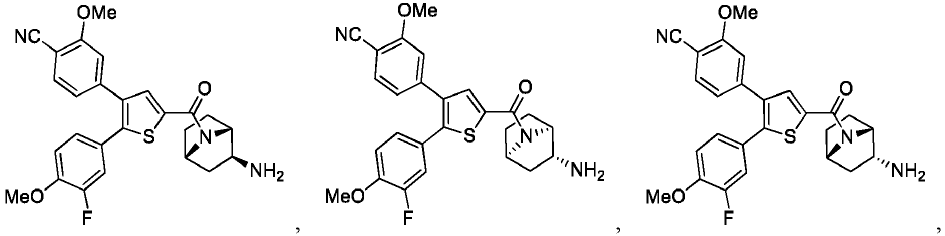

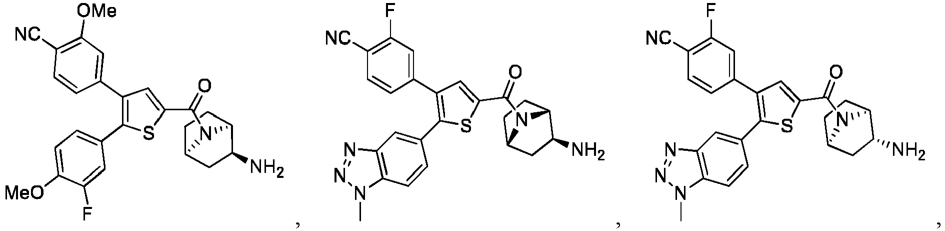

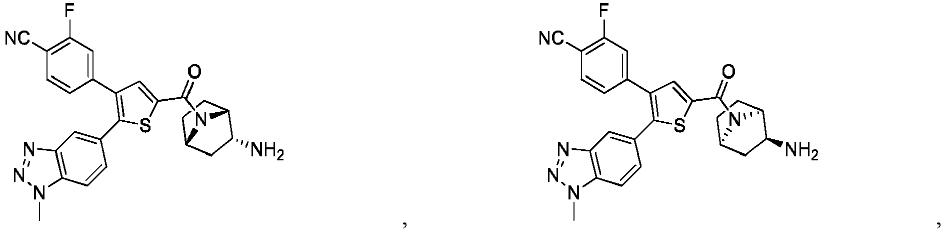

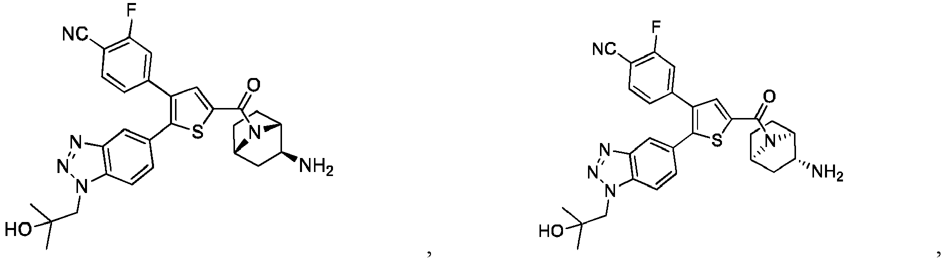

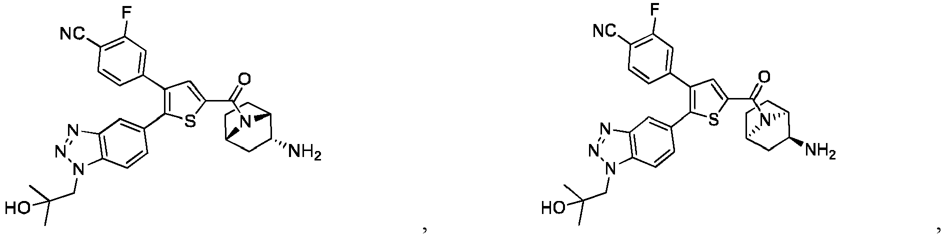

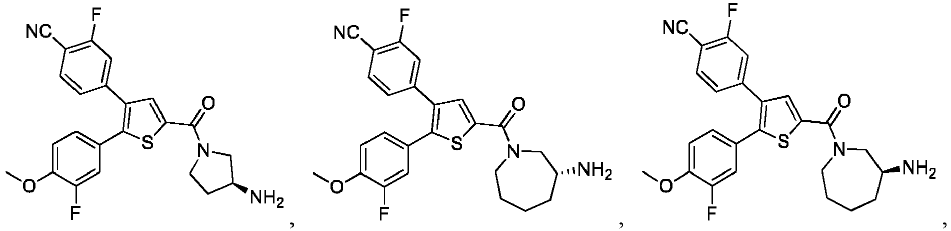

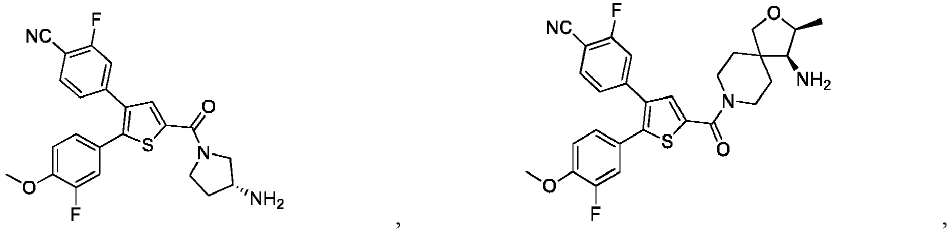

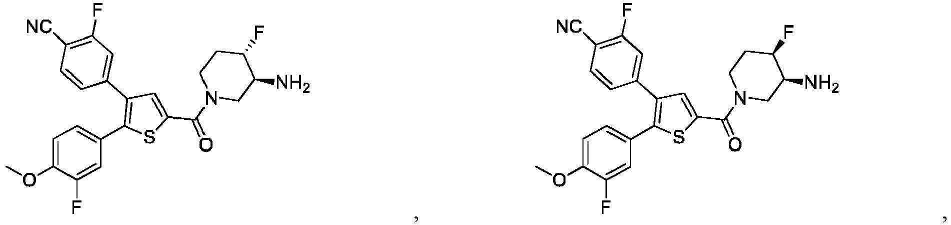

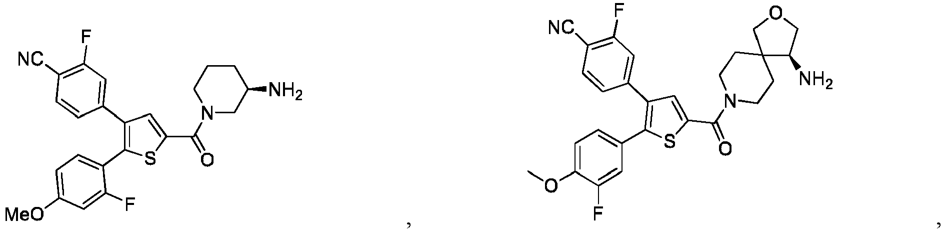

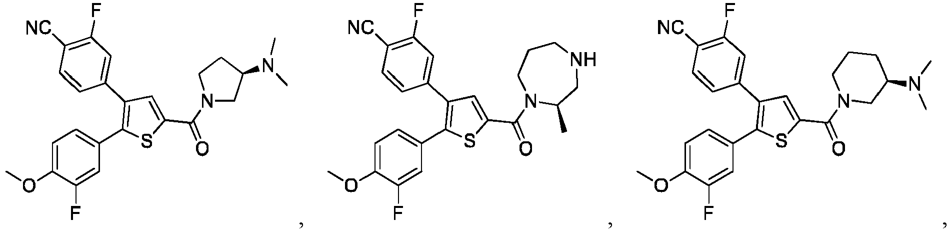

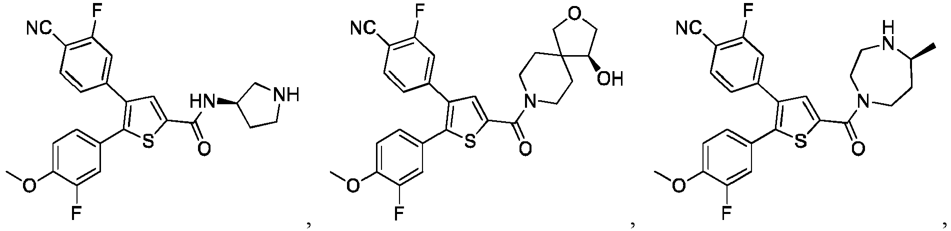

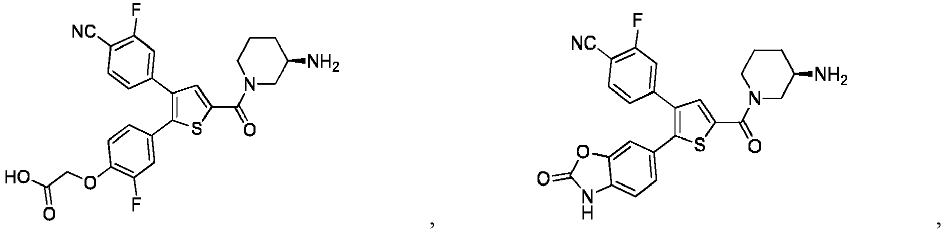

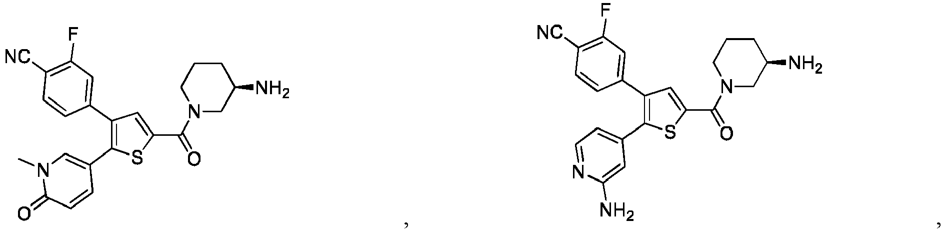

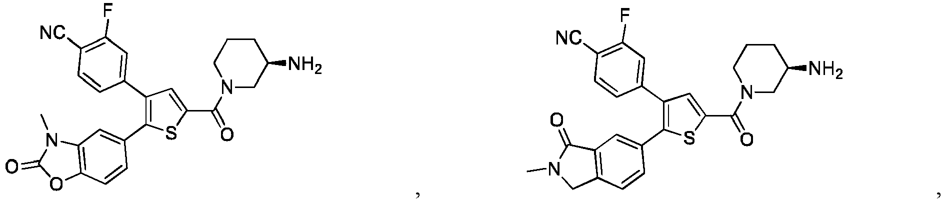

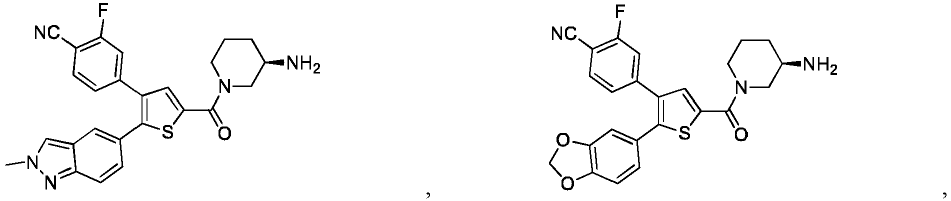

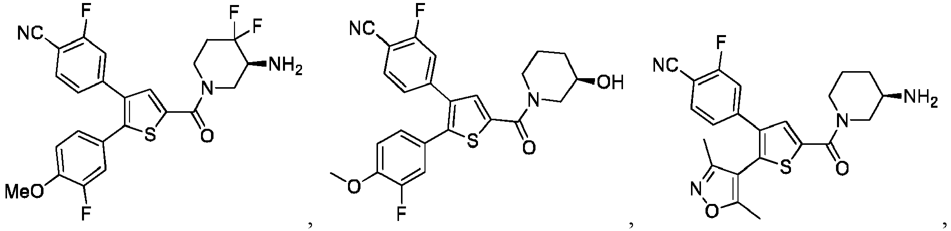

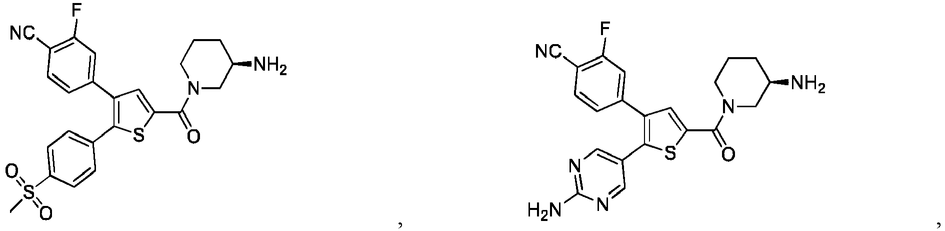

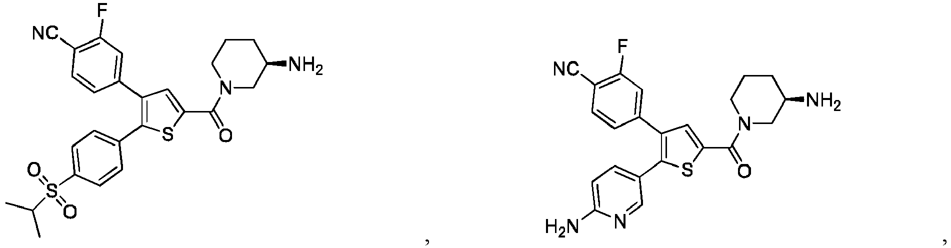

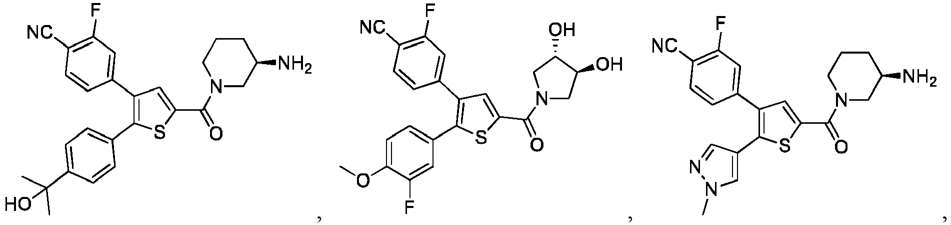

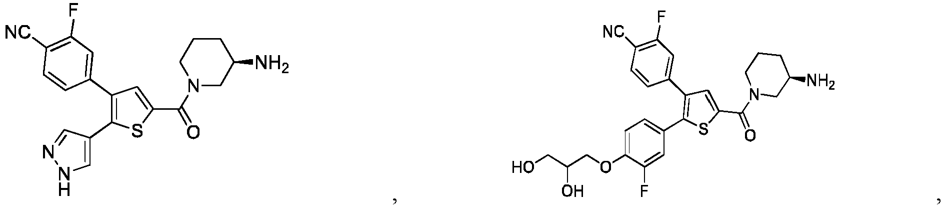

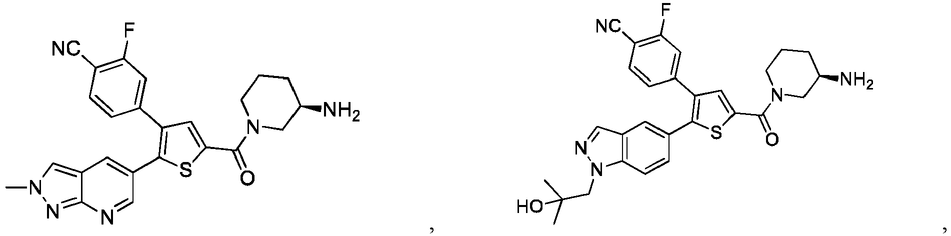

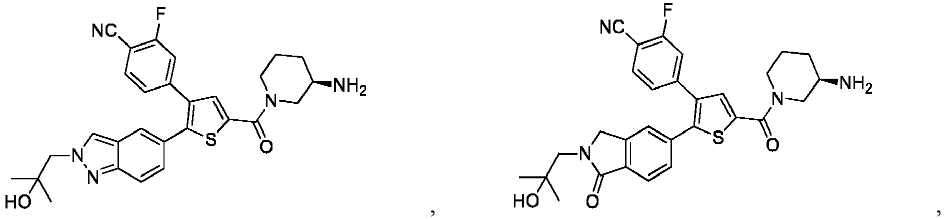

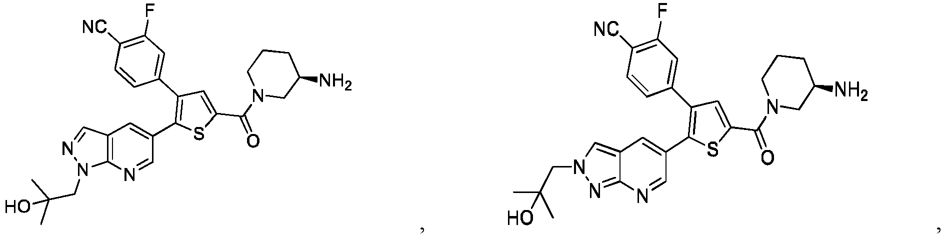

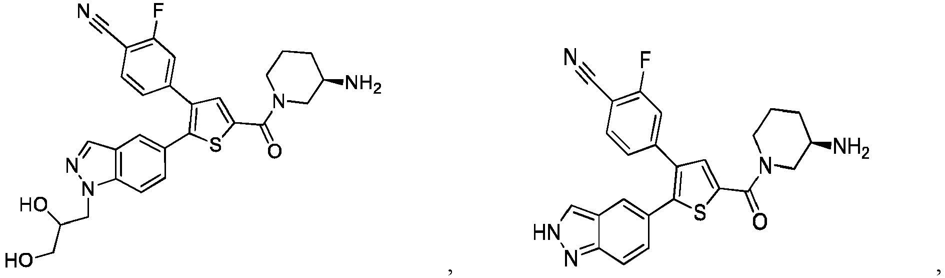

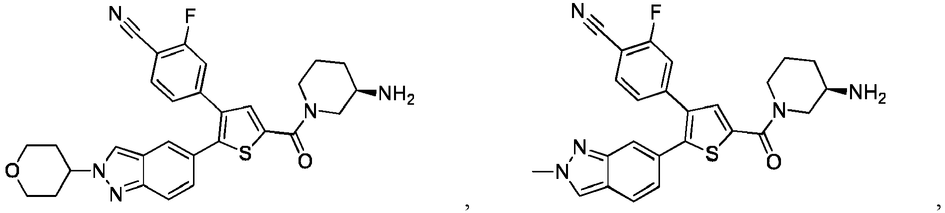

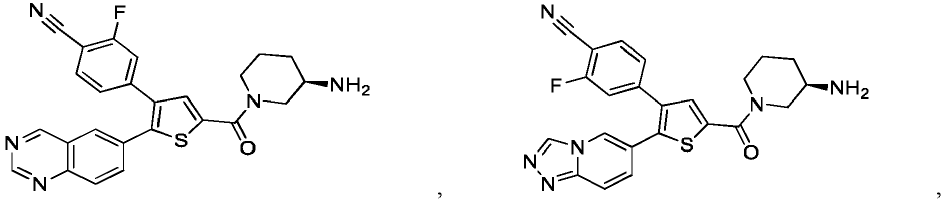

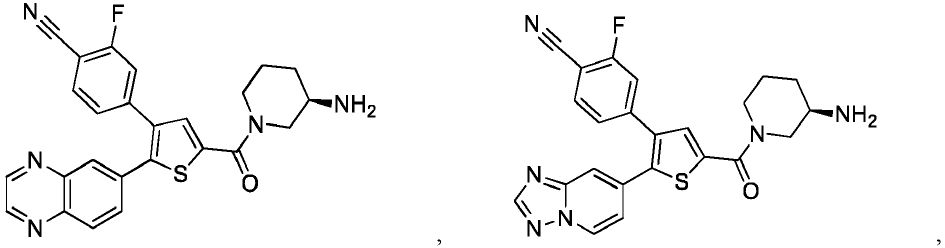

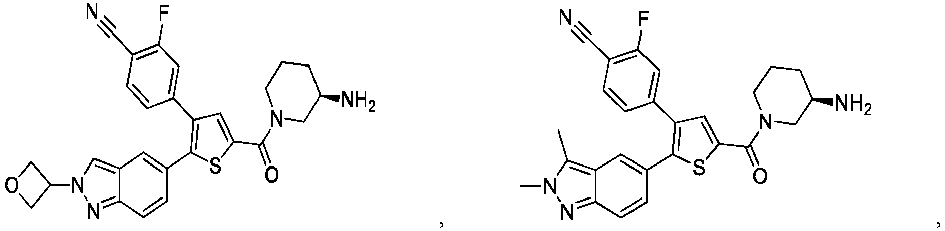

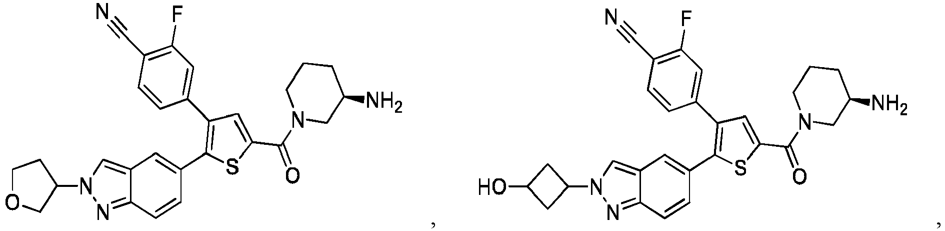

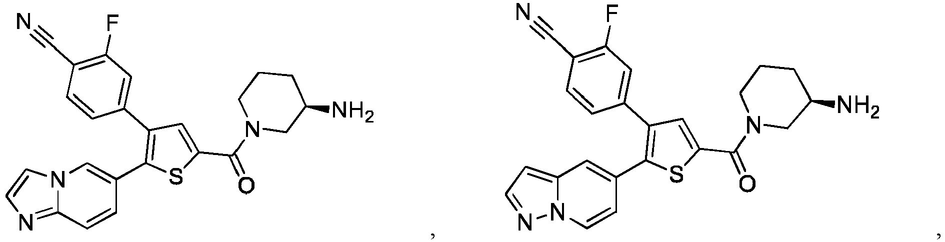

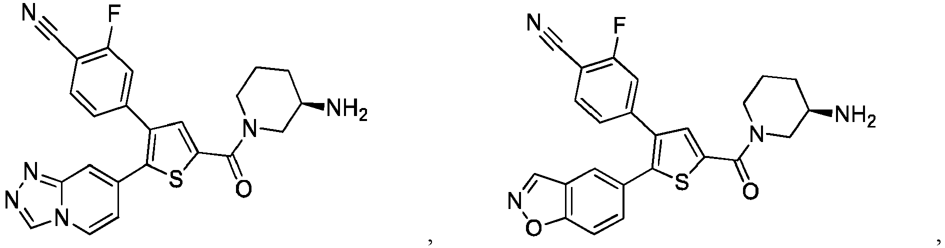

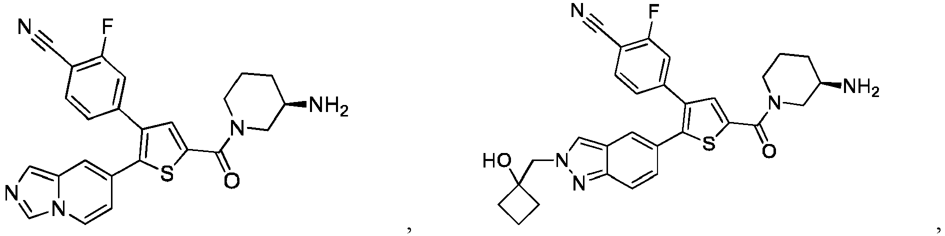

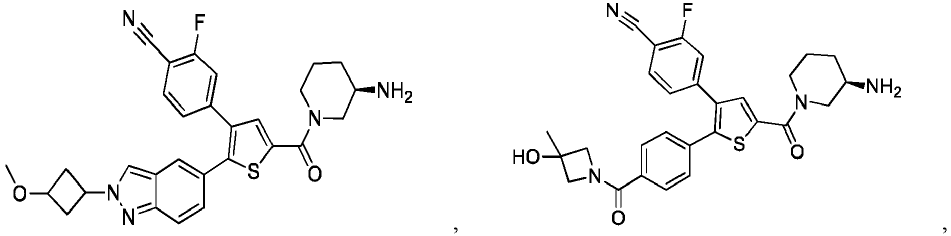

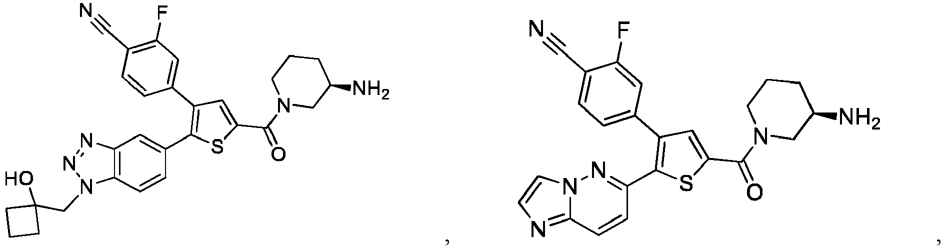

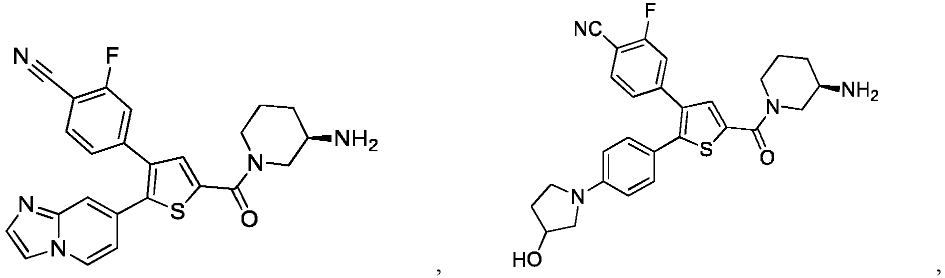

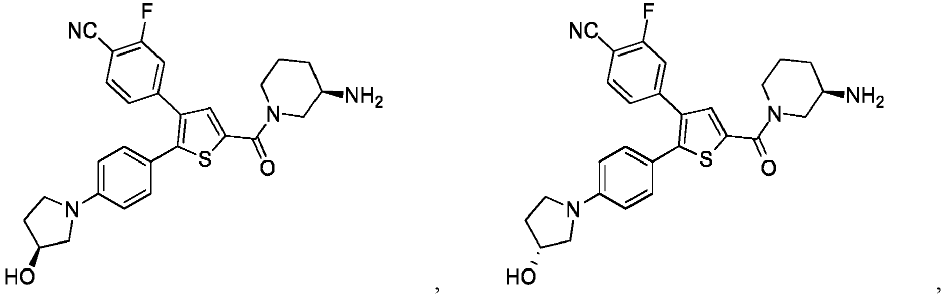

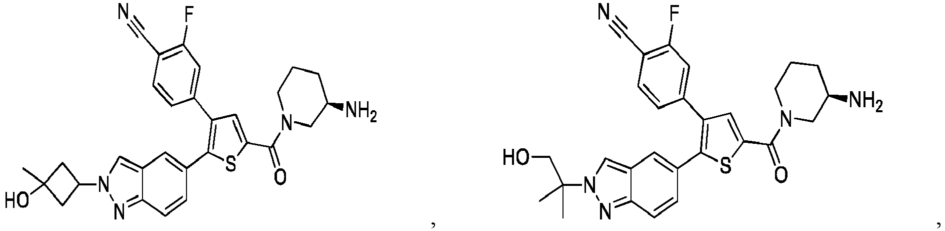

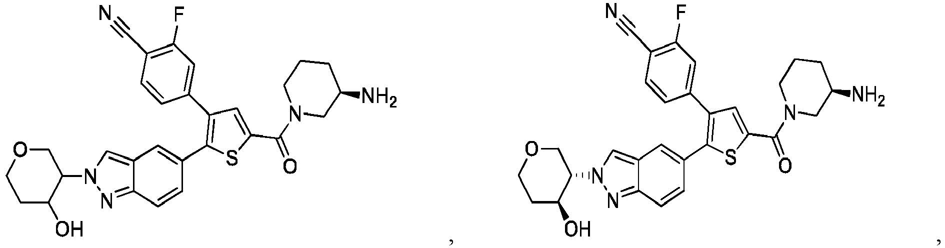

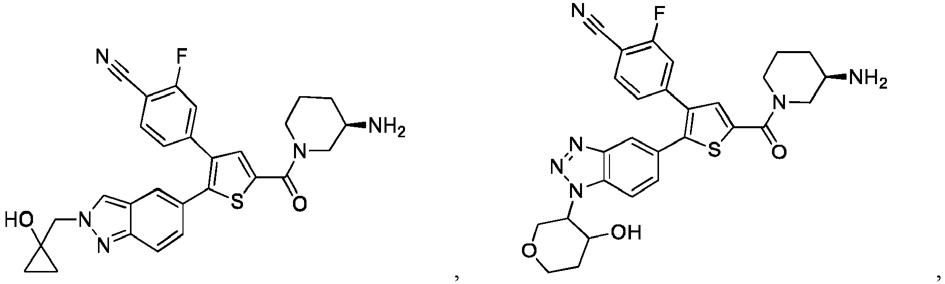

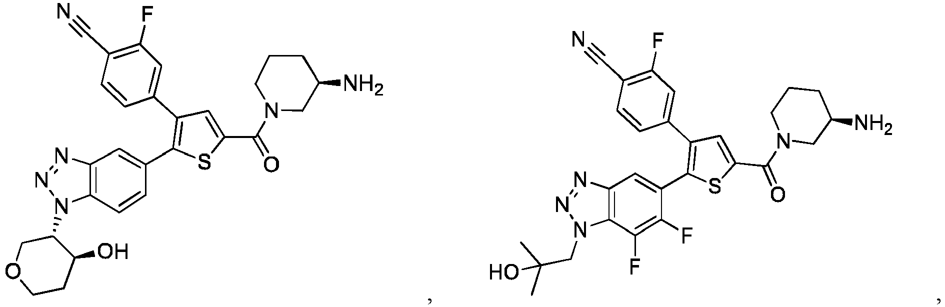

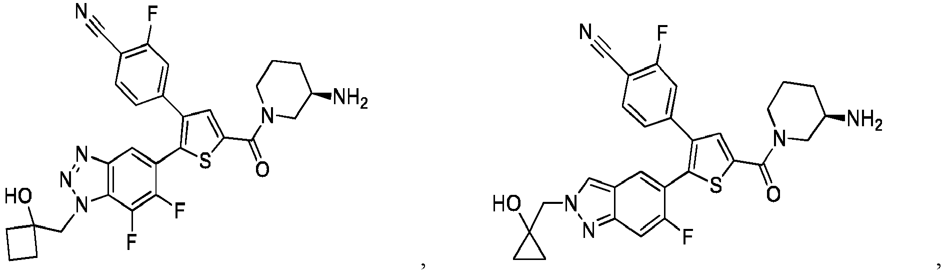

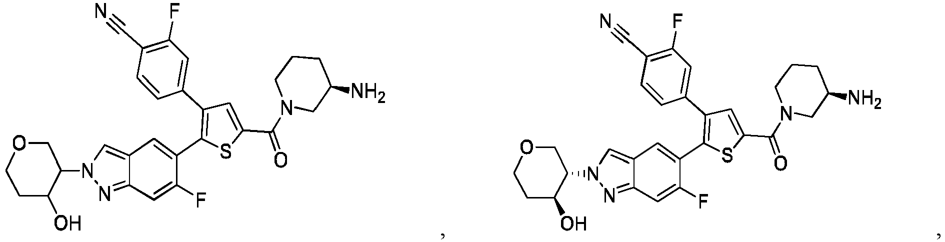

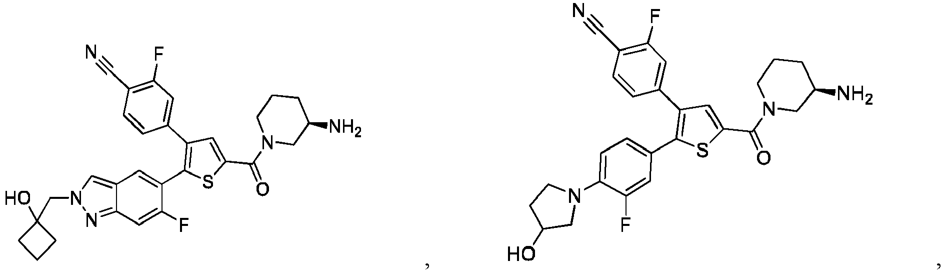

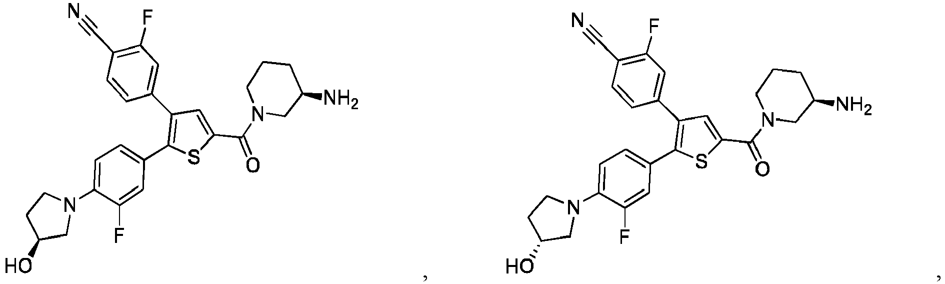

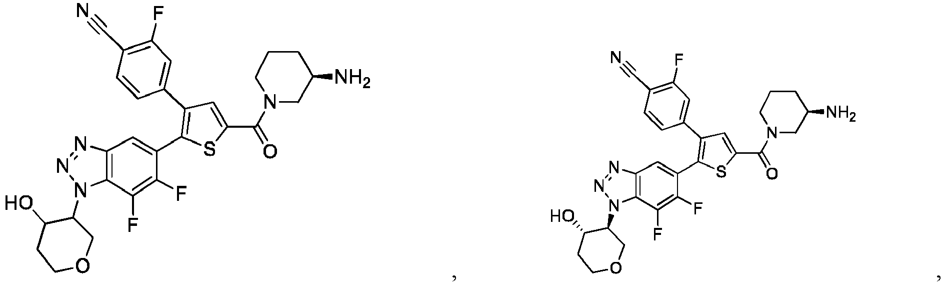

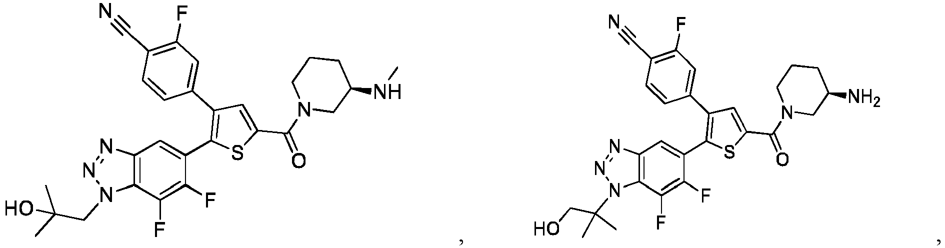

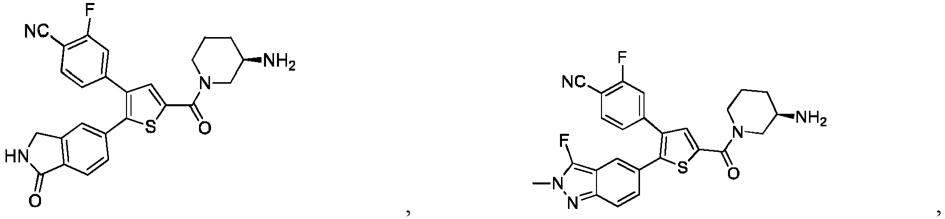

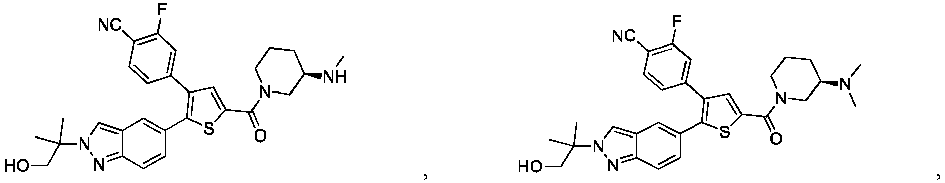

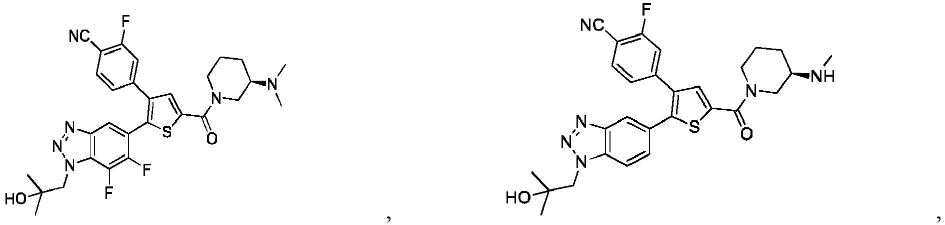

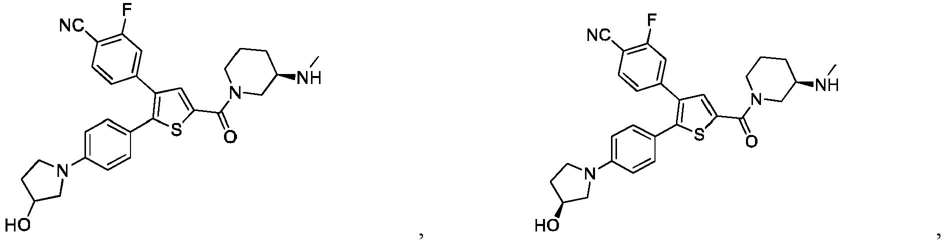

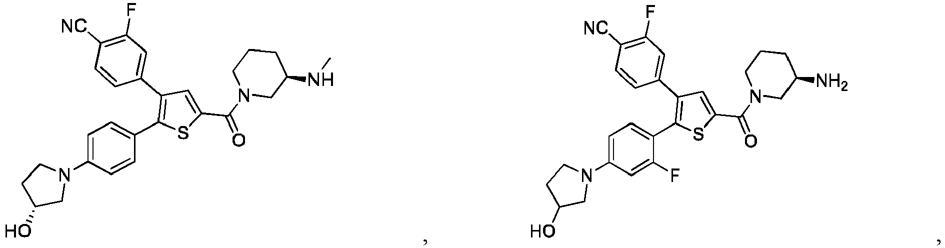

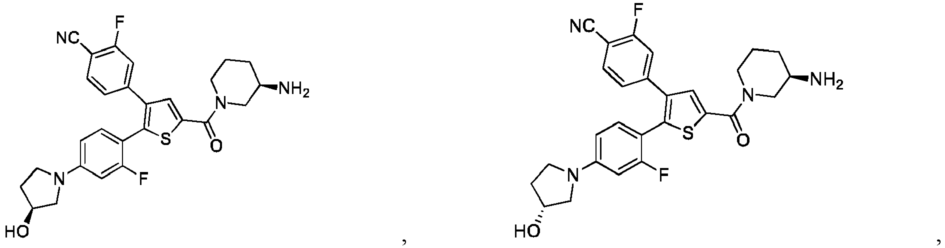

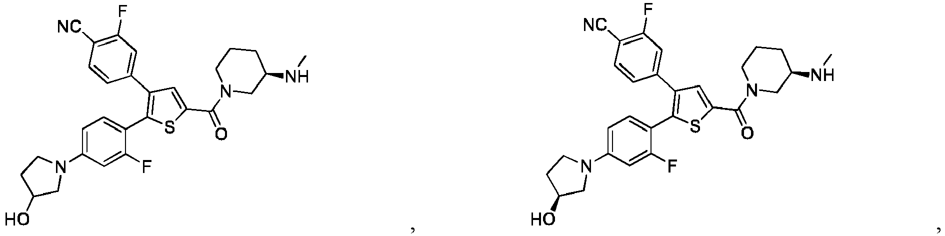

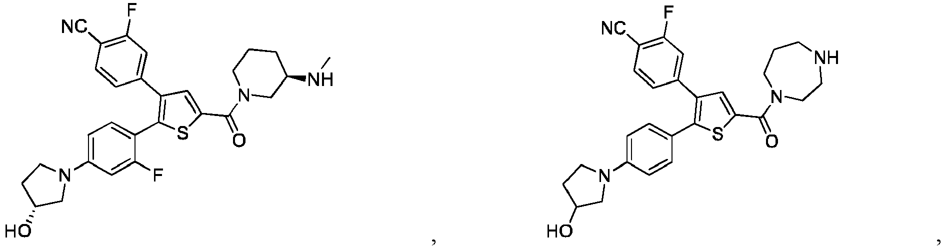

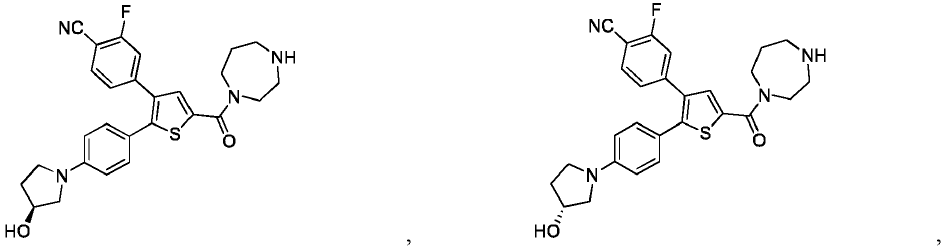

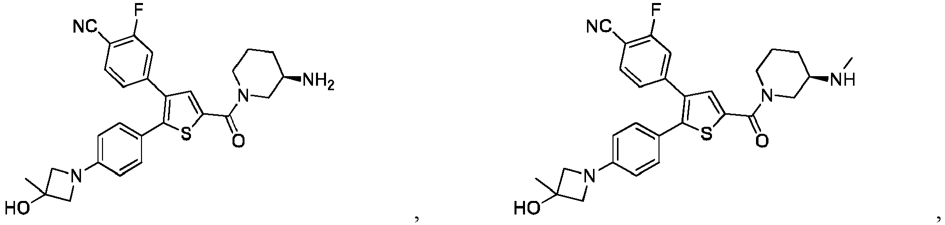

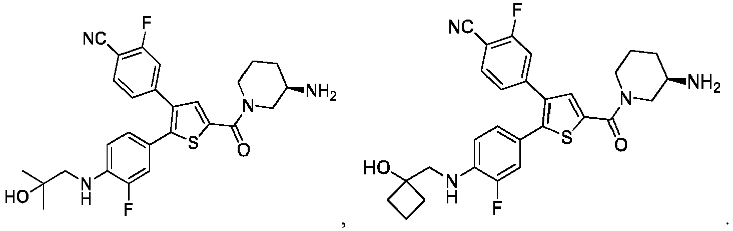

The present disclosure also provides the compound or the pharmaceutically acceptable salt thereof, wherein the salt is hydrochloride.

The present disclosure also provides a use of the compound or the pharmaceutically acceptable salt thereof in the manufacture of a medicament for treating diseases related to LSD1.

### Technical effect

The compounds of the present disclosure can better inhibit the activity of LSD1; the compounds also have obvious inhibitory activity on the proliferation of NCI-H1417 cells, and have good pharmacokinetic properties (including good oral bioavailability, oral exposure, half-life, and clearance rate, etc.); the compounds do not have significant inhibitory effect on the hERG potassium ion channel, thus ensuring high safety; the compounds of the present disclosure have an excellent tumor inhibitory effect on human small cell lung cancer NCI-H1417 xenograft tumor model.

### Definition and description

Unless otherwise specified, the following terms and phrases used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of an amino acid (such as arginine), and a salt of an organic acid such as glucuronic acid. Certain specific compounds of the present disclosure contain both basic and acidic functional groups and thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and"(±)" refers to racemic.

Unless otherwise specified, the absolute configuration of a stereogenic center is represented by a wedged solid bond ( 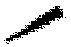 ) and a wedged dashed bond ( 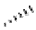 ), and the relative configuration of a stereogenic center is represented by a straight solid bond ( 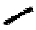 ) and a straight dashed bond ( 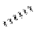 ), a wave line ( 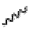 ) is used to represent a wedged solid bond ( 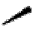 ) or a wedged dashed bond ( 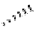 ), or the wave line ( 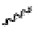 ) is used to represent a straight solid bond ( 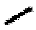 ) and a straight dashed bond ( 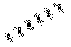 ).

Unless otherwise specified, when double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by a wave line ( 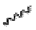 ), this refers to the (Z) isomer, (E) isomer, or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as a mixture of two isomers of formula (C-1) and formula (C-2).

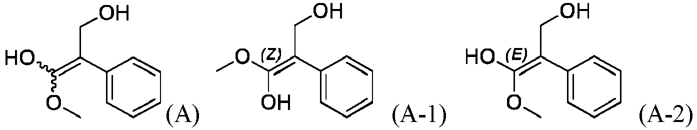

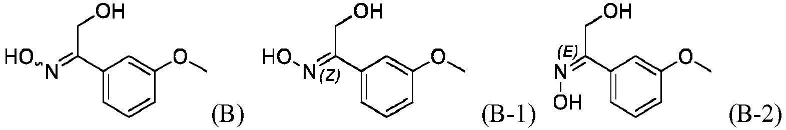

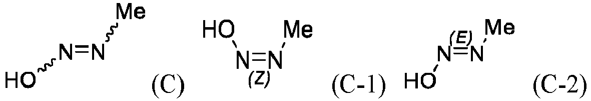

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers and *D* and *L* isomers can be prepared using chiral synthesis or chiral reagents or other conventional techniques. If one kind of enantiomer of a certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom that constitutes the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone.

The term "optionally substituted" means an atom can be substituted by a substituent or not, unless otherwise specified, the type and number of the substituent may be arbitrary as long as it is chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 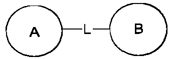 is -M-W-, then -M-W- can link ring A and ring B to form 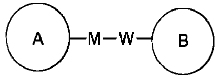 in the direction same as left-to-right reading order, and form 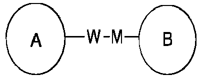 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of chemical bond(s) linking thereto to meet the corresponding valence. The chemical bond between the site and other groups can be represented by a straight solid bond ( 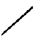 ), a straight dashed bond ( 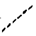 ) or a wavy line ( 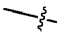 ). For example, the straight solid bond in -OCH₃ means that it is linked to other groups through the oxygen atom in the group; the straight dashed bond in 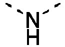 means that it is linked to other groups through the two ends of the nitrogen atom in the group; the wave lines in 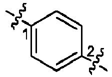 means that the phenyl group is linked to other groups through carbon atoms at position 1 and position 2; 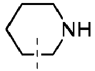 means that it can be linked to other groups through any linkable sites on the piperidinyl by one chemical bond, including at least four types of linkage, including 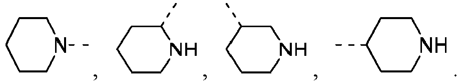 Even though the H atom is drawn on the -N-, 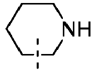 still includes the linkage of 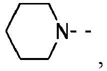 merely when one chemical bond was connected, the H of this site will be reduced by one to the corresponding monovalent piperidinyl.

Unless otherwise specified, the terms "halo" or "halogen" by itself or as a part of another substituent refers to a fluorine, chlorine, bromine, or iodine atom.

Unless otherwise specified, Cₙ₋ₙ₊ₘ or Cₙ₋Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring.

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, C₂₋₃ alkyl, etc.; it can be monovalent (such as methyl), divalent (such as methylene), or multivalent (such as methine). Examples of C₁₋₄ alkyl include, but are not limited to, methyl (Me), ethyl (Et), propyl (including *n*-propyl and isopropyl), butyl (including *n*-butyl, isobutyl, *s*-butyl, and *t*-butyl), etc.

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₂, C₂₋₃, C₃, C₂ alkoxy, etc. Examples of C₁₋₃ alkoxy include, but are not limited to, methoxy, ethoxy, propoxy (including *n*-propoxy and isopropoxy), etc.

Unless otherwise specified, "C₃₋₆ cycloalkyl" refers to a saturated cyclic hydrocarbon group consisting of 3 to 6 carbon atoms, which is monocyclic system and bicyclic systems, and the C₃₋₆ cycloalkyl includes C₃₋₅, C₄₋₅, C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic systems include a spiro ring, a fused ring, and a bridged ring. In addition, concerning the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, etc.

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl,or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for blocking the side reaction on hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed using conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by the direct method (Shelxs97).

The solvents used in the present disclosure are commercially available. The present disclosure adopts the following abbreviations: aq represents water; m-CPBA represents 3-chloroperoxybenzoic acid; eq represents equivalent; Boc represents tert-butoxycarbonyl, which is an amine protecting group.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and specific embodiments thereof have also been disclosed; for those skilled in the art, it is obvious to make various modifications and improvements to the embodiments of the present disclosure without departing from the spirit and scope of the present disclosure.

### Example 1

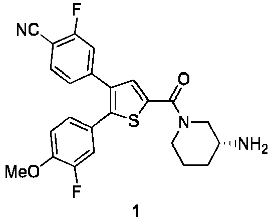

### Synthetic route:

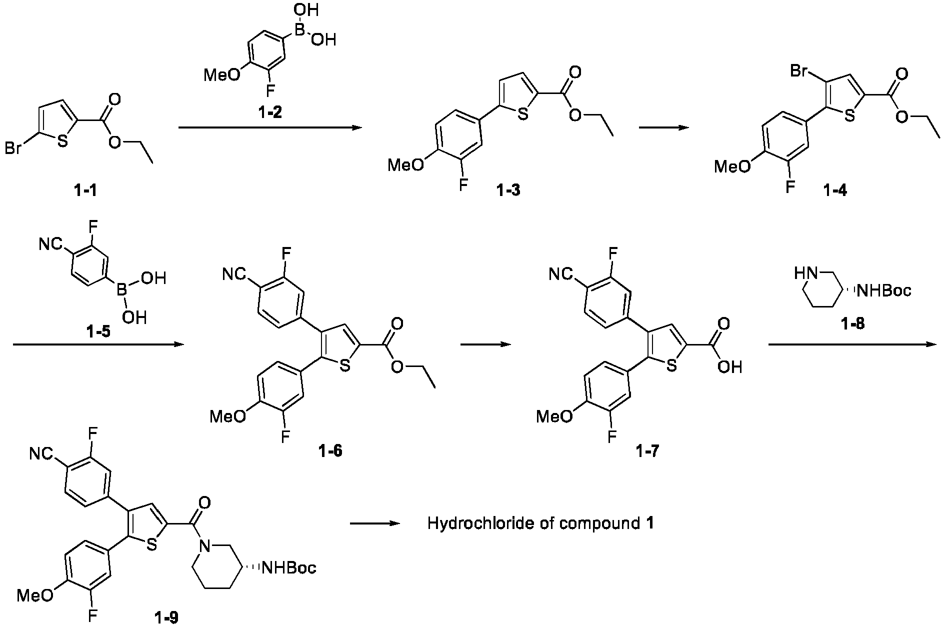

### Step 1

Under nitrogen atmosphere, compound **1-1** (100 mg, 0.425 mmol), compound **1-2** (108 mg, 0.638 mmol), and sodium carbonate (90.2 mg, 0.851 mmol) were dissolved in dioxane (3.00 mL) and water (3.00 mL), and the reaction mixture was added with tetrakis(triphenylphosphine)palladium (49.2 mg, 42.5 µmol), stirred, and reacted for 14 hours at 80°C. The reaction mixture was concentrated under reduced pressure, and the residue was purified by silica gel column chromatography (10/1, petroleum ether/ethyl acetate, R_{f} = 0.35) to obtain compound **1-3.** ¹H NMR (400MHz, CDCl₃) δ = 7.74 (d, *J*=4.0 Hz, 1H), 7.42-7.37 (m, 1H), 7.37-7.33 (m, 1H), 7.18 (d, *J*=4.0 Hz, 1H), 7.03-6.96 (m, 1H), 4.37 (q, *J*=7.2 Hz, 2H), 3.94 (s, 3H), 1.40 (t, *J*=7.2 Hz, 3H). MS-ESI calculated for [M+H]⁺ 281, found 281.

### Step 2

Compound **1-3** (580 mg, 2.07 mmol) was dissolved in *N*,*N*-dimethylformamide (5.00 mL). *N*-Bromosuccinimide (737 mg, 4.14 mmol) was added thereto, and the reaction mixture was stirred and reacted for 14 hours at 25°C. After the reaction mixture was concentrated, the resulting crude product was purified by silica gel column chromatography (5/1, petroleum ether/ethyl acetate, R_{f} = 0.7) to obtain compound **1-4.** ¹H NMR (400MHz, CDCl₃) δ = 7.71 (s, 1H), 7.48-7.45 (m, 1H), 7.43-7.36 (m, 1H), 7.04 (t, *J*=8.6 Hz, 1H), 4.38 (q, *J*=7.2 Hz, 2H), 3.96 (s, 3H), 1.39 (t, *J*=7.2 Hz, 3H). MS-ESI calculated for [M+H]⁺ 359, found 359.

### Step 3

Under nitrogen atmosphere, compound **1-4** (710 mg, 1.98 mmol), compound **1-5** (391 mg, 2.37 mmol), and sodium carbonate (419 mg, 3.95 mmol) were dissolved in ethanol (4.00 mL) and water (1.00 mL), and the reaction mixture was added with bis(di-tert-butyl(4-dimethylaminophenyl)phosphine)dichloropalladium(II) (70.0 mg, 98.8 µmol), stirred, and reacted for 12 hours at 80°C. After the reaction mixture was concentrated, the resulting crude product was purified by silica gel column chromatography (10/1, petroleum ether/ethyl acetate, Rf = 0.6) to obtain compound **1-6.** ¹H NMR (400MHz, CDCl₃) δ = 7.80 (s, 1H), 7.58-7.55 (m, 1H), 7.19-7.10 (m, 2H), 7.04-6.98 (m, 2H), 6.96-6.91 (m, 1H), 4.40 (q, *J*=7.2 Hz, 2H), 3.93 (s, 3H), 1.41 (t, *J*=7.2 Hz, 3H). MS-ESI calculated for [M+H]⁺ 400, found 400.

### Step 4

Compound **1-6** (350 mg, 876 µmol) was dissolved in tetrahydrofuran (3.00 mL) and water (3.00 mL), and lithium hydroxide monohydrate (110 mg, 2.63 mmol) was added thereto, and the reaction mixture was stirred and reacted for 14 hours at 20°C. After the reaction mixture was concentrated, the residue was added with water (10 mL). The mixture was acidified to pH = 6 to 7 with 1 M dilute hydrochloric acid, and extracted with ethyl acetate (5 mL × 3). The organic phases were combined, dried over anhydrous sodium sulfate, filtered, and concentrated to obtain crude compound **1-7**, which was used directly in the next reaction step. ¹H NMR (400MHz, DMSO-*d₆)* δ = 7.90 (s, 1H), 7.89-7.85 (m, 1H), 7.50-7.52 (m, 1H), 7.26-7.16 (m, 3H), 7.10-7.08 (m, 1H), 3.86 (s, 3H). MS-ESI calculated for [M-H]⁺ 370, found 370.

### Step 5

Compound **1-7** (200 mg, 539 µmol), compound **1-8** (108 mg, 539 µmol), and 2-(7-azabenzotriazol-1-yl)-*N*,*N*,*N'*,*N'*-tetramethyluronium hexafluorophosphate (307 mg, 808 µmol) were dissolved in *N*,*N*-dimethylformamide (3.00 mL), and *N*,*N*-diisopropylethylamine (209 mg, 1.62 mmol) was added thereto, and the reaction mixture was stirred and reacted for 12 hours at 20°C. To the reaction mixture were added water (20 mL) and ethyl acetate (5 mL). The organic phase was separated, and the aqueous phase was extracted with ethyl acetate (5 mL × 3). The organic phases were combined, dried over anhydrous sodium sulfate, filtered, and concentrated, and the resulting crude product was purified by column chromatography (20/1, dichloromethane/methanol, R_{f} = 0.5) to obtain compound **1-9.** ¹H NMR (400MHz, CDCl₃) δ = 7.72 (s, 1H), 7.58-7.41 (m, 2H), 7.24 (d, *J*=8.0 Hz, 1H), 7.08-6.98 (m, 2H), 6.96-6.89 (m, 1H), 4.61-4.60 (m, 2H), 4.40-4.36 (m, 1H), 3.93 (s, 3H), 3.74-3.71 (m, 1H), 3.11-2.89 (m, 2H), 2.13-2.04 (m, 1H), 1.93-1.82 (m, 1H), 1.76-1.61 (m, 1H), 1.47 (s, 9H). MS-ESI calculated for [M+H]⁺ 554, found 554.

### Step 6

Compound **1-9** (150 mg, 271 µmol) was dissolved in ethyl acetate (1.00 mL), and hydrochloride ethyl acetate solution (4.00 mL, 4M) was added thereto, and the reaction mixture was stirred and reacted for 10 hours at 20°C. After the reaction mixture was concentrated, the residue was purified by preparative high performance liquid chromatography (3_Phenomenex Luna C18 75 * 30 mm * 3 µm; mobile phase A: hydrochloric acid aqueous solution with a volume fraction of 0.05%; mobile phase B: acetonitrile; B (acetonitrile) %: 25% to 45%, 6.5 minutes) to obtain the hydrochloride of compound **1**. ¹H NMR (400MHz, DMSO-*d₆*) δ = 8.15 (s, 2H), 7.89 (t, *J*=7.2 Hz, 1H), 7.70-7.54 (m, 2H), 7.30-7.15 (m, 3H), 7.09-7.07 (m, 1H), 4.43-4.25 (m, 1H), 4.16-3.96 (m, 1H), 3.86 (s, 3H), 3.31-3.20 (m, 3H), 2.13-1.98 (m, 1H), 1.90-1.74 (m, 1H), 1.72-1.47 (m, 2H). MS-ESI calculated for [M+H]⁺ 454, found 454.

### Example 2

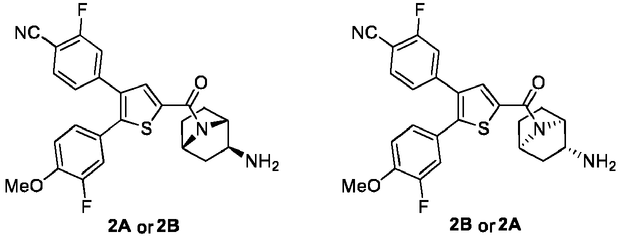

### Synthetic route:

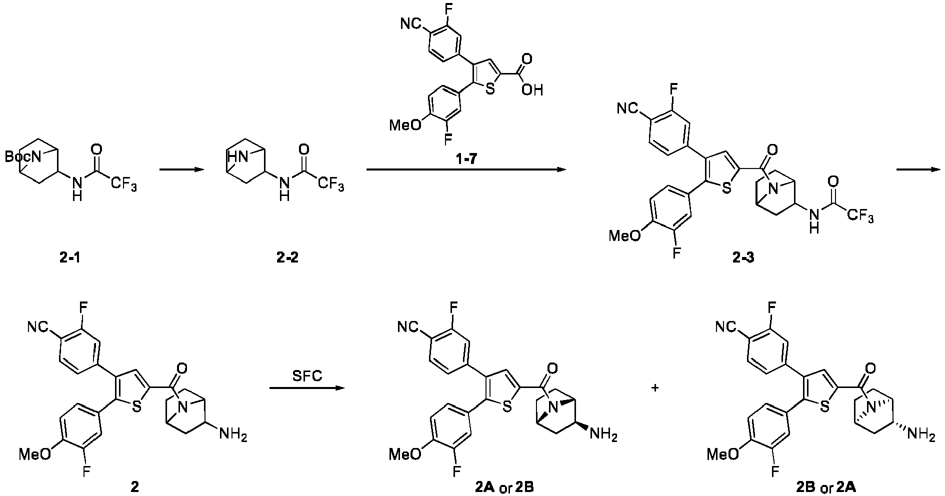

### Step 1

Compound **2-1** (150 mg, 487 µmol) was dissolved in ethyl acetate (1 mL), and hydrochloride ethyl acetate solution (5.00 mL, 4M) was added thereto, and the reaction mixture was stirred and reacted for 14 hours at 20°C. After the reaction mixture was concentrated, the hydrochloride of compound **2-2** was obtained, and the crude product was used directly in the next reaction step without purification. ¹H NMR (400MHz, DMSO-*d₆*) δ = 9.83 (d, *J*=6.0 Hz, 1H), 9.18 (s, 1H), 4.43-4.28 (m, 1H), 4.20 (t, *J*=4.4 Hz, 1H), 4.13 (t, *J*=4.4 Hz, 1H), 2.29-2.16 (m, 1H), 1.88-1.79 (m, 2H), 1.78-1.65 (m, 3H). MS-ESI calculated for [M+H]⁺ 209, found 209.

### Step 2

The hydrochloride of compound **2-2** (116 mg, 474 µmol), 2-(7-azabenzotriazol-1-yl)-*N,N,N',N*'-tetramethyluronium hexafluorophosphate (270 mg, 711 µmol), and *N,N-*diisopropylethylamine (184 mg, 1.42 mmol) were dissolved in *N*,*N*-dimethylformamide (3.00 mL), and compound **1-7** (176 mg, 474 µmol) was added thereto, and the reaction mixture was stirred and reacted for 12 hours at 20°C. To the reaction mixture were added water (20 mL) and ethyl acetate (5 mL). The organic phase was separated, and the aqueous phase was extracted with ethyl acetate (5 mL × 3). The organic phases were combined, dried over anhydrous sodium sulfate, filtered, and concentrated, and the resulting crude product was purified by column chromatography (20/1, dichloromethane/methanol, R_{f} = 0.65) to obtain compound **2-3.** ¹H NMR (400MHz, CDCl₃) δ = 7.57 (dd, *J*=6.8, 7.6 Hz, 1H), 7.53 (s, 1H), 7.19-7.10 (m, 2H), 7.05-7.00 (m, 1H), 7.00-6.98 (m, 1H), 6.97-6.90 (m, 1H), 6.61 (d, *J*=4.8 Hz, 1H), 5.00 (t, *J*=4.8 Hz, 1H), 4.76 (t, *J*=4.8 Hz, 1H), 4.44-4.30 (m, 1H), 3.93 (s, 3H), 2.65-2.50 (m, 1H), 2.05-1.97 (m, 1H), 1.97-1.79 (m, 2H), 1.67-1.59 (m, 2H). MS-ESI calculated for [M+H]⁺ 562, found 562.

### Step 3

Compound **2-3** (70.0 mg, 125 µmol) was dissolved in tetrahydrofuran (1.00 mL) and water (0.500 mL), then sodium hydroxide (15.0 mg, 374 µmol) was added thereto, and the reaction mixture was stirred and reacted for 14 hours at 50°C. To the reaction mixture were added water (5 mL) and ethyl acetate (2 mL). The organic phase was separated, and the aqueous phase was extracted with ethyl acetate (2 mL × 3). The organic phases were combined, dried over anhydrous sodium sulfate, filtered, and concentrated, and the residue was purified by preparative high performance liquid chromatography (3_Phenomenex Luna C18 75 * 30 mm * 3 µm; mobile phase A: hydrochloric acid aqueous solution with a volume fraction of 0.05%; mobile phase B: acetonitrile; B%: 25% to 45%, 6.5 minutes) to obtain the hydrochloride of compound **2**. ¹H NMR (400MHz, DMSO-*d₆*) δ = 8.52 (s, 2H), 7.88 (t, *J*=7.6 Hz, 1H), 7.84 (s, 1H), 7.58 (d, *J*=10.4 Hz, 1H), 7.27-7.15 (m, 3H), 7.10-7.08 (m, 1H), 4.79-4.57 (m, 2H), 3.86 (s, 3H), 3.68-3.66 (m, 1H), 2.37-2.20 (m, 1H), 2.04-1.91 (m, 1H), 1.88-1.67 (m, 3H), 1.46 (dd, *J*=4.0, 12.8 Hz, 1H). MS-ESI calculated for [M+H]⁺ 466, found 466.

### Step 4

Compound **2** was purified by SFC (DAICEL CHIRALPAK IC 250 mm * 30 mm, 10 µm; mobile phase A: ammonia aqueous solution with a volume fraction of 0.1%; mobile phase B: methanol; B (methanol) %: 45% to 45%, 20 minutes) to obtain compounds **2A** and **2B.**

Compound **2A** (retention time: 7.579 minutes). Compound **2A** ¹H NMR (400MHz, CD₃OD) δ = 7.68 (t, *J*=7.6 Hz, 1H), 7.63 (s, 1H), 7.33-7.23 (m, 2H), 7.12-7.02 (m, 3H), 4.64-4.56 (m, 2H), 3.89 (s, 3H), 3.51-3.48 (m, 1H), 2.35-2.21 (m, 1H), 2.19-2.16 (m, 1H), 1.90-1.68 (m, 3H), 1.12-1.08 (m, 1H).MS-ESI calculated for [M+H]⁺ 466, found 466.

Compound **2B** (retention time: 10.758 minutes). Compound **2B** ¹H NMR (400MHz, CD₃OD) δ = 7.73 (s, 1H), 7.69 (t, *J*=7.4 Hz, 1H), 7.33 (d, *J*=10 Hz, 1H), 7.26 (d, *J*= 8.0 Hz, 1H), 7.15-7.00 (m, 3H), 4.83-4.75 (m, 2H), 3.89 (s, 3H), 3.86-3.78 (m, 1H), 2.57-2.42 (m, 1H), 2.09-1.91 (m, 3H), 1.85-1.74 (m, 1H), 1.54-1.46 (m, 1H).MS-ESI calculated for [M+H]⁺ 466, found 466.

### Example 3

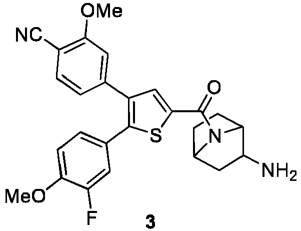

### Synthetic route:

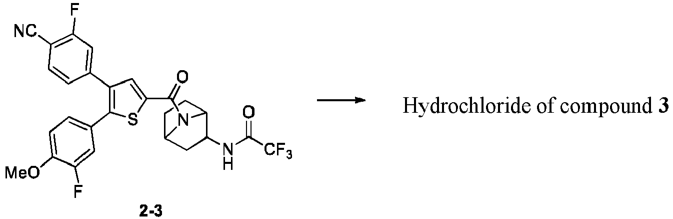

### Step 1

Compound **2-3** (130 mg, 232 µmol) was dissolved in methanol (2.50 mL) and water (1.00 mL), then potassium carbonate (166 mg, 1.20 mmol) was added thereto, and the reaction mixture was stirred and reacted for 10 hours at 20°C. After the reaction mixture was concentrated, the residue was purified by preparative high performance liquid chromatography (3_Phenomenex Luna C18 75 * 30 mm * 3 µm; mobile phase A: hydrochloric acid aqueous solution with a volume fraction of 0.05%; mobile phase B: acetonitrile; B%: 25% to 45%, 6.5 minutes) to obtain the hydrochloride of compound **3**. ¹H NMR (400MHz, DMSO-*d₆*) δ = 8.37 (s, 2H), 7.83 (s, 1H), 7.69 (d, *J*=8.0 Hz, 1H), 7.23-7.14 (m, 3H), 7.08 (dd, *J*=1.2, 8.0 Hz, 1H), 6.94 (dd, *J*=1.2, 8.0 Hz, 1H), 4.73-4.66 (m, 2H), 3.85 (s, 3H), 3.83 (s, 3H), 3.67-3.66 (m, 1H), 2.31-2.21 (m, 1H), 1.99-1.90 (m, 1H), 1.87-1.77 (m, 2H), 1.74-1.66 (m, 1H), 1.42 (dd, *J*=4.4, 12.4 Hz, 1H). MS-ESI calculated for [M+H]⁺ 478, found 478.

### Example 4

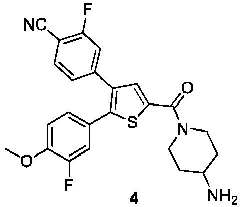

### Synthetic route:

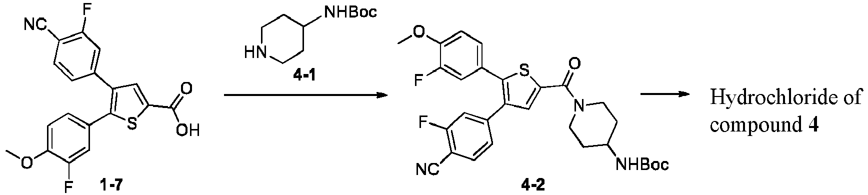

### Step 1

Compound **1-7** (200 mg, 539 µmol), 2-(7-azabenzotriazol-1-yl)-*N*,*N*,*N*',*N*'-tetramethyluronium hexafluorophosphate (307 mg, 808 µmol), and *N*,*N*-diisopropylethylamine (209 mg, 1.62 mmol) were dissolved in *N*,*N*-dimethylformamide (3.00 mL), and then compound **4-1** (108 mg, 539 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.53) to obtain compound **4-2.** MS-ESI calculated for [M-56+H]⁺ 498, found 498.

### Step 2

Compound **4-2** (310 mg, 559.95 µmol) was dissolved in ethyl acetate (3.00 mL), and hydrochloride ethyl acetate solution (4 mol/L, 2.8 mL) was added thereto, and the reaction mixture was stirred and reacted for 12 hours at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%, mobile phase B: acetonitrile B%: 30% to 60%, 9 minutes) to obtain the hydrochloride of compound **4.** ¹H NMR (400MHz, CD₃OD) δ = 7.69 (t, *J*=7.4 Hz, 1H), 7.53 (s, 1H), 7.33 (d, *J*=10.4 Hz, 1H), 7.25 (d, *J*=8.0 Hz, 1H), 7.16-6.99 (m, 2H), 4.58 (d, *J*=12.4 Hz, 2H), 3.90 (s, 3H), 3.50-3.45 (m, 1H), 3.30-3.13 (m, 2H), 2.16-2.13 (m, 2H), 1.73-1.55 (m, 2H). MS-ESI calculated for [M+H]⁺ 454, found 454.

### Example 5

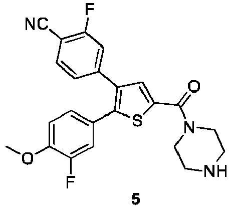

### Synthetic route:

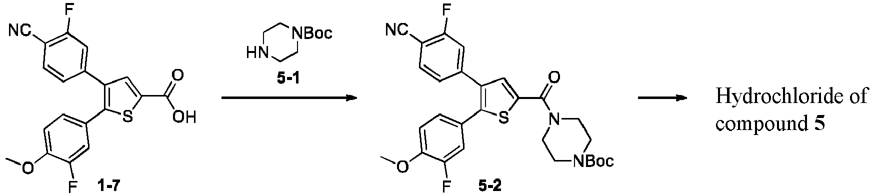

### Step 1

Compound **1-7** (200 mg, 539 µmol), 2-(7-azabenzotriazol-1-yl)-*N*,*N*,*N*',*N*'-tetramethyluronium hexafluorophosphate (307 mg, 808 µmol), and *N*,*N*-diisopropylethylamine (209 mg, 1.62 mmol) were dissolved in *N*,*N*-dimethylformamide (3.00 mL), and then compound **5-1** (100 mg, 539 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.50) to obtain compound **5-2.** MS-ESI calculated for [M-56+H]⁺ 484, found 484.

### Step 2

Compound **5-2** (341 mg, 632 µmol) was dissolved in ethyl acetate (3.00 mL), and hydrochloride ethyl acetate solution (4 mol/L, 3.16 mL) was added thereto, and the reaction mixture was stirred and reacted for 12 hours at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 30% to 60%, 9 minutes) to obtain the hydrochloride of compound **5**. ¹H NMR (400MHz, CD₃OD) δ = 7.70 (t, *J*=7.2 Hz, 1H), 7.61 (s, 1H), 7.35 (d, *J*=10.8 Hz, 1H), 7.26 (d, *J*=8.4 Hz, 1H), 7.16-7.01 (m, 3H), 4.08-4.06 (m, 4H), 3.90 (s, 3H), 3.39-3.33 (m, 4H). MS-ESI calculated for [M+H]⁺ 440, found 440.

### Example 6

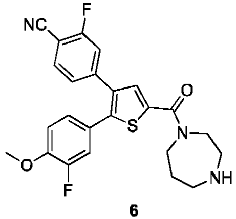

### Synthetic route:

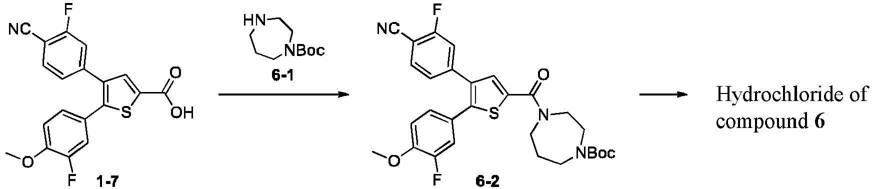

### Step 1

Compound **1-7** (200 mg, 539 µmol), 2-(7-azabenzotriazol-1-yl)-*N*,*N*,*N*',*N*'-tetramethyluronium hexafluorophosphate (307 mg, 808 µmol), and *N*,*N*-diisopropylethylamine (209 mg, 1.62 mmol) were dissolved in *N*,*N*-dimethylformamide (5.00 mL), and then compound **6-1** (108 mg, 539 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.49) to obtain compound **6-2.** MS-ESI calculated for [M-56+H]⁺ 498, found 498.

### Step 2

Compound **6-2** (404 mg, 729.74 µmol) was dissolved in ethyl acetate (3.00 mL), and hydrochloride ethyl acetate solution (4 mol/L, 3.65 mL) was added thereto, and the reaction mixture was stirred and reacted for 12 hours at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 30% to 60%, 9 minutes) to obtain the hydrochloride of compound **6.** ¹H NMR (400MHz, CD₃OD) δ = 7.70 (t, *J*=7.4 Hz, 1H), 7.60 (s, 1H), 7.33 (d, *J*=9.6 Hz, 1H), 7.26 (d, *J*=8.0 Hz, 1H), 7.12-7.02 (m, 3H), 3.10-3.90 (m, 4H), 3.90 (s, 3H), 3.50-3.43 (m, 2H), 3.40-3.36 (m, 2H), 2.28-2.20 (m, 2H). MS-ESI calculated for [M+H]⁺ 454, found 454.

### Example 7

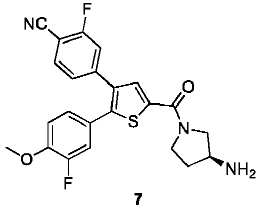

### Synthetic route:

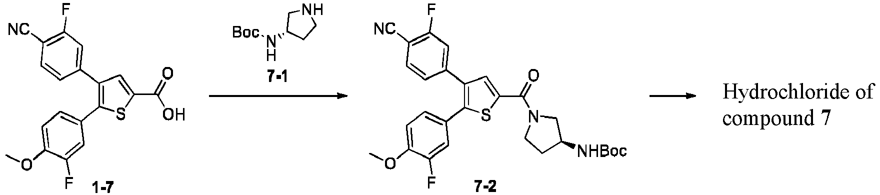

### Step 1

Compound **1-7** (200 mg, 539 µmol), 2-(7-azabenzotriazol-1-yl)-*N*,*N*',*N*'-tetramethyluronium hexafluorophosphate (307 mg, 808 µmol), and *N*,*N*-diisopropylethylamine (209 mg, 1.62 mmol) were dissolved in *N*,*N*-dimethylformamide (5.00 mL), and then compound **7-1** (100 mg, 539 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.57) to obtain compound **7-2.** MS-ESI calculated for [M-56+H]⁺ 484, found 484.

### Step 2

Compound **7-2** (404 mg, 730 µmol) was dissolved in ethyl acetate (3.00 mL), and hydrochloride ethyl acetate solution (4 mol/L, 3.65 mL) was added thereto, and the reaction mixture was stirred and reacted for 12 hours at 20°C. After the reaction mixture was concentrated, the residue was separated by preparative high performance liquid chromatography (chromatographic column: Venusil ASB Phenyl 150 * 30 mm* 5 µm; mobile phase A: hydrochloric acid aqueous solution with a volume fraction of 0.05%; mobile phase B: acetonitrile; B (acetonitrile) %: 30% to 60%, 9 minutes) to obtain the hydrochloride of compound **7.** ¹H NMR (400MHz, CD₃OD) δ = 7.79-7.62 (m, 2H), 7.32 (d, *J*=10.4 Hz, 1H), 7.26 (dd, *J*=1.0, 8.2 Hz, 1H), 7.15-7.00 (m, 3H), 4.15-3.98 (m, 2H), 3.90 (s, 3H), 3.85-3.75 (m, 1H), 2.60-2.40 (m, 1H), 2.35-2.10 (m, 1H). MS-ESI calculated for [M+H]⁺ 440, found 440.

### Example 8

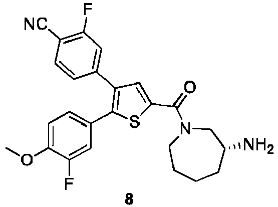

### Synthetic route:

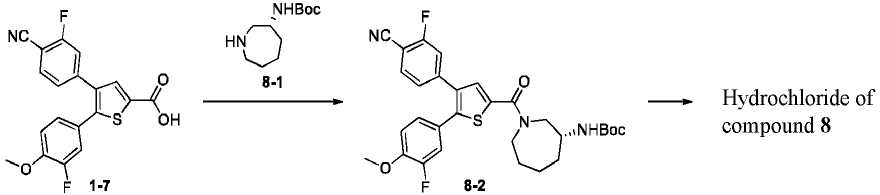

### Step 1

Compound **1-7** (150 mg, 404 µmol), 2-(7-azabenzotriazol-1-yl)-*N*,*N*,*N*',*N*'-tetramethyluronium hexafluorophosphate (230 mg, 606 µmol), and *N*,*N*-diisopropylethylamine (157 mg, 1.21 mmol) were dissolved in *N*,*N*-dimethylformamide (5.00 mL), and then compound **8-1** (86.6 mg, 404 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.49) to obtain compound **8-2.** MS-ESI calculated for [M+H]⁺ 568, found 568.

### Step 2

Compound **8-2** (213 mg, 375 µmol) was dissolved in ethyl acetate (5.00 mL), and hydrochloride ethyl acetate solution (4 mol/L, 1.88 mL) was added thereto, and the reaction mixture was stirred and reacted for 1 hour at 20°C. After the reaction mixture was concentrated, the residue was separated by preparative high performance liquid chromatography (chromatographic column: Venusil ASB Phenyl 150 * 30 mm* 5 µm; mobile phase A: hydrochloric acid aqueous solution with a volume fraction of 0.05%; mobile phase B: acetonitrile; B (acetonitrile) %: 35% to 65%, 9 minutes) to obtain the hydrochloride of compound **8**. ¹H NMR (400MHz, CD₃OD) δ = 7.72 (t, *J*=7.4 Hz, 1H), 7.65 (s, 1H), 7.34 (d, *J*=11.2 Hz, 1H), 7.28 (d, *J*=8.0 Hz, 1H), 7.19-7.03 (m, 3H), 4.22-4.05 (m, 1H), 4.05-3.92 (m, 2H), 3.92 (s, 3H), 3.82-3.70 (m, 1H), 3.67-3.56 (m, 1H), 2.29-2.10 (m, 1H), 2.08-1.90 (m, 2H), 1.90-1.78 (m, 1H), 1.73-1.48 (m, 2H). MS-ESI calculated for [M+H]⁺ 468, found 468.

### Example 9

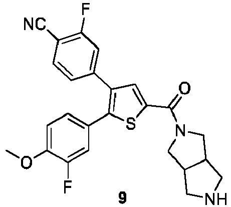

### Synthetic route:

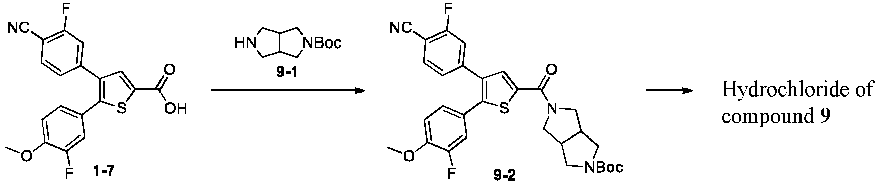

### Step 1

Compound **1-7** (150 mg, 404 µmol), 2-(7-azabenzotriazol-1-yl)-*N*,*N*,*N*',*N*'-tetramethyluronium hexafluorophosphate (230 mg, 606 µmol), and *N*,*N*-diisopropylethylamine (157 mg, 1.21 mmol) were dissolved in *N*,*N*-dimethylformamide (5.00 mL), and then compound **9-1** (85.8 mg, 404 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.49) to obtain compound **9-2.** MS-ESI calculated for [M-56+H]⁺ 510, found 510.

### Step 2

Compound **9-2** (280 mg, 495.02 µmol) was dissolved in ethyl acetate (5 mL), and hydrochloride ethyl acetate solution (4 mol/L, 2.48 mL) was added thereto, and the reaction mixture was stirred and reacted for 1 hour at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 35% to 65%, 9 minutes) to obtain the hydrochloride of compound **9.** ¹H NMR (400MHz, CD₃OD) δ = 7.74-7.65 (m, 2H), 7.33 (d, *J*=10.4 Hz, 1H), 7.25 (d, *J*=1.3, 8.0 Hz, 1H), 7.15-7.00 (m, 3H), 3.90 (s, 3H), 3.62-3.58 (m, 2H), 3.31-3.26 (m, 8H). MS-ESI calculated for [M+H]⁺ 466, found 466.

### Example 10

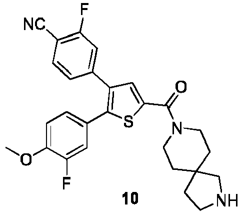

### Synthetic route:

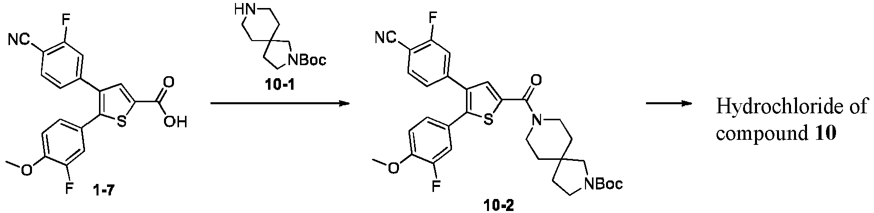

### Step 1

Compound **1-7** (150 mg, 404 µmol), 2-(7-azabenzotriazol-1-yl)-*N*,*N*,*N*',*N*'-tetramethyluronium hexafluorophosphate (230 mg, 606 µmol), and *N*,*N*-diisopropylethylamine (157 mg, 1.21 mmol) were dissolved in *N*,*N*-dimethylformamide (5.00 mL), and then compound **10-1** (97.1 mg, 404 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.49) to obtain compound **10-2.** MS-ESI calculated for [M-56+H]⁺ 538, found 538.

### Step 2

Compound **10-2** (300 mg, 505 µmol) was dissolved in ethyl acetate (5 mL), and hydrochloride ethyl acetate solution (4 mol/L, 2.53 mL) was added thereto, and the reaction mixture was stirred and reacted for 1 hour at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 35% to 65%, 9 minutes) to obtain the hydrochloride of compound **10.** ¹H NMR (400MHz, CD₃OD) δ = 7.51 (s, 1H), 7.31 (d, *J*=10.4 Hz, 1H), 7.25 (d, *J*=8.0 Hz, 1H), 7.16-6.97 (m, 3H), 3.96-3.90 (m, 2H), 3.90 (s, 3H), 3.85-3.67 (m, 2H), 3.43 (t, *J*=7.2 Hz, 2H), 3.25-3.15 (m, 2H), 2.05 (t, *J*=7.6 Hz, 2H), 1.84-1.67 (m, 4H). MS-ESI calculated for [M+H]⁺ 494, found 494.

### Example 11

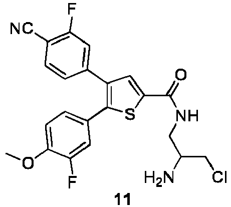

### Synthetic route:

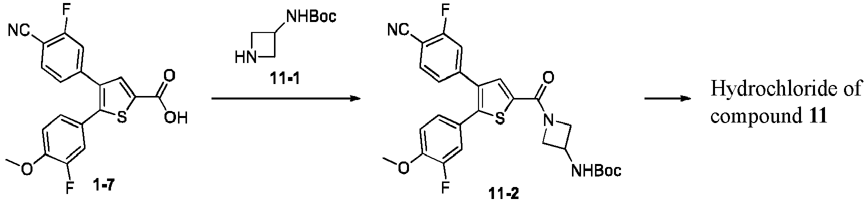

### Step 1

Compound **1-7** (150 mg, 404 µmol), 2-(7-azabenzotriazol-1-yl)-*N*,*N*,*N'*,*N'-*tetramethyluronium hexafluorophosphate (230 mg, 606 µmol), and N,N-diisopropylethylamine (156.61 mg, 1.21 mmol) were dissolved in *N,N*-dimethylformamide (5 mL), and then compound **11-1** (69.57 mg, 403.92 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.49) to obtain compound **11-2.** Compound **11-2** MS-ESI calculated for [M+H]⁺ 526, found 526.

### Step 2

Compound **11-2** (300 mg, 571 µmol) was dissolved in ethyl acetate (5.00 mL), and hydrochloride ethyl acetate solution (4 mol/L, 2.85 mL) was added thereto, and the reaction mixture was stirred and reacted for 1 hour at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 35% to 65%, 9 minutes) to obtain the hydrochloride of compound **11.** ¹H NMR (400MHz, CD₃OD) δ = 7.86 (s, 1H), 7.72 (t, *J*=7.4 Hz, 1H), 7.35-7.22 (m, 2H), 7.17-7.01 (m, 3H), 3.98-3.93 (m, 1H), 3.90 (s, 3H), 3.89-3.77 (m, 2H), 3.77-3.66 (m, 2H). MS-ESI calculated for [M+H]⁺ 462, found 462.

### Example 12

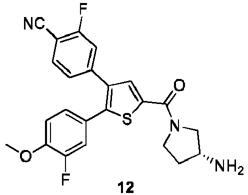

### Synthetic route:

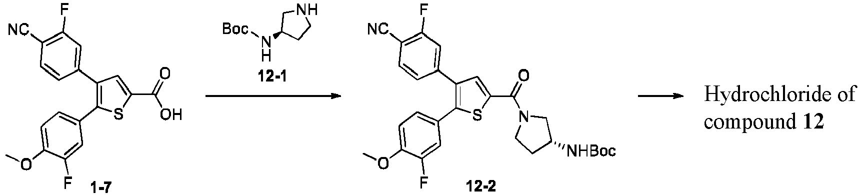

### Step 1

Compound **1-7** (200 mg, 539 µmol), 2-(7-azabenzotriazol-1-yl)-*N,N,N',N'-*tetramethyluronium hexafluorophosphate (307.17 mg, 808 µmol), and *N,N-*diisopropylethylamine (209 mg, 1.62 mmol) were dissolved in N,N-dimethylformamide (5.00 mL), and then compound **12-1** (100 mg, 539 µmol) was added thereto, and the reaction mixture was stirred and reacted for 12 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.57) to obtain compound **12-2.** MS-ESI calculated for [M-56+H]⁺ 484, found 484.

### Step 2

Compound **12-2** (300 mg, 556 µmol) was dissolved in ethyl acetate (10.0 mL), and hydrochloride ethyl acetate solution (4 mol/L, 10.0 mL) was added thereto, and the reaction mixture was stirred and reacted for 12 hours at 20°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 30% to 60%, 9 minutes) to obtain the hydrochloride of compound **12.** ¹H NMR (400MHz, CD₃OD) δ = 7.77-7.66 (m, 2H), 7.32 (dd, *J*=1.2, 10.0 Hz, 1H), 7.26 (dd, *J*=1.2, 8.0 Hz, 1H), 7.16-7.02 (m, 3H), 4.40-3.92 (m, 4H), 3.90 (s, 3H), 3.85-3.81 (m, 1H), 2.60-2.40 (m, 1H), 2.35-2.10 (m, 1H). MS-ESI calculated for [M+H]⁺ 440, found 440.

### Example 13

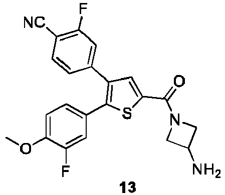

### Synthetic route:

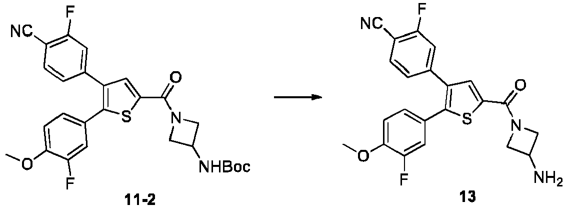

Compound **11-2** (270 mg, 514 µmol) was dissolved in dichloromethane (10.0 mL). Trimethylsilyl trifluoromethanesulfonate (120 mg, 539 µmol) was added thereto, and the reaction mixture was stirred and reacted at 20°C for 12 hours. After the reaction mixture was concentrated, the residue was separated by preparative high performance liquid chromatography (chromatographic column: Phenomenex Gemini-NX 80 * 30 mm * 3 µm; mobile phase A: 10 mM ammonium bicarbonate aqueous solution; mobile phase B: acetonitrile B%: 42% to 72%, 9 minutes) to obtain compound **13.** Compound **13** ¹H NMR (400MHz, CD₃OD) δ = 7.69 (t, *J*=8.0 Hz, 1H), 7.59 (s, 1H), 7.29 (d, *J*=10.0 Hz, 1H), 7.23 (dd, *J*=1.4, 8.2 Hz, 1H), 7.14-6.98 (m, 3H), 4.79 (t, *J*=8.4 Hz, 1H), 4.69-4.54 (m, 1H), 4.46-4.34 (m, 1H), 4.30-4.18 (m, 1H), 3.98-3.92 (m, 1H), 3.89 (s, 3H). MS-ESI calculated for [M+H]⁺ 426, found 426.

### Example 14

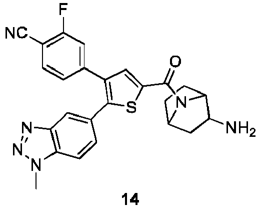

### Synthetic route:

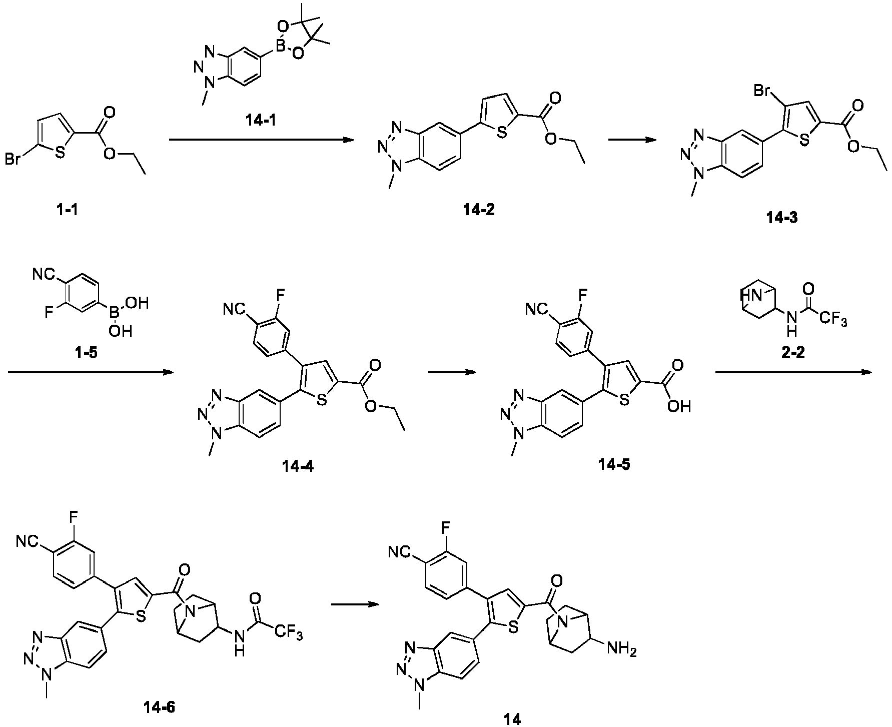

### Step 1

Under nitrogen atmosphere, compound **1-1** (992 mg, 4.22 mmol) and compound **14-1** (729 mg, 2.81 mmol) were dissolved in dioxane (10.0 mL) and water (2.00 mL), and then potassium carbonate (778 mg, 5.63 mmol) and tetrakis(triphenylphosphine)palladium (325 mg, 281 µmol) were added thereto. The reaction mixture was stirred and reacted for 12 hours at 80°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.3) to obtain compound **14-2.** MS-ESI calculated for [M+H]⁺ 288, found 288.

### Step 2

Compound **14-2** (500 mg, 1.74 mmol) was dissolved in *N*,*N*-dimethylformamide (5.00 mL). *N*-Bromosuccinimide (619 mg, 3.48 mmol) was added thereto, and the reaction mixture was stirred and reacted for 12 hours at 25°C. The reaction mixture was concentrated under reduced pressure, and the residue was purified by silica gel column chromatography (1/1, petroleum ether/ethyl acetate, R_{f} = 0.55) to obtain compound **14-3.** MS-ESI calculated for [M+H]⁺ 366, found 366.

### Step 3

Under nitrogen atmosphere, compound **14-3** (730 mg, 1.99 mmol) and compound **1-5** (329 mg, 1.99 mmol) were dissolved in ethanol (10.0 mL) and water (2.00 mL), and then sodium carbonate (423 mg, 3.99 mmol) and bis(di-tert-butyl(4-dimethylaminophenyl)phosphine)dichloropalladium(II) (72.9 mg, 99.7 µmol) were added thereto. The reaction mixture was stirred and reacted for 12 hours at 80°C. After the reaction mixture was concentrated to remove the ethanol, the reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f}= 0.2) to obtain compound **14-4.** ¹H NMR (400MHz, CD₃Cl) δ = 8.08 (s, 1H), 7.89-7.78 (m, 1H), 7.63-7.42 (m, 2H), 7.33 (dd, *J*=1.4, 8.6 Hz, 1H), 7.22-7.01 (m, 2H), 4.47-4.38 (m, 2H), 4.34 (s, 3H), 1.46-1.39 (m, 3H). MS-ESI calculated for [M+H]⁺ 407, found 407.

### Step 4

Compound **14-4** (271 mg, 667 µmol) was dissolved in tetrahydrofuran (3.00 mL) and water (3.00 mL), and lithium hydroxide monohydrate (83.9 mg, 2.00 mmol) was added thereto, and the reaction mixture was stirred and reacted for 12 hours at 20°C. After the reaction mixture was concentrated to remove the tetrahydrofuran, the residue was added with water (50 mL). The mixture was acidified to pH = 6 with 1 M dilute hydrochloric acid, and extracted with ethyl acetate (50 mL × 3). The organic phases were combined, washed with saturated brine (10 mL), dried over anhydrous sodium sulfate, filtered, and concentrated to obtain crude compound **14-5.** MS-ESI calculated for [M-H]⁺ 379, found 379.

### Step 5

Compound **14-5** (200 mg, 529 µmol) was dissolved in *N*,*N*-dimethylformamide (5.00 mL). 2-(7-Azabenzotriazol-1-yl)-*N,N,N',N'*-tetramethyluronium hexafluorophosphate (301 mg, 793 µmol) and N,N-diisopropylethylamine (342 mg, 2.64 mmol) were added thereto, and then compound **2-2** (93.6 mg, 450 µmol) was added thereto, and the reaction mixture was stirred and reacted for 4 hours at 20°C. The reaction mixture was added with water (50 mL), and extracted with ethyl acetate (50 mL × 2). The organic phase was washed with saturated brine (10 mL), dried over anhydrous sodium sulfate, filtered, and concentrated under reduced pressure. The residue was purified by silica gel column chromatography (1/1, petroleum ether/ethyl acetate, R_{f} = 0.51) to obtain compound **14-6.** MS-ESI calculated for [M+H]⁺ 569, found 569.

### Step 6

Compound **14-6** (160 mg, 281 µmol) was dissolved in tetrahydrofuran (10.0 mL) and water (5.00 mL), then sodium hydroxide (33.8 mg, 844 µmol) was added thereto, and the reaction mixture was stirred and reacted for 12 hours at 50°C. The reaction mixture was added with water (50 mL), and extracted with ethyl acetate (50 mL × 3). The organic phase was washed with saturated brine (10 mL), dried over anhydrous sodium sulfate, filtered, and concentrated under reduced pressure. The residue was purified by preparative high performance liquid chromatography (chromatographic column: Phenomenex Gemini-NX 80 * 30 mm * 3 µm; mobile phase A: 10 mM ammonium bicarbonate aqueous solution; mobile phase B: acetonitrile B%: 33% to 63%, 9 minutes) to obtain compound **14.** ¹H NMR (400MHz, CDCl₃) δ = 8.06 (s, 1H), 7.55 (s, 1H), 7.54-7.47 (m, 2H), 7.33 (dd, *J*=1.6, 8.8 Hz, 1H), 7.16-7.06 (m, 2H), 4.74-4.64 (m, 1H), 4.55 (s, 1H), 4.34 (s, 3H), 3.71-3.61 (m, 1H), 2.47-2.38 (m, 1H), 2.31-2.19 (m, 1H), 1.99-1.92 (m, 1H), 1.87-1.81 (m, 1H), 1.29-1.24 (m, 1H), 1.02-0.99 (m, 1H). MS-ESI calculated for [M+H]⁺ 473, found 473.

### Example 15

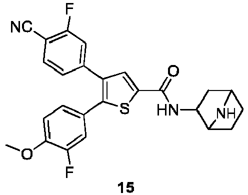

### Synthetic route:

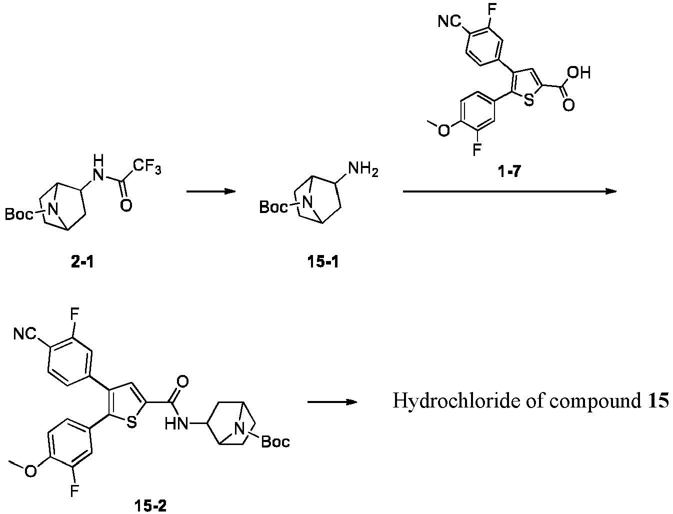

### Step 1

Compound **2-1** (100 mg, 324 µmol) was dissolved in tetrahydrofuran (5.00 mL) and water (1.00 mL), then sodium hydroxide (26.0 mg, 650 µmol) was added thereto, and the reaction mixture was stirred and reacted for 12 hours at 50°C. The reaction mixture was added with water (10 mL) and extracted with ethyl acetate (20 mL × 2). The organic phase was washed with saturated brine (30 mL), dried over anhydrous sodium sulfate, filtered, and concentrated under reduced pressure to obtain compound **15-1.** MS-ESI calculated for [M+H]⁺ 213, found 213.

### Step 2

Compound **1-7** (70.0 mg, 188 µmol) was dissolved in anhydrous *N,N-*dimethylformamide (3.00 mL). Compound **15-1** (44.0 mg, 207 µmol), 2-(7-azabenzotriazol-1-yl)-*N,N,N',N'*-tetramethyluronium hexafluorophosphate (108 mg, 283 µmol), and *N,N-*diisopropylethylamine (97.5 mg, 754 µmol) were added thereto, and the reaction mixture was stirred for 12 hours at 25°C. The reaction mixture was added with water (30 mL) and filtered, and the filter cake was washed with water (20 mL) and dried under vacuum to obtain compound **15-2.** MS-ESI calculated for [M+Na]⁺ 588, found 588.

### Step 3

Compound **15-2** (100 mg, 177 µmol) was dissolved in ethyl acetate (5.00 mL), and hydrochloride ethyl acetate solution (4 mol/L, 5 mL) was added thereto. The reaction mixture was stirred and reacted for 2 hours at 25°C. The reaction mixture was concentrated under reduced pressure, and the residue was purified by preparative high performance liquid chromatography (Phenomenex Gemini-NX C18 75 * 30 mm*3 µm; mobile phase A: hydrochloric acid aqueous solution with a volume fraction of 0.05%; mobile phase B: acetonitrile; B%: 29% to 49%, 7 minutes) to obtain the hydrochloride of compound **15.** ¹H NMR (400MHz, CD₃OD) δ = 8.01 (s, 1H), 7.76 - 7.74 (m, 1H), 7.33-7.27 (m, 2H), 7.14 - 7.06 (m, 3H), 4.57 - 4.52 (m, 1H), 4.46 (t, *J*=4.4 Hz, 1H), 4.27 (t, *J*=4.8 Hz, 1H), 3.92 (s, 3H), 2.51-2.43 (m, 1H), 2.18-2.14 (m, 1H), 1.99-1.94 (m, 3H), 1.86-1.81 (m, 1H). MS-ESI calculated for [M+H]⁺ 466, found 466.

### Example 16

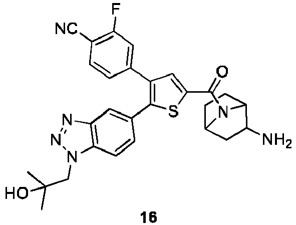

### Synthetic route:

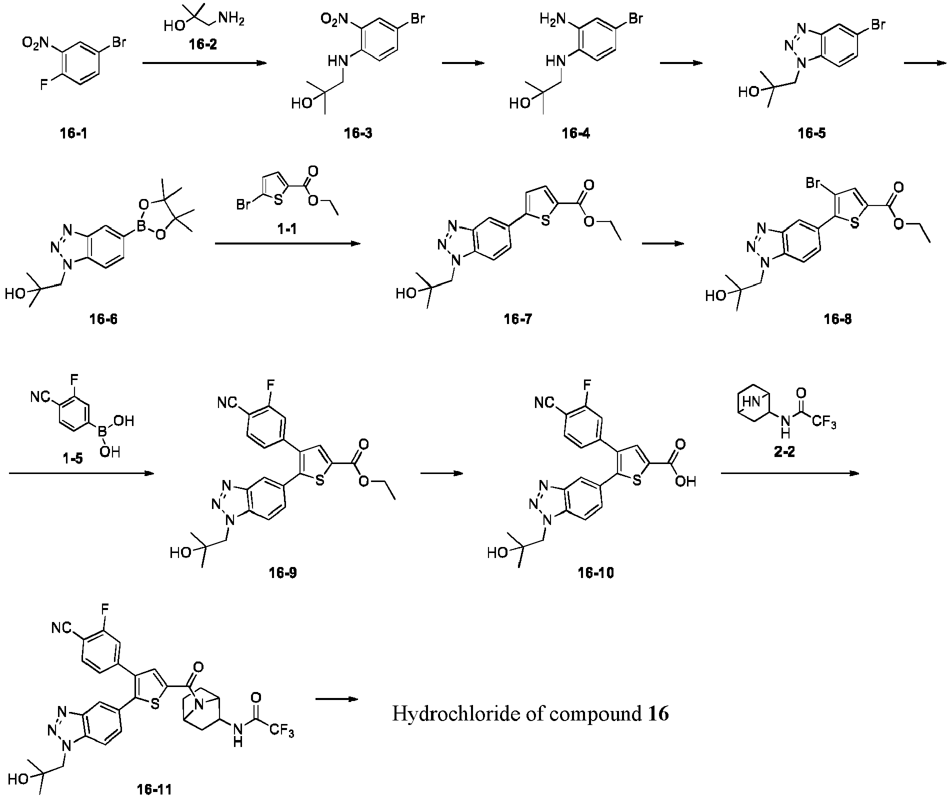

### Step 1

Compound **16-1** (10.0 g, 45.5 mmol) was dissolved in *N,N*-dimethylformamide (120 mL), then **16-2** (4.86 g, 54.6 mmol) and *N,N*-diisopropylethylamine (11.8 g, 90.9 mmol) were added thereto, and the reaction mixture was stirred and reacted for 16 hours at 80°C. To the stirred water (600 mL) was added the reaction mixture. The resulting suspension was filtered, and the filter cake was washed with water (100 mL) and dried under vacuum to obtain compound **16-3.** ¹H NMR (400MHz, CDCl₃) δ = 8.28 (s, 1H), 8.24 (d, *J*=2.8 Hz, 1H), 7.40 (dd, *J*=2.4, 9.2 Hz, 1H), 6.76 (d, *J*=9.2 Hz, 1H), 3.22 (d, *J*=5.6 Hz, 2H), 1.30 (s, 6H). MS-ESI calculated for [M+H]⁺ 289, found 289.

### Step 2

Compound **16-3** (3.00 g, 10.4 mmol) was dissolved in concentrated hydrochloric acid (30 mL), then tin dichloride dihydrate (14.1 g, 62.3 mmol) was added thereto, and the reaction mixture was stirred and reacted for 0.5 hours at 25°C. The pH of the reaction mixture was adjusted to 13 by adding sodium hydroxide (1 mol/L aqueous solution). The resulting suspension was filtered, and the collected filter cake was washed with water (50 mL) and dried under vacuum. The dried filter cake was purified by silica gel column chromatography (2/1, petroleum ether/ethyl acetate, R_{f} = 0.3) to obtain compound **16-4.** ¹H NMR (400MHz, CDCl₃) δ = 6.82-6.80 (m, 1H), 6.76 (d, *J*=2.4 Hz, 1H), 6.46 (d, *J*=8.4 Hz, 1H), 3.41-3.28 (m, 3H), 2.95 (s, 2H), 1.26 (s, 6H). MS-ESI calculated for [M+H]⁺ 261, found 261.

### Step 3

Compound **16-4** (1.41 g, 5.44 mmol) was dissolved in acetic acid (20 mL), and a solution of sodium nitrite (564 mg, 8.17 mmol) in water (10 mL) was added dropwise thereto, and the reaction mixture was stirred for 5 hours at 25°C. The reaction mixture was added with water (30 mL), and extracted with ethyl acetate (50 mL × 2). The organic phase was sequentially washed with saturated sodium bicarbonate aqueous solution (100 mL) and saturated brine (100 mL), dried over anhydrous sodium sulfate, filtered, and concentrated under reduced pressure, and the residue was purified by silica gel column chromatography (1/1, petroleum ether/ethyl acetate, R_{f} = 0.30) to obtain compound **16-5.** ¹H NMR (400MHz, CDCl₃) δ = 8.11-8.10 (m, 1H), 7.50-7.49 (m, 2H), 4.52 (s, 2H), 1.25 (s, 6H). MS-ESI calculated for [M+H]⁺ 272, found 272.

### Step 4

Under nitrogen atmosphere, compound **16-5** (1.66 g, 5.77 mmol) was dissolved in 1,4-dioxane (30 mL), and then bis(pinacolato)diboron (1.61 g, 6.35 mmol), potassium acetate (1.14 g, 11.6 mmol), and [1,1'-bis(diphenylphosphino)ferrocene]dichloropalladium(II) (427 mg, 583 µmol) were added thereto. The reaction mixture was stirred for 12 hours at 90°C. The reaction mixture was filtered through diatomite, and the filtrate was concentrated under reduced pressure, and the residue was purified by silica gel column chromatography (1/1, petroleum ether/ethyl acetate, R_{f} = 0.50) to obtain compound **16-6.** ¹H NMR (400MHz, CDCl₃) δ = 8.50 (s, 1H), 7.84 (d, *J*=8.0 Hz, 1H), 7.56 (d, *J*=8.4 Hz, 1H), 4.55 (s, 2H), 1.30 (s, 12H), 1.23 (s, 6H).MS-ESI calculated for [M+H]⁺ 318, found 318.

### Step 5

Under nitrogen atmosphere, compound **1-1** (900 mg, 3.83 mmol) was dissolved in 1,4-dioxane (20 mL) and water (4 mL), and then **16-6** (1.28 g, 4.04 mmol), potassium phosphate (1.63 g, 7.66 mmol), and 1,1'-bis (di-tert-butylphosphino)ferrocene dichloropalladium(II) (250 mg, 383 µmol) were added thereto. The reaction mixture was stirred for 12 hours at 90°C. The reaction mixture was filtered through diatomite, and the filtrate was concentrated under reduced pressure, and the residue was purified by silica gel column chromatography (1/1, petroleum ether/ethyl acetate, R_{f} = 0.20) to obtain compound **16-7.** MS-ESI calculated for [M+H]⁺ 346, found 346.

### Step 6

Compound **16-7** (487 mg, 1.41 mmol) and *N*-bromosuccinimide (500 mg, 2.81 mmol) were dissolved in acetonitrile (10.0 mL), and the reaction mixture was stirred for 24 hours at 80°C. The reaction mixture was added with ethyl acetate (20 mL), and sequentially washed with water (20 mL × 1) and saturated brine (20 mL × 1). The organic phase was dried over anhydrous sodium sulfate, filtered, and the filtrate was concentrated under reduced pressure, and the residue was purified by silica gel column chromatography (1/1, petroleum ether/ethyl acetate, R_{f} = 0.50) to obtain compound **16-8.** ¹H NMR (400MHz, CDCl₃) δ = 8.39-8.38 (m, 1H), 7.80-7.77 (m, 3H), 4.67 (s, 2H), 4.43-4.38 (m, 2H), 1.44-1.40 (m, 3H), 1.37 (s, 6H). MS-ESI calculated for [M+H]⁺ 426, found 426.

### Step 7

Under nitrogen atmosphere, compound **16-8** (730 mg, 1.72 mmol) was dissolved in 1,4-dioxane (10.0 mL) and water (2.00 mL), and then compound **1-5** (300 mg, 1.82 mmol), potassium phosphate (730 mg, 3.44 mmol), and 1,1'-bis (di-tert-butylphosphino)ferrocene dichloropalladium(II) (112 mg, 172 µmol) were added thereto. The reaction mixture was stirred for 12 hours at 90°C. The reaction mixture was filtered through diatomite, and the filtrate was concentrated under reduced pressure, and the residue was purified by silica gel column chromatography (1/1, petroleum ether/ethyl acetate, R_{f} = 0.43) to obtain compound **16-9.** ¹H NMR (400MHz, CDCl₃) δ = 8.06 (s, 1H), 7.87 (s, 1H), 7.67-7.65 (m, 1H), 7.54-7.52 (m, 1H), 7.35-7.33 (m, 1H), 7.17-7.11 (m, 1H), 4.64 (s, 2H), 4.45-4.40 (m, 2H), 1.45-1.42 (m, 3H), 1.37 (s, 6H).MS-ESI calculated for [M+H]⁺ 465, found 465.

### Step 8

Compound **16-9** (150 mg, 296 µmol) was dissolved in tetrahydrofuran (5.00 mL) and water (1.00 mL), then lithium hydroxide monohydrate (26.0 mg, 619 µmol) was added thereto, and the reaction mixture was stirred and reacted for 12 hours at 28°C. The pH of the reaction mixture was adjusted to 4 by adding dilute hydrochloric acid (1 N), and the mixture was extracted with ethyl acetate (30 mL × 2). The organic phase was washed with saturated brine (50 mL), dried over anhydrous sodium sulfate, filtered, and concentrated under reduced pressure to obtain compound **16-10.** MS-ESI calculated for [M+H]⁺ 437, found 437.

### Step 9

Compound **16-10** (188 mg, 431 µmol) was dissolved in anhydrous *N,N-*dimethylformamide (6.00 mL), and then compound **2-2** (107 mg, 437 µmol), 2-(7-azabenzotriazol-1-yl)-*N,N,N',N'*-tetramethyluronium hexafluorophosphate (246 mg, 646 µmol), and *N,N*-diisopropylethylamine (223 mg, 1.72 mmol) were added thereto, and the reaction mixture was stirred for 12 hours at 28°C. To water (30 mL) was added the reaction mixture. The resulting suspension was filtered, and the collected filter cake was washed with water (20 mL) and dried under vacuum to obtain compound **16-11.** MS-ESI calculated for [M+Na]⁺ 627, found 627.

### Step 10

Compound **16-11** (83.0 mg, 132 µmol) was dissolved in methanol (3.00 mL) and water (3.00 mL), then potassium carbonate (36.6 mg, 265 µmol) was added thereto, and the reaction mixture was stirred for 12 hours at 28°C. The reaction mixture was extracted with ethyl acetate (30 mL × 2), and the organic phase was washed with saturated brine (50 mL), dried over anhydrous sodium sulfate, filtered, and concentrated under reduced pressure. The residue was purified by preparative high performance liquid chromatography (Phenomenex Luna C18 75 * 30 mm* 3 µm; mobile phase A: hydrochloric acid aqueous solution with a volume fraction of 0.05%; mobile phase B: acetonitrile; B%: 18% to 38%, 6.5 minutes) to obtain the hydrochloride of compound **16.** ¹H NMR (400MHz, CD₃OD) δ = 7.97 (s, 1H), 7.87-7.81 (m, 2H), 7.67-7.64 (m, 1H), 7.42-7.34 (m, 2H), 7.28-7.19 (m, 1H), 4.96-4.93 (m, 2H), 4.69 (s, 2H), 3.89-3.87 (m, 1H), 2.56-2.52 (m, 1H), 2.06-1.97 (m, 3H), 1.85-1.83 (m, 1H), 1.56-1.54 (m, 1H), 1.29 (s, 6H).MS-ESI calculated for [M+H]⁺ 531, found 531.

### Example 17

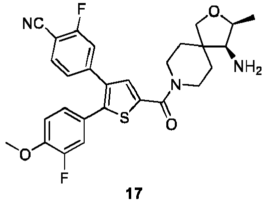

### Synthetic route:

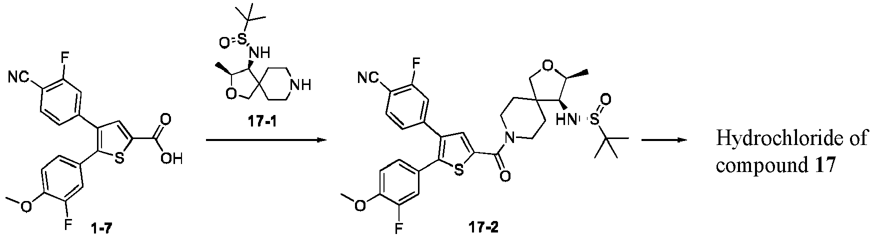

### Step 1

Compound **1-7** (217 mg, 583 µmol), 2-(7-azabenzotriazol-1-yl)-*N,N,N',N'-*tetramethyluronium hexafluorophosphate (332 mg, 875 µmol), and *N,N*-diisopropylethylamine (226 mg, 1.75 mmol) were dissolved in *N,N*-dimethylformamide (5.00 mL), and then compound **17-1** (160 mg, 583 µmol) was added thereto, and the reaction mixture was stirred and reacted for 12 hours at 25°C. The reaction mixture was added with water (100 mL), and extracted with ethyl acetate (20 mL × 3). The organic phase was washed with saturated brine (100 mL), dried over anhydrous sodium sulfate, filtered, and concentrated under reduced pressure. The residue was purified by silica gel column chromatography (1/1, petroleum ether/ethyl acetate, R_{f} = 0.14) to obtain compound **17-2.** MS-ESI calculated for [M+H]⁺ 628, found 628.

### Step 2

Compound **17-2** (250 mg, 398 µmol) was dissolved in ethyl acetate (3.00 mL), and hydrochloride ethyl acetate solution (4 mol/L, 5.00 mL) was added thereto, and the reaction mixture was stirred and reacted for 5 hours at 25°C. After the reaction mixture was concentrated, the residue was separated by preparative high performance liquid chromatography (chromatographic column: Venusil ASB Phenyl 150 * 30 mm * 5 µm; mobile phase A: hydrochloric acid aqueous solution with a volume fraction of 0.05%; mobile phase B: acetonitrile B%: 35% to 65%, 9 minutes) to obtain the hydrochloride of compound **17.** ¹H NMR (400MHz, CD₃OD) δ = 7.70 (t, *J*=7.6 Hz, 1H), 7.52 (s, 1H), 7.32 (d, *J*=10.4 Hz, 1H), 7.25 (d, *J*=8.4 Hz, 1H), 7.15 - 7.03 (m, 3H), 4.90 - 4.89(m, 1H), 4.39 - 4.28 (m, 3H), 4.01 - 3.99 (m, 1H), 3.90 - 3.83 (m, 4H), 3.47 - 3.46 (m, 1H), 1.97 - 1.66 (m, 4H), 1.32 (d, *J*=6.6 Hz, 3H). MS-ESI calculated for [M+H]⁺ 524, found 524.

### Example 18

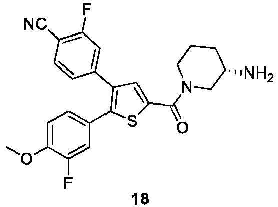

### Synthetic route:

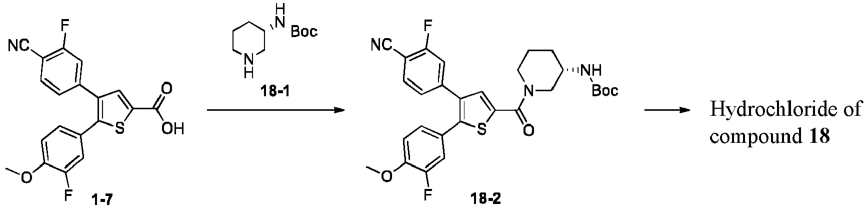

### Step 1

Compound **1-7** (200 mg, 539 µmol), 2-(7-azabenzotriazol-1-yl)-*N,N,N',N'-*tetramethyluronium hexafluorophosphate (307 mg, 808 µmol), and *N,N*-diisopropylethylamine (209 mg, 1.62 mmol) were dissolved in *N,N*-dimethylformamide (3.00 mL), and then compound **18-1** (108 mg, 539 µmol) was added thereto, and the reaction mixture was stirred and reacted for 12 hours at 25°C. The reaction mixture was added with water (50 mL), and extracted with ethyl acetate (20 mL × 3). The organic phase was washed with saturated brine (50 mL), dried over anhydrous sodium sulfate, filtered, and concentrated under reduced pressure. The residue was purified by silica gel column chromatography (1/1, petroleum ether/ethyl acetate, R_{f} = 0.41) to obtain compound **18-2.** MS-ESI calculated for [M-56+H]⁺ 498, found 498.

### Step 2

Compound **18-2** (265 mg, 479 µmol) was dissolved in ethyl acetate (5 mL), and hydrochloride ethyl acetate solution (4 mol/L, 5.00 mL) was added thereto, and the reaction mixture was stirred and reacted for 2 hours at 25°C. After the reaction mixture was concentrated, the residue was separated by preparative high performance liquid chromatography (chromatographic column: Phenomenex Synergi C18 150 * 30 mm * 4 µm; mobile phase A: hydrochloric acid aqueous solution with a volume fraction of 0.05%; mobile phase B: acetonitrile B%: 34% to 64%, 9 minutes) to obtain the hydrochloride of compound **18.** ¹H NMR (400MHz, CD₃OD) δ = 7.69 (t, *J*=7.4 Hz, 1H), 7.56 (s, 1H), 7.33 (d, *J*=10.4 Hz, 1H), 7.26 (d, *J*=8.0 Hz, 1H), 7.15 - 7.01 (m, 3H), 4.46 - 4.32 (m, 1H), 4.21 - 4.09 (m, 1H), 3.90 (s, 3H), 3.59 - 3.36 (m, 3H), 2.25 - 2.15 (m, 1H), 1.97 - 1.64 (m, 3H). MS-ESI calculated for [M+H]⁺ 454, found 454.

### Example 19

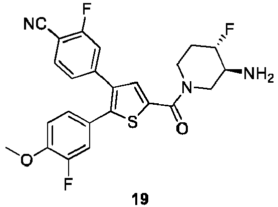

### Synthetic route:

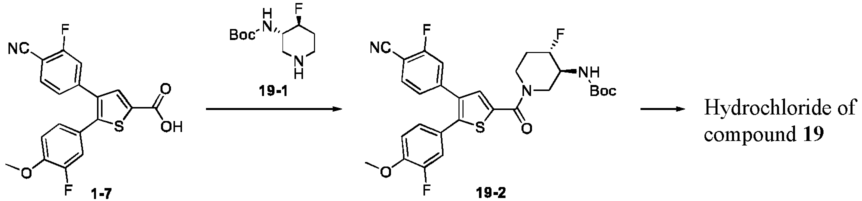

### Step 1

Compound **1-7** (180 mg, 485 µmol), 2-(7-azabenzotriazol-1-yl)-*N,N,N',N'-*tetramethyluronium hexafluorophosphate (276 mg, 727 µmol), and *N,N*-diisopropylethylamine (188 mg, 1.45 mmol) were dissolved in *N,N*-dimethylformamide (3.00 mL), and then compound **19-1** (116 mg, 533 µmol) was added thereto, and the reaction mixture was stirred and reacted for 12 hours at 25°C. The reaction mixture was added with water (50 mL), and extracted with ethyl acetate (20 mL × 3). The organic phase was washed with saturated brine (50 mL), dried over anhydrous sodium sulfate, filtered, and concentrated under reduced pressure. The residue was purified by silica gel column chromatography (1/1, petroleum ether/ethyl acetate, R_{f} = 0.50) to obtain compound **19-2.** MS-ESI calculated for [M-56+H]⁺ 516, found 516.

### Step 2

Compound **19-2** (180 mg, 315 µmol) was dissolved in ethyl acetate (10.0 mL), and hydrochloride ethyl acetate solution (4 mol/L, 4.50 mL) was added thereto, and the reaction mixture was stirred and reacted for 6 hours at 25°C. After the reaction mixture was concentrated, the residue was separated by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile B%: 16% to 46%, 10 minutes) to obtain the hydrochloride of compound **19**. ¹H NMR (400MHz, CD₃OD) δ = 7.73 - 7.66 (m, 1H), 7.61 (s, 1H), 7.35 (d, *J*=10.4 Hz, 1H), 7.27 (d, *J*=8.0 Hz, 1H), 7.15 - 7.02 (m, 3H), 4.83 - 4.68 (m, 2H), 4.52 - 4.47 (m, 1H), 3.90 (s, 3H), 3.61 - 3.45 (m, 1H), 3.43 - 3.34 (m, 1H), 3.29 - 3.21 (m, 1H), 2.38 - 2.26 (m, 1H), 2.00 - 1.82 (m, 1H). MS-ESI calculated for [M+H]⁺ 472, found 472.

### Example 20

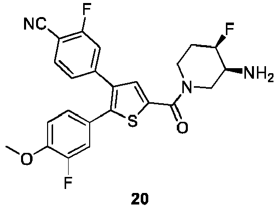

### Synthetic route:

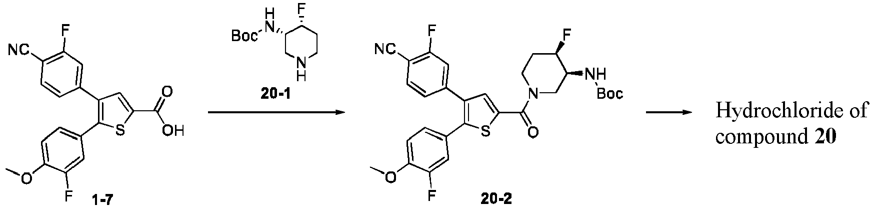

### Step 1

Compound **1-7** (180 mg, 485 µmol), 2-(7-azabenzotriazol-1-yl)-*N,N,N',N'-*tetramethyluronium hexafluorophosphate (276 mg, 727 µmol), and N,N-diisopropylethylamine (188 mg, 1.45 mmol) were dissolved in *N,N-*dimethylformamide (3.00 mL), and then compound **20-1** (116 mg, 533 µmol) was added thereto, and the reaction mixture was stirred and reacted for 12 hours at 25°C. The reaction mixture was added with water (50 mL), and extracted with ethyl acetate (20 mL × 3). The organic phase was washed with saturated brine (50 mL), dried over anhydrous sodium sulfate, filtered, and concentrated under reduced pressure. The residue was purified by silica gel column chromatography (1/1, petroleum ether/ethyl acetate, R_{f} = 0.55) to obtain compound **20-2.** MS-ESI calculated for [M-56+H]⁺ 516, found 516.

### Step 2

Compound **20-2** (140 mg, 245 µmol) was dissolved in ethyl acetate (10.0 mL), and hydrochloride ethyl acetate solution (4 mol/L, 3.50 mL) was added thereto, and the reaction mixture was stirred and reacted for 6 hours at 25°C. After the reaction mixture was concentrated, the residue was separated by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile B%: 16% to 46%, 10 minutes) to obtain the hydrochloride of compound **20.** ¹H NMR (400MHz, CD₃OD) δ = 7.72 - 7.68 (m, 1H), 7.60 (s, 1H), 7.34 (d, *J*=10.4 Hz, 1H), 7.26 (d, *J*=8.0 Hz, 1H), 7.15 - 7.03 (m, 3H), 5.27 - 5.06 (m, 1H), 4.49 - 4.44 (m, 1H), 4.26 - 4.22 (m, 1H), 3.90 (s, 3H), 3.78 - 3.53 (m, 3H), 2.28 - 1.96 (m, 2H). MS-ESI calculated for [M+H]⁺ 472, found 472.

### Example 21

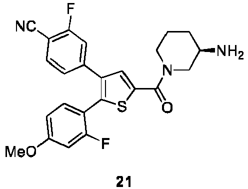

### Synthetic route:

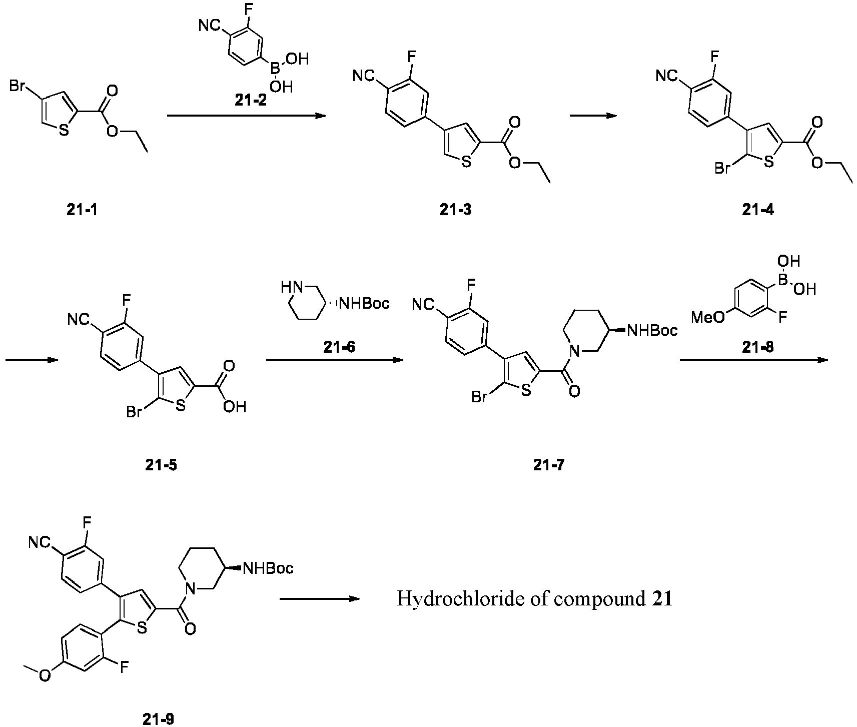

### Step 1

Under nitrogen atmosphere, compound **21-1** (15.0 g, 63.8 mmol), compound **21-2** (12.6 g, 76.6 mmol), and sodium carbonate (13.5 g, 128 mmol) were dissolved in dioxane (120 mL) and water (30 mL), and the reaction mixture was added with [1,1'-bis(diphenylphosphino)ferrocene]dichloropalladium(II) (2.33 g, 3.19 mmol), stirred, and reacted for 12 hours at 100°C. The reaction mixture was concentrated under reduced pressure, and the residue was purified by silica gel column chromatography (4/1, petroleum ether/ethyl acetate, R_{f} = 0.53) to obtain compound **21-3.** ¹H NMR (400MHz, CD₃OD) δ = 8.23 (dd, *J*=1.6, 6.4 Hz, 2H), 7.83 - 7.69 (m, 3H), 4.38 (q, *J*=7.2 Hz, 2H), 1.39 (t, *J*=7.2 Hz, 3H).

### Step 2

Compound **21-3** (10.0 g, 3.21 mmol) was dissolved in acetic acid (120 mL) and sulfuric acid (50.0 mL). N-Bromosuccinimide (12.9 g, 72.6 mmol) was added thereto, and the reaction mixture was stirred and reacted for 12 hours at 25°C. The reaction mixture was poured into water, filtered, and the filter cake was dried, and the resulting crude product was purified by silica gel column chromatography (3/1, petroleum ether/ethyl acetate, R_{f} = 0.72) to obtain compound **21-4.** ¹H NMR (400MHz, CD₃Cl) δ = 7.75 - 7.68 (m, 2H), 7.49 - 7.43 (m, 2H), 4.38 (q, *J*=7.2 Hz, 2H), 1.39 (t, *J*=7.2 Hz, 3H).

### Step 3

Compound **21-4** (10.8 g, 30.5 mmol) was dissolved in tetrahydrofuran (100 mL) and water (100 mL), and lithium hydroxide monohydrate (3.84 g, 91.5 mmol) was added thereto, and the reaction mixture was stirred and reacted for 14 hours at 25°C. After the reaction mixture was concentrated, the residue was added with water (200 mL). The mixture was acidified to pH = 3 to 4 with 1 M dilute hydrochloric acid, and extracted with ethyl acetate (200 mL × 3). The organic phases were combined, dried over anhydrous sodium sulfate, filtered, and concentrated under reduced pressure to obtain crude compound **21-5**, which was used directly in the next reaction step.

### Step 4

Compound **21-5** (6.00 g, 15.3 mmol) and compound **21-6** (3.67 g, 18.3 mmol) were dissolved in *N*,*N*-dimethylformamide (150 mL), and then 2-(7-azabenzotriazol-1-yl)-*N*,*N*,*N*',*N*'-tetramethyluronium hexafluorophosphate (8.71 g, 22.9 mmol) and *N,N-*diisopropylethylamine (209 mg, 1.62 mmol) were added thereto, and the reaction mixture was stirred and reacted for 12 hours at 25°C. The reaction mixture was added with water (200 mL), and extracted with ethyl acetate (200 mL × 2). The organic phases were combined, washed with saturated brine (200 mL × 1), dried over anhydrous sodium sulfate, filtered, and concentrated. The resulting crude product was purified by column chromatography (1/2, petroleum ether/ethyl acetate, R_{f} = 0.25) to obtain compound **21-7.** ¹H NMR (400MHz, CD₃Cl) δ = 7.85 - 7.53 (m, 4H), 4.72 - 4.22 (m, 2H), 3.71 -3.60 (m, 1H), 3.07 -2.94 (m, 2H), 2.15 - 1.78 (m, 2H), 1.73 - 1.49 (m, 2H), 1.43 (s, 9H). MS-ESI calculated for [M+H]⁺ 510, found 510.

### Step 5

Under nitrogen atmosphere, compound **21-7** (50.0 mg, 98.4 µmol), compound **21-8** (20.0 mg, 118 µmol), and sodium carbonate (20.8 mg, 197 µmol) were dissolved in dioxane (8.00 mL) and water (2.00 mL), and the reaction mixture was added with [1,1'-bis(diphenylphosphino)ferrocene]dichloropalladium(II) (7.20 mg, 9.83 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.57) to obtain compound **21-9.** ¹H NMR (400MHz, CD₃OD) δ = 7.89 - 7.69 (m, 1H), 7.64 - 7.44 (m, 2H), 7.32 -7.24 (m, 2H), 6.83 (dd, *J*=2.4, 8.8 Hz, 1H), 6.74 (dd, *J*=2.4, 12.0 Hz, 1H), 4.49 - 4.37 (m, 1H), 4.33 - 4.36 (m, 1H), 3.83 (s, 3H), 3.71 - 3.55 (m, 1H),3.12 - 2.99(m,2 H), 2.04 - 1.96 (m, 1H), 1.93 -1.88 (m, 1H), 1.69 - 1.53 (m, 2H), 1.54 (s, 9H). MS-ESI calculated for [M+Na]⁺ 576, found 576.

### Step 6

Compound **21-9** (50.0 mg, 90.3 µmol) was dissolved in ethyl acetate (15.0 mL), and hydrochloride ethyl acetate solution (4 mol/L, 226 µL) was added thereto, and the reaction mixture was stirred and reacted for 12 hours at 25°C. After the reaction mixture was concentrated, the residue was purified by preparative high performance liquid chromatography (chromatographic column: Phenomenex Synergi C18 150 * 30 mm * 4 µm; mobile phase A: hydrochloric acid aqueous solution with a volume fraction of 0.05%; mobile phase B: acetonitrile; B%: 36% to 66%, 9 minutes) to obtain the hydrochloride of compound **21.** ¹H NMR (400MHz, CD₃OD) δ = 7.73 - 7.65 (m, 1H), 7.64 (s, 1H), 7.38 - 7.28 (m, 2H), 7.25 (dd, *J*=1.6, 8.0 Hz, 1H), 6.87 (dd, *J*=2.4, 8.4 Hz, 1H), 6.78 (dd, *J*=2.4, 12.0 Hz, 1H), 4.39 (d, *J*=11.2 Hz, 1H), 4.19 - 4.13 (m, 1H), 3.86 (s, 3H), 3.61 - 3.39 (m, 3H), 2.23 - 2.19 (m, 1H), 1.97 - 1.86 (m, 1H), 1.85 - 1.69 (m, 2H). MS-ESI calculated for [M+H]⁺ 454, found 454.

### Example 22

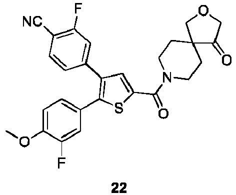

### Synthetic route:

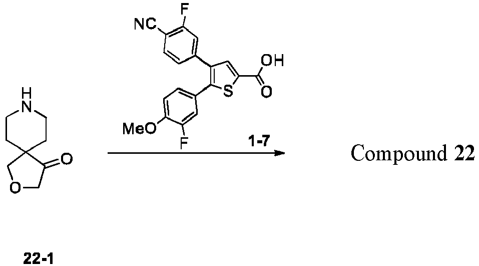

Compound **1-7** (190 mg, 0.512 mmol) was dissolved in anhydrous dichloromethane (5 mL), and then triethylamine (152.87 mg, 1.18 mmol), compound **22-1** (98.1 mg, 0.512 mmol), and 2-(7-azabenzotriazol-l-yl)-*N*,*N*,*N*',*N*'-tetramethyluronium hexafluorophosphate (292 mg, 0.767 mmol) were added thereto, and the reaction mixture was stirred for 12 hours at 25°C. The reaction mixture was added with water (20 mL) and extracted with dichloromethane (20 mL × 1). The organic phase was washed with saturated brine (20 mL × 1), dried over anhydrous sodium sulfate, filtered, and concentrated under reduced pressure. The residue was separated by preparative high performance liquid chromatography (chromatographic column: Phenomenex Synergi C18 150 * 30 mm * 4 µm; mobile phase: mobile phase A: hydrochloric acid aqueous solution with a volume fraction of 0.05%; mobile phase B: acetonitrile; B%: 54% to 84%, 9 minutes) to obtain compound **22.** ¹H NMR (400MHz, CD₃OD) δ 7.68 (t, J=7.6 Hz, 1H), 7.51 (s, 1H), 7.34 - 7.29 (m, 1H), 7.27 - 7.22 (m, 1H), 7.14 - 7.02 (m, 3H), 4.20 - 4.10 (m, 4H), 4.05 (s, 2H), 3.89 (s, 3H), 3.70 - 3.50 (m, 2H), 1.88 - 1.78 (m, 2H), 1.77 - 1.67 (m, 2H). MS-ESI calculated for [M+H]⁺ 509, found 509.

### Example 23

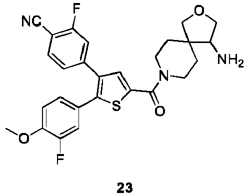

### Synthetic route:

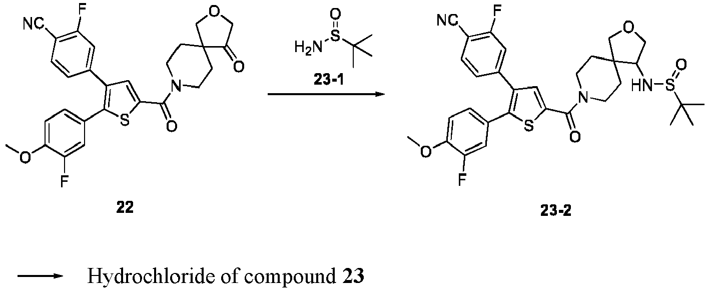

### Step 1

Under nitrogen atmosphere, compound **22** (130 mg, 0.256 mmol) was dissolved in anhydrous tetrahydrofuran (10 mL), and then titanium ethoxide (233 mg, 1.02 mmol) and tert-butanesulfinamide (**23-1**) (62.0 mg, 0.511 mmol) were added thereto, and the reaction mixture was stirred for 12 hours at 85°C, then cooled to -5°C, and methanol (73.7 mg, 2.30 mmol) was added thereto, and then lithium borohydride (62.0 mg, 0.511 mmol) was slowly added thereto. The reaction mixture was stirred for 1 hour at -5°C, cooled to 0°C, added dropwise with saturated ammonium chloride aqueous solution (20 mL), then added with ethyl acetate (20 mL), and filtrated. The filter cake was washed with ethyl acetate (10 mL). The organic phase was washed with saturated brine (20 mL × 1), dried over anhydrous sodium sulfate, filtered, concentrated under reduced pressure, and the residue was separated by silica gel column chromatography (10/1, dichloromethane/methanol, R_{f} = 0.43) to obtain compound **23-2.** MS-ESI calculated for [M+H]⁺ 614, found 614.

### Step 2

Compound **23-2** (60.0 mg, 0.098 mmol) was dissolved in ethyl acetate (5 mL), and hydrochloride ethyl acetate solution (4 mol/L, 5 mL) was added dropwise thereto at 25°C. The reaction mixture was stirred for 12 hours at 25°C and concentrated under reduced pressure. The residue was separated by preparative high performance liquid chromatography (chromatographic column: Phenomenex Synergi C18 150 * 30 mm* 4 µm; mobile phase: mobile phase A: hydrochloric acid aqueous solution with a volume fraction of 0.05%; mobile phase B: acetonitrile; B%: 32% to 62%, 9 minutes) to obtain the hydrochloride of compound 23. ¹H NMR (400MHz, CD₃OD) δ 7.69 (t, J=7.6 Hz, 1H), 7.54 - 7.50 (m, 1H), 7.35 - 7.30 (m, 1H), 7.27 - 7.23 (m, 1H), 7.15 - 7.09 (m, 1H), 7.08 - 7.03 (m, 2H), 4.41 - 4.24 (m, 2H), 4.22 - 4.16 (m, 1H), 4.01 - 3.96 (m, 1H), 3.94 - 3.91 (m, 1H), 3.90 (s, 3H), 3.86 - 3.82 (m, 1H), 3.64 - 3.60 (m, 1H), 3.52 - 3.37 (m, 1H), 1.96 - 1.73 (m, 4H). MS-ESI calculated for [M+H]⁺ 510, found 510.

### Example 24

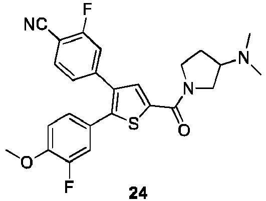

### Synthetic route:

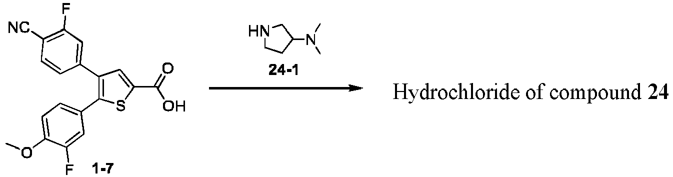

Under nitrogen atmosphere, compound **1-7** (100 mg, 269 µmol), 2-(7-azabenzotriazol-1-yl)-*N*,*N*,*N'*,*N'*-tetramethyluronium hexafluorophosphate (154 mg, 404 µmol), and *N,N-*diisopropylethylamine (104 mg, 807 µmol) were dissolved in *N*,*N*-dimethylformamide (10.00 mL), and then compound **24-1** (36.9 mg, 323 µmol) was added thereto, and the reaction mixture was stirred and reacted for 12 hours at 25°C. The reaction mixture was added with water (50.0 mL), and extracted with ethyl acetate (50.0 mL × 2). The organic phase was washed with saturated brine (50.0 mL × 1), dried over anhydrous sodium sulfate, filtered, and concentrated under reduced pressure. The residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 14% to 44%, 10 minutes) to obtain the hydrochloride of compound **24.** ¹H NMR (400MHz, CD₃OD) δ = 7.74 (s, 1H), 7.72 - 7.67 (m, 1H), 7.34 (d, *J*=10.0 Hz, 1H), 7.26 (d, *J*=7.6 Hz, 1H), 7.16 - 7.01 (m, 3H), 4.53 - 4.13 (m, 2H), 4.10 - 3.95 (m, 2H), 3.90 (s, 3H), 3.87 - 3.79 (m, 1H), 3.00 (s, 6H), 2.63 - 2.59 (m, 1H), 2.36 - 2.31 (m, 1H). MS-ESI calculated for [M+H]⁺ 468, found 468.

### Example 25

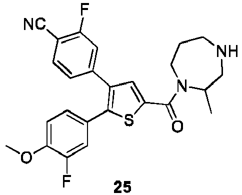

### Synthetic route:

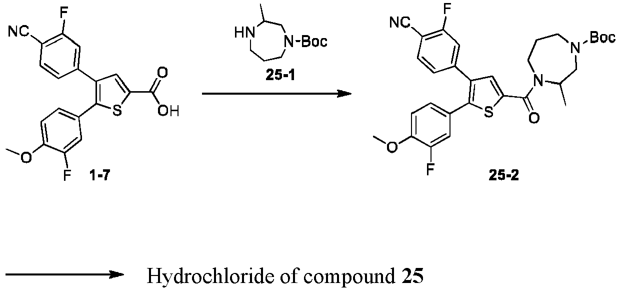

### Step 1

Compound **1-7** (100 mg, 269 µmol), 2-(7-azabenzotriazol-1-yl)-*N*,*N*,*N'*,*N'-*tetramethyluronium hexafluorophosphate (154 mg, 404 µmol), and *N*,*N*-diisopropylethylamine (104 mg, 807 µmol) were dissolved in *N*,*N*-dimethylformamide (20.0 mL), then compound **25-1** (69.3 mg, 323 µmol) was added thereto, and the reaction mixture was stirred and reacted for 6 hours at 25°C. The reaction mixture was added with water (30.0 mL), and extracted with ethyl acetate (30.0 mL × 2). The organic phase was washed with saturated brine (30.0 mL × 1), dried over anhydrous sodium sulfate, filtered, and concentrated under reduced pressure. The residue was purified by silica gel column chromatography (1/1, petroleum ether/ethyl acetate, R_{f} = 0.50) to obtain compound **25-2.** MS-ESI calculated for [M-56+H]⁺ 512, found 512.

### Step 2

Compound **25-2** (147 mg, 259 µmol) was dissolved in ethyl acetate (30.0 mL), and hydrochloride ethyl acetate solution (4 mol/L, 324 µL) was added thereto, and the reaction mixture was stirred and reacted for 6 hours at 25°C. After the reaction mixture was concentrated, the residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 17% to 47%, 10 minutes) to obtain the hydrochloride of compound **25.** ¹H NMR (400MHz, CD₃OD) δ = 7.76 - 7.69 (m, 1H), 7.59 (s, 1H), 7.35 (dd, *J*=1.2, 10.2 Hz, 1H), 7.28 (dd, *J*=1.6, 8.0 Hz, 1H), 7.17 - 7.05 (m, 3H), 5.03 - 4.92 (m, 1H), 4.86 - 4.74 (m, 1H), 4.3 -4.22 (m, 1H), 3.92 (s, 3H), 3.79 -3.70 (m, 1H), 3.58-3.48 (m, 1H), 3.29 - 3.22 (m, 1H), 3.21 - 3.08 (m, 1H), 2.28 - 2.05 (m, 2H), 1.42 -1.28 (s, 3H). MS-ESI calculated for [M+H]⁺ 468, found 468.

### Example 26

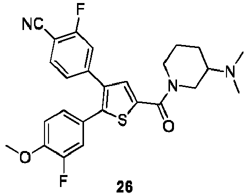

### Synthetic route:

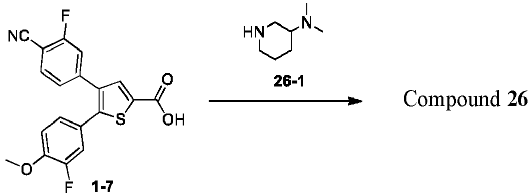

Under nitrogen atmosphere, compound **1-7** (100 mg, 269 µmol), 2-(7-azabenzotriazol-1-yl)-*N*,*N*,*N'*,*N'*-tetramethyluronium hexafluorophosphate (154 mg, 404 µmol), and *N,N-*diisopropylethylamine (104 mg, 807 µmol) were dissolved in *N*,*N*-dimethylformamide (30.0 mL), and then compound **26-1** (41.4 mg, 323 µmol) was added thereto, and the reaction mixture was stirred and reacted for 12 hours at 25°C. The reaction mixture was added with water (50 mL), and extracted with ethyl acetate (50.0 mL × 2). The organic phase was washed with saturated brine (50.0 mL × 1), dried over anhydrous sodium sulfate, filtered, and concentrated under reduced pressure. The residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 16% to 46%, 10 minutes) to obtain the hydrochloride of compound **26.** ¹H NMR (400MHz, CD₃OD) δ = 7.69 (t, *J*=7.6 Hz, 1H), 7.59 (s, 1H), 7.34 (d, *J*=10.4 Hz, 1H), 7.27 (d, *J*=8.0 Hz, 1H), 7.15 - 7.01 (m, 3H), 4.55 (d, *J*=11.6 Hz, 1H), 4.25 (d, *J*=11.2 Hz, 1H), 3.89 (s, 3H), 3.65 - 3.55 (m, 1H), 3.54 - 3.38 (m, 2H), 3.00 (s, 3H), 2.95 (s, 3H), 2.29 - 2.25 (m, 1H), 2.03 -1.90 (m, 2H), 1.78 - 1.68 (m, 1H). MS-ESI calculated for [M+H]⁺ 482, found 482.

### Example 27

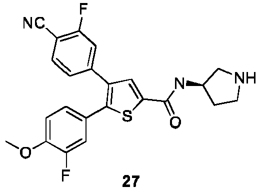

### Synthetic route:

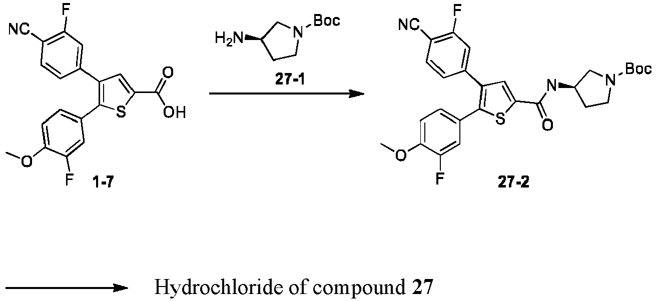

### Step 1

Compound **1-7** (100 mg, 269 µmol), 2-(7-azabenzotriazol-1-yl)-*N,N,N',N'-*tetramethyluronium hexafluorophosphate (154 mg, 404 µmol), and N,N-diisopropylethylamine (104 mg, 807 µmol) were dissolved in *N,N*-dimethylformamide (10.0 mL), then compound **27-1** (60.2 mg, 323 µmol) was added thereto, and the reaction mixture was stirred and reacted at 25°C for 12 hours. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, R_{f} = 0.43) to obtain compound **27-2.** MS-ESI calculated for [M-56+H]⁺ 484, found 484.

### Step 2

Compound **27-2** (120 mg, 259 µmol) was dissolved in ethyl acetate (30.0 mL), and hydrochloride ethyl acetate solution (4 mol/L, 278 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 17% to 47%, 10 minutes) to obtain the hydrochloride of compound **27**. ¹H NMR (400MHz, CD₃OD) δ = 8.01 (s, 1H), 7.71 (t, *J*=7.6 Hz, 1H), 7.36 - 7.21 (m, 2H), 7.18 - 6.99 (m, 3H), 4.72 - 4.62 (m, 1H), 3.91 (s, 3H), 3.68 - 3.55 (m, 2H), 3.52 - 3.37 (m, 2H), 2.48 - 2.39 (m, 1H), 2.30 - 2.22 (m, 1H). MS-ESI calculated for [M+H]⁺ 440, found 440.

### Example 28

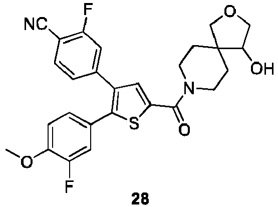

### Synthetic route:

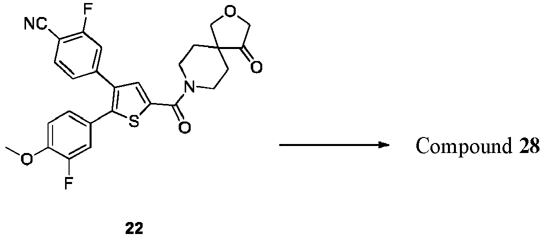

Compound **22** (80.0 mg, 0.157 mmol) was dissolved in anhydrous tetrahydrofuran (5.00 mL), and sodium borohydride (11.9 mg, 0.315 mmol) was added thereto. The reaction mixture was stirred for 12 hours at 25°C, cooled to 0°C, added dropwise with saturated ammonium chloride aqueous solution (20 mL), and then added with ethyl acetate (20 mL). The organic phase was washed with saturated brine (20 mL × 1), dried over anhydrous sodium sulfate, filtered, concentrated under reduced pressure, and the residue was separated by preparative high performance liquid chromatography (chromatographic column: Phenomenex Synergi C18 150 * 30 mm * 4 µm; mobile phase: mobile phase A: hydrochloric acid aqueous solution with a volume fraction of 0.05%; mobile phase B: acetonitrile; B%: 49% to 79%, 9 minutes) to obtain compound **28.** ¹H NMR (400MHz, CD₃OD) δ 7.72 - 7.66 (m, 1H), 7.50 (s, 1H), 7.32 (dd, J=1.2, 10.4 Hz, 1H), 7.25 (dd, J=1.6, 8.0 Hz, 1H), 7.15 - 7.02 (m, 3H), 4.16 - 4.10 (m, 1H), 4.09 - 4.05 (m, 1H), 3.99 - 3.91 (m, 2H), 3.90 (s, 3H), 3.80 - 3.77 (m, 1H), 3.76 - 3.57 (m, 4H), 1.97 - 1.88 (m, 1H), 1.70 - 1.60 (m, 3H). MS-ESI calculated for [M+H]⁺ 511, found 511.

### Example 29

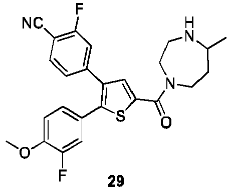

### Synthetic route:

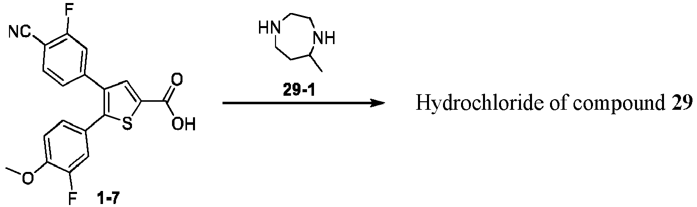

Compound **1-7** (100 mg, 269 µmol), 2-(7-azabenzotriazol-1-yl)-*N,N,N',N'-*tetramethyluronium hexafluorophosphate (154 mg, 404 µmol), and *N,N*-diisopropylethylamine (104 mg, 807 µmol) were dissolved in *N,N*-dimethylformamide (10.0 mL), then compound **29-1** (61.5 mg, 539 µmol) was added thereto, and the reaction mixture was stirred and reacted at 25°C for 12 hours. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by preparative high performance liquid chromatography (chromatographic column: Phenomenex Gemini-NX 80 * 30 mm * 3 µm; mobile phase A: 10 mM NH₄HCO₃ aqueous solution; mobile phase B: acetonitrile; B%: 40% to 70%, 9 minutes), and 200 µL of 2N hydrochloric acid aqueous solution was added thereto to obtain the hydrochloride of compound **29.** ¹H NMR (400MHz, CD₃OD) δ = 7.71 -7.67 (m, 1H), 7.61 (s, 1H), 7.33 (dd, *J*=1.2, 10.4 Hz, 1H), 7.25 (dd, *J*=1.2, 8.4 Hz, 1H), 7.15 - 7.01 (m, 3H), 4.17 - 4.10 (m, 2H), 3.99 - 3.92 (m, 1H), 3.90 (s, 3H), 3.83 - 3.60 (m, 1H), 3.66 - 3.50 (m, 2H), 3.4 -3.34 (m, 1H), 2.26 - 2.00 (m, 2H), 1.42 (d, *J*=6.4 Hz, 3H). MS-ESI calculated for [M+H]⁺ 468, found 468.

### Example 30

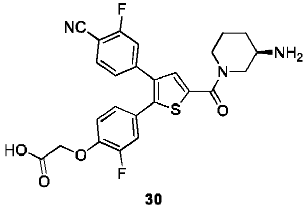

### Synthetic route:

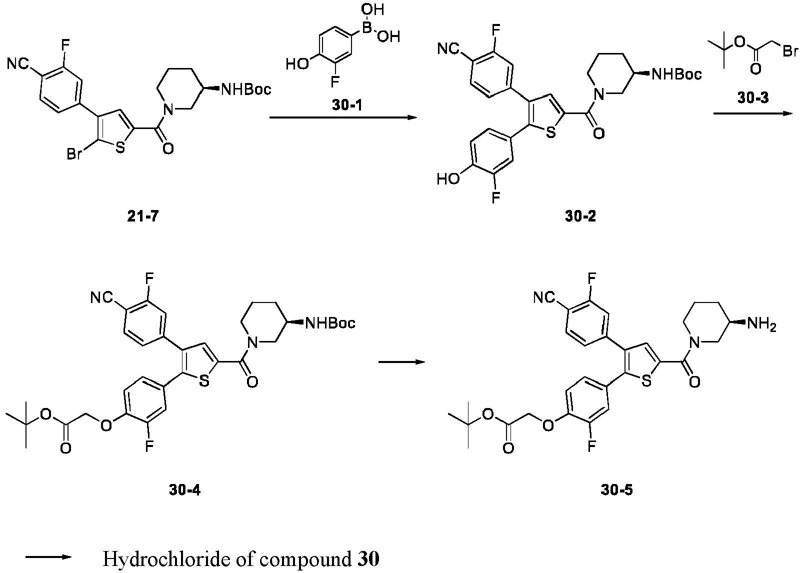

### Step 1

Under nitrogen atmosphere, compound **21-7** (130 mg, 256 µmol), compound **30-1** (47.8 mg, 309 µmol), and sodium carbonate (54.2 mg, 511 µmol) were dissolved in dioxane (8.00 mL) and water (2.00 mL), and the reaction mixture was added with [1,1'-bis(diphenylphosphino)ferrocene]dichloropalladium(II) (18.7 mg, 25.6 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.43) to obtain compound **30-2.** ¹H NMR (400MHz, CD₃OD) δ = 7.85 - 7.45 (m, 3H), 7.28 (d, *J*=8.0 Hz, 1H), 7.04 - 6.97 (m, 1H), 6.96 - 6.88 (m, 2H), 4.50 -4.39 (m, 1H), 4.32 - 4.16 (m, 1H), 3.67 -3.56 (m, 1H), 3.21 -3.01 (m, 2H), 2.07 - 1.97 (m, 1H), 1.93 - 1.82 (m, 1H), 1.71 - 1.52 (m, 2H), 1.42 (s, 9H). MS-ESI calculated for [M-56+H]⁺ 484, found 484.

### Step 2

Compound **30-2** (66.0 mg, 122 µmol) was dissolved in *N*,*N*-dimethylformamide (5.00 mL), and the reaction mixture was added with **30-3** (28.6 mg, 147 µmol) and potassium carbonate (33.8 mg, 245 µmol), stirred, and reacted at 60°C for 12 hours. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/2, petroleum ether/ethyl acetate, R_{f} = 0.42) to obtain compound **30-4**. ¹H NMR (400MHz, CD₃OD) δ = 7.88 - 7.43 (m, 3H), 7.28 (d, *J*=8.0 Hz, 1H), 7.11 - 7.05 (m, 1H), 7.06 - 6.96 (m, 2H), 4.70 (s, 2H), 4.44 - 4.39 (m, 1H), 4.23 - 4.20 (m, 1H), 3.63 -3.60 (m, 1H), 3.23 - 2.97 (m, 2H), 2.03 - 2.00 (m, 1H), 1.94 - 1.82 (m, 1H), 1.71 - 1.54 (m, 2H), 1.48 (s, 9H), 1.42 (s, 9H). MS-ESI calculated for [M+H]⁺ 654, found 654.

### Step 3

Compound **30-4** (50.0 mg, 259 µmol) was dissolved in ethyl acetate (5.00 mL), and hydrochloride ethyl acetate solution (4 mol/L, 191 µL) was added thereto, and the reaction mixture was stirred and reacted for 12 hours at 25°C. The reaction mixture was concentrated to obtain crude compound **30-5**, which was used directly in the next reaction step. MS-ESI calculated for [M+H]⁺ 554, found 554.

### Step 4

Compound **30-5** (50.0 mg, 84.7 µmol) was dissolved in dichloromethane (5.00 mL), and trifluoroacetic acid (2.00 mL) was added thereto, and the reaction mixture was stirred and reacted for 2 hours at 25°C. After the reaction mixture was concentrated, the residue was separated by preparative high performance liquid chromatography (chromatographic column: Phenomenex Synergi C18 150 * 30 mm * 4 µm; mobile phase A: hydrochloric acid aqueous solution with a volume fraction of 0.05%; mobile phase B: acetonitrile; B%: 17% to 47%, 10 minutes) to obtain the hydrochloride of compound **30**. ¹H NMR (400MHz, CD₃OD) δ = 7.70 (t, *J*=7.6 Hz, 1H), 7.56 (s, 1H), 7.34 (d, *J*=10.4 Hz, 1H), 7.25 (dd, *J*=1.2, 8.2 Hz, 1H), 7.13 - 6.97 (m, 3H), 4.78 (s, 2H), 4.37 (d, *J*=11.6 Hz, 1H), 4.14 (d, *J*=13.6 Hz, 1H), 3.61 - 3.35 (m, 3H), 2.26 - 2.14 (m, 1H), 1.95 - 1.84 (m, 1H), 1.83 - 1.67 (m, 2H). MS-ESI calculated for [M+H]⁺ 498, found 498.

### Example 31

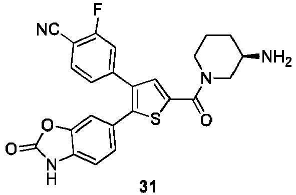

### Synthetic route:

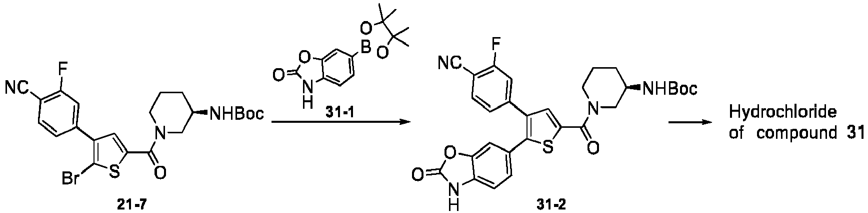

### Step 1

Under nitrogen atmosphere, compound **21-7** (81.1 mg, 160 µmol), compound **31-1** (50.0 mg, 192 µmol), and sodium carbonate (33.8 mg, 319 µmol) were dissolved in dioxane (4.00 mL) and water (1.00 mL), and the reaction mixture was added with [1,1'-bis(diphenylphosphino)ferrocene]dichloropalladium(II) (11.7 mg, 16.0 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.14) to obtain compound **31-2.** MS-ESI calculated for [M-56+H]⁺ 507, found 507.

### Step 2

Compound **31-2** (50 mg, 88.8 µmol) was dissolved in ethyl acetate (20.0 mL), and hydrochloride ethyl acetate solution (4 mol/L, 222 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 8% to 38%, 10 minutes) to obtain the hydrochloride of compound **31.** ¹H NMR (400MHz, CD₃OD) δ = 7.72 -7.65 (m, 1H), 7.60 (s, 1H), 7.39 - 7.33 (m, 1H), 7.28 - 7.24 (m, 1H), 7.21 (s, 1H), 7.16 - 7.05 (m, 2H), 4.42 - 4.35 (m, 1H), 4.20 - 4.13 (m, 1H), 3.54 - 3.38 (m, 3H), 2.30 - 2.19 (m, 1H), 1.97 - 1.89 (m, 1H), 1.85 - 1.68 (m, 2H). MS-ESI calculated for [M+H]⁺ 463, found 463.

### Example 32

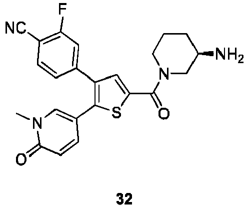

### Synthetic route:

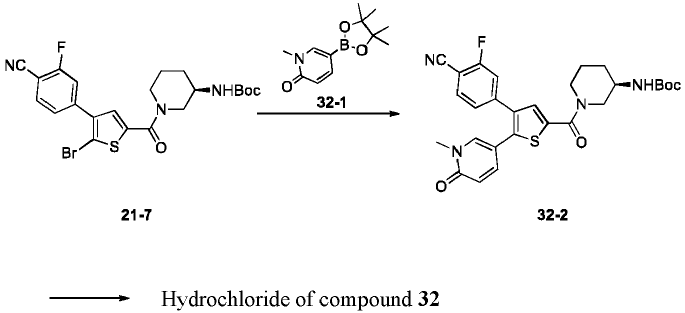

### Step 1

Under nitrogen atmosphere, compound **21-7** (180 mg, 354 µmol), compound **32-1** (100 mg, 192 µmol), and sodium carbonate (75.1 mg, 709 µmol) were dissolved in dioxane (8.00 mL) and water (2.00 mL), and the reaction mixture was added with [1,1'-bis(diphenylphosphino)ferrocene]dichloropalladium(II) (25.9 mg, 35.5 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.14) to obtain compound **32-2.** MS-ESI calculated for [M+H]⁺ 537, found 537.

### Step 2

Compound **32-2** (50.0 mg, 93.18 µmol) was dissolved in ethyl acetate (10.00 mL), and hydrochloride ethyl acetate solution (4 mol/L, 234 µL) was added thereto, and the reaction mixture was stirred and reacted for 12 hours at 25°C. After the reaction mixture was concentrated, the residue was purified by preparative high performance liquid chromatography (chromatographic column: Phenomenex Synergi C18 150 * 30 mm * 4 µm, mobile phase A: hydrochloric acid aqueous solution with a volume fraction of 0.05%; mobile phase B: acetonitrile; B%: 23% to 53%, 9 minutes) to obtain the hydrochloride of compound **32.** ¹H NMR (400MHz, CD₃OD) δ = 7.91 (d, *J*=2.4 Hz, 1H), 7.74 (t, *J*=7.6 Hz, 1H), 7.59 (s, 1H), 7.45 (d, *J*=10.4 Hz, 1H), 7.36 (d, *J*=8.0 Hz, 1H), 7.28 (dd, *J*=2.4, 9.6 Hz, 1H), 6.51 (d, *J*=9.6 Hz, 1H), 4.39 (d, *J*=11.2 Hz, 1H), 4.13 (d, *J*=13.2 Hz, 1H), 3.59 (s, 3H), 3.53 - 3.37 (m, 3H), 2.23 -2.17 (m, 1H), 1.96 - 1.85 (m, 1H), 1.82 - 1.65 (m, 2H). MS-ESI calculated for [M+H]⁺ 437, found 437.

### Example 33

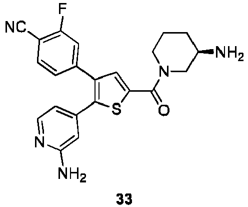

### Synthetic route:

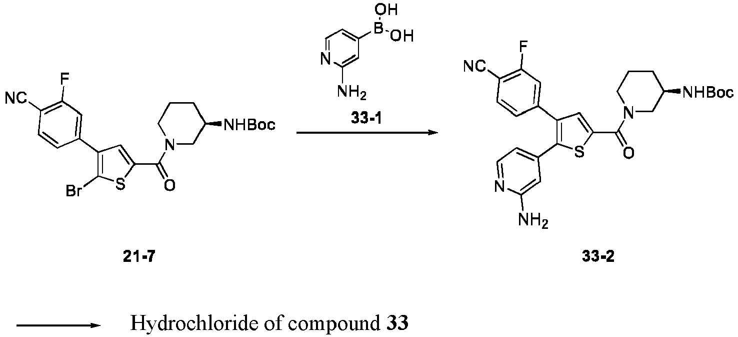

### Step 1

Under nitrogen atmosphere, compound **21-7** (154 mg, 302 µmol), compound **33-1** (50.0 mg, 363 µmol), and sodium carbonate (64.0 mg, 604 µmol) were dissolved in dioxane (4.00 mL) and water (1.00 mL), and the reaction mixture was added with [1,1'-bis(diphenylphosphino)ferrocene]dichloropalladium(II) (22.1 mg, 30.2 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.17) to obtain compound **33-2.** MS-ESI calculated for [M+H]⁺ 522, found 522.

### Step 2

Compound **33-2** (80.0 mg, 153 µmol) was dissolved in ethyl acetate (5.00 mL), and hydrochloride ethyl acetate solution (4 mol/L, 383 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 1% to 30%, 10 minutes) to obtain the hydrochloride of compound **33.** ¹H NMR (400MHz, CD₃OD) δ = 7.87 - 7.74 (m, 2H), 7.64 (s, 1H), 7.52 (d, J=10.0 Hz, 1H), 7.39 (dd, *J*=1.2, 8.0 Hz, 1H), 6.96 (d, *J*=1.2 Hz, 1H), 6.70 (dd, *J*=1.6, 6.8 Hz, 1H), 4.39 (d, *J*=11.6 Hz, 1H), 4.25 - 3.99 (m, 1H), 3.56 - 3.36 (m, 3H), 2.26 - 2.12 m, 1H), 1.93 - 1.80 (m, 1H), 1.79 - 1.62 (m, 2H). MS-ESI calculated for [M+H]⁺ 422, found 422.

### Example 34

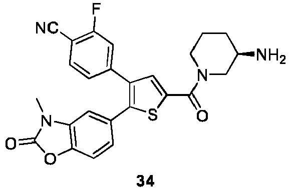

### Synthetic route:

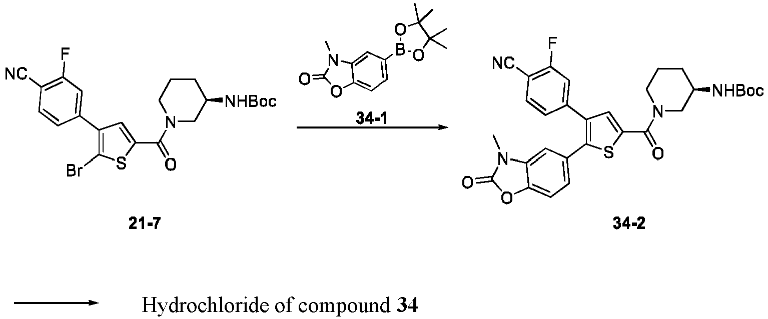

### Step 1

Under nitrogen atmosphere, compound **21-7** (100 mg, 197 µmol), compound **34-1** (64.9 mg, 236 µmol), and sodium carbonate (41.7 mg, 393 µmol) were dissolved in dioxane (4.00 mL) and water (1.00 mL), and the reaction mixture was added with [1,1'-bis(diphenylphosphino)ferrocene]dichloropalladium(II) (14.4 mg, 19.7 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.08) to obtain compound **34-2.** MS-ESI calculated for [M-56+H]⁺ 521, found 521.

### Step 2

Compound **34-2** (50.0 mg, 32.1 µmol) was dissolved in ethyl acetate (5.00 mL), and hydrochloride ethyl acetate solution (4 mol/L, 80.0 µL) was added thereto, and the reaction mixture was stirred and reacted for 12 hours at 25°C. After the reaction mixture was concentrated, the residue was purified by preparative high performance liquid chromatography (chromatographic column: Phenomenex Synergi C18 150 * 30 mm * 4 µm, mobile phase A: hydrochloric acid aqueous solution with a volume fraction of 0.05%; mobile phase B: acetonitrile; B%: 35% to 65%, 9 minutes) to obtain the hydrochloride of compound **34.** ¹H NMR (400MHz, CD₃OD) δ = 7.71 - 7.64 (m, 1H), 7.61 (s, 1H), 7.35 (dd, *J*=1.2, 10.4 Hz, 1H), 7.29 - 7.22 (m, 2H), 7.17 (d, *J*=2.0 Hz, 1H), 7.06 (dd, *J*=2.0, 8.4 Hz, 1H), 4.39 (d, *J*=11.2 Hz, 1H), 4.16 (d, *J*=13.6 Hz, 1H), 3.64 - 3.39 (m, 3H), 3.35 (s, 3H), 2.25 - 2.14 (m, 1H), 1.97 - 1.85 (m, 1H), 1.83 - 1.68 (m, 2H). MS-ESI calculated for [M+H]⁺ 477, found 477.

### Example 35

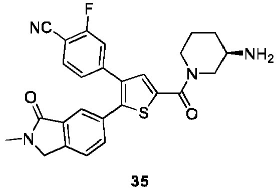

### Synthetic route:

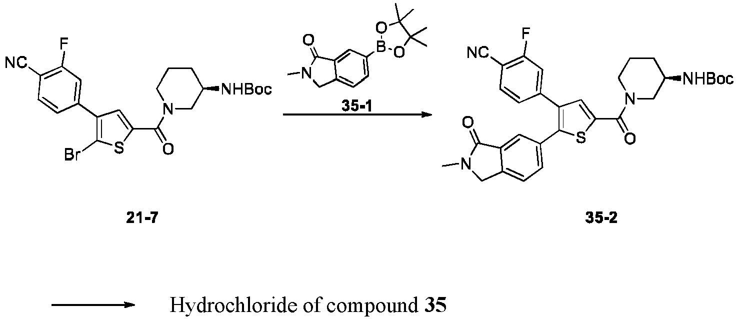

### Step 1

Under nitrogen atmosphere, compound **21-7** (77.6 mg, 153 µmol), compound **35-1** (50.0 mg, 183 µmol), and sodium carbonate (32.3 mg, 305 µmol) were dissolved in dioxane (4.00 mL) and water (1.00 mL), and the reaction mixture was added with [1,1'-bis(diphenylphosphino)ferrocene]dichloropalladium(II) (11.1 mg, 15.2 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.54) to obtain compound **35-2.** MS-ESI calculated for [M-56+H]⁺ 519, found 519.

### Step 2

Compound **35-2** (65.0 mg, 32.1 µmol) was dissolved in ethyl acetate (5.00 mL), and hydrochloride ethyl acetate solution (4 mol/L, 287 µL) was added thereto, and the reaction mixture was stirred and reacted for 2 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **35.** ¹H NMR (400MHz, CD₃OD) δ = 7.73 - 7.65 (m, 2H), 7.64 - 7.60 (m, 2H), 7.56 (dd, *J*=1.6, 8.0 Hz, 1H), 7.34 (dd, *J*=1.2, 10.4 Hz, 1H), 7.25 (dd, *J*=1.2, 8.0 Hz, 1H), 4.57 (s, 2H), 4.40 (d, *J*=12.0 Hz, 1H), 4.17 (d, J=12.4 Hz, 1H), 3.63 - 3.41 (m, 3H), 3.22 (s, 3H), 2.30 - 2.14 (m, 1H), 2.01 - 1.87 (m, 1H), 1.84 - 1.65 (m, 2H). MS-ESI calculated for [M+H]⁺ 475, found 475.

### Example 36

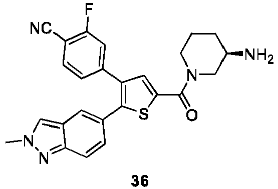

### Synthetic route:

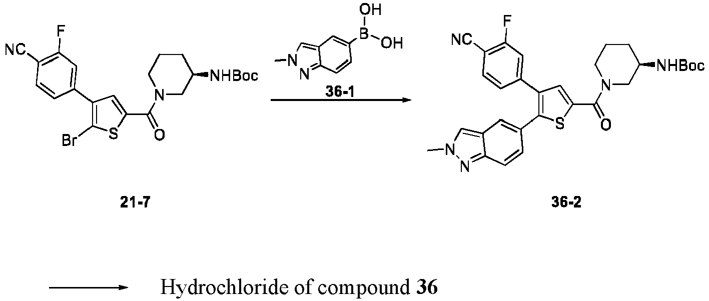

### Step 1

Under nitrogen atmosphere, compound **21-7** (241 mg, 474 µmol), compound **36-1** (100 mg, 568 µmol), and sodium carbonate (100 mg, 947 µmol) were dissolved in dioxane (8.00 mL) and water (2.00 mL), and the reaction mixture was added with [1,1'-bis(diphenylphosphino)ferrocene]dichloropalladium(II) (34.6 mg, 47.3 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.29) to obtain compound **36-2.** MS-ESI calculated for [M+H]⁺ 560, found 560.

### Step 2

Compound **36-2** (200 mg, 357 µmol) was dissolved in ethyl acetate (5.00 mL), and hydrochloride ethyl acetate solution (4 mol/L, 893 µL) was added thereto, and the reaction mixture was stirred and reacted for 2 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 16% to 46%, 10 minutes) to obtain the hydrochloride of compound **36.** ¹H NMR (400MHz, CD₃OD) δ = 8.58 (s, 1H), 7.93 (s, 1H), 7.74 - 7.61 (m, 3H), 7.43 - 7.33 (m, 2H), 7.30 (d, *J*=8.0 Hz, 1H), 4.45 (d, *J*=12.0 Hz, 1H), 4.34 (s, 3H), 4.20 (d, *J*=13.6 Hz, 1H), 3.70 - 3.39 (m, 3H), 2.27 -2.20 (m, 1H), 1.97 - 1.92 (m, 1H), 1.88 - 1.65 (m, 2H). MS-ESI calculated for [M+H]⁺ 460, found 460.

### Example 37

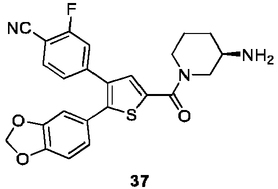

### Synthetic route:

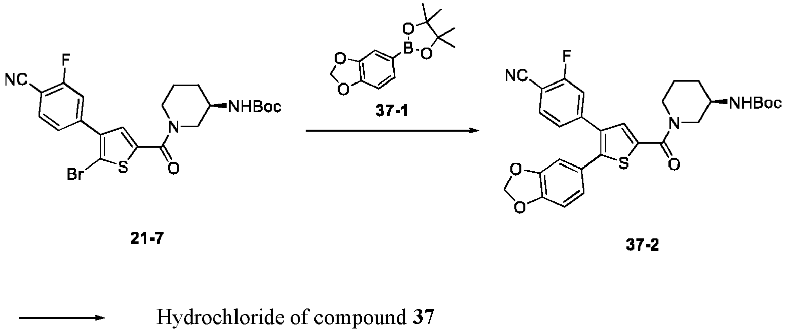

### Step 1

Under nitrogen atmosphere, compound **21-7** (86.4 mg, 166 µmol), compound **37-1** (50.0 mg, 568 µmol), and sodium carbonate (35.2 mg, 333 µmol) were dissolved in dioxane (8.00 mL) and water (2.00 mL), and the reaction mixture was added with [1,1'-bis(diphenylphosphino)ferrocene]dichloropalladium(II) (12.2 mg, 16.7 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.38) to obtain compound **37-2.** MS-ESI calculated for [M-56+H]⁺ 494, found 494.

### Step 2

Compound **37-2** (62.0 mg, 113 µmol) was dissolved in ethyl acetate (10.0 mL), and hydrochloride ethyl acetate solution (4 mol/L, 282 µL) was added thereto, and the reaction mixture was stirred and reacted for 12 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 16% to 46%, 10 minutes) to obtain the hydrochloride of compound **37.** ¹H NMR (400MHz, CD₃OD) δ = 7.71 (t, *J*=8.0 Hz,, 1H), 7.57 (s, 1H), 7.33 (dd, *J*=1.6, 10.4 Hz, 1H), 7.29 (dd, *J*=1.2, 8.0 Hz, 1H), 6.91 - 6.81 (m, 2H), 6.75 (d, *J*=1.6 Hz, 1H), 6.02 (s, 2H), 4.39 (d, *J*=12.4 Hz, 1H), 4.16 (d, *J*=13.6 Hz, 1H), 3.65 - 3.38 (m, 3H), 2.29 - 2.16 (m, 1H), 1.99 - 1.88 (m, 1H), 1.82 - 1.60 (m, 2H). MS-ESI calculated for [M+H]⁺ 450, found 450.

### Example 38

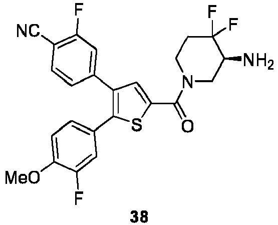

### Synthetic route:

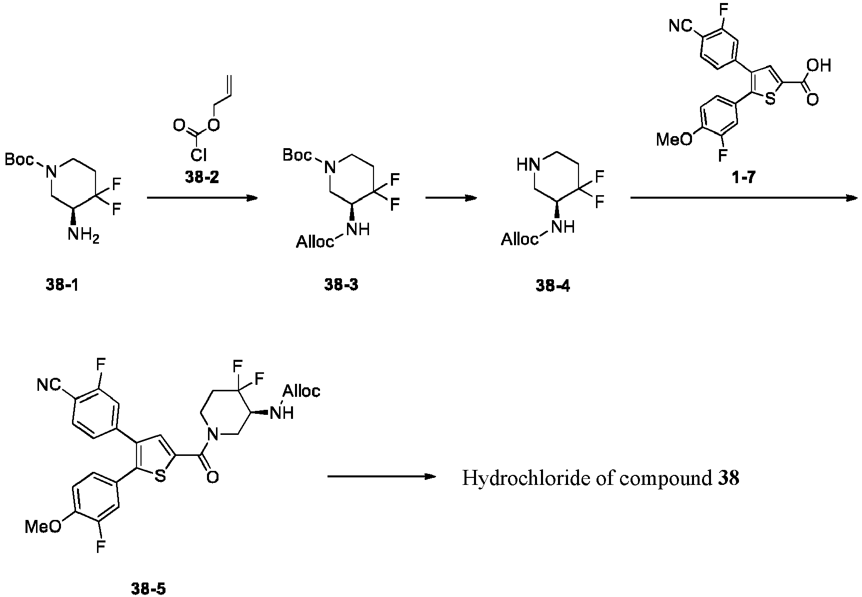

### Step 1

Under nitrogen atmosphere, compound **38-1** (100 mg, 0.423 mmol) was dissolved in anhydrous tetrahydrofuran (10.0 mL), and then sodium bicarbonate (178 mg, 2.12 mmol) and allyl chloroformate **(38-2)** (56.1 mg, 0.466 mmol) were added thereto, and the reaction mixture was stirred for 12 hours at 25°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (2/1, petroleum ether/ethyl acetate, R_{f} = 0.70) to obtain compound **38-3.** ¹H NMR (400MHz, CDCl₃) δ 6.01 - 5.88 (m, 1H), 5.38 - 5.22 (m, 2H), 5.01 - 4.91 (m, 1H), 4.52 - 4.54 (m, 2H), 4.20 - 4.00 (m, 2H), 3.98 - 3.90 (m, 1H), 3.20 - 3.10 (m, 1H), 3.06 - 2.94 (m, 1H), 2.19 - 2.08 (m, 1H), 2.02 - 1.86 (m, 1H), 1.48 (s, 9H).

### Step 2

Compound **38-3** (125 mg, 0.390 mmol) was dissolved in ethyl acetate (5.00 mL), and hydrochloride ethyl acetate solution (4 mol/L, 5.00 mL) was added dropwise thereto at 25°C. The reaction mixture was stirred for 2 hours at 25°C and concentrated under reduced pressure to obtain crude compound **38-4.** ¹H NMR (400MHz, CD₃OD) δ 6.00 - 5.89 (m, 1H), 5.36 - 5.29 (m, 1H), 5.21 (dd, J=1.6, 10.4 Hz, 1H), 4.63 - 4.55 (m, 2H), 4.42 - 4.28 (m, 1H), 3.58 - 3.49 (m, 2H), 3.27 - 3.16 (m, 1H), 3.11 (t, J=12.4 Hz, 1H), 2.56 - 2.44 (m, 1H), 2.38 - 2.18 (m, 1H).

### Step 3

Under nitrogen atmosphere, compound **1-7** (140 mg, 0.377 mmol) was dissolved in anhydrous *N*,*N-*dimethylformamide (5.00 mL), and then triethylamine (191 mg, 1.88 mmol), compound **38-4** (99.6 mg, 0.452 mmol), and 2-(7-azabenzotriazol-1-yl)-*N,N,N',N'-*tetramethyluronium hexafluorophosphate (215 mg, 0.565 mmol) were added thereto. The reaction mixture was stirred for 12 hours at 25°C. The reaction mixture was added with water (20 mL), and extracted with dichloromethane (20 mL × 1). The organic phase was washed with saturated brine (20 mL × 1), dried over anhydrous sodium sulfate, filtered, and concentrated under reduced pressure, and the residue was purified by silica gel column chromatography (1/1, petroleum ether/ethyl acetate, R_{f} = 0.51) to obtain compound **38-5.** MS-ESI calculated for [M+H]⁺ 574, found 574.

### Step 4

Under nitrogen atmosphere, compound **38-5** (130 mg, 0.227 mmol) was dissolved in anhydrous tetrahydrofuran (5 mL), and then ethylenediamine (111 mg, 1.52 mmol) and tetrakis(triphenylphosphine)palladium (26.2 mg, 0.227 mmol) were added thereto, and the reaction mixture was stirred for 12 hours at 65°C, and concentrated under reduced pressure. The residue was purified by silica gel column chromatography (10/1, dichloromethane/methanol, R_{f} = 0.41), and then separated by preparative high performance liquid chromatography (chromatographic column: Phenomenex Genimi NX C18 150 * 40 mm * 5 µm; mobile phase: mobile phase A: ammonia aqueous solution with a volume fraction of 0.04% and 10 mmol/L sodium bicarbonate aqueous solution; mobile phase B: acetonitrile; B%: 35% to 65%, 10 min) to obtain the compound, which was added with hydrochloric acid aqueous solution (4 mol/L, 2 mL) to obtain the hydrochloride of compound **38.** ¹H NMR (400MHz, CD₃OD) δ 7.74 - 7.67 (m, 1H), 7.63 (s, 1H), 7.35 (dd, *J*=1.2, 10.4 Hz, 1H), 7.26 (dd, *J*=1.6, 8.0 Hz, 1H), 7.16 - 7.03 (m, 3H), 4.70 - 4.62 (m, 1H), 4.50 - 4.42 (m, 1H), 4.00 - 3.91 (m, 1H), 3.90 (s, 3H), 3.63 - 3.55 (m, 1H), 3.53 - 3.45 (m, 1H), 2.42 - 2.22 (m, 2H). MS-ESI calculated for [M+H]⁺ 490, found 490.

### Example 39

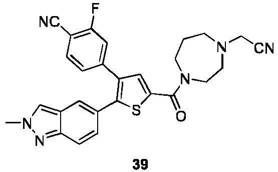

### Synthetic route:

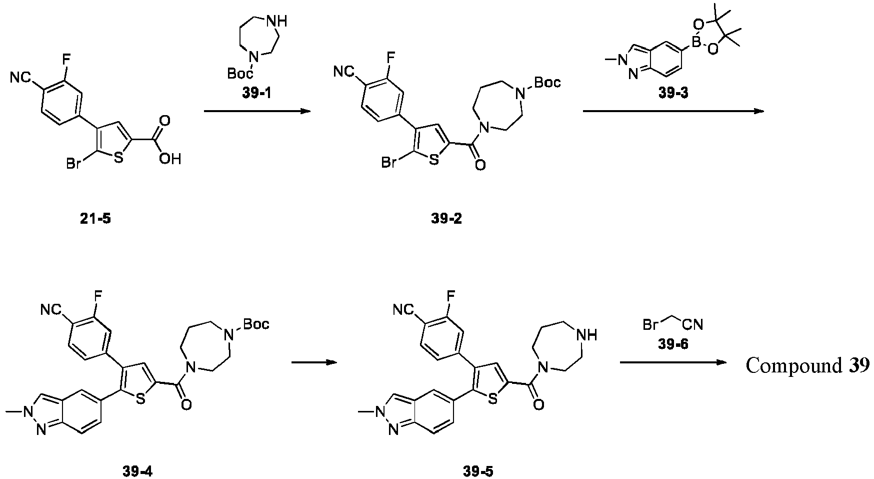

### Step 1

Under nitrogen atmosphere, compound **21-5** (160 mg, 0.491 mmol) was dissolved in anhydrous dichloromethane (10.0 mL), and then triethylamine (149 mg, 1.47 mmol), 1-boc-hexahydro-1,4-diazepine (118 mg, 0.589 mmol), and 2-(7-azabenzotriazol-1-yl)-*N,N,N',N'-*tetramethyluronium hexafluorophosphate (280 mg, 0.736 mmol) were added thereto. The reaction mixture was stirred for 2 hours at 25°C and concentrated under reduced pressure. The residue was purified by silica gel column chromatography (1/1, petroleum ether/ethyl acetate, Rf= 0.34) to obtain compound **39-2.** MS-ESI calculated for [M+H]⁺ 509, found 509.

### Step 2

Under nitrogen atmosphere, compound **39-2** (180 mg, 0.354 mmol) was dissolved in 1,4-dioxane (10.0 mL) and water (2.00 mL), and the reaction mixture was added with 2-methyl-5-(tetramethyl-1,3,2-dioxaborolan-2-yl)-2H-indazole (91.4 mg, 0.354 mmol), potassium phosphate (225 mg, 1.06 mmol), and [1,1'-bis(diphenylphosphino)ferrocene]dichloropalladium(II) (25.9 mg, 0.035 mmol), stirred for 12 hours at 100°C, added with water (20 mL), and extracted with ethyl acetate (20 mL × 1). The organic phase was washed with saturated brine (20 mL × 3), dried over anhydrous sodium sulfate, filtered, and concentrated under reduced pressure. The residue was purified by silica gel column chromatography (1/1, petroleum ether/ethyl acetate, R_{f} = 0.07) to obtain compound **39-4.** MS-ESI calculated for [M+H]⁺ 560, found 560.

### Step 3

Compound **39-4** (110 mg, 0.197 mmol) was dissolved in ethyl acetate (5.00 mL), and hydrochloride ethyl acetate solution (4 mol/L, 5.00 mL) was added dropwise thereto at 25°C. The reaction mixture was stirred for 12 hours at 25°C and concentrated under reduced pressure to obtain compound **39-5.** MS-ESI calculated for [M+H]⁺ 460, found 460.

### Step 4

Under nitrogen atmosphere, compound **39-5** (80.0 mg, 0.161 mmol) was dissolved in anhydrous *N*,*N-*dimethylformamide (3.00 mL), and then potassium carbonate (66.9 mg, 0.484 mmol) and bromoacetonitrile (29.0 mg, 0.242 mmol) were added thereto, and the reaction mixture was stirred for 12 hours at 25°C, filtered, and the residue was separated by preparative high performance liquid chromatography (chromatographic column: Phenomenex Genimi NX C18 150 * 40 mm * 5 µm; mobile phase: mobile phase A: ammonia aqueous solution with a volume fraction of 0.04% and 10 mmol/L sodium bicarbonate aqueous solution; mobile phase B: acetonitrile; B%: 30 to 60%, 10 minutes) to obtain compound **39.** ¹H NMR (400MHz, CD₃OD) δ 8.40 (s, 1H), 7.87 - 7.77 (m, 2H), 7.73 - 7.62 (m, 1H), 7.61 - 7.52 (m, 2H), 7.27 - 7.21 (m, 1H), 7.05 - 6.99 (m, 1H), 4.18 (s, 3H), 4.01 - 3.85 (br s, 2H), 3.81 (s, 2H), 3.76 - 3.58 (m, 2H), 2.92 - 2.75 (m, 2H), 2.72 - 2.64 (m, 2H), 2.07 - 1.71 (m, 2H). MS-ESI calculated for [M+H]⁺ 499, found 499.

### Example 40

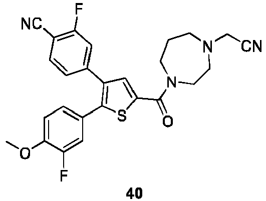

### Synthetic route:

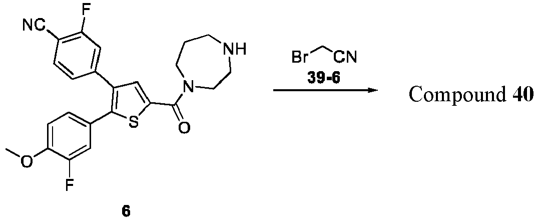

Under nitrogen atmosphere, compound **6** (50.0 mg, 0.102 mmol) was dissolved in anhydrous *N*,*N-*dimethylformamide (3.00 mL), and then potassium carbonate (42.3 mg, 0.306 mmol) and bromoacetonitrile (13.5 mg, 0.112 mmol) were added thereto, and the reaction mixture was stirred for 12 hours at 25°C, filtered, and the residue was separated by preparative high performance liquid chromatography (chromatographic column: Phenomenex Genimi NX C18 150 * 40 mm * 5 µm; mobile phase: mobile phase A: 10 mmol/L sodium bicarbonate aqueous solution; mobile phase B: acetonitrile; B%: 43 to 73%, 9 minutes) to obtain compound **40.** ¹H NMR (400MHz, CD₃OD) δ 7.72 - 7.64 (m, 1H), 7.58 - 7.49 (m, 1H), 7.30 (dd, *J*=1.6, 10.4 Hz, 1H), 7.24 (dd, *J*=1.6, 8.0 Hz, 1H), 7.13 - 7.00 (m, 3H), 4.04 - 3.90 (m, 2H), 3.89 (s, 3H), 3.88 - 3.76 (m, 2H), 3.75 (s, 2H), 2.96 - 2.89 (m, 2H), 2.85 - 2.74 (m, 2H), 2.09 - 1.95 (m, 2H). MS-ESI calculated for [M+H]⁺ 493, found 493.

### Example 41

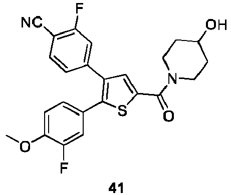

### Synthetic route:

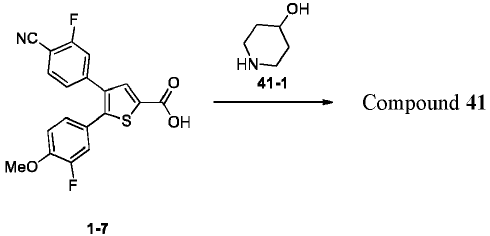

Under nitrogen atmosphere, compound **1-7** (50.0 mg, 0.135 mmol) was dissolved in anhydrous *N,N*-dimethylformamide (3.00 mL), and then triethylamine (40.9 mg, 0.404 mmol), 4-hydroxypiperidine **(41-1)** (16.3 mg, 0.162 mmol), and 2-(7-azabenzotriazol-1-yl)-*N,N,N',N'-*tetramethyluronium hexafluorophosphate (76.8 mg, 0.202 mmol) were added thereto. The reaction mixture was stirred for 12 hours at 25°C, and filtered. The residue was separated by preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 35 to 65%, 10 minutes) to obtain compound **41.** ¹H NMR (400MHz, CD₃OD) δ 7.72 - 7.65 (m, 1H), 7.49 (s, 1H), 7.31 (dd, *J*=1.6, 10.4 Hz, 1H), 7.25 (dd, J=1.6, 8.0 Hz, 1H), 7.14 - 7.02 (m, 3H), 4.21 - 4.10 (m, 2H), 3.98 - 3.91 (m, 1H), 3.90 (s, 3H), 3.57 - 3.41 (m, 2H), 2.01 - 1.89 (m, 2H), 1.64 - 1.50 (m, 2H). MS-ESI calculated for [M+H]⁺ 455, found 455.

### Example 42

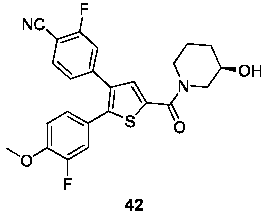

### Synthetic route:

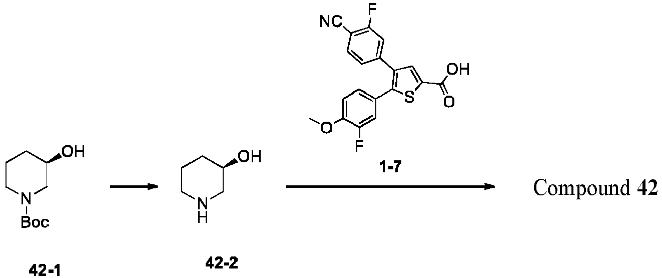

### Step 1

Compound **42-1** (1.00 g, 0.497 mmol) was dissolved in ethyl acetate (10.0 mL), and hydrochloride ethyl acetate solution (4 mol/L, 10 mL) was added dropwise thereto at 25°C. The reaction mixture was stirred for 12 hours at 25°C and concentrated under reduced pressure to obtain crude compound **42-2.** ¹H NMR (400MHz, CDOD₃) δ 4.10 - 3.94 (m, 1H), 3.23 - 3.00 (m, 4H), 2.16 - 2.03 (m, 1H), 1.93 - 1.80 (m, 1H), 1.79 - 1.65 (m, 2H).

### Step 2

Under nitrogen atmosphere, compound **1-7** (50.0 mg, 0.135 mmol) was dissolved in anhydrous *N,N-*dimethylformamide (3.00 mL), and then triethylamine (40.9 mg, 0.404 mmol), compound **42-2** (22.2 mg, 0.162 mmol), and 2-(7-azabenzotriazol-1-yl)-*N,N,N',N'-*tetramethyluronium hexafluorophosphate (76.8 mg, 0.202 mmol) were added thereto. The reaction mixture was stirred for 12 hours at 25°C, and filtered. The residue was separated by preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 35 to 65%, 10 minutes) to obtain compound **42.** ¹H NMR (400MHz, CD₃OD) δ 7.70 - 7.64 (m, 1H), 7.52 (s, 1H), 7.29 (dd, *J*=1.6, 10.4 Hz, 1H), 7.24 (dd, *J*=1.6, 8.0 Hz, 1H), 7.12 - 7.00 (m, 3H), 4.09 - 3.94 (m, 1H), 3.89 (s, 3H), 3.87 - 3.73 (m, 2H), 3.65 - 3.34 (m, 2H), 2.03 - 1.85 (m, 2H), 1.72 - 1.53 (m, 2H). MS-ESI calculated for [M+H]⁺ 455, found 455.

### Example 43

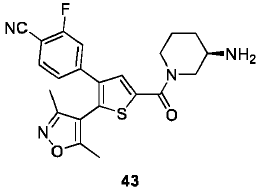

### Synthetic route:

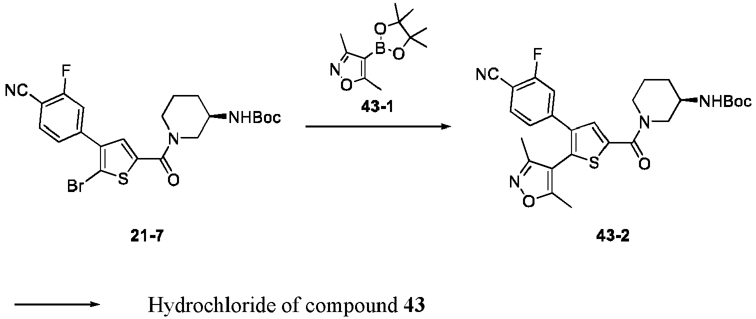

### Step 1

Under nitrogen atmosphere, compound **21-7** (190 mg, 374 µmol), compound **43-1** (100 mg, 448 µmol), and sodium carbonate (79.2 mg, 747 µmol) were dissolved in dioxane (8.00 mL) and water (2.00 mL), and the reaction mixture was added with [1,1'-bis(diphenylphosphino)ferrocene]dichloropalladium(II) (27.3 mg, 37.4 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.55) to obtain compound **43-2.** MS-ESI calculated for [M+H]⁺ 525, found 525.

### Step 2

Compound **43-2** (140 mg, 267 µmol) was dissolved in ethyl acetate (20.0 mL), and hydrochloride ethyl acetate solution (4 mol/L, 667 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **43.** ¹H NMR (400MHz, CD₃OD) δ = 7.81 - 7.74 (m, 1H), 7.71 (s, 1H), 7.41 (dd, *J*=1.2, 10.3 Hz, 1H), 7.29 (dd, *J*=1.6, 8.0 Hz, 1H), 4.39 (d, *J*=10.4 Hz, 1H), 4.16 (d, *J*=12.8 Hz, 1H), 3.67 - 3.39 (m, 3H), 2.25 (s, 3H), 2.23 - 2.15 (m, 1H), 1.99 (s, 3H), 1.94 - 1.86 (m, 1H), 1.84 - 1.71 (m, 2H). MS-ESI calculated for [M+H]⁺ 425, found 425.

### Example 44

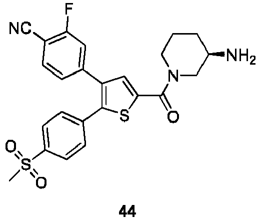

### Synthetic route:

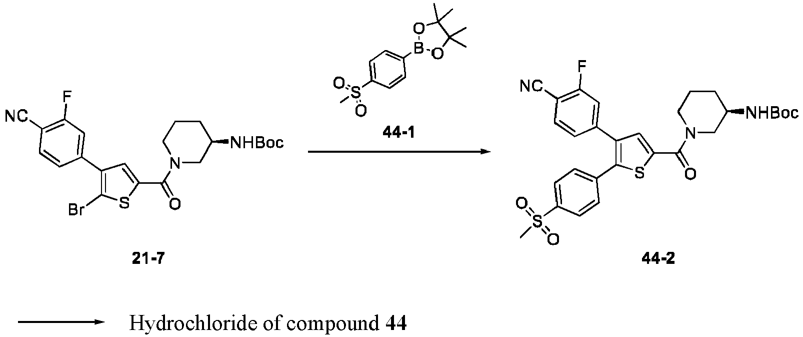

### Step 1

Under nitrogen atmosphere, compound **21-7** (75.1 mg, 148 µmol), compound **44-1** (50.0 mg, 177 µmol), and sodium carbonate (31.3 mg, 295 µmol) were dissolved in dioxane (8.00 mL) and water (2.00 mL), and the reaction mixture was added with [1,1'-bis(diphenylphosphino)ferrocene]dichloropalladium(II) (10.8 mg, 14.8 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.25) to obtain compound **44-2.** MS-ESI calculated for [M+H]⁺ 584, found 584.

### Step 2

Compound **44-2** (75.0 mg, 128 µmol) was dissolved in ethyl acetate (10.0 mL), and hydrochloride ethyl acetate solution (4 mol/L, 321 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **44.** ¹H NMR (400MHz, CD₃OD) δ = 7.98 (d, *J*=8.4 Hz, 2H), 7.73 (t, *J*=7.4 Hz, 1H), 7.67 - 7.54 (m, 3H), 7.40 (d, *J*=10.4 Hz, 1H), 7.26 (d, *J*=8.0 Hz, 1H), 4.40 (d, *J*=12.4 Hz, 1H), 4.16 (d, *J*=13.2 Hz, 1H), 3.67 - 3.42 (m, 3H), 3.17 (s, 3H), 2.28 - 2.15 (m, 1H), 1.99 - 1.88 (m, 1H), 1.83 - 1.69 (m, 2H). MS-ESI calculated for [M+H]⁺ 484, found 484.

### Example 45

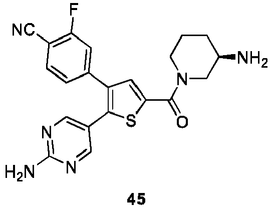

### Synthetic route:

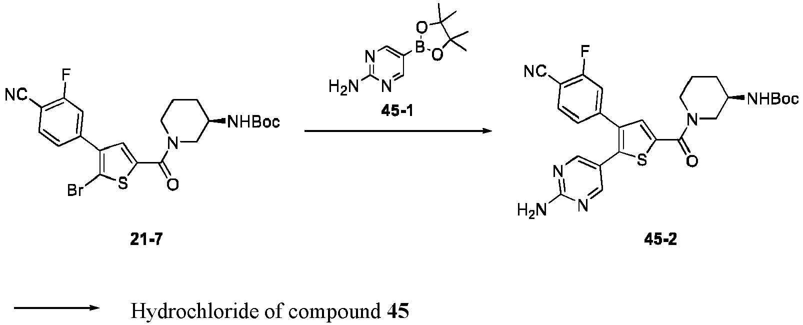

### Step 1

Under nitrogen atmosphere, compound **21-7** (100 mg, 197 µmol), compound **45-1** (52.2 mg, 236 µmol), and sodium carbonate (41.7 mg, 393 µmol) were dissolved in dioxane (8.00 mL) and water (2.00 mL), and the reaction mixture was added with [1,1'-bis(diphenylphosphino)ferrocene]dichloropalladium(II) (14.4 mg, 19.7 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.07) to obtain compound **45-2.** MS-ESI calculated for [M+Na]⁺ 545, found 545.

### Step 2

Compound **45-2** (54.0 mg, 103 µmol) was dissolved in ethyl acetate (10.00 mL), and hydrochloride ethyl acetate solution (4 mol/L, 258 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 1% to 30%, 10 minutes) to obtain the hydrochloride of compound **45.** ¹H NMR (400MHz, CD₃OD) δ = 8.51 (s, 2H), 7.81 (t, *J*=8.0 Hz, 1H), 7.66 (s, 1H), 7.55 (d, *J*=8.0 Hz, 1H), 7.42 (d, *J*=8.0 Hz, 1H), 4.43 (d, *J*=12.0 Hz, 1H), 4.20 - 4.09 (m, 1H), 3.58 - 3.41 (m, 3H), 2.28 - 2.17 (m, 1H), 1.97 - 1.89 (m, 1H), 1.85 - 1.72 (m, 2H). MS-ESI calculated for [M+H]⁺ 423, found 423.

### Example 46

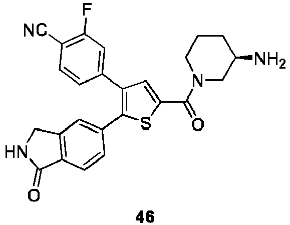

### Synthetic route:

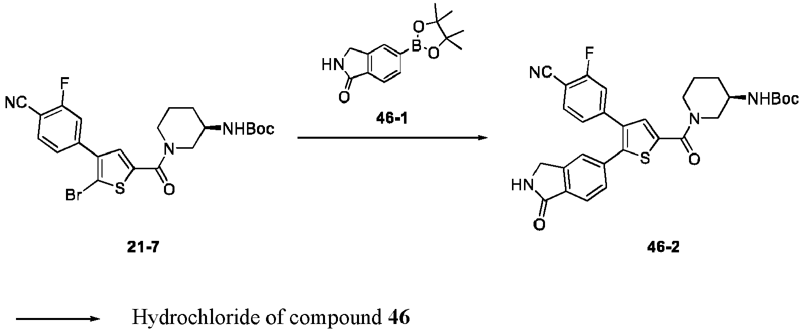

### Step 1

Under nitrogen atmosphere, compound **21-7** (100 mg, 197 µmol), compound **46-1** (61.2 mg, 236 µmol), and sodium carbonate (41.7 mg, 393 µmol) were dissolved in dioxane (4.00 mL) and water (1.00 mL), and the reaction mixture was added with [1,1'-bis(diphenylphosphino)ferrocene]dichloropalladium(II) (14.4 mg, 19.7 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.06) to obtain compound **46-2.** MS-ESI calculated for [M+H]⁺ 561, found 561.

### Step 2

Compound **46-2** (50.0 mg, 89.2 µmol) was dissolved in ethyl acetate (5.00 mL), and hydrochloride ethyl acetate solution (4 mol/L, 223 µL) was added thereto, and the reaction mixture was stirred and reacted for 12 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 5% to 35%, 10 minutes) to obtain the hydrochloride of compound **46.** ¹H NMR (400MHz, CD₃OD) δ = 7.78 (d, *J*=8.0 Hz, 1H), 7.68 (t, *J*=8.0 Hz, 1H), 7.62 (s, 1H), 7.58 (s, 1H), 7.44 (d, *J*=8.0 Hz, 1H), 7.36 (d, *J*=8.0 Hz, 1H), 7.23 (d, *J*=8.0 Hz, 1H), 4.45 (s, 2H), 4.38 (d, *J*=12.0 Hz, 1H), 4.15 (d, *J*=12.0 Hz, 1H), 3.59 - 3.38 (m, 3H), 2.24 - 2.17 (m, 1H), 1.94 - 1.86 (m, 1H), 1.81 - 1.70 (m, 2H). MS-ESI calculated for [M+H]⁺ 461, found 461.

### Example 47

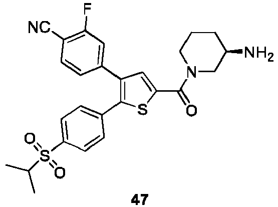

### Synthetic route:

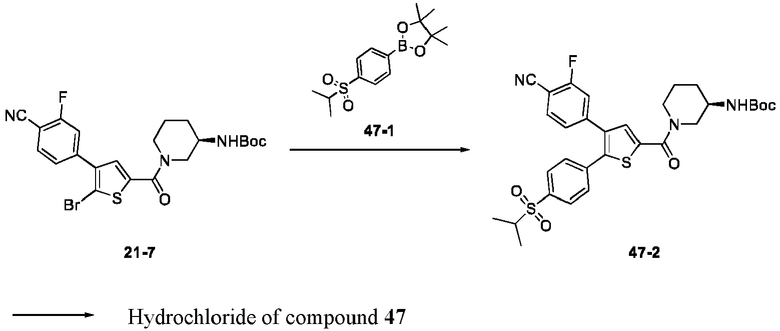

### Step 1

Under nitrogen atmosphere, compound **21-7** (163 mg, 322 µmol), compound **47-1** (100 mg, 322 µmol), and sodium carbonate (68.3 mg, 644 µmol) were dissolved in dioxane (32.0 mL) and water (4.00 mL), and the reaction mixture was added with [1,1'-bis(diphenylphosphino)ferrocene]dichloropalladium(II) (23.6 mg, 32.2 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.45) to obtain compound **47-2.** MS-ESI calculated for [M+H]⁺ 612, found 612.

### Step 2

Compound **47-2** (140 mg, 229 µmol) was dissolved in ethyl acetate (20.0 mL), and hydrochloride ethyl acetate solution (4 mol/L, 572 µL) was added thereto, and the reaction mixture was stirred and reacted for 24 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **47.** ¹H NMR (400MHz, CD₃OD) δ = 7.91 (d, *J*=8.4 Hz, 2H), 7.73 (t, *J*=7.6 Hz, 1H), 7.67 - 7.57 (m, 3H), 7.37 (d, *J*=10.4 Hz, 1H), 7.27 (dd, *J*=1.2, 8.0 Hz, 1H), 4.40 (d, *J*=11.2 Hz, 1H), 4.16 (d, *J*=13.2 Hz, 1H), 3.60 - 3.36 (m, 4H), 2.26 - 2.13 (m, 1H), 1.97 - 1.91 (m, 1H), 1.85 - 1.69 (m, 2H), 1.29 (s, 3H), 1.28 (s, 3H). MS-ESI calculated for [M+H]⁺ 512, found 512.

### Example 48

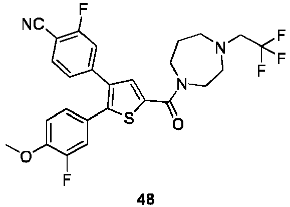

### Synthetic route:

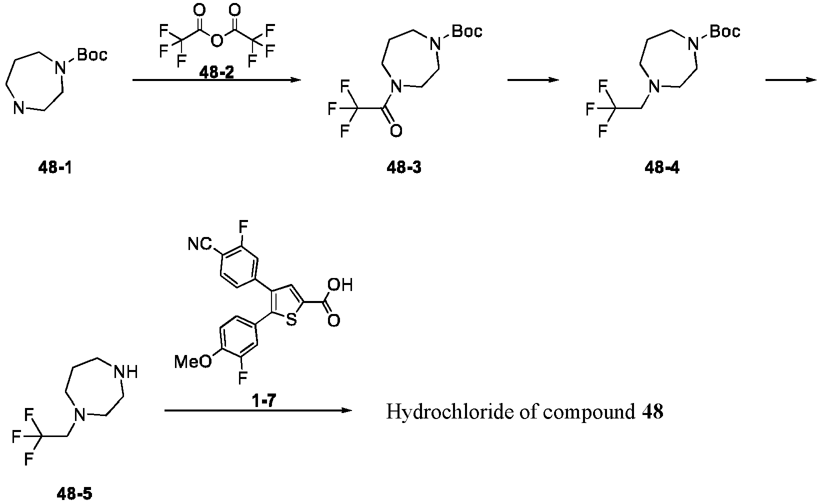

### Step 1

Under nitrogen atmosphere, compound **48-1** (500 mg, 2.50 mmol) was dissolved in anhydrous dichloromethane (10.0 mL), and triethylamine (379 mg, 2.12 mmol) was added thereto. Trifluoromethanesulfonic anhydride **(48-2)** (629 mg, 3.00 mmol) was added dropwise thereto at 0°C, and the reaction mixture was stirred at 25°C for 12 hours, added with water (20 mL), and extracted with dichloromethane (20 mL × 1). The organic phase was washed with saturated brine (20 mL × 1), dried over anhydrous sodium sulfate, filtered, and concentrated under reduced pressure, and the residue was purified by silica gel column chromatography (4/1, petroleum ether/ethyl acetate, R_{f} = 0.65) to obtain compound **48-3.** ¹H NMR (400MHz, CDCl₃) δ 3.76 - 3.53 (m, 6H), 3.52 - 3.36 (m, 2H), 2.00 - 1.89 (m, 2H), 1.47 (s, 9H).

### Step 2

Under nitrogen atmosphere, compound **48-3** (250 mg, 0.844 mmol) was dissolved in anhydrous tetrahydrofuran (10.0 mL), and borane-dimethylsulfide (10 mol/L, 422 µL) was added dropwise thereto at 0°C. The reaction mixture was stirred at 65°C for 12 hours, cooled to 0°C, added with methanol (2 mL), stirred at 65°C for 1 hour, and then concentrated under reduced pressure. The residue was purified by silica gel column chromatography (4/1, petroleum ether/ethyl acetate, R_{f} = 0.30) to obtain compound **48-4.** ¹H NMR (400MHz, CDCl₃) δ 3.53 - 3.38 (m, 4H), 3.14 (q, J=9.2 Hz, 2H), 2.98 - 2.83 (m, 4H), 1.88 - 1.74 (m, 2H), 1.47 (s, 9H).

### Step 3

Compound **48-4** (200 mg, 0.708 mmol) was dissolved in ethyl acetate (5.00 mL), and hydrochloride ethyl acetate solution (4 mol/L, 5 mL) was added dropwise thereto at 25°C. The reaction mixture was stirred for 12 hours at 25°C and concentrated under reduced pressure to obtain crude compound **48-5.** ¹H NMR (400MHz, CDOD₃) δ 3.70 (q, J=9.2 Hz, 2H), 3.45 - 3.41 (m, 1H), 3.42 (s, 3H), 3.39 - 3.34 (m, 2H), 3.27 (t, J=6.0 Hz, 2H), 2.17 - 2.10 (m, 2H).

### Step 4

Under nitrogen atmosphere, compound **1-7** (45 mg, 0.121 mmol) was dissolved in anhydrous *N*,*N-*dimethylformamide (2 mL), and then triethylamine (36.8 mg, 0.364 mmol), compound **48-5** (31.8 mg, 0.145 mmol), and 2-(7-azabenzotriazol-1-yl)-*N,N,N',N'-*tetramethyluronium hexafluorophosphate (69.1 mg, 0.182 mmol) were added thereto. The reaction mixture was stirred for 12 hours at 25°C, and concentrated under reduced pressure. The residue was separated by preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 50% to 80%, 10 minutes) to obtain the hydrochloride of compound **48.** ¹H NMR (400MHz, CD₃OD) δ 7.71 - 7.64 (m, 1H), 7.57 -7.49 (m, 1H), 7.33 - 7.27 (m, 1H), 7.26 - 7.22 (m, 1H), 7.14 - 7.00 (m, 3H), 4.04 - 3.92 (m, 2H), 3.89 (s, 3H), 3.87 - 3.71 (m, 2H), 3.54 - 3.35 (m, 2H), 3.20 - 3.12 (m, 2H), 3.10 - 2.97 (m, 2H), 2.09 - 1.94 (m, 2H). MS-ESI calculated for [M+H]⁺ 536, found 536.

### Example 49

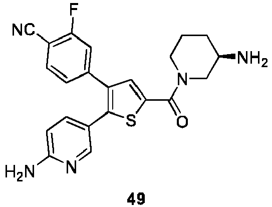

### Synthetic route:

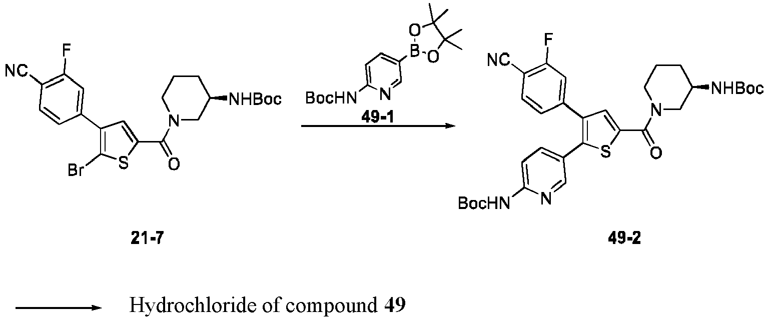

### Step 1

Under nitrogen atmosphere, compound **21-7** (92.6 mg, 182 µmol), compound **49-1** (70.0 mg, 219 µmol), and sodium carbonate (38.6 mg, 364 µmol) were dissolved in dioxane (16.0 mL) and water (4.00 mL), and the reaction mixture was added with [1,1'-bis(diphenylphosphino)ferrocene]dichloropalladium(II) (13.3 mg, 18.2 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.50) to obtain compound **49-2.** MS-ESI calculated for [M+H]⁺ 622, found 622.

### Step 2

Compound **49-2** (100 mg, 161 µmol) was dissolved in ethyl acetate (20.0 mL), and hydrochloride ethyl acetate solution (4 mol/L, 402 µL) was added thereto, and the reaction mixture was stirred and reacted for 12 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 1% to 30%, 10 minutes) to obtain the hydrochloride of compound **49.** ¹H NMR (400MHz, CD₃OD) δ = 7.95 (s, 1H), 7.85 - 7.71 (m, 2H), 7.64 (s, 1H), 7.50 (d, *J*=10.0 Hz, 1H), 7.39 (d, *J*=7.6 Hz, 1H), 7.04 (d, *J*=9.2 Hz, 1H), 4.43 (d, *J*=11.6 Hz, 1H), 4.16 (d, *J*=9.6 Hz, 1H), 3.61 - 3.40 (m, 3H), 2.31 - 2.13 (m, 1H), 2.01 - 1.88 (m, 1H), 1.87 - 1.64 (m, 2H). MS-ESI calculated for [M+H]⁺ 422, found 422.

### Example 50

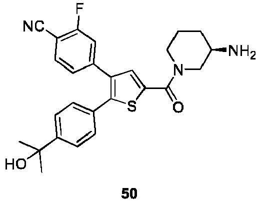

### Synthetic route:

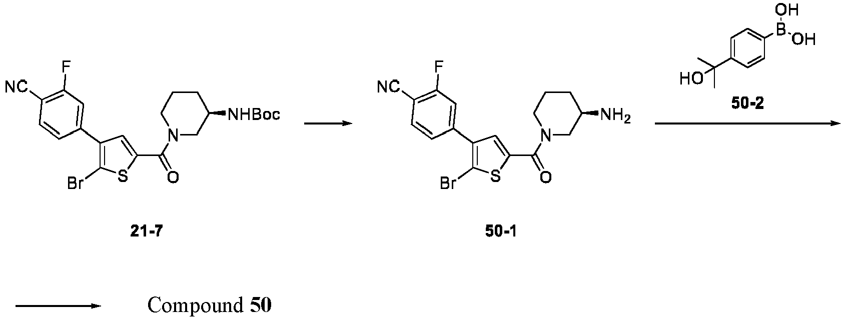

### Step 1

Compound **21-7** (500 mg, 983 µmol) was dissolved in ethyl acetate (30.0 mL), and hydrochloride ethyl acetate solution (4 mol/L, 2.46 mL) was added thereto, and the reaction mixture was stirred and reacted for 12 hours at 25°C. The reaction mixture was concentrated to obtain crude compound **50-1,** which was directly used in the next reaction step. MS-ESI calculated for [M+H]⁺ 410, found 410.

### Step 2

Under nitrogen atmosphere, compound **50-1** (50.0 mg, 122 µmol), compound **50-2** (26.5 mg, 147 µmol), and sodium carbonate (26.0 mg, 245 µmol) were dissolved in dioxane (8.00 mL) and water (2.00 mL), and the reaction mixture was added with [1,1'-bis(diphenylphosphino)ferrocene]dichloropalladium(II) (8.96 mg, 12.3 µmol), and the reaction mixture was stirred and reacted for 12 hours at 100°C. The reaction mixture was concentrated under reduced pressure, and the residue was purified by preparative high performance liquid chromatography (chromatographic column: Phenomenex Genimi NX C18 150 * 40 mm * 5 µm, mobile phase A: ammonia aqueous solution with a volume fraction of 0.05%; mobile phase B: 10 mM ammonium bicarbonate solution in acetonitrile; B%: 28% to 58%, 10 minutes) to obtain compound **50.** ¹H NMR (400MHz, CD₃OD) δ = 7.67 (t, *J*=7.6 Hz, 1H), 7.58 - 7.48 (m, 3H), 7.36 - 7.22 (m, 4H), 4.59 (br, 1H), 4.40 - 4.32 (m, 1H), 4.28 -4.20 (m, 1H), 3.09 - 2.82 (m, 2H), 2.14 -2.00 (m, 1H), 1.94 - 1.79 (m, 1H), 1.69 - 1.57 (m, 1H), 1.54 (s, 6H), 1.50 - 1.40 (m, 1H). MS-ESI calculated for [M+H]⁺ 464, found 464.

### Example 51

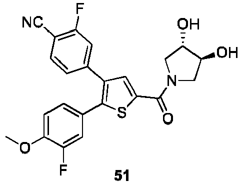

### Synthetic route:

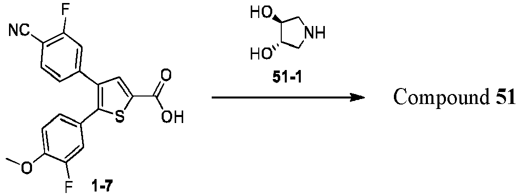

Under nitrogen atmosphere, compound **1-7** (150 mg, 404 µmol), 2-(7-azabenzotriazol-1-yl)-*N,N,N',N*'-tetramethyluronium hexafluorophosphate (230 mg, 606 µmol), and *N,N-*diisopropylethylamine (156 mg, 1.21 mmol) were dissolved in *N*,*N-*dimethylformamide (10.0 mL), and then compound **51-1** (50.0 mg, 485 µmol) was added thereto, and the reaction mixture was stirred and reacted for 12 hours at 25°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by preparative high performance liquid chromatography (chromatographic column: Xtimate C18 150 * 40 mm * 5 µm; mobile phase A: hydrochloric acid aqueous solution with a volume fraction of 0.05%; mobile phase B: acetonitrile; B%: 27% to 57%, 10 minutes) to obtain compound **51.** ¹H NMR (400MHz, CD₃OD) δ = 7.72 - 7.61 (m, 2H), 7.30 (dd, *J*=1.2, 10.0 Hz,, 1H), 7.25 (dd, *J*=1.2, 8.0 Hz, 1H), 7.13 - 7.00 (m, 3H), 4.27 - 4.20 (m, 1H), 4.19 - 4.10 (m, 2H), 3.89 (s, 3H), 3.86 - 3.79 (m, 2H), 3.65 - 3.55 (m, 1H). MS-ESI calculated for [M+H]⁺ 457, found 457.

### Example 52

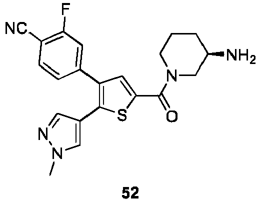

### Synthetic route:

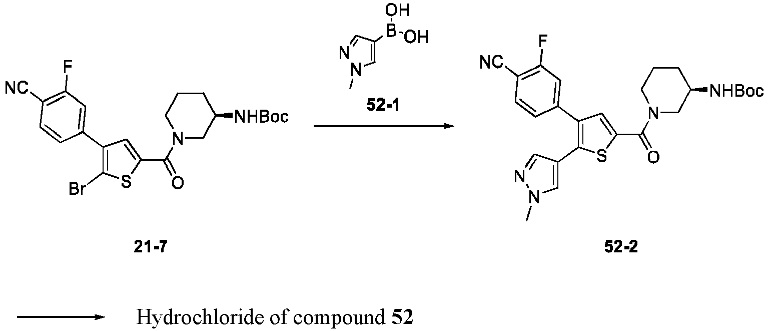

### Step 1

Under nitrogen atmosphere, compound **21-7** (168 mg, 331 µmol), compound **52-1** (50.0 mg, 397 µmol), and sodium carbonate (70.1 mg, 662 µmol) were dissolved in dioxane (32.0 mL) and water (8.00 mL), and the reaction mixture was added with [1,1'-bis(diphenylphosphino)ferrocene]dichloropalladium(II) (24.2 mg, 33.1 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.28) to obtain compound **52-2.** MS-ESI calculated for [M+Na]⁺ 532, found 532.

### Step 2

Compound **52-2** (110 mg, 162 µmol) was dissolved in ethyl acetate (20.0 mL), and hydrochloride ethyl acetate solution (4 mol/L, 401 µL) was added thereto, and the reaction mixture was stirred and reacted for 12 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 7% to 37%, 10 minutes) to obtain the hydrochloride of compound **52.** ¹H NMR (400MHz, CD₃OD) δ = 7.76 (t, *J*=7.6 Hz, 1H), 7.69 (s, 1H), 7.49 (s, 1H), 7.45 - 7.34 (m, 3H), 4.37 (d, *J*=11.2 Hz, 1H), 4.14 (d, *J*=13.6 Hz, 1H), 3.88 (s, 3H), 3.58 - 3.37 (m, 3H), 2.27 - 2.14 (m, 1H), 1.92 - 1.87 (m, 1H), 1.81 - 1.64 (m, 2H). MS-ESI calculated for [M+H]⁺ 410, found 410.

### Example 53

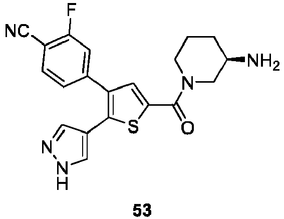

### Synthetic route:

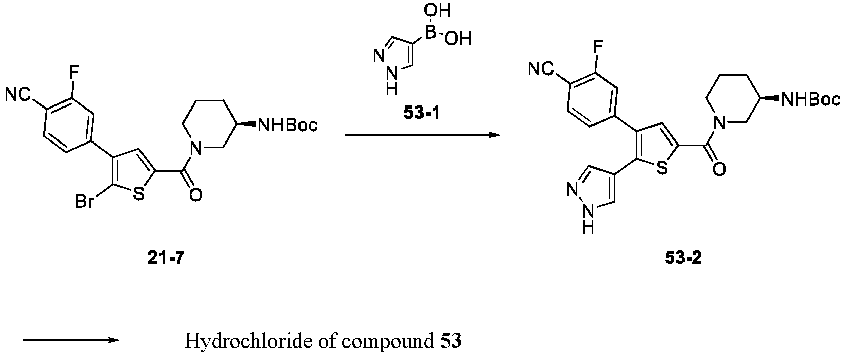

### Step 1

Under nitrogen atmosphere, compound **21-7** (300 mg, 590 µmol), compound **53-1** (79.2 mg, 708 µmol), and sodium carbonate (125 mg, 1.18 mmol) were dissolved in dioxane (32.0 mL) and water (4.00 mL), and the reaction mixture was added with [1,1'-bis(diphenylphosphino)ferrocene]dichloropalladium(II) (43.2 mg, 59.1 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.18) to obtain compound **53-2.** MS-ESI calculated for [M+Na]⁺ 518, found 518.

### Step 2

Compound **53-2** (70.0 mg, 141 µmol) was dissolved in ethyl acetate (15.0 mL), and hydrochloride ethyl acetate solution (4 mol/L, 353 µL) was added thereto, and the reaction mixture was stirred and reacted for 2 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 5% to 35%, 10 minutes) to obtain the hydrochloride of compound **53.** ¹H NMR (400MHz, CD₃OD) δ = 7.76 (t, *J*=7.6 Hz, 1H), 7.64 (s, 2H), 7.51 (s, 1H), 7.45 - 7.34 (m, 2H), 4.38 (d, *J*=11.2 Hz, 1H), 4.23 - 4.07 (m, 1H), 3.59 - 3.36 (m, 3H), 2.26 - 2.14 (m, 1H), 1.96 - 1.85 (m, 1H), 1.81 - 1.63 (m, 2H). MS-ESI calculated for [M+H]⁺ 396, found 396.

### Example 54

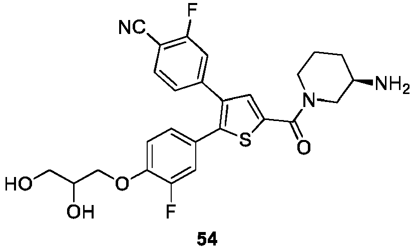

### Synthetic route:

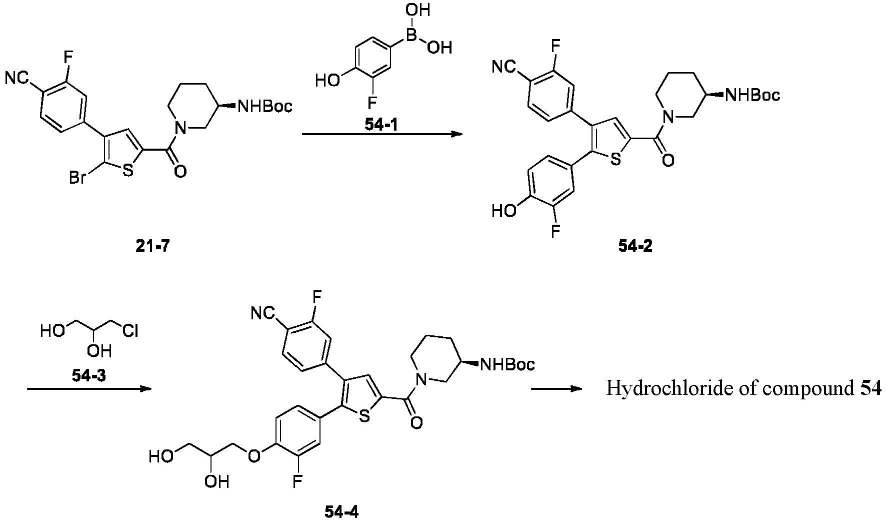

### Step 1

Under nitrogen atmosphere, compound **21-7** (326 mg, 641 µmol), compound **54-1** (120 mg, 770 µmol), and sodium carbonate (136 mg, 1.28 mmol) were dissolved in dioxane (32.00 mL) and water (8.00 mL), and the reaction mixture was added with [1,1'-bis(diphenylphosphino)ferrocene]dichloropalladium(II) (46.9 mg, 64.1 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.25) to obtain compound **54-2.** MS-ESI calculated for [M+Na]⁺ 562, found 562.

### Step 2

Compound **54-2** (50.0 mg, 92.6 µmol) was dissolved in ethanol (10.0 mL), and then 1.00 mL of sodium hydroxide (33.8 mg, 844 µmol) aqueous solution was added thereto. The reaction mixture was stirred and reacted for 2 hours at 78°C and then cooled to 50°C. 10.0 mL of a solution of compound **54-3** (51.2 mg, 463 µmol) in ethanol was added slowly in batches. The reaction mixture was stirred and reacted for another 15 hours at 78°C. The reaction mixture was added with water (50 mL) and extracted with ethyl acetate (50 mL × 2). The organic phase was washed with saturated brine (50 mL × 1), dried over anhydrous sodium sulfate, filtered, and concentrated under reduced pressure to obtain crude compound **54-4,** which was used directly in the next reaction step. MS-ESI calculated for [M+H]⁺ 558, found 558.

### Step 3

Compound **54-4** (65.0 mg, 106 µmol) was dissolved in dichloromethane (10.0 mL), and trifluoroacetic acid (78.4 µL, 1.06 mmol) was added thereto, and the reaction mixture was stirred and reacted for 1 hour at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 50%, 10 minutes) to obtain the hydrochloride of compound **54.** ¹H NMR (400MHz, CD₃OD) δ = 7.69 (t, *J*=7.6 Hz, 1H), 7.56 (s, 1H), 7.33 (d, *J*=10.4 Hz, 1H), 7.26 (d, *J*=8.0 Hz, 1H), 7.17 - 7.10 (t, *J*=8.8 Hz, 1H), 7.09 - 7.00 (m, 2H), 4.39 (d, *J*=12.0 Hz, 1H), 4.17 - 4.10 (m, 2H), 4.09 - 4.03 (m, 1H), 4.02 - 3.97 (m, 1H), 3.72 - 3.63 (m, 2H), 3.57 - 3.37 (m, 3H), 2.26 - 2.16 (m, 1H), 1.98 - 1.86 (m, 1H), 1.84 - 1.66 (m, 2H). MS-ESI calculated for [M+H]⁺ 514, found 514.

### Example 55

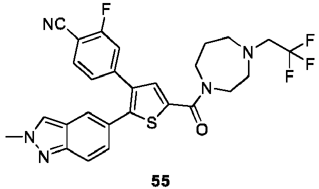

### Synthetic route:

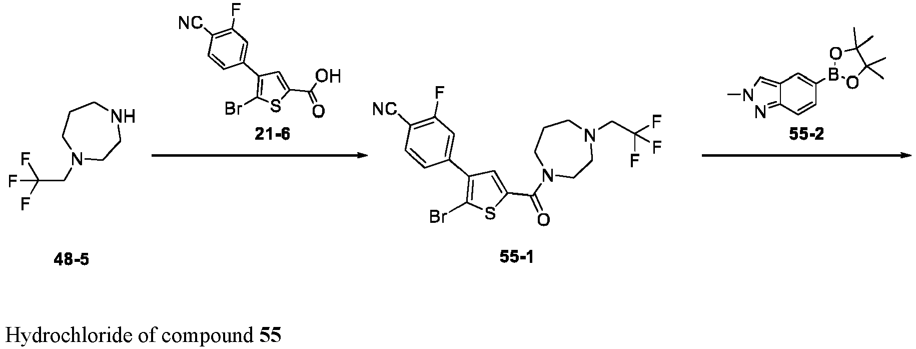

### Step 1

Under nitrogen atmosphere, compound **21-6** (130 mg, 0.399 mmol) was dissolved in anhydrous *N*,*N-*dimethylformamide (2.00 mL), and then triethylamine (121 mg, 1.20 mmol), compound **48-5** (105 mg, 0.478 mmol), and 2-(7-azabenzotriazol-1-yl)-*N,N,N',N'-*tetramethyluronium hexafluorophosphate (227 mg, 0.598 mmol) were added thereto. The reaction mixture was stirred for 12 hours at 25°C. The reaction mixture was added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.20) to obtain compound **55-1.** MS-ESI calculated for [M+H]⁺ 491, found 491.

### Step 2

Under nitrogen atmosphere, compound **55-1** (185 mg, 0.377 mmol), compound **55-2** (97.4 mg, 0.377 mmol), and potassium phosphate (240 mg, 1.13 mmol) were dissolved in dioxane (10.0 mL) and water (2.00 mL), and the reaction mixture was added with [1,1'-bis(diphenylphosphino)ferrocene]dichloropalladium(II) (27.6 mg, 0.0377 mmol), and the reaction mixture was stirred and reacted for 12 hours at 100°C. The reaction mixture was added with water (20 mL), and extracted with ethyl acetate (20 mL × 1). The organic phase was washed with saturated brine (20 mL × 3), dried over anhydrous sodium sulfate, filtered, and concentrated under reduced pressure. The residue was purified by silica gel column chromatography (1/1, petroleum ether/ethyl acetate, Rf = 0.17), then subjected to preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 40% to 70%, 10 minutes) to obtain the hydrochloride of compound **55.** ¹H NMR (400MHz, CD₃OD) δ 8.33 (s, 1H), 7.80 (s, 1H), 7.68 - 7.57 (m, 3H), 7.35 - 7.29 (m, 1H), 7.28 - 7.24 (m, 1H), 7.21 - 7.15 (m, 1H), 4.25 (s, 3H), 4.22 - 3.85 (br s, 6H), 3.64 - 3.38 (m, 4H), 2.40 - 2.17 (br s, 2H). MS-ESI calculated for [M+H]⁺ 542, found 542.

### Example 56

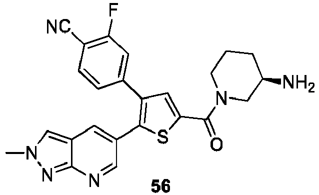

### Synthetic route:

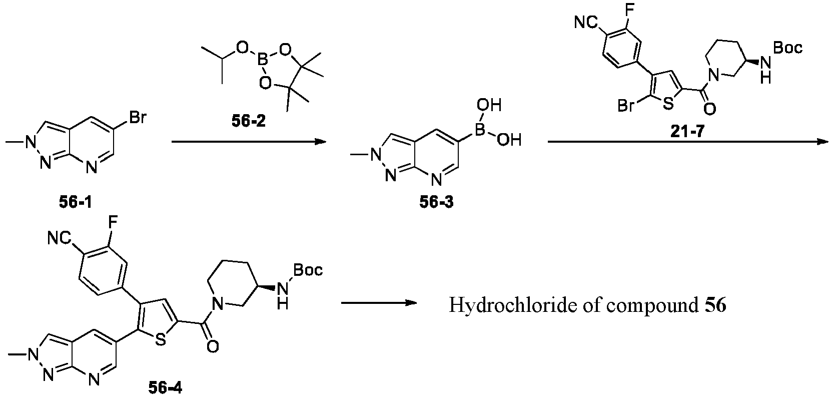

### Step 1

Under nitrogen atmosphere, compound **56-1** (100 mg, 0.472 mmol), compound **56-2** (132 mg, 0.707 mmol) were dissolved in anhydrous tetrahydrofuran (5.00 mL), then n-butyllithium (2.5 mol/L n-hexane solution, 377 µL) was added dropwise thereto at -78°C. The reaction mixture was stirred and reacted for 12 hours at 25°C, added with water (20 mL), and extracted with ethyl acetate (20 mL × 1). The aqueous phase was concentrated under reduced pressure to obtain compound **56-3.** MS-ESI calculated for [M+H]⁺ 178, found 178.

### Step 2

Under nitrogen atmosphere, compound **21-7** (110 mg, 0.216 mmol), compound **56-3** (57.4 mg, 0.325 mmol), and potassium phosphate (138 mg, 0.649 mmol) were dissolved in dioxane (10.0 mL) and water (2.00 mL), and the reaction mixture was added with [1,1'-bis(diphenylphosphino)ferrocene]dichloropalladium(II) (15.8 mg, 0.216 mmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/2, petroleum ether/ethyl acetate, R_{f} = 0.19) to obtain compound **56-4.** MS-ESI calculated for [M+H]⁺ 561, found 561.

### Step 3

Compound **56-4** (115 mg, 0.205 mmol) was dissolved in ethyl acetate (5.00 mL), and hydrochloride ethyl acetate solution (4 mol/L, 5 mL) was added dropwise thereto at 25°C. The reaction mixture was stirred for 12 hours at 25°C. The reaction mixture was concentrated under reduced pressure, and the residue was separated by preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 3% to 33%, 10 minutes) to obtain the hydrochloride of compound **56.** ¹H NMR (400MHz, CD₃OD) δ 8.93 - 8.80 (m, 3H), 7.74 - 7.66 (m, 2H), 7.49 (d, *J*=10.0 Hz, 1H), 7.32 (d, *J*=8.0 Hz, 1H), 4.46 - 4.43 (m, 1H), 4.39 (s, 3H), 4.24 - 4.11 (m, 1H), 3.58 - 3.41 (m, 3H), 2.27 - 2.17 (m, 1H), 1.98 - 1.88 (m, 1H), 1.86 - 1.69 (m, 2H). MS-ESI calculated for [M+H]⁺ 461, found 461.

### Example 57

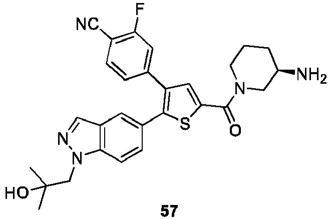

### Synthetic route:

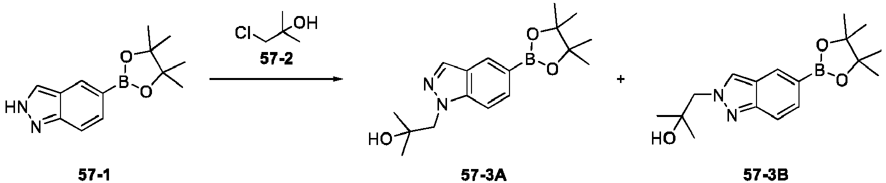

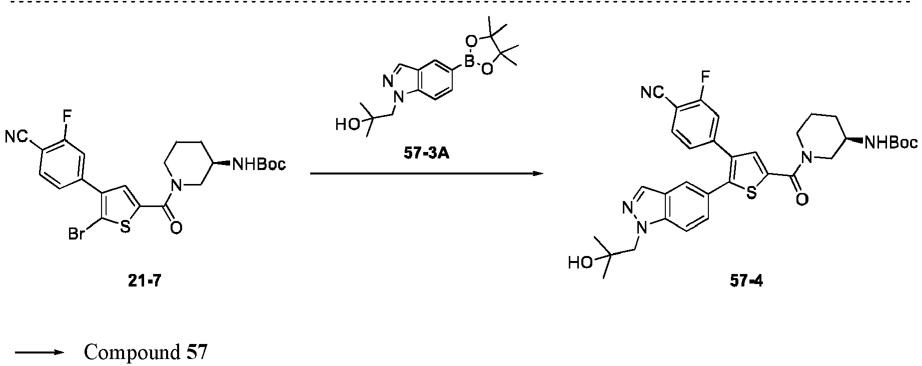

### Step 1

Compounds **57-1** (100 mg, 410 µmol) and **57-2** (210 µL, 2.05 mmol) were dissolved in *N*,*N-*dimethylformamide (2.00 mL), and potassium carbonate (283 mg, 2.05 mmol) was added thereto, and the reaction mixture was stirred and reacted for 48 hours at 100°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, Rf_{P1} = 0.67, Rf_{P2} = 0.47) to obtain compounds **57-3A** and **57-3B.** Compound **57-3A** MS-ESI calculated for [M+H]⁺ 317, found 317; compound **57-3B** MS-ESI calculated for [M+H]⁺ 317, found 317.

### Step 2

Under nitrogen atmosphere, compound **21-7** (68.3 mg, 134 µmol), compound **57-3A** (104 mg, 161 µmol), and sodium carbonate (28.5 mg, 269 µmol) were dissolved in dioxane (8.00 mL) and water (2.00 mL), and the reaction mixture was added with [1,1'-bis(diphenylphosphino)ferrocene]dichloropalladium(II) (9.83 mg, 13.4 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.19) to obtain compound **57-4.** ¹H NMR (400MHz, CD₃OD) δ = 8.05 (s, 1H), 7.78 (s, 1H), 7.62 (d, J=8.8 Hz, 1H), 7.58 - 7.48 (m, 2H), 7.30 - 7.19 (m, 2H), 6.81 - 6.78 (m, 1H), 4.53 - 4.42 (m, 1H), 4.37 (s, 2H), 4.30 - 4.20 (m, 1H), 3.69 - 3.56 (m, 1H), 3.19 - 3.05 (m, 2H), 2.10 - 2.00 (m, 1H), 1.94 - 1.83 (m, 1H), 1.72 - 1.53 (m, 2H), 1.43 (s, 9H), 1.24 (s, 6H). MS-ESI calculated for [M+H]⁺ 618, found 618.

### Step 3

Compound **57-4** (37.0 mg, 59.9 µmol) was dissolved in dichloromethane (10.0 mL), and the reaction mixture was added with trimethylsilyl trifluoromethanesulfonate (21.6 µL, 120 µmol) at 0°C, stirred, and reacted for 1 hour at 25°C. The reaction mixture was added with saturated sodium bicarbonate aqueous solution (20 mL), and extracted with dichloromethane (20 mL × 3). The organic phase was washed with saturated brine (20.0 mL × 1), dried over anhydrous sodium sulfate, filtered, and concentrated under reduced pressure. The residue was purified by preparative high performance liquid chromatography (chromatographic column: Welch Xtimate C18 150 * 25 mm * 5 µm, mobile phase A: 10 mM ammonium bicarbonate aqueous solution; mobile phase B: acetonitrile; B%: 35% to 65%, 9 minutes) to obtain compound 57. ¹H NMR (400MHz, CD₃OD) δ = 8.06 (s, 1H), 7.79 (s, 1H), 7.67 - 7.59 (m, 2H), 7.55 (s, 1H), 7.32 (d, *J*=10.4 Hz, 1H), 7.28 - 7.21 (m, 2H), 4.41 - 4.32 (m, 3H), 4.28 - 4.20 (m, 1H), 3.24 - 3.08 (m, 1H), 3.07 - 3.00 (m, 1H), 2.93 - 2.83 (m, 1H), 2.13 - 1.99 (m, 1H), 1.92 - 1.79 (m, 1H), 1.73 - 1.55 (m, 1H), 1.51 - 1.37 (m, 1H), 1.24 (s, 6H). MS-ESI calculated for [M+H]⁺ 518, found 518.

### Example 58

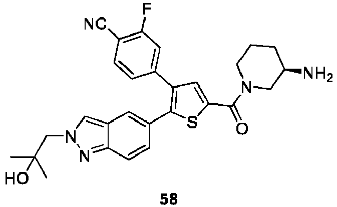

### Synthetic route:

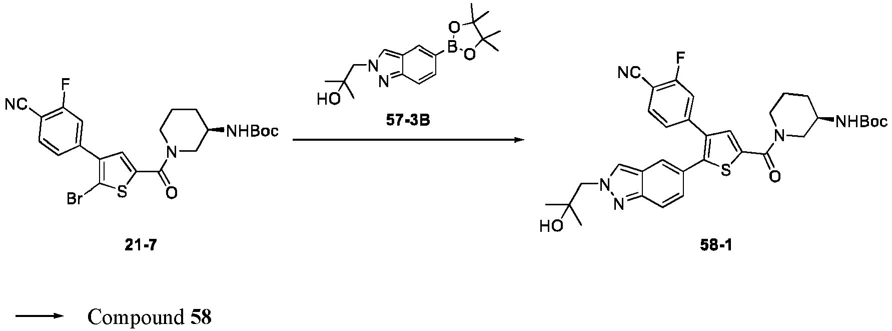

### Step 1

Under nitrogen atmosphere, compound **21-7** (16.9 mg, 33 µmol), compound **57-3B** (15.0 mg, 39.9 µmol), and sodium carbonate (7.04 mg, 66.4 µmol) were dissolved in dioxane (4.00 mL) and water (1.00 mL), and the reaction mixture was added with [1,1'-bis(diphenylphosphino)ferrocene]dichloropalladium(II) (2.43 mg, 3.32 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.14) to obtain compound **58-1.** ¹H NMR (400MHz, CD₃OD) δ = 8.30 (s, 1H), 7.81 (s, 1H), 7.71 - 7.48 (m, 3H), 7.31 (d, J=8.0 Hz, 1H), 7.13 (d, *J*=8.8 Hz, 1H), 6.81 (d, *J*=6.4 Hz, 1H), 4.54 - 4.44 (m, 1H), 4.42 (s, 2H), 4.35 - 4.20 (m, 1H), 3.74 - 3.58 (m, 1H), 3.21 - 3.04 (m, 2H), 2.12 - 1.98 (m, 1H), 1.94 - 1.84 (m, 1H), 1.73 - 1.55 (m, 2H), 1.45 (s, 9H), 1.24 (s, 6H). MS-ESI calculated for [M+H]⁺ 618, found 618.

### Step 3

Compound **58-1** (18.0 mg, 29.1 µmol) was dissolved in dichloromethane (10.0 mL), and the reaction mixture was added with trimethylsilyl trifluoromethanesulfonate (10.5 µL, 58.3 µmol) at 0°C, stirred, and reacted for 1 hour at 25°C. The reaction mixture was added with saturated sodium bicarbonate aqueous solution (20 mL), and extracted with dichloromethane (20 mL × 3). The organic phase was washed with saturated brine (20.0 mL × 1), dried over anhydrous sodium sulfate, filtered, and concentrated under reduced pressure. The residue was purified by preparative high performance liquid chromatography (chromatographic column: Welch Xtimate C18 150 * 25 mm * 5 µm, mobile phase A: 10 mM ammonium bicarbonate aqueous solution; mobile phase B: acetonitrile; B%: 36% to 66%, 9 minutes) to obtain compound **58.** ¹H NMR (400MHz, CD₃OD) δ = 8.31 (s, 1H), 7.82 (s, 1H), 7.70 - 7.59 (m, 2H), 7.58 (s, 1H), 7.37 (d, *J*=10.0 Hz, 1H), 7.30 (d, *J*=8.0 Hz, 1H), 7.15 (d, *J*=8.8 Hz, 1H), 4.44 - 4.35 (m, 3H), 4.29 - 4.21 (m, 1H), 3.26 - 3.14 (m, 1H), 3.09 - 2.98 (m, 1H), 2.93 - 2.85 (m, 1H), 2.11 - 2.06 (m, 1H), 1.95 - 1.84 (m, 1H), 1.69 - 1.58 (m, 1H), 1.54 - 1.42 (m, 1H), 1.24 (s, 6H). MS-ESI calculated for [M+H]⁺ 518, found 518.

### Example 59

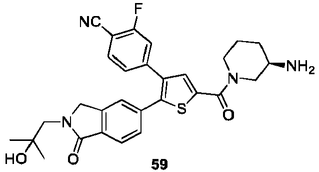

### Synthetic route:

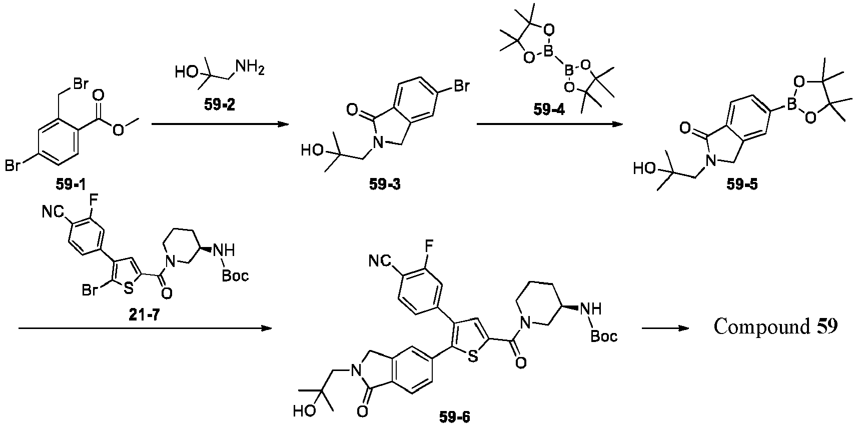

### Step 1

Under nitrogen atmosphere, compound **59-1** (810 mg, 2.63 mmol) was dissolved in anhydrous toluene (20.0 mL), and then triethylamine (840 mg, 8.30 mmol) and 1-amino-2-methylpropan-2-ol **(16-2)** (281 mg, 3.16 mmol) were added thereto. The reaction mixture was stirred for 4 hours at 110°C, and then concentrated under reduced pressure. The residue was purified by silica gel column chromatography (10/1, dichloromethane/methanol, Rf= 0.18) to obtain compound **59-3.** ¹H NMR (400MHz, CDCl₃) δ 7.74 - 7.70 (m, 1H), 7.64 - 7.59 (m, 2H), 4.59 (s, 2H), 3.60 (s, 2H), 1.29 (s, 6H).

### Step 2

Under nitrogen atmosphere, compound **59-3** (720 mg, 2.53 mmol), bis(pinacolato)diboron **(59-4)** (772 mg, 3.04 mmol), and potassium acetate (746 mg, 7.60 mmol) were dissolved in dioxane (10.0 mL), and the reaction mixture was added with [1,1'-bis(diphenylphosphino)ferrocene]dichloropalladium(II) (185 mg, 0.253 mmol), and the reaction mixture was stirred and reacted for 2 hours at 95°C, and concentrated under reduced pressure. The residue was purified by silica gel column chromatography (0/1, petroleum ether/ethyl acetate, R_{f} = 0.26) to obtain compound **59-5.** ¹H NMR (400MHz, CDCl₃) δ 7.94 - 7.84 (m, 3H), 4.59 (s, 2H), 3.63 (s, 2H), 1.37 (s, 9H), 1.30 (s, 6H).

### Step 3

Under nitrogen atmosphere, compound **21-7** (150 mg, 0.295 mmol), compound **59-5** (107 mg, 0.325 mmol), and sodium carbonate (93.8 mg, 0.885 mmol) were dissolved in dioxane (10.0 mL) and water (2.00 mL), and the reaction mixture was added with [1,1'-bis(diphenylphosphino)ferrocene]dichloropalladium(II) (21.6 mg, 0.295 mmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/2, petroleum ether/ethyl acetate, R_{f} = 0.19) to obtain compound **59-6.** MS-ESI calculated for [M+H]⁺ 633, found 633.

### Step 4

Under nitrogen atmosphere, compound **59-6** (160 mg, 0.253 mmol) was dissolved in anhydrous dichloromethane (5 mL), and trimethylsilyl trifluoromethanesulfonate (112 mg, 0.506 mmol) was added dropwise at 0°C. The reaction mixture was stirred for 1 hour at 25°C, added with saturated sodium bicarbonate aqueous solution (20 mL), and extracted with dichloromethane (20 mL × 1). The organic phase was dried over anhydrous sodium sulfate, filtered, concentrated under reduced pressure, and separated by preparative high performance liquid chromatography (chromatographic column: Phenomenex Genimi NX C18 150 * 40 mm * 5 µm; mobile phase: mobile phase A: ammonia aqueous solution with a volume fraction of 0.04% and 10 mmol/L sodium bicarbonate aqueous solution; mobile phase B: acetonitrile; B%: 20 to 50%, 10 minutes) to obtain compound **59.** ¹H NMR (400MHz, CD₃OD) δ 7.74 (d, *J*=8.0 Hz, 1H), 7.68 - 7.63 (m, 1H), 7.57 - 7.52 (m, 2H), 7.43 - 7.32 (m, 2H), 7.25 - 7.19 (m, 1H), 4.69 (s, 2H), 4.40 - 4.33 (m, 1H), 4.29 - 4.22 (m, 1H), 3.58 (s, 2H), 3.30 - 3.09 (m, 1H), 3.07 - 2.92 (m, 1H), 2.91 - 2.80 (m, 1H), 2.10 - 2.02 (m, 1H), 1.90 - 1.80 (m, 1H), 1.69 - 1.55 (m, 1H), 1.51 - 1.37 (m, 1H), 1.23 (s, 6H). MS-ESI calculated for [M+H]⁺ 533, found 533.

### Example 60

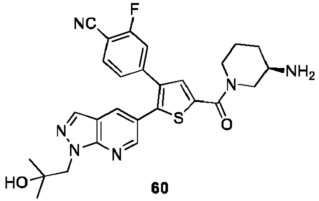

### Synthetic route:

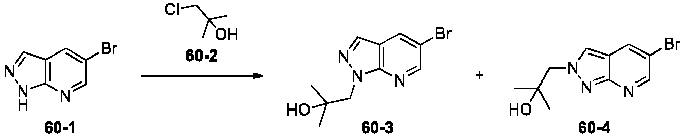

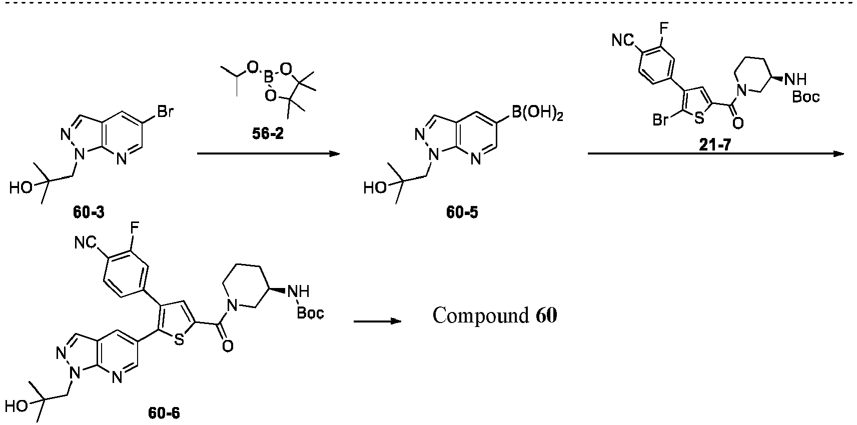

### Step 1

Under nitrogen atmosphere, compound **60-1** (2.10 g, 10.6 mmol) was dissolved in anhydrous *N*,*N-*dimethylformamide (20.0 mL), and then potassium carbonate (4.40 g, 31.8 mmol) and 1-amino-2-methylpropan-2-ol (2.30 g, 21.2 mmol) were added thereto, and the reaction mixture was stirred for 12 hours at 110°C, added with water (40 mL), and extracted with ethyl acetate (40 mL × 1). The organic phase was washed with saturated brine (40 mL × 1), dried over anhydrous sodium sulfate, filtered, and concentrated under reduced pressure. The residue was purified by silica gel column chromatography (1/2, petroleum ether/ethyl acetate, R_{f} = 0.66, 0.33) to obtain compound **60-3** and compound **60-4.** Compound **60-3** ¹H NMR (400MHz, CDCl₃) δ 8.52 (d, *J*=2.0 Hz, 1H), 8.20 (d, *J*=2.0 Hz, 1H), 7.98 (s, 1H), 4.50 (s, 2H), 4.00 (s, 1H), 1.18 (s, 6H). MS-ESI calculated for [M+H]⁺ 271, found 271. Compound **60-4** ¹H NMR (400MHz, CDCl₃) δ 8.67 (d, *J*=2.0 Hz, 1H), 8.20 (d, *J*=2.4 Hz, 1H), 7.98 (s, 1H), 4.39 (s, 2H), 3.98 (s, 1H), 1.22 (s, 6H). MS-ESI calculated for [M+H]⁺ 271, found 271.

### Step 2

Under nitrogen atmosphere, compound **60-3** (100 mg, 0.370 mmol), 2-isopropoxy-4,4,5,5-tetramethyl-1,3,2-dioxaborolane **(56-2)** (103 mg, 0.555 mmol) were dissolved in anhydrous tetrahydrofuran (5.00 mL), and n-butyllithium (2.5 mol/L n-hexane solution, 592 µL) was added dropwise thereto at -78°C. The reaction mixture was stirred and reacted for 12 hours at 25°C, added with water (20 mL), and extracted with ethyl acetate (20 mL × 1). The aqueous phase was concentrated under reduced pressure to obtain compound **60-5.** MS-ESI calculated for [M+H]⁺ 236, found 236.

### Step 3

Under nitrogen atmosphere, compound **21-7** (100 mg, 0.197 mmol), compound **60-5** (64.7 mg, 0.275 mmol), and sodium carbonate (62.5 mg, 0.590 mmol) were dissolved in dioxane (10.0 mL) and water (2.00 mL), and the reaction mixture was added with [1,1'-bis(diphenylphosphino)ferrocene]dichloropalladium(II) (14.4 mg, 0.197 mmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/2, petroleum ether/ethyl acetate, R_{f} = 0.19) to obtain compound **60-6.** MS-ESI calculated for [M+H]⁺ 619, found 619.

### Step 4

Under nitrogen atmosphere, compound **60-6** (60.0 mg, 0.097 mmol) was dissolved in anhydrous dichloromethane (5.00 mL), and trimethylsilyl trifluoromethanesulfonate (43.1 mg, 0.194 mmol) was added dropwise at 0°C. The reaction mixture was stirred for 1 hour at 25°C, added with saturated sodium bicarbonate aqueous solution (20 mL), and extracted with dichloromethane (20 mL × 1). The organic phase was dried over anhydrous sodium sulfate, filtered, concentrated under reduced pressure, and separated by preparative high performance liquid chromatography (chromatographic column: Phenomenex Genimi NX C18 150 * 40 mm * 5 µm; mobile phase: mobile phase A: ammonia aqueous solution with a volume fraction of 0.04% and 10 mmol/L sodium bicarbonate aqueous solution; mobile phase B: acetonitrile; B%: 20 to 50%, 10 minutes) to obtain compound **60.** ¹H NMR (400MHz, CD₃OD) δ 8.38 (d, *J*=2.0 Hz, 1H), 8.24 (d, *J*=2.0 Hz, 1H), 8.14 (s, 1H), 7.68 - 7.63 (m, 1H), 7.59 (s, 1H), 7.36 (d, *J*=10.0 Hz, 1H), 7.26 - 7.21 (m, 1H), 4.50 (s, 2H), 4.43 - 4.32 (m, 1H), 4.30 - 4.21 (m, 1H), 3.29 - 3.10 (m, 1H), 3.08 - 2.92 (m, 1H), 2.91 - 2.82 (m, 1H), 2.10 - 2.02 (m, 1H), 1.92 - 1.82 (m, 1H), 1.69 - 1.56 (m, 1H), 1.51 - 1.38 (m, 1H), 1.23 (s, 6H). MS-ESI calculated for [M+H]⁺ 519, found 519.

### Example 61

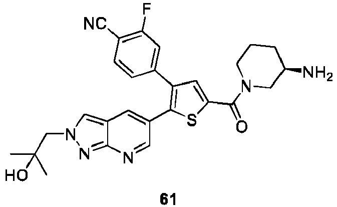

### Synthetic route:

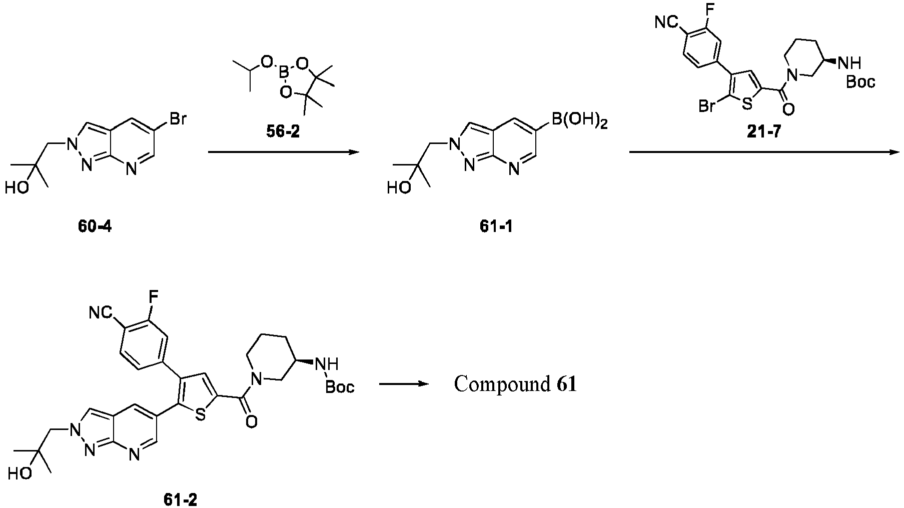

### Step 1

Under nitrogen atmosphere, compound **60-4** (120 mg, 0.444 mmol), 2-isopropoxy-4,4,5,5-tetramethyl-1,3,2-dioxaborolane (124 mg, 0.666 mmol) were dissolved in anhydrous tetrahydrofuran (5.00 mL), then n-butyllithium (2.5 mol/L n-hexane solution, 711 µL) was added dropwise thereto at -78°C. The reaction mixture was stirred and reacted for 12 hours at 25°C, added with water (20 mL), and extracted with ethyl acetate (20 mL × 1). The aqueous phase was concentrated under reduced pressure to obtain compound **61-1.** MS-ESI calculated for [M+H]⁺ 236, found 236.

### Step 2

Under nitrogen atmosphere, compound **21-7** (140 mg, 0.275 mmol), compound **61-1** (90.6 mg, 0.386 mmol), and sodium carbonate (87.6 mg, 0.826 mmol) were dissolved in dioxane (10.0 mL) and water (2.00 mL), and the reaction mixture was added with [1,1'-bis(diphenylphosphino)ferrocene]dichloropalladium(II) (20.2 mg, 0.275 mmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/2, petroleum ether/ethyl acetate, R_{f} = 0.19) to obtain compound **61-2.** MS-ESI calculated for [M+H]⁺ 619, found 619.

### Step 3

Under nitrogen atmosphere, compound **61-2** (60.0 mg, 0.097 mmol) was dissolved in anhydrous dichloromethane (10.0 mL), and trimethylsilyl trifluoromethanesulfonate (43.1 mg, 0.194 mmol) was added dropwise at 0°C. The reaction mixture was stirred for 1 hour at 25°C, added with saturated sodium bicarbonate aqueous solution (20 mL), and extracted with dichloromethane (20 mL × 1). The organic phase was dried over anhydrous sodium sulfate, filtered, concentrated under reduced pressure, and separated by preparative high performance liquid chromatography (chromatographic column: Phenomenex Genimi NX C18 150 * 40 mm * 5 µm; mobile phase: mobile phase A: ammonia aqueous solution with a volume fraction of 0.04% and 10 mmol/L sodium bicarbonate aqueous solution; mobile phase B: acetonitrile; B%: 15 to 45%, 10 minutes) to obtain compound **61.** ¹H NMR (400MHz, CD₃OD) δ 8.40 - 8.36 (m, 2H), 8.32 (d, *J*=2.0 Hz, 1H), 7.70 - 7.64 (m, 1H), 7.59 (s, 1H), 7.40 (d, *J*=10.4 Hz, 1H), 7.29 - 7.23 (m, 1H), 4.45 (s, 2H), 4.40 - 4.33 (m, 1H), 4.30 - 4.23 (m, 1H), 3.29 - 3.09 (m, 1H), 3.08 - 2.94 (m, 1H), 2.92 - 2.82 (m, 1H), 2.11 - 2.01 (m, 1H), 1.92 - 1.84 (m, 1H), 1.69 - 1.56 (m, 1H), 1.51 - 1.40 (m, 1H), 1.25 (s, 6H). MS-ESI calculated for [M+H]⁺ 519, found 519.

### Example 62

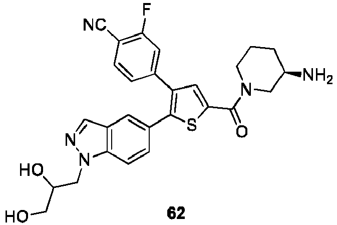

### Synthetic route:

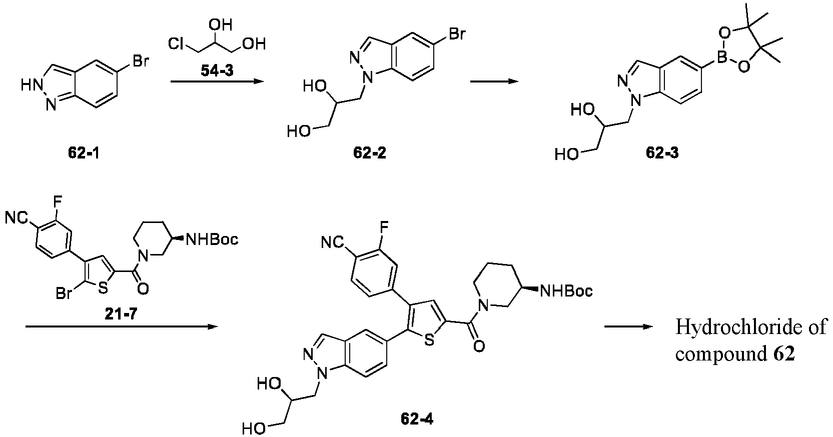

### Step 1

Compounds **62-1** (4.00 g, 20.3 mmol) and **54-3** (6.73 g, 60.9 mmol) were dissolved in ethanol (30.0 mL), and sodium hydroxide (2.44 g, 60.9 mmol) was added thereto, and the reaction mixture was stirred and reacted for 12 hours at 78°C. The reaction mixture was added with water (100 mL), and extracted with ethyl acetate (100 mL × 2). The organic phase was washed with saturated brine (100 mL × 1), dried over anhydrous sodium sulfate, filtered, and concentrated under reduced pressure. The residue was purified by silica gel column chromatography (1/2, petroleum ether/ethyl acetate, R_{f} = 0.34) to obtain compound **62-2.** MS-ESI calculated for [M+H]⁺ 273, found 273.

### Step 2

Under nitrogen atmosphere, compound **62-2** (1.00 g, 3.69 mmol) and bis(pinacolato)diboron (2.81 g, 11.1 mol) were dissolved in dioxane (20.0 mL), and the reaction mixture was added with potassium acetate (1.09 g, 11.1 mol) and [1,1'-bis(diphenylphosphino)ferrocene]dichloropalladium(II) (269 mg, 369 µmol). The reaction mixture was stirred and reacted at 100°C for 12 hours. The reaction mixture was concentrated under reduced pressure, and the residue was purified by silica gel column chromatography (1/2, petroleum ether/ethyl acetate, R_{f} = 0.31) to obtain compound **62-3.** ¹H NMR (400MHz, CD₃OD) δ = 8.22 (s, 1H), 8.09 (s, 1H), 7.76 (d, *J*=8.4 Hz, 1H), 7.61 (d, *J*=8.4 Hz, 1H), 4.59 - 4.50 (m, 1H), 4.49 - 4.38 (m, 1H), 4.17 - 4.07 (m, 1H), 3.82 - 3.71 (m, 1H), 3.47 - 3.40 (m, 1H), 1.38 (s, 12H). MS-ESI calculated for [M+H]⁺ 319, found 319.

### Step 3

Under nitrogen atmosphere, compound **21-7** (639 mg, 1.26 mmol), compound **62-3** (480 mg, 1.51 mmol), and sodium carbonate (400 mg, 3.77 mmol) were dissolved in dioxane (24.0 mL) and water (6.00 mL), and the reaction mixture was added with [1,1'-bis(diphenylphosphino)ferrocene]dichloropalladium(II) (92.0 mg, 126 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.07) to obtain compound **62-4.** MS-ESI calculated for [M+H]⁺ 620, found 620.

### Step 4

Compound **62-4** (200 mg, 323 µmol) was dissolved in ethyl acetate (15.0 mL), and hydrochloride ethyl acetate solution (4 mol/L, 807 µL) was added thereto, and the reaction mixture was stirred and reacted for 3 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 5% to 35%, 10 minutes) to obtain the hydrochloride of compound **62.** ¹H NMR (400MHz, CD₃OD) δ = 8.07 (s, 1H), 7.79 (s, 1H), 7.69 - 7.59 (m, 3H), 7.33 - 7.21 (m, 3H), 4.64 - 4.51 (m, 1H), 4.49 - 4.37 (m, 2H), 4.24 - 4.04 (m, 2H), 3.64 - 3.38 (m, 5H), 2.29 - 2.15 (m, 1H), 1.97 -1.86 (m, 1H), 1.84 - 1.68 (m, 2H). MS-ESI calculated for [M+H]⁺ 520, found 520.

### Example 63

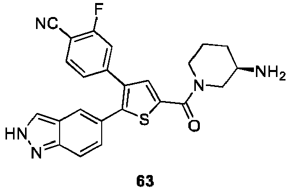

### Synthetic route:

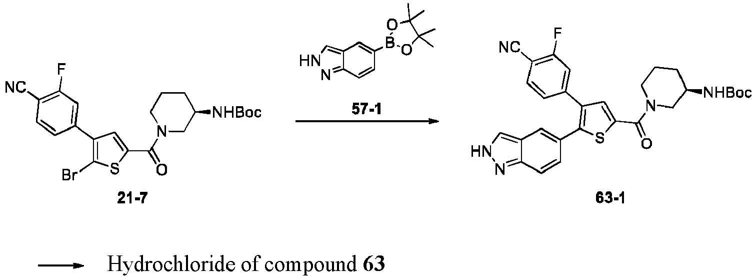

### Step 1

Under nitrogen atmosphere, compound **21-7** (300 mg, 590 µmol), compound **57-1** (173 mg, 708 µmol), and sodium carbonate (125 mg, 1.18 mmol) were dissolved in dioxane (32.0 mL) and water (8.00 mL), and the reaction mixture was added with [1,1'-bis(diphenylphosphino)ferrocene]dichloropalladium(II) (43.2 mg, 59.0 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.18) to obtain compound **63-1.** MS-ESI calculated for [M+H]⁺ 546, found 546.

### Step 2

Compound **63-1** (80.0 mg, 147 µmol) was dissolved in ethyl acetate (10.0 mL), and hydrochloride ethyl acetate solution (4 mol/L, 367 µL) was added thereto, and the reaction mixture was stirred and reacted for 12 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 8% to 38%, 10 minutes) to obtain the hydrochloride of compound **63.** ¹H NMR (400MHz, CD₃OD) δ = 8.13 (s, 1H), 7.86 (s, 1H), 7.71 - 7.61 (m, 2H), 7.57 (d, *J*=8.8 Hz, 1H), 7.35 (d, *J*=10.4 Hz, 1H), 7.29 - 7.24 (m, 2H), 4.44 - 4.39 (m, 1H), 4.21 - 4.16 (m, 1H), 3.65 - 3.40 (m, 3H), 2.27 - 2.17 (m, 1H), 2.01 - 1.88 (m, 1H), 1.84 - 1.68 (m, 2H). MS-ESI calculated for [M+H]⁺ 446, found 446.

### Example 64

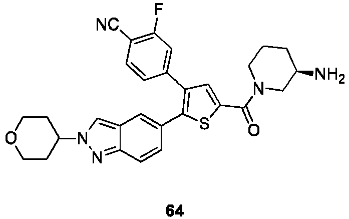

### Synthetic route:

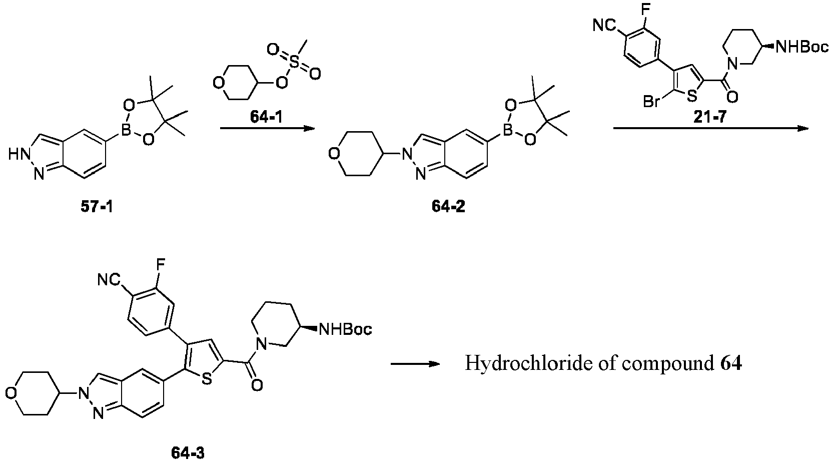

### Step 1

Compounds **57-1** (400 mg, 1.64 mmol) and **64-1** (886 mg, 4.92 mmol) were dissolved in *N*,*N-*dimethylformamide (20.0 mL), and then cesium carbonate (534 mg, 1.64 mmol) was added thereto, and the reaction mixture was stirred and reacted for 12 hours at 100°C. The reaction mixture was added with water (80 mL), and extracted with ethyl acetate (80 mL × 2). The organic phase was washed with saturated brine (80 mL × 1), dried over anhydrous sodium sulfate, filtered, and concentrated under reduced pressure. The residue was purified by silica gel column chromatography (3/1, petroleum ether/ethyl acetate, R_{f} = 0.38) to obtain compound **64-2.** MS-ESI calculated for [M+H]⁺ 329, found 329.

### Step 2

Under nitrogen atmosphere, compound **21-7** (150 mg, 295 µmol), compound **64-2** (150 mg, 457 µmol), and sodium carbonate (80.7 mg, 762 µmol) were dissolved in dioxane (4.00 mL) and water (1.00 mL), and the reaction mixture was added with [1,1'-bis(diphenylphosphino)ferrocene]dichloropalladium(II) (27.9 mg, 38.1 µmol), and the reaction mixture was stirred and reacted for 12 hours at 100°C. The reaction mixture was concentrated under reduced pressure, and the residue was purified by silica gel column chromatography (2/1, petroleum ether/ethyl acetate, R_{f} = 0.34) to obtain compound **64-3.** MS-ESI calculated for [M+H]⁺ 630, found 630.

### Step 3

Compound **64-3** (112 mg, 178 µmol) was dissolved in ethyl acetate (1.00 mL), and hydrochloride ethyl acetate solution (4 mol/L, 445 µL) was added thereto, and the reaction mixture was stirred and reacted for 2 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **64.** ¹H NMR (400MHz, CD₃OD) δ = 8.39 (s, 1H), 7.78 (s, 1H), 7.68 - 7.58 (m, 3H), 7.34 (d, *J*=10.4 Hz, 1H), 7.26 (d, *J*=8.0 Hz, 1H), 7.15 (d, *J*=8.8 Hz, 1H), 4.45 - 4.36 (m, 1H), 4.24 - 4.07 (m, 3H), 3.70 - 3.58 (m, 2H), 3.59 - 3.38 (m, 3H), 2.33 - 2.12 (m, 6H), 1.94 - 1.89 (m, 1H), 1.83 - 1.68 (m, 2H). MS-ESI calculated for [M+H]⁺ 530, found 530.

### Example 65

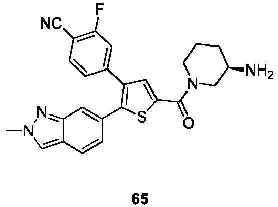

### Synthetic route:

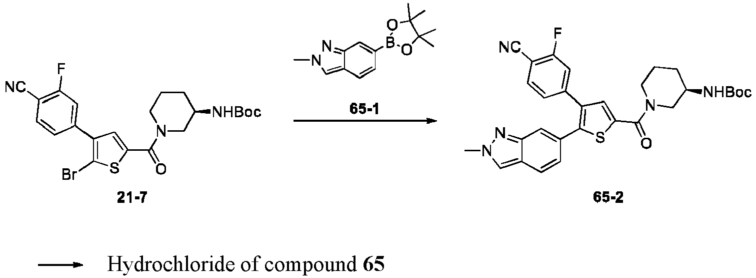

### Step 1

Under nitrogen atmosphere, compound **21-7** (300 mg, 590 µmol), compound **65-1** (228 mg, 885 µmol), and sodium carbonate (125 mg, 1.18 mmol) were dissolved in dioxane (24.0 mL) and water (6.00 mL), and the reaction mixture was added with [1,1'-bis(diphenylphosphino)ferrocene]dichloropalladium(II) (43.2 mg, 59.0 µmol), and the reaction mixture was stirred and reacted for 12 hours at 100°C. The reaction mixture was concentrated under reduced pressure, and the residue was purified by silica gel column chromatography (0/1, petroleum ether/ethyl acetate, R_{f} = 0.49) to obtain compound **65-2.** MS-ESI calculated for [M-56+H]⁺ 504, found 504.

### Step 2

Compound **65-2** (222 mg, 397 µmol) was dissolved in ethyl acetate (15.0 mL), and hydrochloride ethyl acetate solution (4 mol/L, 992 µL) was added thereto, and the reaction mixture was stirred and reacted for 12 hours at 25°C. After the reaction mixture was concentrated under reduced pressure, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 12% to 52%, 10 minutes) to obtain the hydrochloride of compound **65.** ¹H NMR (400MHz, CD₃OD) δ = 8.34 (s, 1H), 7.75 (d, *J*=8.4 Hz, 1H), 7.69 - 7.58 (m, 3H), 7.33 (d, *J*=10.4 Hz, 1H), 7.28 (d, *J*=8.0 Hz, 1H), 7.00 (d, *J*=8.8 Hz, 1H), 4.44 - 4.35 (m, 1H), 4.25 (s, 3H), 4.21 - 4.10 (m, 1H), 3.62 - 3.38 (m, 3H), 2.27 - 2.14 (m, 1H), 1.97 - 1.86 (m, 1H), 1.84 - 1.68 (m, 2H). MS-ESI calculated for [M+H]⁺ 460, found 460.

### Example 66

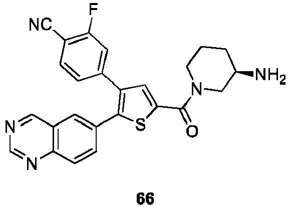

### Synthetic route:

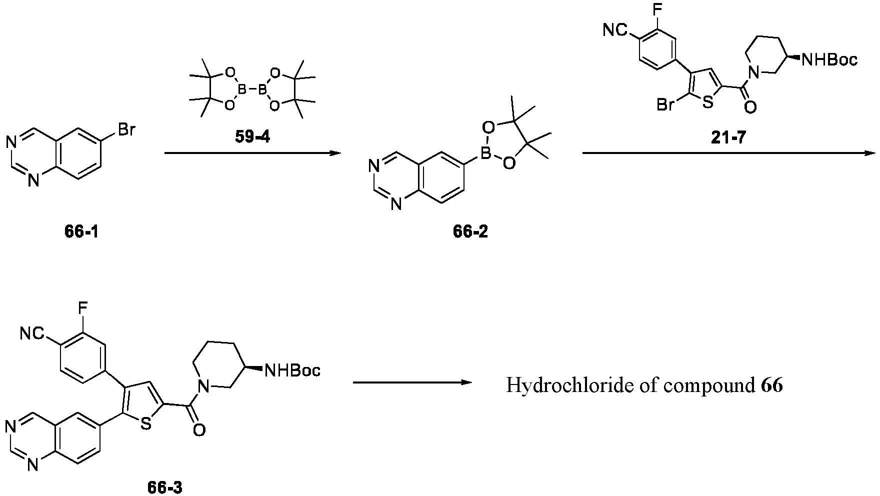

### Step 1

Under nitrogen atmosphere, compound **66-1** (200 mg, 957 µmol), compound **59-4** (292 mg, 1.15 mmol), and potassium acetate (188 mg, 1.91 mmol), and [1,1'-bis(diphenylphosphino)ferrocene]dichloropalladium(II) dichloromethane (70.0 mg, 95.7 µmol) were dissolved in dioxane (5.00 mL). The reaction mixture was stirred and reacted for 12 hours at 90°C. The reaction mixture was concentrated under reduced pressure, and the residue was purified by preparative silica gel thin layer chromatography (1/1, petroleum ether/ethyl acetate, R_{f} = 0.50) to obtain compound **66-2.** ¹H NMR (400MHz, CDCl₃) δ = 9.37 (s, 1H), 9.29 (s, 1H), 8.39 (s, 1H), 8.04 - 8.25 (m, 1H), 7.97 (d, *J*= 8.4 Hz, 1H), 1.33 (s, 12H).

### Step 2

Under nitrogen atmosphere, compound **66-2** (121 mg, 472 µmol), compound **21-7** (200 mg, 394 µmol), potassium phosphate (167 mg, 787 µmol), and [1,1'-bis(di-tert-butylphosphino)ferrocene]dichloropalladium(II) (25.6 mg, 39.3 µmol) were dissolved in dioxane (8.00 mL) and water (1.60 mL), and the reaction mixture was stirred and reacted for 12 hours at 90°C. The reaction mixture was filtered through diatomite, and the filtrate was concentrated and purified by preparative silica gel thin layer chromatography (20/1, dichloromethane/methanol, R_{f} = 0.24) to obtain compound **66-3.** MS-ESI calculated for [M+H]⁺ 558, found 558.

### Step 3

Compound **66-3** (160 mg, 287 µmol) was dissolved in ethyl acetate (700 µL), and hydrochloride ethyl acetate solution (4 mol/L, 287 µL) was added thereto at 0°C, and the reaction mixture was stirred and reacted for 12 hours at 11°C. The reaction mixture was filtrated. The solid was washed with ethyl acetate (400 µL). The residue was purified by preparative high performance liquid chromatography (chromatographic column: 3_Phenomenex Luna C18 150 * 25 mm * 10 µm, mobile phase A: hydrochloric acid aqueous solution with a volume fraction of 0.05%; mobile phase B: acetonitrile; B%: 3% to 33%, 10 minutes) to obtain the hydrochloride of compound **66.** ¹H NMR (400MHz, DMSO-*d*₆) δ = 9.65 (s, 1H), 9.34 (s, 1H), 8.28 - 8.24 (m, 4H), 7.99 (d, *J*=8.8 Hz, 1H), 7.84 (t, *J*=8.8 Hz, 1H), 7.80 - 7.77 (m, 1H), 7.74 (s, 1H), 7.70 - 7.67 (m, 1H), 7.27 - 7.24 (m, 1H), 4.36 - 4.34 (m, 1H), 4.09 - 4.05 (m, 1H), 3.37 - 3.23 (m, 3H), 2.08 - 2.04 (m, 1H), 1.87 - 1.82 (m, 1H), 1.69 - 1.57 (m, 2H). MS-ESI calculated for [M+H]⁺ 458, found 458.

### Example 67

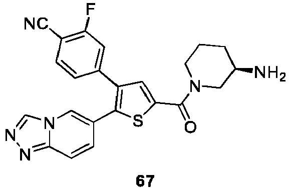

### Synthetic route:

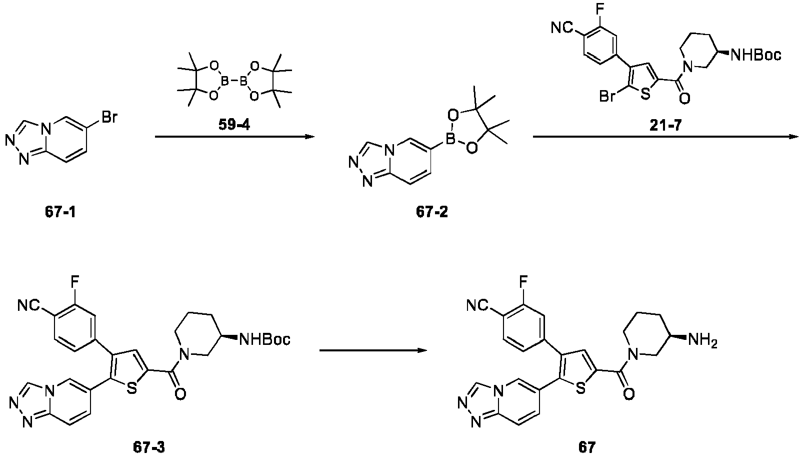

### Step 1

Under nitrogen atmosphere, compound **67-1** (95.0 mg, 984 µmol), compound **59-4** (300 mg, 1.18 mmol), potassium acetate (193 mg, 1.97 mmol), and [1,1'-bis(diphenylphosphino)ferrocene]dichloropalladium(II) dichloromethane (80.4 mg, 98.5 µmol) were dissolved in dioxane (7.00 mL). The reaction mixture was stirred and reacted for 12 hours at 85°C. The reaction mixture was filtered through diatomite, and the filtrate was concentrated to obtain crude compound **67-2,** which was used directly in the next reaction step.

### Step 2

Under nitrogen atmosphere, compound **67-2** (240 mg, 979 µmol), compound **21-7** (527 mg, 979 µmol), potassium phosphate (416 mg, 1.96 mmol), and [1,1'-bis(di-tert-butylphosphino)ferrocene]dichloropalladium(II) (63.8 mg, 97.9 µmol) were dissolved in dioxane (8.00 mL) and water (1.60 mL), and the reaction mixture was stirred and reacted for 12 hours at 85°C. The reaction mixture was filtered through diatomite, and the filtrate was concentrated and purified by preparative silica gel thin layer chromatography (20/1, dichloromethane/methanol, R_{f} = 0.26) to obtain compound **67-3.** MS-ESI calculated for [M+H]⁺ 547, found 547.

### Step 3

Compound **67-3** (240 mg, 363 µmol) was dissolved in ethyl acetate (6.00 mL), and hydrochloride ethyl acetate solution (4 mol/L, 342 µL) was added thereto at 0°C, and the reaction mixture was stirred and reacted for 12 hours at 11°C. The reaction mixture was filtered. The solid was washed with ethyl acetate (400 µL), and the residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%; mobile phase B: acetonitrile; B%: 11% to 31%, 6 minutes); the purified solution was adjusted to pH = 9 to 10 with ammonia water, extracted with dichloromethane/methanol (10/1, 90 mL), dried over anhydrous sodium sulfate, filtered, and concentrated to obtain compound **67.** ¹H NMR (400MHz, CD₃OD) δ = 9.19 (s, 1H), 8.70 - 8.69 (m, 1H), 7.72 - 7.69 (m, 2H), 7.61 (s, 1H), 7.50 - 7.48 (m, 1H), 7.34 - 7.31 (m, 1H), 7.22 - 7.19 (m, 1H), 4.35 - 4.29 (m, 1H), 4.26 - 4.21 (m, 1H), 3.25 - 3.16 (m, 1H), 3.05 - 3.00 (m, 1H), 2.91 - 2.84 (m, 1H), 2.08 - 2.04 (m, 1H), 1.90 - 1.84 (m, 1H), 1.67 - 1.57 (m, 1H), 1.50 - 1.40 (m, 1H). MS-ESI calculated for [M+H]⁺ 447, found 447.

### Example 68

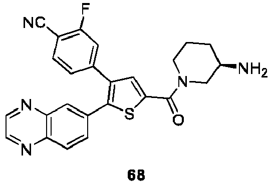

### Synthetic route:

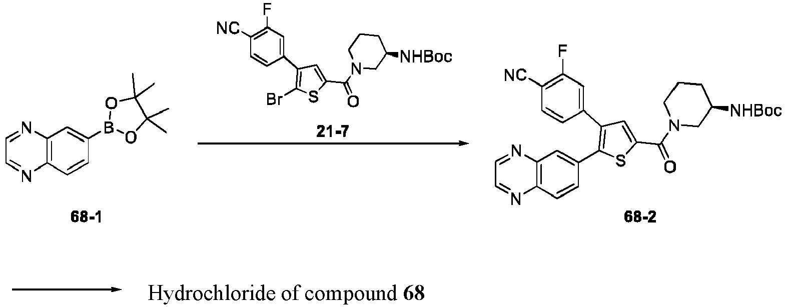

### Step 1

Under nitrogen atmosphere, compound **68-1** (121 mg, 471 µmol), compound **21-7** (212 mg, 394 µmol), potassium phosphate (167 mg, 785 µmol), and [1,1'-bis(di-tert-butylphosphino)ferrocene]dichloropalladium(II) (25.6 mg, 39.2 µmol) were dissolved in dioxane (8.00 mL) and water (1.60 mL), and the reaction mixture was stirred and reacted for 12 hours at 80°C. The reaction mixture was filtered through diatomite, and the filtrate was concentrated and purified by preparative silica gel thin layer chromatography (20/1, dichloromethane/methanol, Rf = 0.25) to obtain compound **68-2.** ¹H NMR (400MHz, CDCl₃) δ = 8.88 (s, 2H), 8.16 - 8.12 (m, 1H), 8.06 (d, *J*=8.4 Hz, 1H), 7.83 (s, 1H), 7.64 - 7.59 (m, 2H), 7.45 (t, *J*=7.2 Hz, 1H), 7.24 (d, *J*=8.0 Hz, 1H), 4.72 - 4.59 (m, 2H), 4.48 - 4.39 (m, 1H), 3.75 - 3.71 (m, 1H), 3.05 - 2.93 (m, 2H), 2.12 - 2.09 (m, 1H), 1.93 - 1.88 (m, 1H), 1.75 - 1.66 (m, 1H), 1.48 (s, 9H). MS-ESI calculated for [M+H]⁺ 558, found 558.

### Step 2

Compound **68-2** (180 mg, 317 µmol) was dissolved in ethyl acetate (1.50 mL), and hydrochloride ethyl acetate solution (4 mol/L, 317 µL) was added thereto at 0°C, and the reaction mixture was stirred and reacted for 12 hours at 11°C. The reaction mixture was filtered. The solid was washed with ethyl acetate (0.4 mL), and the residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%; mobile phase B: acetonitrile; B%: 0% to 40%, 6.5 minutes) to obtain the hydrochloride of compound **68.** ¹H NMR (400MHz, DMSO-*d*₆) δ = 8.99 (s, 2H), 8.34 - 8.29 (m, 3H), 8.10 - 8.08 (m, 2H), 7.89 - 7.84 (m, 1H), 7.74 (s, 1H), 7.72 - 7.65 (m, 2H), 4.37 - 4.35 (m, 1H), 4.10 - 4.06 (m, 1H), 3.38 - 3.33 (m, 3H), 2.09 - 2.05 (m, 1H), 1.86 - 1.83 (m, 1H), 1.70 - 1.57 (m, 2H). MS-ESI calculated for [M+H]⁺ 458, found 458.

### Example 69

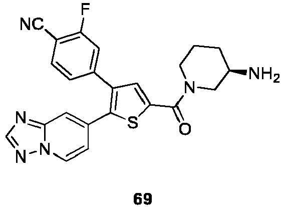

### Synthetic route:

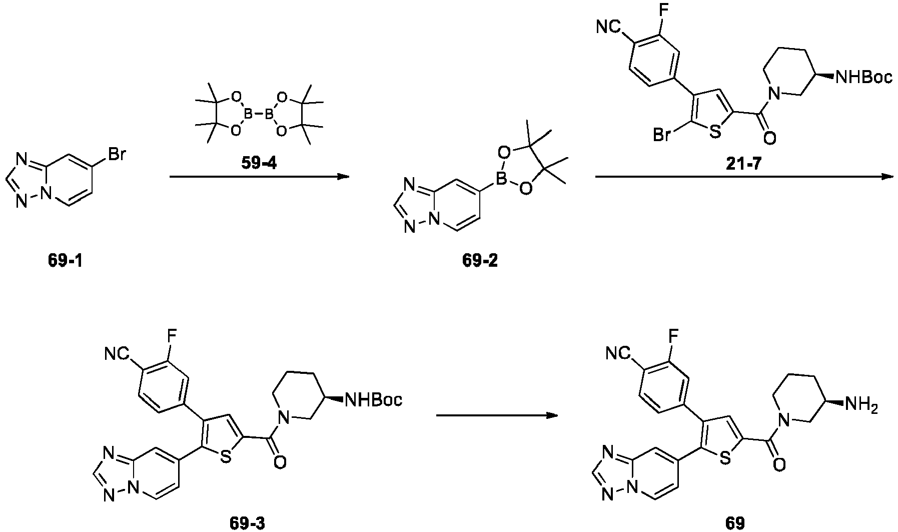

### Step 1

Under nitrogen atmosphere, compound **69-1** (117 mg, 599 µmol), compound **59-4** (180 mg, 709 µmol), and potassium acetate (116 mg, 1.18 mmol), and [1,1'-bis(diphenylphosphino)ferrocene]dichloropalladium(II) dichloromethane (48.3 mg, 59.1 µmol) were dissolved in dioxane (4.00 mL). The reaction mixture was stirred and reacted for 12 hours at 85°C. The reaction mixture was filtered through diatomite, and the filtrate was concentrated to obtain crude compound **69-2,** which was used directly in the next reaction step.

### Step 2

Under nitrogen atmosphere, compounds **69-2** (150 mg, 612 µmol), **21-7** (323 mg, 612 µmol), potassium phosphate (260 mg, 1.22 mmol), and [1,1'-bis(di-tert-butylphosphino)ferrocene]dichloropalladium(II) (39.9 mg, 61.2 µmol) were dissolved in dioxane (8.00 mL) and water (1.60 mL), and the reaction mixture was stirred and reacted for 12 hours at 90°C. The reaction mixture was filtered through diatomite, and the filtrate was concentrated and purified by preparative silica gel thin layer chromatography (20/1, dichloromethane/methanol, R_{f} = 0.26) to obtain compound **69-3.** MS-ESI calculated for [M+H]⁺ 547, found 547.

### Step 3

Compound **69-3** (210 mg, 358 µmol) was dissolved in ethyl acetate (6.00 mL), and hydrochloride ethyl acetate solution (4 mol/L, 287 µL) was added thereto at 0°C, and the reaction mixture was stirred and reacted for 12 hours at 11°C. The reaction mixture was filtered. The solid was washed with ethyl acetate (0.4 mL), and the residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%; mobile phase B: acetonitrile; B%: 21% to 31%, 6 minutes); the purified solution was adjusted to pH = 9 to 10 with ammonia water, extracted with dichloromethane/methanol (10/1, 70 mL), dried over anhydrous sodium sulfate, filtered, and concentrated to obtain compound **69.** ¹H NMR (400MHz, DMSO-*d*₆) δ = 8.94 - 8.90 (m, 1H), 8.57 (s, 1H), 7.91 - 7.87 (m, 2H), 7.70 - 7.67 (m, 2H), 7.34 - 7.32 (m, 1H), 6.95 - 6.93 (m, 1H), 4.19 - 4.06 (m, 2H), 3.30 (s, 2H), 3.18 - 3.04 (m, 1H), 2.78 - 2.72 (m, 1H), 1.90 - 1.87 (m, 1H), 1.79 - 1.73 (m, 2H), 1.53 - 1.43 (m, 1H), 1.34 - 1.24 (m, 1H). MS-ESI calculated for [M+H]⁺ 447, found 447.

### Example 70

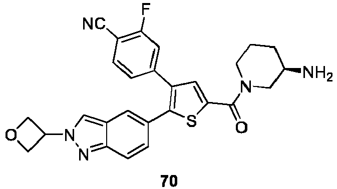

### Synthetic route:

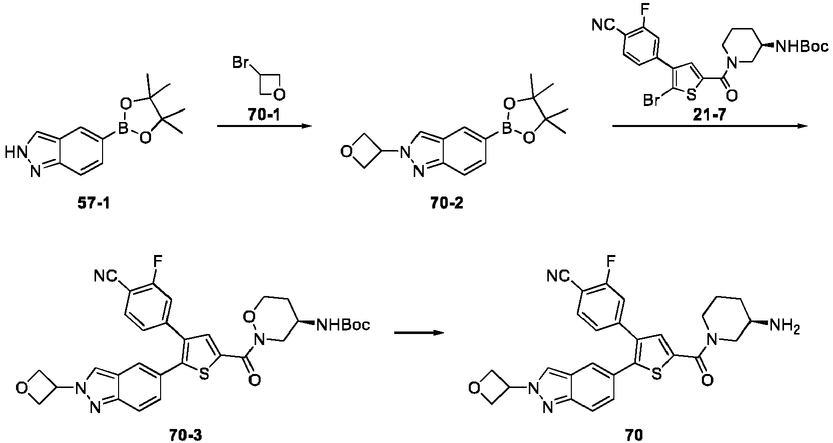

### Step 1

Compounds **57-1** (1.45 g, 5.94 mmol) and **70-1** (2.44 g, 17.8 mmol) were dissolved in *N*,*N-*dimethylformamide (30.0 mL), and then potassium carbonate (2.46 g, 17.8 mmol) was added thereto, and the reaction mixture was stirred and reacted for 12 hours at 100°C. The reaction mixture was added with water (150 mL), and extracted with ethyl acetate (150 mL × 2). The organic phase was washed with saturated brine (150 mL × 1), dried over anhydrous sodium sulfate, filtered, and concentrated under reduced pressure. The residue was purified by silica gel column chromatography (3/1, petroleum ether/ethyl acetate, R_{f} = 0.42) to obtain compound **70-2.** MS-ESI calculated for [M+H]⁺ 301, found 301.

### Step 2

Under nitrogen atmosphere, compound **21-7** (291 mg, 572 µmol), compound **70-2** (340 mg, 686 µmol), and sodium carbonate (182 mg, 1.72 mmol) were dissolved in dioxane (24.0 mL) and water (6.00 mL), and the reaction mixture was added with [1,1'-bis(diphenylphosphino)ferrocene]dichloropalladium(II) (41.9 mg, 57.2 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.32) to obtain compound **70-3.** MS-ESI calculated for [M+H]⁺ 602, found 602.

### Step 3

Compound **70-3** (100 mg, 166 µmol) was dissolved in dichloromethane (1.00 mL), and trifluoroacetic acid (1.00 mL) was added thereto, and the reaction mixture was stirred and reacted for 1 hour at 25°C. The reaction mixture was concentrated, added with water (3.00 mL), and then adjusted to pH = 8 with ammonia water. The residue was purified by preparative high performance liquid chromatography (chromatographic column: Phenomenex Gemini-NX 80 * 30 mm * 3 µm, mobile phase A: mobile phase A: 10 mM ammonium bicarbonate aqueous solution; mobile phase B: acetonitrile; B%: 30% to 60%, 9 minutes) to obtain compound **70.** ¹H NMR (400MHz, CD₃OD) δ = 8.41 (s, 1H), 7.78 (s, 1H), 7.70 - 7.60 (m, 2H), 7.56 (s, 1H), 7.33 (d, *J*=10.4 Hz, 1H), 7.27 (dd, *J*=1.2, 8.0 Hz, 1H), 7.15 (dd, *J*=1.2, 8.8 Hz, 1H), 5.90 - 5.83 (m, 1H), 5.20 - 5.13 (m, 4H), 4.40 -4.33 (m, 1H), 4.30 - 4.24 (m, 1H), 3.25 - 3.10 (m, 1H), 3.06 - 2.93 (m, 1H), 2.92 - 2.81 (m, 1H), 2.12 - 2.01 (m, 1H), 1.91 - 1.81 (m, 1H), 1.71 - 1.56 (m, 1H), 1.53 - 1.38 (m, 1H). MS-ESI calculated for [M+H]⁺ 502, found 502.

### Example 71

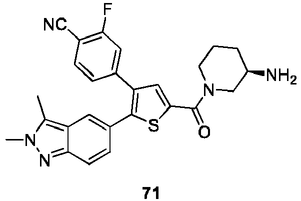

### Synthetic route:

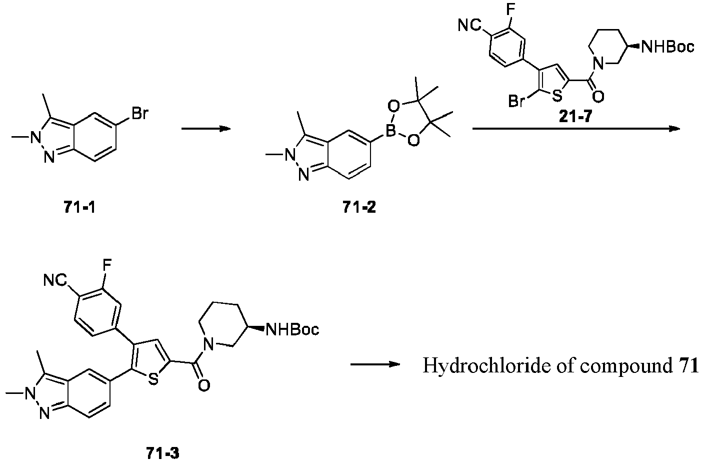

### Step 1

Under nitrogen atmosphere, compound **71-1** (1.00 g, 4.44 mmol) and bis(pinacolato)diboron (3.38 g, 13.3 mmol), and potassium acetate (1.31 g, 13.3 mmol) were dissolved in dioxane (30.0 mL), and the reaction mixture was added with [1,1'-bis(diphenylphosphino)ferrocene]dichloropalladium(II) (325 mg, 444 µmol). The reaction mixture was stirred and reacted at 100°C for 12 hours. The reaction mixture was concentrated under reduced pressure, and the residue was purified by silica gel column chromatography (2/1, petroleum ether/ethyl acetate, R_{f} = 0.37) to obtain compound **71-2.** ¹H NMR (400MHz, CD₃OD) δ = 8.13 (s, 1H), 7.58 (dd, *J*=0.8, 8.8 Hz, 1H), 7.45 (dd, *J*=0.8, 8.8 Hz, 1H), 4.08 (s, 3H), 2.65 (s, 3H), 1.36 (s, 12H). MS-ESI calculated for [M+H]⁺ 273, found 273.

### Step 2

Under nitrogen atmosphere, compound **21-7** (467 mg, 918 µmol), compound **71-2** (300 mg, 1.10 mmol), and sodium carbonate (195 mg, 1.84 mmol) were dissolved in dioxane (24.0 mL) and water (6.00 mL), and the reaction mixture was added with [1,1'-bis(diphenylphosphino)ferrocene]dichloropalladium(II) (67.2 mg, 91.9 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.32) to obtain compound **71-3.** MS-ESI calculated for [M+H]⁺ 574, found 574.

### Step 3

Compound **71-3** (325 mg, 566 µmol) was dissolved in ethyl acetate (5.00 mL), and hydrochloride ethyl acetate solution (4 mol/L, 1.42 mL) was added thereto, and the reaction mixture was stirred and reacted for 1 hour at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 50%, 10 minutes) to obtain the hydrochloride of compound **71.** ¹H NMR (400MHz, CD₃OD) δ = 8.06 (s, 1H), 7.69 - 7.59 (m, 3H), 7.44 (d, *J*=8.8 Hz, 1H), 7.36 - 7.26 (m, 2H), 4.48 - 4.41 (m, 1H), 4.25 (s, 3H), 4.25 - 4.16 (m, 1H), 3.65 - 3.38 (m, 3H), 2.79 (s, 3H), 2.30 - 2.14 (m, 1H), 2.00 - 1.89 (m, 1H), 1.87 - 1.67 (m, 2H). MS-ESI calculated for [M+H]⁺ 474, found 474.

### Example 72

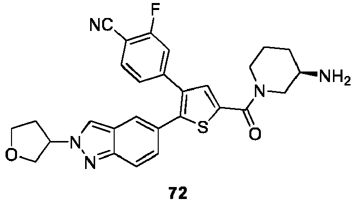

### Synthetic route:

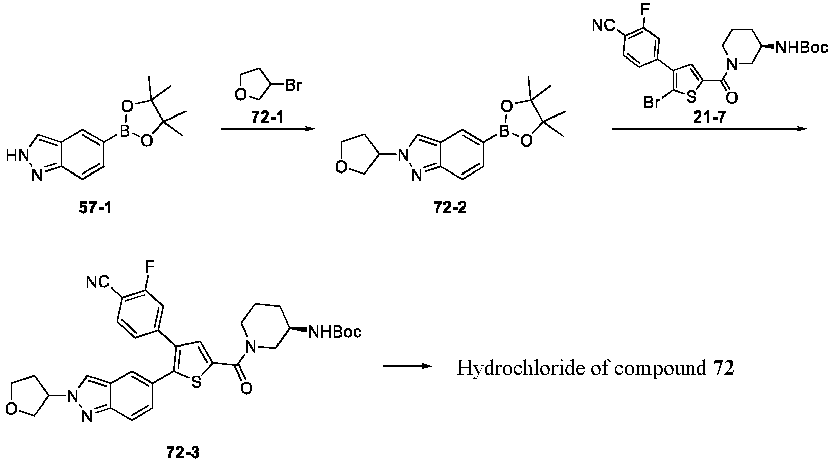

### Step 1

Compounds **57-1** (862 mg, 3.53 mmol) and **72-1** (1.60 g, 10.6 mmol) were dissolved in *N,N-*dimethylformamide (20.0 mL), and then potassium carbonate (1.46 g, 10.6 mmol) was added thereto, and the reaction mixture was stirred and reacted for 12 hours at 100°C. The reaction mixture was added with water (150 mL), and extracted with ethyl acetate (150 mL × 2). The organic phase was washed with saturated brine (150 mL × 1), dried over anhydrous sodium sulfate, filtered, and concentrated under reduced pressure. The residue was purified by silica gel column chromatography (2/1, petroleum ether/ethyl acetate, R_{f} = 0.19) to obtain compound **72-2.** ¹H NMR (400MHz, CD₃OD) δ = 8.35 (s, 1H), 8.20 (s, 1H), 7.63 - 7.53 (m, 2H), 5.37 - 5.28 (m, 1H), 4.28 - 4.10 (m, 3H), 4.01 - 3.91 (m, 1H), 2.67 - 2.54 (m, 1H), 2.50 - 2.39 (m, 1H), 1.36 (s, 12H). MS-ESI calculated for [M+H]⁺ 315, found 315.

### Step 2

Under nitrogen atmosphere, compound **21-7** (351 mg, 690 µmol), compound **72-2** (260 mg, 828 µmol), and sodium carbonate (146 mg, 1.38 mmol) were dissolved in dioxane (16.0 mL) and water (4.00 mL), and the reaction mixture was added with [1,1'-bis(diphenylphosphino)ferrocene]dichloropalladium(II) (50.5 mg, 69.0 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.32) to obtain compound **72-3.** MS-ESI calculated for [M+H]⁺ 616, found 616.

### Step 3

Compound **72-3** (340 mg, 552 µmol) was dissolved in ethyl acetate (5.00 mL), and hydrochloride ethyl acetate solution (4 mol/L, 1.38 mL) was added thereto, and the reaction mixture was stirred and reacted for 2 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 5% to 50%, 10 minutes) to obtain the hydrochloride of compound **72.** ¹H NMR (400MHz, CD₃OD) δ = 8.38 (s, 1H), 7.79 (s, 1H), 7.70 - 7.58 (m, 3H), 7.34 (d, *J*=10.4 Hz, 1H), 7.28 (d, *J*=8.4 Hz, 1H), 7.14 (d, *J*=8.8 Hz, 1H), 5.41 - 5.32 (m, 1H), 4.45 - 4.42 (m, 1H), 4.29 - 4.13 (m, 4H), 4.01 - 3.95 (m, 1H), 3.61 - 3.39 (m, 3H), 2.72 - 2.59 (m, 1H), 2.54 - 2.41 (m, 1H), 2.45 - 2.21 (m, 1H), 1.99 - 1.89 (m, 1H), 1.87 - 1.70 (m, 2H). MS-ESI calculated for [M+H]⁺ 516, found 516.

### Example 73

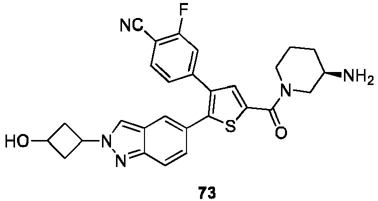

### Synthetic route:

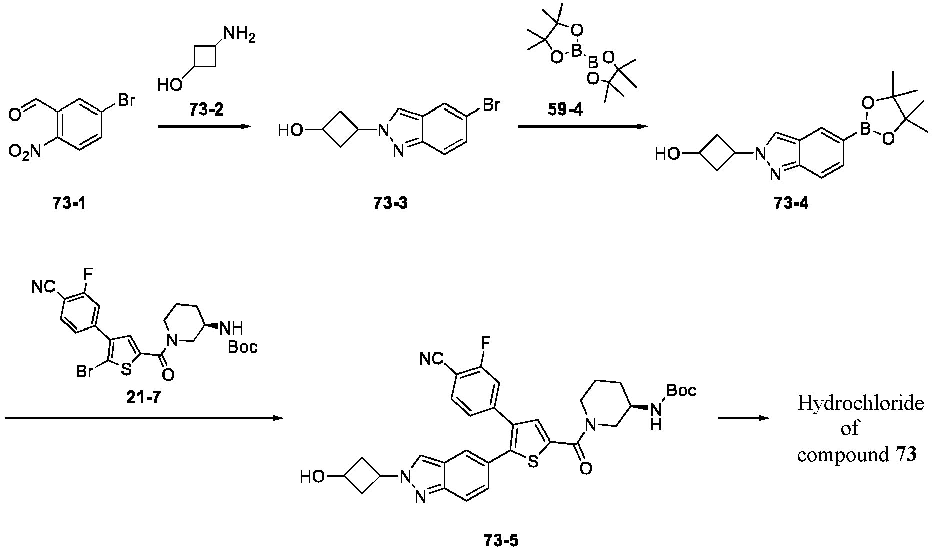

### Step 1

Under nitrogen atmosphere, compound **73-1** (100 mg, 0.435 mmol) was dissolved in isopropanol (10.0 mL), and 3-aminocyclobutanol (41.7 mg, 0.478 mmol) was added thereto, and the reaction mixture was stirred for 4 hours at 80°C, added with tributylphosphine (264 mg, 1.30 mmol) at 0°C, stirred for 12 hours at 80°C, added with saturated ammonium chloride (30 mL), and extracted with ethyl acetate (30 mL × 1). The organic phase was washed with saturated brine (30 mL × 1), dried over anhydrous sodium sulfate, filtered, concentrated under reduced pressure, and the residue was purified by silica gel column chromatography (1/1, petroleum ether/ethyl acetate, R_{f} = 0.12) to obtain compound **73-3.** ¹H NMR (400MHz, CDCl₃) δ = 7.96 - 7.87 (m, 1H), 7.85 - 7.77 (m, 1H), 7.65 - 7.56 (m, 1H), 7.41 - 7.32 (m, 1H), 5.31 - 5.19 (m, 0.4H), 4.92 - 4.80 (m, 0.4H), 4.74 - 4.62 (m, 0.6H), 4.35 - 4.26 (m, 0.6H), 3.16 - 2.97 (m, 2H), 2.70 - 2.62 (m, 2H). MS-ESI calculated for [M+H]⁺ 267, found 267.

### Step 2

Under nitrogen atmosphere, compound **73-3** (100 mg, 0.374 mmol), bis(pinacolato)diboron (285 mg, 1.12 mmol), and potassium acetate (184 mg, 1.87 mmol) were dissolved in dioxane (10.0 mL), and the reaction mixture was added with [1,1'-bis(diphenylphosphino)ferrocene]dichloropalladium(II) (54.8 mg, 74.9 µmol), stirred and reacted for 12 hours at 95°C, and concentrated under reduced pressure. The residue was purified by silica gel column chromatography (1/1, petroleum ether/ethyl acetate, R_{f} = 0.60) to obtain compound **73-4.** ¹H NMR (400MHz, CDCl₃) δ = 8.23 (s, 1H), 8.02 - 7.96 (m, 1H), 7.71 - 7.66 (m, 2H), 5.31 - 5.23 (m, 0.4H), 4.92 - 4.83 (m, 0.4H), 4.76 - 4.68 (m, 0.6H), 4.36 - 4.28 (m, 0.6H), 3.19 - 2.99 (m, 2H), 2.75 - 2.61 (m, 2H), 1.37 (s, 12H). MS-ESI calculated for [M+H]⁺ 315, found 315.

### Step 3

Under nitrogen atmosphere, compound **21-7** (145 mg, 0.285 mmol), compound **73-4** (98.6 mg, 0.314 mmol), and sodium carbonate (90.7 mg, 0.856 mmol) were dissolved in dioxane (8.00 mL) and water (2.00 mL), and the reaction mixture was added with [1,1'-bis(di-tert-butylphosphino)ferrocene]dichloropalladium(II) (18.6 mg, 28.5 µmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure, and the residue was purified by silica gel column chromatography (1/1, petroleum ether/ethyl acetate, Rf= 0.21) to obtain compound **73-5.** MS-ESI calculated for [M+H]⁺ 616, found 616.

### Step 4

Under nitrogen atmosphere, compound **73-5** (61.0 mg, 99.1 µmol) was dissolved in anhydrous dichloromethane (5.00 mL), and trimethylsilyl trifluoromethanesulfonate (44.0 mg, 198 µmol) was added dropwise at 0°C. The reaction mixture was stirred for 1 hour at 25°C, added with water (1 mL), concentrated under reduced pressure, and then separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 5 to 35%, 10 minutes) to obtain the hydrochloride of compound **73.** ¹H NMR (400MHz, CD₃OD) δ = 8.49 - 8.43 (m, 1H), 7.81 (s, 1H), 7.68 - 7.58 (m, 3H), 7.38 - 7.17 (m, 3H), 5.38 - 5.28 (m, 0.4H), 4.76 - 4.68 (m, 1H), 4.45 - 4.35 (m, 1H), 4.27 - 4.19 (m, 1H), 4.18 - 4.14 (m, 0.6H), 3.59 - 3.38 (m, 3H), 3.07 - 2.89 (m, 2H), 2.70 - 2.55 (m, 2H), 2.25 - 2.15 (m, 1H), 1.98 - 1.87 (m, 1H), 1.86 - 1.68 (m, 2H). MS-ESI calculated for [M+H]⁺ 516, found 516.

### Example 74

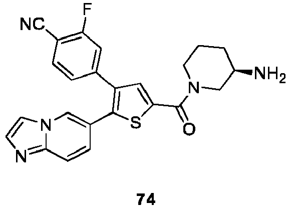

### Synthetic route:

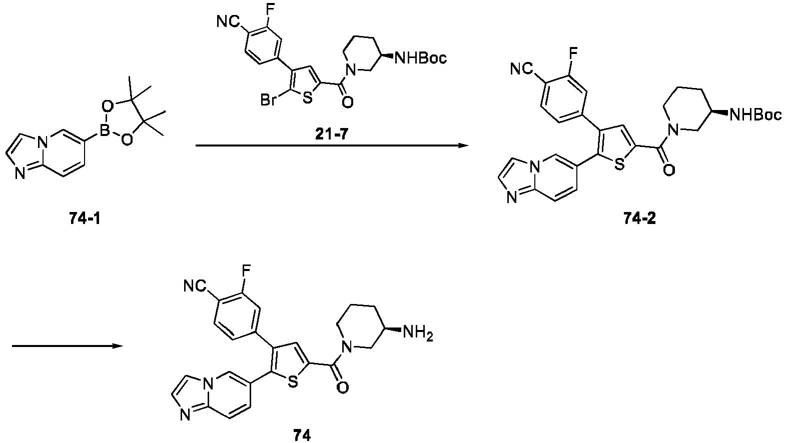

### Step 1

Under nitrogen atmosphere, compound **74-1** (150 mg, 615 µmol), compound **21-7** (331 mg, 615 µmol), potassium phosphate (261 mg, 1.23 mmol), and [1,1'-bis(di-tert-butylphosphino)ferrocene]dichloropalladium(II) (40.1 mg, 61.5 µmol) were dissolved in dioxane (4.00 mL) and water (0.800 mL), and the reaction mixture was stirred and reacted for 12 hours at 85°C. The reaction mixture was filtered through diatomite, and the filtrate was concentrated and purified by preparative silica gel thin layer chromatography (20/1, dichloromethane/methanol, R_{f} = 0.24) to obtain compound **74-2.** MS-ESI calculated for [M+H]⁺ 546, found 546.

### Step 2

Compound **74-2** (80 mg, 113 µmol) was dissolved in ethyl acetate (1.5 mL), and hydrochloride ethyl acetate solution (4 mol/L, 287 µL) was added thereto at 0°C, and the reaction mixture was stirred and reacted for 12 hours at 11°C. The reaction mixture was filtered. The solid was washed with ethyl acetate (0.400 mL), and the residue was purified by preparative high performance liquid chromatography (chromatographic column: Waters Xbridge 150 * 25 mm * 5 µm; mobile phase: mobile phase A: ammonia aqueous solution with a volume fraction of 0.05%; mobile phase B: acetonitrile; B%: acetonitrile 17% to 47%, retention time of 9 minutes) to obtain compound **74.** ¹H NMR (400MHz, CD₃OD) δ = 8.64 - 8.63 (m, 1H), 7.90 - 7.88 (m, 1H), 7.70 - 7.67 (m, 1H), 7.64 - 7.60 (m, 1H), 7.59 (s, 1H), 7.52 (d, *J*=9.6 Hz, 1H), 7.47 - 7.44 (m, 1H), 7.32 - 7.29 (m, 1H), 7.10 - 7.07 (m, 1H), 4.60 - 4.57 (m, 1H), 4.37 - 4.33 (m, 1H), 4.27 - 4.22 (m, 1H), 3.04 - 2.96 (m, 1H) , 2.91 - 2.85 (m, 1H) , 2.07 - 2.03 (m, 1H), 1.89 - 1.84 (m, 1H), 1.65 - 1.58 (m, 1H), 1.50 - 1.44 (m, 1H). MS-ESI calculated for [M+H]⁺ 446, found 446.

### Example 75

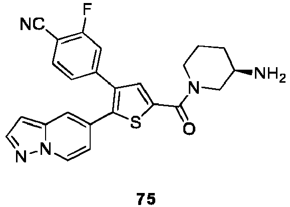

### Synthetic route:

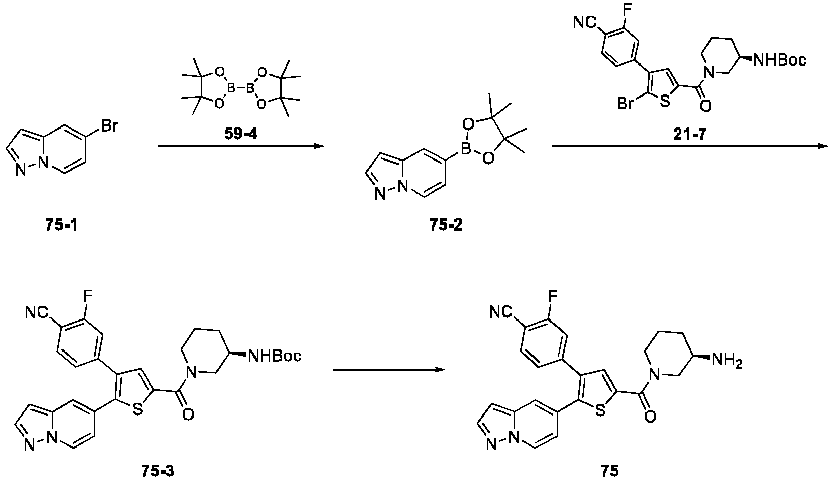

### Step 1

Under nitrogen atmosphere, compound **75-1** (116 mg, 589 µmol), compound **59-4** (179 mg, 706 µmol), potassium acetate (116 mg, 1.18 mmol), and [1,1'-bis(diphenylphosphino)ferrocene]dichloropalladium(II) dichloromethane (48.1 mg, 58.9 µmol) were dissolved in dioxane (4.00 mL). The reaction mixture was stirred and reacted for 12 hours at 85°C. The reaction mixture was filtered through diatomite, and the filtrate was concentrated to obtain crude compound **75-2,** which was used directly in the next reaction step.

### Step 2

Under nitrogen atmosphere, compound **75-2** (144 mg, 590 µmol), compound **21-7** (317 mg, 589.93 µmol), potassium phosphate (250 mg, 1.18 mmol), and [1,1'-bis(di-tert-butylphosphino)ferrocene]dichloropalladium(II) (38.5 mg, 59.0 µmol) were dissolved in dioxane (8.00 mL) and water (1.60 mL), and the reaction mixture was stirred and reacted for 12 hours at 90°C. The reaction mixture was filtered through diatomite, and the filtrate was concentrated and purified by preparative silica gel thin layer chromatography (20/1, dichloromethane/methanol, Rf = 0.27) to obtain compound **75-3.** MS-ESI calculated for [M+H]⁺ 546, found 546.

### Step 3

Compound **75-3** (300 mg, 493 µmol) was dissolved in ethyl acetate (6.00 mL), and hydrochloride ethyl acetate solution (4 mol/L, 287 µL) was added thereto at 0°C, and the reaction mixture was stirred and reacted for 12 hours at 11°C. The reaction mixture was filtered, and the solid was washed with ethyl acetate (0.4 mL). The residue was purified by preparative high performance liquid chromatography (chromatographic column: Waters Xbridge 150 * 25 mm * 5 µm; mobile phase: mobile phase A: ammonia aqueous solution with a volume fraction of 0.05%, mobile phase B: acetonitrile; B%: 25% to 55%, 9 minutes) to obtain compound 75. ¹H NMR (400MHz, CDCl₃) δ = 8.39 - 8.37 (m, 1H), 8.00 - 7.99 (m, 1H), 7.58 - 7.54 (m, 2H), 7.40 (s, 1H), 7.24 - 7.16 (m, 2H), 6.59 - 6.57 (m, 1H), 6.49 - 6.47 (m, 1H), 4.20 - 4.05 (m, 2H), 3.35 - 3.26 (m, 2H), 3.17 - 3.13 (m, 1H), 2.08 - 1.88 (m, 4H). MS-ESI calculated for [M+H]⁺ 446, found 446.

### Example 76

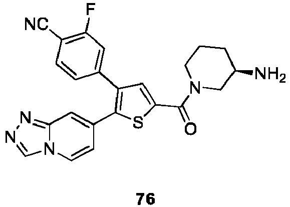

### Synthetic route:

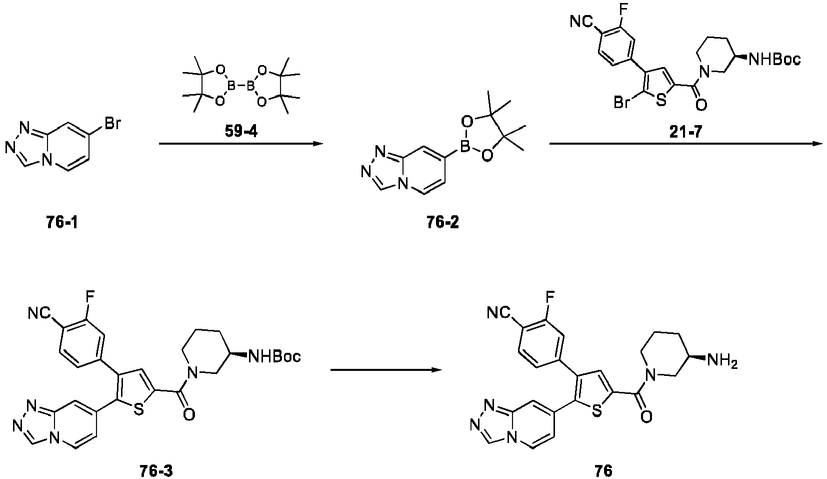

### Step 1

Under nitrogen atmosphere, compound **76-1** (500 mg, 2.52 mmol), compound **59-4** (769 mg, 3.03 mmol), potassium acetate (496 mg, 5.05 mmol), and [1,1'-bis(diphenylphosphino)ferrocene]dichloropalladium(II) dichloromethane (206 mg, 253 µmol) were dissolved in dioxane (16.0 mL). The reaction mixture was stirred and reacted for 12 hours at 80°C. The reaction mixture was filtered through diatomite, and the filtrate was concentrated to obtain crude compound **76-2,** which was used directly in the next reaction step.

### Step 2

Under nitrogen atmosphere, compound **76-2** (620 mg, 2.53 mmol), compound **21-7** (1.36 g, 2.53 mmol), potassium phosphate (1.07 g, 5.06 mmol), and [1,1'-bis(di-tert-butylphosphino)ferrocene]dichloropalladium(II) (165 mg, 253 µmol) were dissolved in dioxane (16.0 mL) and water (3.20 mL), and the reaction mixture was stirred and reacted for 12 hours at 90°C. The reaction mixture was filtered through diatomite, and the filtrate was concentrated and purified by silica gel column chromatography (2/3/1, petroleum ether/ethyl acetate/ethanol) to obtain compound **76-3.** MS-ESI calculated for [M+H]⁺ 547, found 547.

### Step 3

Compound **76-3** (1.30 g, 1.95 mmol) was dissolved in ethyl acetate (15.0 mL), and hydrochloride ethyl acetate solution (4 mol/L, 1.46 mL) was added thereto at 0°C, and the reaction mixture was stirred and reacted for 12 hours at 11°C. The reaction mixture was filtered. The solid was washed with ethyl acetate (1 mL), and the residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%; mobile phase B: acetonitrile; B%: acetonitrile 13% to 23%, 6 minutes); the purified solution was adjusted to pH = 9 to 10 with ammonia water, extracted with dichloromethane/methanol (10/1, 180 mL), dried over anhydrous sodium sulfate, filtered, and concentrated to obtain compound **76.** ¹H NMR (400MHz, DMSO-*d*₆) δ = 9.28 (s, 1H), 8.50 - 8.48 (m, 1H), 7.93 - 7.86 (m, 2H), 7.70 - 7.66 (m, 2H), 7.35 - 7.33 (m, 1H), 6.69 - 6.67 (m, 1H), 4.16 - 4.05 (m, 2H), 3.12 - 3.02 (m, 2H), 2.77 - 2.67 (m, 1H), 1.89 - 1.85 (m, 1H), 1.77 - 1.66 (m, 3H), 1.50 - 1.42 (m, 1H), 1.32 - 1.23 (m, 1H). MS-ESI calculated for [M+H]⁺ 447, found 447.

### Example 77

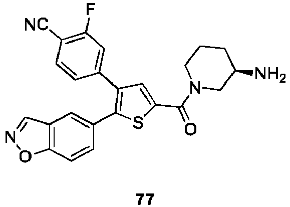

### Synthetic route:

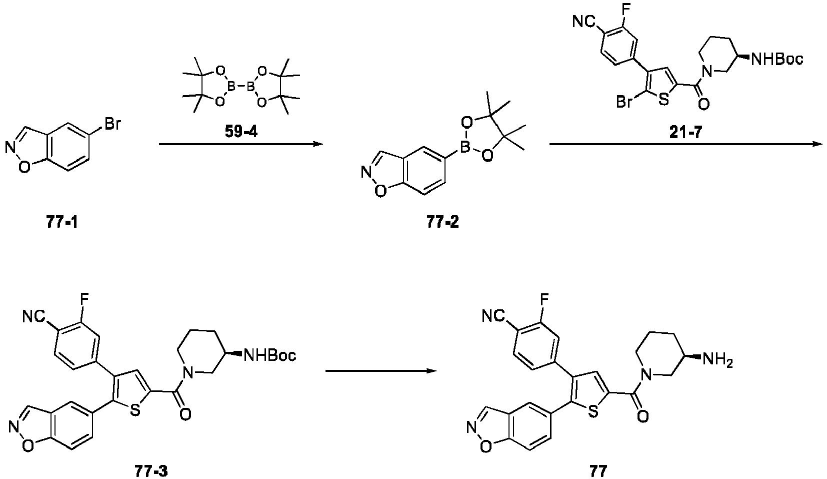

### Step 1

Under nitrogen atmosphere, compound **77-1** (117 mg, 591 µmol), compound 59-4 (180.05 mg, 709.03 µmol), potassium acetate (116 mg, 1.18 mmol), and [1,1'-bis(diphenylphosphino)ferrocene]dichloropalladium(II) dichloromethane (48.3 mg, 59.1 µmol) were dissolved in dioxane (4.00 mL). The reaction mixture was stirred and reacted for 12 hours at 85°C. The reaction mixture was filtered through diatomite, and the filtrate was concentrated to obtain crude compound **77-2,** which was used directly in the next reaction step.

### Step 2

Under nitrogen atmosphere, compound **77-2** (145 mg, 592 µmol), compound **21-7** (318 mg, 592 µmol), potassium phosphate (251 mg, 1.18 mmol), and [1,1'-bis(di-tert-butylphosphino)ferrocene]dichloropalladium(II) (38.6 mg, 59.2 µmol) were dissolved in dioxane (8.00 mL) and water (1.60 mL), and the reaction mixture was stirred and reacted for 12 hours at 80°C. The reaction mixture was filtered through diatomite, and the filtrate was concentrated and purified by preparative silica gel thin layer chromatography (20/1, dichloromethane/methanol, R_{f} = 0.26) to obtain compound **77-3.** MS-ESI calculated for [M+H]⁺ 491, found 491.

### Step 3

Compound **77-3** (200 mg, 261 µmol) was dissolved in ethyl acetate (2.00 mL), and hydrochloride ethyl acetate solution (44 mol/L, 816 µL) was added thereto at 0°C, and the reaction mixture was stirred and reacted for 12 hours at 11°C. The reaction mixture was filtered, and the solid was washed with ethyl acetate (0.4 mL). The residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%, mobile phase B: acetonitrile; B%: acetonitrile 23% to 33%, 6 minutes); the purified solution was adjusted to pH = 9 to 10 with ammonia water, extracted with dichloromethane/methanol (10/1, 60 mL), dried over anhydrous sodium sulfate, filtrated, and concentrated to obtain compound 77. ¹H NMR (400MHz, CD₃OD) δ = 7.71 - 7.67 (m, 1H), 7.51 (s, 1H), 7.40 (d, *J*=2.0 Hz, 1H), 7.35 - 7.33 (m, 1H), 7.28 - 7.20 (m, 2H), 6.78 (d, *J*=8.4 Hz, 1H), 4.62 - 4.59 (m, 1H), 4.37 - 4.33 (m, 1H), 4.20 - 4.16 (m, 1H), 3.27 - 3.21 (m, 1H), 3.17 - 3.14 (m, 1H), 2.15 - 2.12 (m, 1H), 1.78 - 1.86 (m, 1H), 1.69 - 1.60 (m, 2H). MS-ESI calculated for [M+H]⁺ 447, found 447.

### Example 78

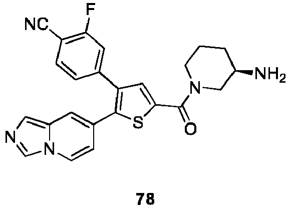

### Synthetic route:

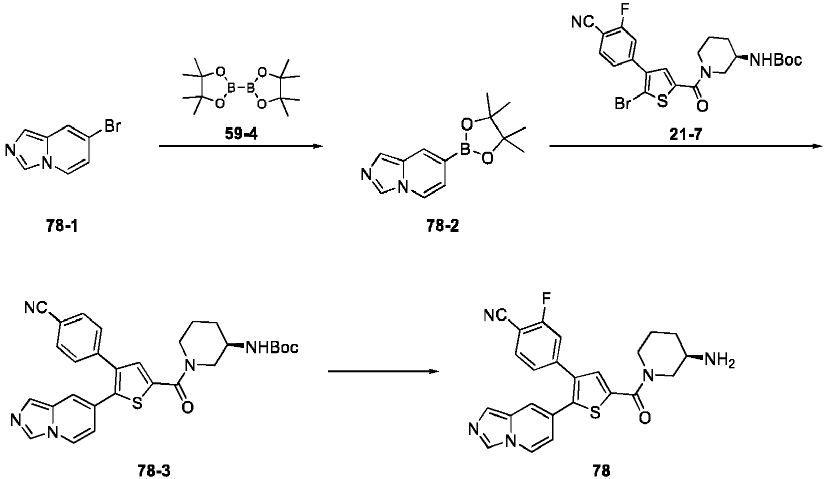

### Step 1

Under nitrogen atmosphere, compound **78-1** (130 mg, 660 µmol), compound **59-4** (201 mg, 792 µmol), potassium acetate (130 mg, 1.32 mmol), and [1,1'-bis(diphenylphosphino)ferrocene]dichloropalladium(II) dichloromethane (53.9 mg, 66.0 µmol) were dissolved in dioxane (4.00 mL). The reaction mixture was stirred and reacted for 12 hours at 85°C. The reaction mixture was filtered through diatomite, and the filtrate was concentrated to obtain crude compound **78-2,** which was used directly in the next reaction step.

### Step 2

Under nitrogen atmosphere, compound **78-2** (160 mg, 655 µmol), compound **21-7** (353 mg, 655 µmol), potassium phosphate (278 mg, 1.31 mmol), and [1,1'-bis(di-tert-butylphosphino)ferrocene]dichloropalladium(II) (42.7 mg, 65.6 µmol) were dissolved in dioxane (6.00 mL) and water (1.60 mL), and the reaction mixture was stirred and reacted for 12 hours at 85°C. The reaction mixture was filtered through diatomite, and the filtrate was concentrated and purified by preparative silica gel thin layer chromatography (20/1, dichloromethane/methanol, Rf = 0.30) to obtain compound **78-3.** MS-ESI calculated for [M+H]⁺ 546, found 546.

### Step 3

Compound **78-3** (200 mg, 367 µmol) was dissolved in ethyl acetate (4.00 mL), and hydrochloride ethyl acetate solution (4 mol/L, 367 µL) was added thereto at 0°C, and the reaction mixture was stirred and reacted for 12 hours at 11°C. The reaction mixture was filtered, and the solid was washed with ethyl acetate (0.4 mL). The residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%, mobile phase B: acetonitrile; B%: 5% to 25%, 6 minutes); the purified solution was adjusted to pH = 9 to 10 with ammonia water, extracted with dichloromethane/methanol (10/1, 90 mL), dried over anhydrous sodium sulfate, filtrated, and concentrated to obtain compound **78.** ¹H NMR (400MHz, CD₃OD) δ = 8.34 (s, 1H), 8.17 (d, *J*=7.6 Hz, 1H), 7.74 - 7.67 (m, 2H), 7.55 (s, 1H), 7.47 - 7.44 (m, 2H), 7.36 -7.34 (m, 1H), 6.42 - 6.40 (m, 1H), 4.36 - 4.33 (m, 1H), 4.28 - 4.20 (m, 1H), 3.24 - 3.14 (m, 1H), 3.05 - 2.95 (m, 1H), 2.91 - 2.85 (m, 1H), 2.08 - 2.04 (m, 1H), 1.89 - 1.83 (m, 1H), 1.67 - 1.56 (m, 1H), 1.50 - 1.40 (m, 1H). MS-ESI calculated for [M+H]⁺ 446, found 446.

### Example 79

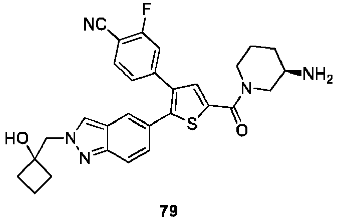

### Synthetic route:

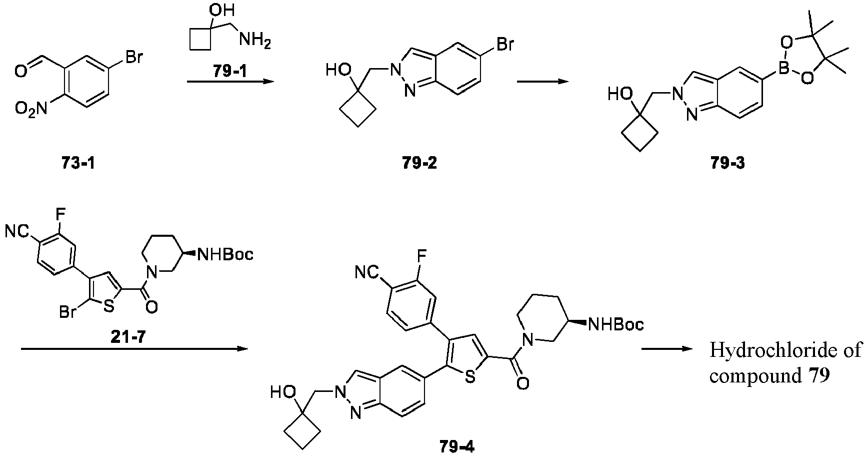

### Step 1

Compound **73-1** (3.79 g, 16.5 mmol) was dissolved in isopropanol (20.0 mL), then **79-1** (2.00 g, 19.8 mmol) was added thereto, and the reaction mixture was stirred at 80°C for 4 hours, then cooled to 25°C, added dropwise with tributylphosphine (12.2 mL, 49.4 mmol), and stirred and reacted for 12 hours at 80°C. The reaction mixture was added with water (80 mL), and extracted with ethyl acetate (80 mL × 2). The organic phase was washed with saturated brine (80 mL × 1), dried over anhydrous sodium sulfate, filtered, and concentrated under reduced pressure. The residue was purified by silica gel column chromatography (3/1, petroleum ether/ethyl acetate, Rf = 0.22) to obtain compound **79-2.** ¹H NMR (400MHz, CD₃OD) δ = 8.23 (s, 1H), 7.89 (d, *J*=1.2 Hz, 1H), 7.54 (d, *J*=9.2 Hz, 1H), 7.34 (dd, *J*=1.6, 9.2 Hz, 1H), 4.53 (s, 2H), 2.25 - 2.14 (m, 2H), 2.12 - 1.98 (m, 2H), 1.85 - 1.74 (m, 1H), 1.72 - 1.61 (m, 1H). MS-ESI calculated for [M+H]⁺ 281, found 281.

### Step 2

Under nitrogen atmosphere, compound **79-2** (1.60 g, 5.69 mmol), bis(pinacolato)diboron (4.34 g, 17.1 mmol), and potassium acetate (1.68 g, 17.1 mmol) were dissolved in dioxane (30.0 mL), and the reaction mixture was added with [1,1'-bis(diphenylphosphino)ferrocene]dichloropalladium(II) (833 mg, 1.14 mmol). The reaction mixture was stirred and reacted at 100°C for 12 hours. The reaction mixture was concentrated under reduced pressure, and the residue was purified by silica gel column chromatography (1/1, petroleum ether/ethyl acetate, R_{f} = 0.29) to obtain compound **79-3.** ¹H NMR (400MHz, CD₃OD) δ = 8.30 (s, 1H), 8.21 (s, 1H), 7.63 - 7.52 (m, 2H), 4.53 (s, 2H), 2.24 - 2.14 (m, 2H), 2.10 - 2.02 (m, 2H), 1.87 - 1.75 (m, 1H), 1.72 - 1.62 (m, 1H), 1.35 (s, 12H). MS-ESI calculated for [M+H]⁺ 329, found 329.

### Step 3

Under nitrogen atmosphere, compound **21-7** (697 mg, 1.37 mmol), compound **79-3** (900 mg, 2.74 mmol), and sodium carbonate (436 mg, 4.11 mmol) were dissolved in dioxane (24.0 mL) and water (6.00 mL), and the reaction mixture was added with [1,1'-bis(diphenylphosphino)ferrocene]dichloropalladium(II) (100 mg, 137 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.22) to obtain compound **79-4.** MS-ESI calculated for [M+H]⁺ 630, found 630.

### Step 4

Compound **79-4** (300 mg, 476 µmol) was dissolved in ethyl acetate (15.0 mL), and hydrochloride ethyl acetate solution (4 mol/L, 1.19 mL) was added thereto, and the reaction mixture was stirred and reacted for 3 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 5% to 35%, 10 minutes) to obtain the hydrochloride of compound **79.** ¹H NMR (400MHz, CD₃OD) δ = 8.63 (s, 1H), 7.94 (s, 1H), 7.75 - 7.57 (m, 3H), 7.43 - 7.22 (m, 3H), 4.66 (s, 2H), 4.47 - 4.37 (m, 1H), 4.23 - 4.10 (m, 1H), 3.61 - 3.39 (m, 3H), 2.29 - 2.16 (m, 3H), 2.15 - 2.02 (m, 2H), 1.98 - 1.67 (m, 5H). MS-ESI calculated for [M+H]⁺ 530, found 530.

### Example 80

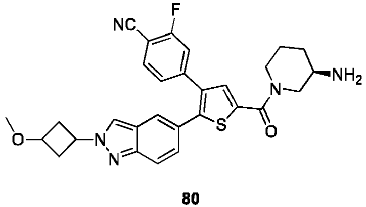

### Synthetic route:

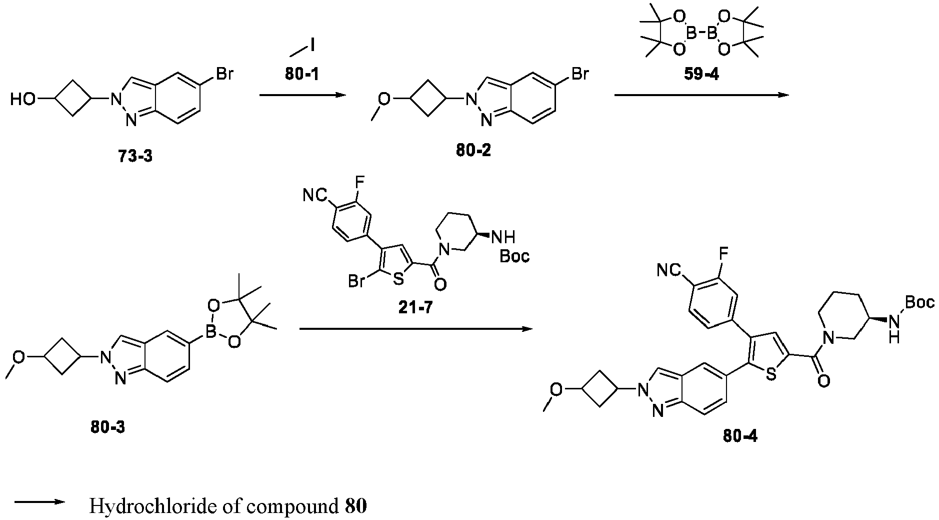

### Step 1

Under nitrogen atmosphere, compound **73-3** (1.00 g, 3.74 mmol) was dissolved in anhydrous dichloromethane (10.0 mL), and iodomethane (5.31 g, 37.4 mmol) and silver oxide (1.74 g, 7.49 mmol) were added thereto. The reaction mixture was stirred for 12 hours at 25°C, and concentrated under reduced pressure. The residue was purified by silica gel column chromatography (1/1, petroleum ether/ethyl acetate, R_{f} = 0.42, 0.51) to obtain compound **80-2.** MS-ESI calculated for [M+H]⁺ 281, found 281.

### Step 2

Under nitrogen atmosphere, compound **80-2** (100 mg, 356 µmol), bis(pinacolato)diboron (271 mg, 1.07 mmol), and potassium acetate (175 mg, 1.78 mmol) were dissolved in dioxane (10.0 mL), and the reaction mixture was added with [1,1'-bis(diphenylphosphino)ferrocene]dichloropalladium(II) (52.1 mg, 71.1 µmol), and the reaction mixture was stirred and reacted for 12 hours at 95°C, and concentrated under reduced pressure. The residue was purified by silica gel column chromatography (1/1, petroleum ether/ethyl acetate, R_{f} = 0.60) to obtain compound **80-3.** MS-ESI calculated for [M+H]⁺ 329, found 329.

### Step 3

Under nitrogen atmosphere, compound **21-7** (150 mg, 295 µmol), compound **80-3** (107 mg, 325 µmol), and sodium carbonate (93.8 mg, 885 µmol) were dissolved in dioxane (8.00 mL) and water (2.00 mL), and the reaction mixture was added with [1,1'-bis(di-tert-butylphosphino)ferrocene]dichloropalladium(II) (19.2 mg, 29.5 µmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure, and the residue was purified by silica gel column chromatography (1/1, petroleum ether/ethyl acetate, Rf = 0.21) to obtain compound **80-4.** MS-ESI calculated for [M+H]⁺ 630, found 630.

### Step 4

Under nitrogen atmosphere, compound 80-4 (130 mg, 0.206 mmol) was dissolved in anhydrous dichloromethane (5.00 mL), and trimethylsilyl trifluoromethanesulfonate (91.8 mg, 0.413 mmol) was added dropwise at 0°C. The reaction mixture was stirred for 1 hour at 25°C, added with water (1 mL), concentrated under reduced pressure, and then separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 15 to 45%, 10 minutes) to obtain the hydrochloride of compound **80.** ¹H NMR (400MHz, CD₃OD) δ = 8.72 - 8.61 (m, 1H), 7.87 (s, 1H), 7.70 - 7.58 (m, 3H), 7.37 - 7.21 (m, 3H), 5.40 - 5.32 (m, 0.3H), 4.91 - 4.85 (m, 0.7H), 4.54 - 4.40 (m, 1H), 4.31 - 4.26 (m, 0.3H), 4.24 - 4.12 (m, 1H), 3.98 - 3.89 (m, 0.7H), 3.58 - 3.40 (m, 3H), 3.34 - 3.32 (m, 3H), 3.09 - 3.01 (m, 1.4H), 2.95 - 2.87 (m, 0.6H), 2.76 - 2.68 (m, 0.6H), 2.65 - 2.55 (m, 1.4H), 2.30 - 2.16 (m, 1H), 2.00 - 1.88 (m, 1H), 1.87 - 1.69 (m, 2H). MS-ESI calculated for [M+H]⁺ 530, found 530.

### Example 81

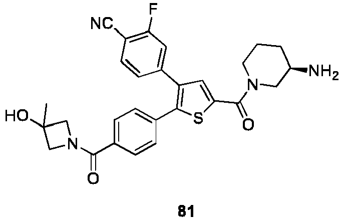

### Synthetic route:

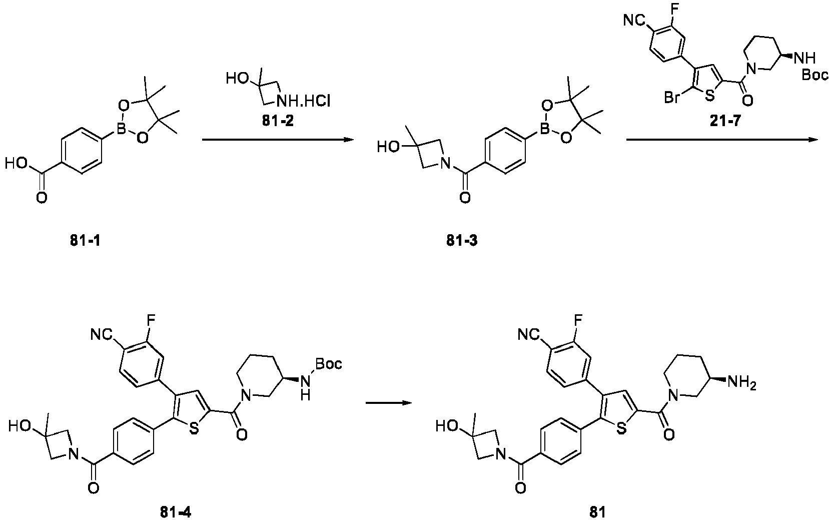

### Step 1

Under nitrogen atmosphere, compound **81-1** (200 mg, 0.806 mmol) was dissolved in anhydrous dichloromethane (10.0 mL), and then triethylamine (326 mg, 3.22 mmol), **81-2** (130 mg, 1.05 mmol), and 2-(7-azabenzotriazol-1-yl)-*N*,*N*,*N',N'*-tetramethyluronium hexafluorophosphate (399 mg, 1.05 mmol) were added thereto. The reaction mixture was stirred for 2 hours at 25°C. The reaction mixture was concentrated under reduced pressure, and the residue was purified by silica gel column chromatography (1/1, petroleum ether/ethyl acetate, R_{f} = 0.26) to obtain compound **81-3.** MS-ESI calculated for [M+H]⁺ 318, found 318.

### Step 2

Under nitrogen atmosphere, compound **21-7** (260 mg, 0.511 mmol), compound **81-3** (243 mg, 0.767 mmol), and sodium carbonate (163 mg, 1.53 mmol) were dissolved in dioxane (8.00 mL) and water (2.00 mL), and the reaction mixture was added with [1,1'-bis(di-tert-butylphosphino)ferrocene]dichloropalladium(II) (33.3 mg, 51.1 µmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **81-4.** MS-ESI calculated for [M+H]⁺ 619, found 619.

### Step 3

Compound **81-4** (200 mg, 0.323 mmol) was dissolved in ethyl acetate (5.00 mL), and hydrochloride ethyl acetate solution (4 mol/L, 5.00 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX 80 * 40 mm * 3 µm; mobile phase: mobile phase A: ammonia aqueous solution with a volume fraction of 0.05%; mobile phase B: acetonitrile; B%: 29 to 59%, 8 minutes) to obtain compound **81.** ¹H NMR (400MHz, CD₃OD) δ = 7.71 - 7.62 (m, 3H), 7.54 (s, 1H), 7.43 - 7.37 (m, 2H), 7.32 (d, *J*=11.2 Hz, 1H), 7.24 (d, *J*=8.0 Hz, 1H), 4.40 - 4.30 (m, 1H), 4.28 - 4.17 (m, 3H), 4.10 - 4.00 (m, 2H), 3.29 - 3.09 (m, 1H), 3.08 - 2.92 (m, 1H), 2.91 - 2.80 (m, 1H), 2.11 - 2.01 (m, 1H), 1.90 - 1.80 (m, 1H), 1.67 - 1.55 (m, 1H), 1.50 (s, 3H), 1.47 - 1.38 (m, 1H). MS-ESI calculated for [M+H]⁺ 519, found 519.

### Example 82

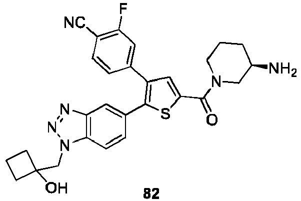

### Synthetic route:

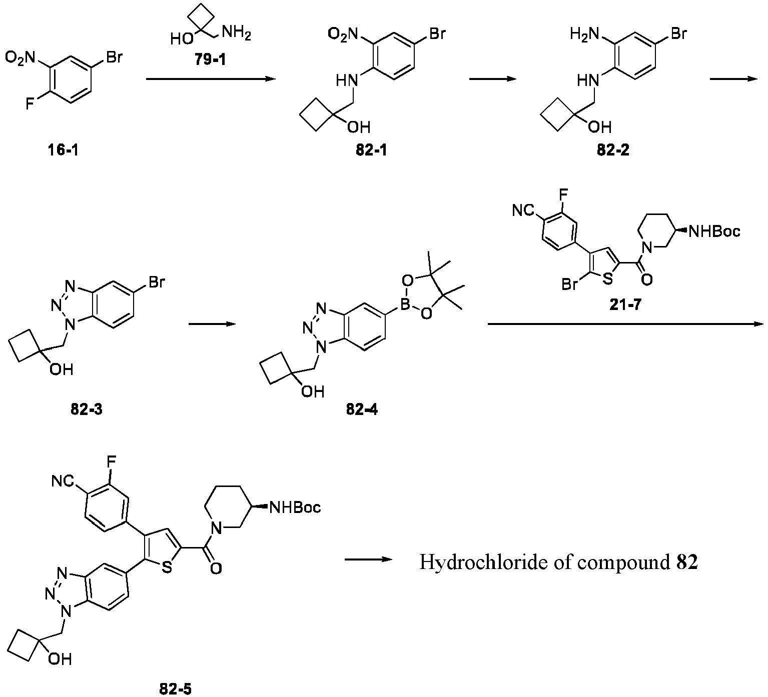

### Step 1

Compounds **16-1** (9.06 g, 41.2 mmol) and **79-1** (5.00 g, 49.4 mmol) were dissolved in *N*,*N*-dimethylformamide (40.0 mL), and *N*,*N*-diisopropylethylamine (10.7 g, 82.4 mmol) was added thereto, and the reaction mixture was stirred and reacted for 12 hours at 80°C. The reaction mixture was added with water (150 mL), and extracted with ethyl acetate (150 mL × 2). The organic phase was washed with saturated brine (150 mL × 1), dried over anhydrous sodium sulfate, filtered, and concentrated under reduced pressure. The residue was purified by silica gel column chromatography (1/1, petroleum ether/ethyl acetate, R_{f} = 0.48) to obtain compound **82-1.** ¹H NMR (400MHz, CD₃OD) δ = 8.20 (d, *J*=2.4 Hz, 1H), 7.54 (dd, *J*=2.4, 9.2 Hz, 1H), 7.03 (d, *J*=9.2 Hz, 1H), 3.44 (s, 2H), 2.22 - 2.08 (m, 4H), 1.89 - 1.75 (m, 1H), 1.73 - 1.58 (m, 1H). MS-ESI calculated for [M+H]⁺ 301, found 301.

### Step 2

Compound **82-1** (3.00 g, 9.96 mmol) was dissolved in concentrated hydrochloric acid (20.0 mL), then tin dichloride (6.74 g, 29.9 mmol) was added thereto, and the reaction mixture was stirred and reacted for 1 hour at 25°C. The reaction mixture was adjusted to pH = 13 with sodium hydroxide (1 mol/L aqueous solution), and extracted with ethyl acetate (150 mL × 2). The organic phase was washed with saturated brine (150 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **82-2.** ¹H NMR (400MHz, CD₃OD) δ = 6.83 (d, *J*=2.4 Hz, 1H), 6.77 (dd, *J*=2.4, 8.4 Hz, 1H), 6.54 (d, *J*=8.4 Hz, 1H), 3.17 (s, 2H), 2.23 - 2.04 (m, 4H), 1.87 - 1.74 (m, 1H), 1.69 - 1.55 (m, 1H). MS-ESI calculated for [M+H]⁺ 271, found 271.

### Step 3

Compound **82-2** (2.30 g, 8.48 mmol) was dissolved in acetic acid (20.0 mL), and a solution of sodium nitrite (878 mg, 12.7 mmol) in water (10.0 mL) was added dropwise thereto, and the reaction mixture was stirred for 4 hours at 25°C. The reaction mixture was added with water (50 mL), and extracted with ethyl acetate (50 mL × 2). The organic phase was washed with saturated ammonium chloride aqueous solution (50 mL), dried over anhydrous sodium sulfate, filtered, and concentrated under reduced pressure. The residue was purified by silica gel column chromatography (1/1, petroleum ether/ethyl acetate, R_{f} = 0.59) to obtain compound **82-3.** ¹H NMR (400MHz, CD₃OD) δ = 8.16 (d, *J*=1.6 Hz, 1H), 7.81 (d, *J*=8.8 Hz, 1H), 7.64 (dd, *J*=1.6, 8.8 Hz, 1H), 4.82 (s, 2H), 2.35 - 2.29 (m, 2H), 2.15 - 2.01 (m, 2H), 1.92 - 1.71 (m, 2H). MS-ESI calculated for [M+H]⁺ 282, found 282.

### Step 4

Under nitrogen atmosphere, compound **82-3** (0.90 g, 3.19 mmol), bis(pinacolato)diboron (2.43 g, 9.57 mmol), and potassium acetate (939 mg, 9.57 mmol) were dissolved in dioxane (20.0 mL), and the reaction mixture was added with [1,1'-bis(diphenylphosphino)ferrocene]dichloropalladium(II) (233 mg, 319 µmol). The reaction mixture was stirred and reacted at 100°C for 12 hours. The reaction mixture was concentrated under reduced pressure, and the residue was purified by silica gel column chromatography (1/1, petroleum ether/ethyl acetate, R_{f} = 0.37) to obtain compound **82-4.** ¹H NMR (400MHz, CD₃OD) δ = 8.35 (s, 1H), 7.87 - 7.78 (m, 2H), 4.80 (s, 2H), 2.35 - 2.28 (m, 2H), 2.11 - 2.01 (m, 2H), 1.89 - 1.68 (m, 2H), 1.38 (s, 12H). MS-ESI calculated for [M+H]⁺ 330, found 330.

### Step 5

Under nitrogen atmosphere, compound **21-7** (321 mg, 632 µmol), compound **82-4** (250 mg, 759 µmol), and sodium carbonate (201 mg, 1.90 mmol) were dissolved in dioxane (16.0 mL) and water (4.00 mL), and the reaction mixture was added with [1,1'-bis(diphenylphosphino)ferrocene]dichloropalladium(II) (46.3 mg, 63.3 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.35) to obtain compound **82-5.** MS-ESI calculated for [M+H]⁺ 631, found 631.

### Step 6

Compound **82-5** (370 mg, 587 µmol) was dissolved in ethyl acetate (10.0 mL), and hydrochloride ethyl acetate solution (4 mol/L, 1.47 mL) was added thereto, and the reaction mixture was stirred and reacted for 3 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **82.** ¹H NMR (400MHz, CD₃OD) δ = 7.97 (s, 1H), 7.87 (d, *J*=8.4 Hz, 1H), 7.70 - 7.63 (m, 2H), 7.46 - 7.34 (m, 2H), 7.27 (d, *J*=7.6 Hz, 1H), 4.85 (s, 2H), 4.46 - 4.40 (m, 1H), 4.21 - 4.17 (m, 1H), 3.63 - 3.40 (m, 3H), 2.38 - 2.30 (m, 2H), 2.25 - 2.21 (m, 1H), 2.15 - 2.03 (m, 2H), 1.98 - 1.90 (m, 1H), 1.89 - 1.71 (m, 4H). MS-ESI calculated for [M+H]⁺ 531, found 531.

### Example 83

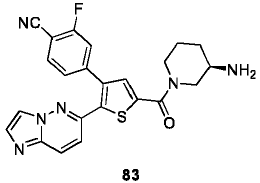

### Synthetic route:

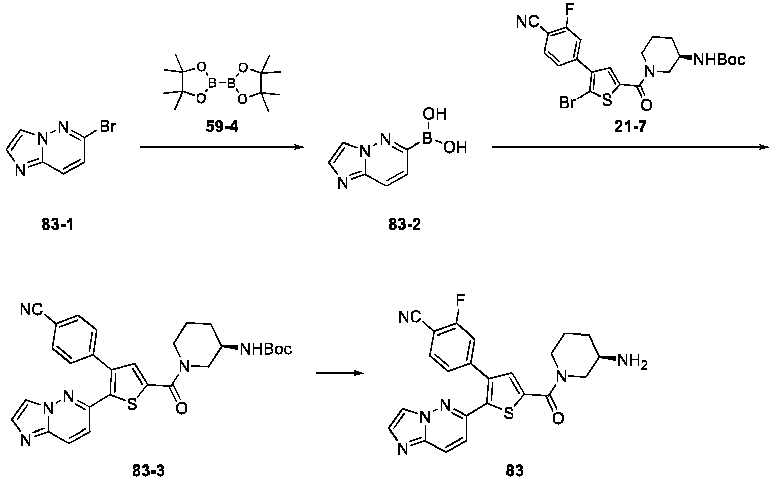

### Step 1

Under nitrogen atmosphere, compound **83-1** (200 mg, 1.01 mmol), compound **59-4** (308 mg, 1.21 mmol), potassium acetate (198 mg, 2.02 mmol), and [1,1'-bis(diphenylphosphino)ferrocene]dichloropalladium(II) dichloromethane (82.5 mg, 101 µmol) were dissolved in dioxane (7.00 mL). The reaction mixture was stirred and reacted for 12 hours at 90°C. The reaction mixture was filtered, and the solid was washed with dichloromethane (3 mL) and dried to obtain crude compound **83-2,** which was used directly in the next reaction step.

### Step 2

Under nitrogen atmosphere, compound **83-2** (170 mg, 1.04 mmol), compound **21-7** (561 mg, 1.04 mmol), potassium phosphate (443 mg, 2.09 mmol), and [1,1'-bis(di-tert-butylphosphino)ferrocene]dichloropalladium(II) (68.0 mg, 104 µmol) were dissolved in dioxane (10.0 mL) and water (2.00 mL), and the reaction mixture was stirred and reacted for 12 hours at 90°C. The reaction mixture was filtered through diatomite, and the filtrate was concentrated and purified by preparative silica gel thin layer chromatography (20/1, dichloromethane/methanol, R_{f} = 0.27) to obtain compound **83-3.** MS-ESI calculated for [M+H]⁺ 547, found 547.

### Step 3

Compound **83-3** (120 mg, 203 µmol) was dissolved in ethyl acetate (2.00 mL), and hydrochloride ethyl acetate solution (4 mol/L, 203 µL) was added thereto at 0°C, and the reaction mixture was stirred and reacted for 12 hours at 11°C. The reaction mixture was filtered, and the solid was washed with ethyl acetate (0.4 mL). The residue was purified by preparative high performance liquid chromatography (chromatographic column: Waters Xbridge150 * 25 mm * 5 µm; mobile phase: mobile phase A: ammonia aqueous solution with a volume fraction of 0.05%, mobile phase B: acetonitrile; B%: acetonitrile 18% to 48%, 9 minutes) to obtain compound **83.** ¹H NMR (400MHz, CD₃OD) δ = 8.17 (s, 1H), 7.90 (d, *J*=9.6 Hz, 1H), 7.80 - 7.75 (m, 2H), 7.56 - 7.54 (m, 2H), 7.39 - 7.36 (s, 1H), 6.99 (d, *J*=9.6 Hz, 1H), 4.33 - 4.23 (m, 2H), 3.14 - 2.99 (m, 2H), 2.90 - 2.84 (m, 1H), 2.08 - 2.04 (m, 1H), 1.89 - 1.84 (m, 1H), 1.68 - 1.58 (m, 1H), 1.50 - 1.44 (m, 1H). MS-ESI calculated for [M+H]⁺ 447, found 447.

### Example 84

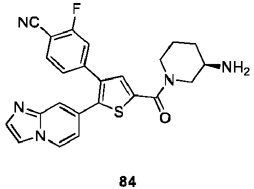

### Synthetic route:

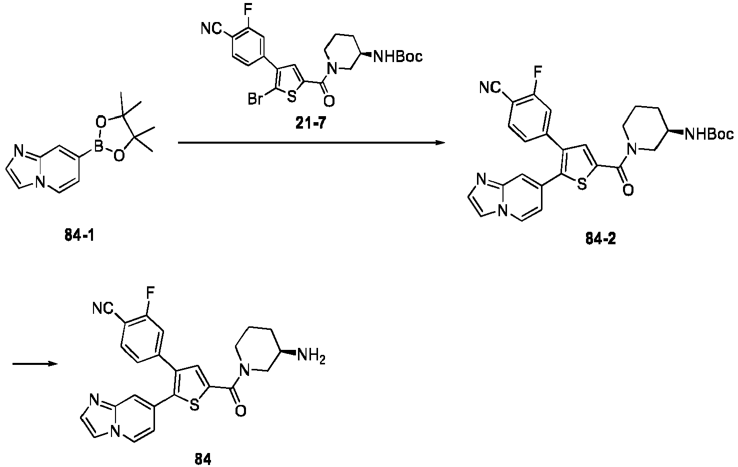

### Step 1

Under nitrogen atmosphere, compound **84-1** (111 mg, 454 µmol), compound **21-7** (244 mg, 454 µmol), potassium phosphate (193 mg, 908 µmol), and [1,1'-bis(di-tert-butylphosphino)ferrocene]dichloropalladium(II) (29.6 mg, 45.4 µmol) were dissolved in dioxane (8.00 mL) and water (1.60 mL), and the reaction mixture was stirred and reacted for 12 hours at 80°C. The reaction mixture was filtered through diatomite, and the filtrate was concentrated and purified by preparative silica gel thin layer chromatography (20/1, dichloromethane/methanol, R_{f} = 0.27) to obtain compound **84-2.** MS-ESI calculated for [M+H]⁺ 546, found 546.

### Step 2

Compound **84-2** (160 mg, 250 µmol) was dissolved in ethyl acetate (2.00 mL), and hydrochloride ethyl acetate solution (4 mol/L, 223 µL) was added thereto at 0°C, and the reaction mixture was stirred and reacted for 12 hours at 11°C. The reaction mixture was filtered. The solid was washed with ethyl acetate (0.4 mL), and the residue was purified by preparative high performance liquid chromatography (chromatographic column: 3_Phenomenex Luna C18 75 * 30 mm * 3 µm; mobile phase: mobile phase A: hydrochloric acid aqueous solution with a volume fraction of 0.05%; mobile phase B: acetonitrile; B%: 2% to 22%, retention time of 6 minutes); the purified solution was adjusted to pH = 9 to 10 with ammonia water, extracted with dichloromethane/methanol (10/1, 60 mL), dried over anhydrous sodium sulfate, filtered, and concentrated to obtain compound **84.** ¹H NMR (400MHz, CD₃OD) δ = 8.42 (d, *J*=7.2 Hz, 1H), 7.88 (s, 1H), 7.73 - 7.70 (m, 1H), 7.63 - 7.62 (m, 1H), 7.57- 7.54 (m, 2H), 7.48 - 7.45 (m, 1H), 7.34 - 7.32 (m, 1H), 6.78 - 6.76 (m, 1H), 4.37 - 4.21 (m, 2H), 3.14 - 3.13 (m, 1H), 3.03 - 2.96 (m, 1H), 2.90 - 2.84 (m, 1H), 2.08 - 2.04 (m, 1H), 1.89 - 1.84 (m, 1H), 1.67 - 1.57 (m, 1H) 1.50 - 1.40 (m, 1H). MS-ESI calculated for [M+H]⁺ 446, found 446.

### Example 85

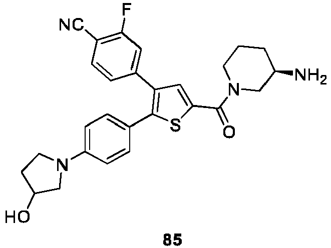

### Synthetic route:

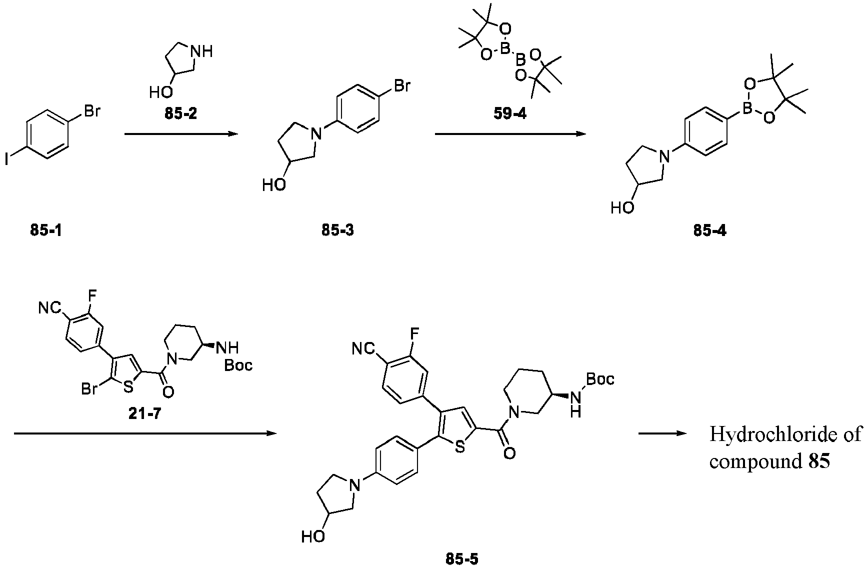

### Step 1

Under nitrogen atmosphere, compound **85-1** (3.0 g, 10.6 mmol) and compound **85-2** (1.85 g, 21.2 mmol) were dissolved in *N*,*N*-dimethylethanolamine (30.0 mL), and then potassium phosphate monohydrate (4.88 g, 21.2 mmol) and cuprous iodide (404 mg, 2.12 mmol) were added thereto, and the reaction mixture was stirred and reacted for 60 hours at 55°C. The reaction mixture was added with saturated ammonium chloride (50 mL), and extracted with dichloromethane (50 mL × 2). The organic phase was washed with saturated brine (50 mL), dried over anhydrous sodium sulfate, filtered, and concentrated under reduced pressure. The residue was purified by silica gel column chromatography (3/1, petroleum ether/ethyl acetate, R_{f} = 0.36) to obtain compound **85-3.** ¹H NMR (400MHz, CD₃OD) δ = 7.24 (d, *J*=8.8 Hz, 2H), 6.47 (d, *J*=8.8 Hz, 2H), 4.55 - 4.48 (m, 1H), 3.49 - 3.36 (m, 2H), 3.30 - 3.26 (m, 1H), 3.18 - 3.13 (m, 1H), 2.21 - 2.09 (m, 1H), 2.07 - 1.96 (m, 1H). MS-ESI calculated for [M+H]⁺ 242, found 242.

### Step 2

Under nitrogen atmosphere, compound **85-3** (550 mg, 2.27 mmol), bis(pinacolato)diboron (1.73 g, 6.82 mmol), and potassium acetate (669 mg, 6.82 mmol) were dissolved in dioxane (10.0 mL), and the reaction mixture was added with [1,1'-bis(diphenylphosphino)ferrocene]dichloropalladium(II) (166 mg, 0.227 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.60) to obtain compound **85-4.** MS-ESI calculated for [M+H]⁺ 290, found 290.

### Step 3

Under nitrogen atmosphere, compound **21-7** (300 mg, 0.590 mmol), compound **85-4** (256 mg, 0.885 mmol), and sodium carbonate (188 mg, 1.77 mmol) were dissolved in dioxane (8.00 mL) and water (2.00 mL), and the reaction mixture was added with [1,1'-bis(di-tert-butylphosphino)ferrocene]dichloropalladium(II) (38.5 mg, 0.059 mmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f}= 0.21) to obtain compound **85-5.** MS-ESI calculated for [M+H]⁺ 591, found 591.

### Step 4

Compound **85-5** (220 mg, 0.372 mmol) was dissolved in ethyl acetate (10.0 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 14 to 44%, 10 minutes) to obtain the hydrochloride of compound **85.** ¹H NMR (400MHz, CD₃OD) δ = 7.72 - 7.65 (m, 1H), 7.57 (s, 1H), 7.35 - 7.26 (m, 4H), 7.07 (d, *J*=8.4 Hz, 2H), 4.67 - 4.59 (m, 1H), 4.45 - 4.35 (m, 1H), 4.21 - 4.11 (m, 1H), 3.75 - 3.58 (m, 3H), 3.55 - 3.36 (m, 4H), 2.35 - 2.25 (m, 1H), 2.24 - 2.09 (m, 2H), 1.96 - 1.86 (m, 1H), 1.84 - 1.67 (m, 2H). MS-ESI calculated for [M+H]⁺ 491, found 491.

### Example 86

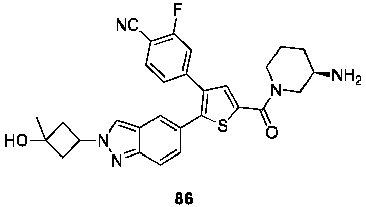

### Synthetic route:

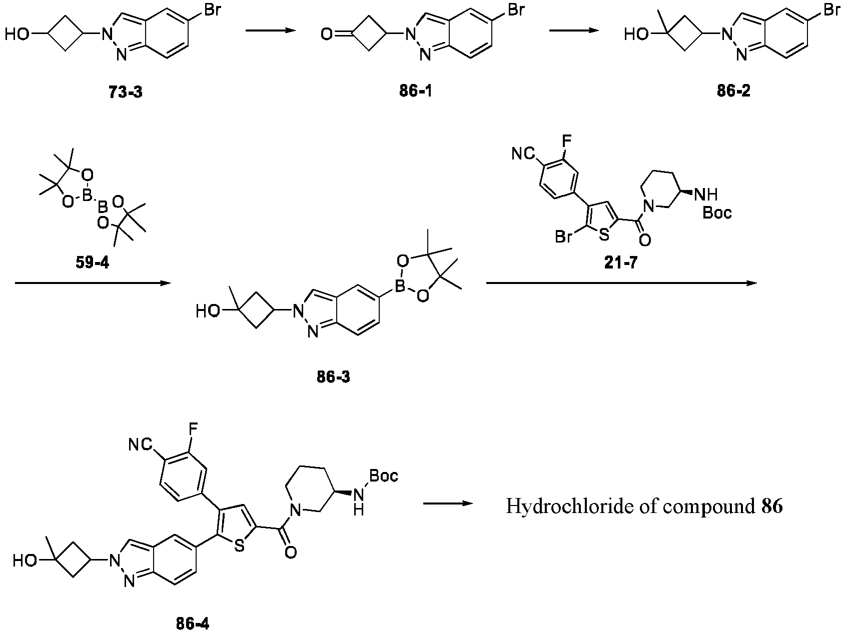

### Step 1

Under nitrogen atmosphere, compound **73-3** (900 mg, 3.37 mmol) was dissolved in anhydrous dichloromethane (10.0 mL), and Dess-Martin periodinane (1.71 g, 4.04 mmol) was added thereto. The reaction mixture was stirred for 12 hours at 25°C, and concentrated under reduced pressure. The residue was purified by silica gel column chromatography (1/1, petroleum ether/ethyl acetate, R_{f} = 0.79) to obtain compound **86-1.** MS-ESI calculated for [M+H]⁺ 266, found 266.

### Step 2

Under nitrogen atmosphere, compound **86-1** (580 mg, 1.64 mmol) was dissolved in anhydrous tetrahydrofuran (10.0 mL), and methyl lithium (1.6 mol/L ether solution, 2.36 mL) was added thereto at 0°C, and the reaction mixture was stirred for 12 hours at 25°C, added with saturated ammonium chloride aqueous solution (30 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.20) to obtain compound **86-2.** ¹H NMR (400MHz, CDCl₃) δ = 7.89 (s, 1H), 7.77 (d, *J*=1.6 Hz, 1H), 7.57 (d, *J*=8.8 Hz, 1H), 7.31 (dd, *J*=2.0, 8.8 Hz, 1H), 4.76 - 4.68 (m, 1H), 2.82 (d, *J*=7.6 Hz, 4H), 1.50 (s, 3H). MS-ESI calculated for [M+H]⁺ 281, found 281.

### Step 3

Under nitrogen atmosphere, compound **86-2** (110 mg, 0.391 mmol), bis(pinacolato)diboron (298 mg, 1.17 mmol), and potassium acetate (192 mg, 1.96 mmol) were dissolved in dioxane (10.0 mL), and the reaction mixture was added with [1,1'-bis(diphenylphosphino)ferrocene]dichloropalladium(II) (57.3 mg, 78.3 µmol), and the reaction mixture was stirred and reacted for 12 hours at 95°C, and concentrated under reduced pressure. The residue was purified by silica gel column chromatography (1/1, petroleum ether/ethyl acetate, Rf = 0.60) to obtain compound **86-3.** MS-ESI calculated for [M+H]⁺ 329, found 329.

### Step 4

Under nitrogen atmosphere, compound **21-7** (120 mg, 0.236 mmol), compound **86-3** (85.2 mg, 0.260 mmol), and sodium carbonate (75.1 mg, 0.708 mmol) were dissolved in dioxane (8.00 mL) and water (2.00 mL), and the reaction mixture was added with [1,1'-bis(di-tert-butylphosphino)ferrocene]dichloropalladium(II) (15.4 mg, 0.024 mmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.21) to obtain compound **86-4.** MS-ESI calculated for [M+H]⁺ 630, found 630.

### Step 5

Under nitrogen atmosphere, compound **86-4** (70.0 mg, 0.111 mmol) was dissolved in anhydrous dichloromethane (5.00 mL), and trimethylsilyl trifluoromethanesulfonate (49.4 mg, 0.222 mmol) was added dropwise at 0°C. The reaction mixture was stirred for 1 hour at 25°C, added with water (1 mL), concentrated under reduced pressure, and then separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **86.** ¹H NMR (400MHz, CD₃OD) δ = 8.51 (s, 1H), 7.82 (s, 1H), 7.67 - 7.60 (m, 3H), 7.33 (d, *J*=10.0 Hz, 1H), 7.29 - 7.20 (m, 2H), 4.87 - 4.84 (m, 1H), 4.47 - 4.37 (m, 1H), 4.24 - 4.14 (m, 1H), 3.56 - 3.39 (m, 3H), 2.78 (d, *J*=8.0 Hz, 4H), 2.27 - 2.16 (m, 1H), 1.98 - 1.89 (m, 1H), 1.85 - 1.69 (m, 2H), 1.49 (s, 3H). MS-ESI calculated for [M+H]⁺ 530, found 530.

### Example 87

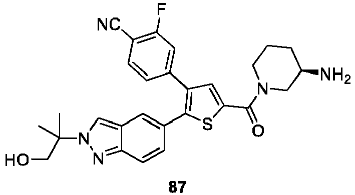

### Synthetic route:

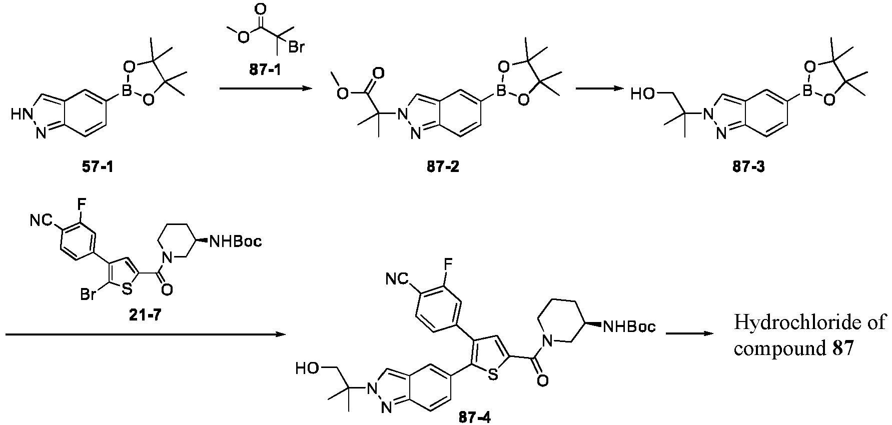

### Step 1

Compounds **57-1** (2.50 g, 10.2 mmol) and **87-1** (5.56 g, 30.7 mmol) were dissolved in acetonitrile (40.0 mL), and potassium carbonate (4.25 g, 30.7 mmol) and potassium iodide (850 mg, 5.12 mmol) were added thereto, and the reaction mixture was stirred and reacted for 12 hours at 85°C. The reaction mixture was concentrated under reduced pressure, and the residue was purified by silica gel column chromatography (1/1, petroleum ether/ethyl acetate, Rf = 0.38) to obtain compound **87-2.** ¹H NMR (400MHz, CD₃OD) δ = 8.48 (s, 1H), 8.24 (s, 1H), 7.60 (d, *J*=8.8 Hz, 1H), 7.54 (d, *J*=8.8 Hz, 1H), 3.69 (s, 3H), 1.97 (s, 6H), 1.36 (s, 12H). MS-ESI calculated for [M+H]⁺ 345, found 345.

### Step 2

Lithium borohydride (285 mg, 13.1 mmol) was dissolved in tetrahydrofuran (30.0 mL), and a solution of **87-2** (1.50 g, 4.36 mmol) in tetrahydrofuran (30.0 mL) was added dropwise thereto, and the reaction mixture was stirred and reacted for 0.5 hours at 25°C, then stirred and reacted for another 12 hours at 70°C. The reaction mixture was added dropwise with saturated ammonium chloride aqueous solution (30 mL) at 0°C, and extracted with ethyl acetate (30 mL × 2). The organic phases were combined, dried over anhydrous sodium sulfate, filtered, and concentrated to obtain crude compound **87-3,** which was used directly in the next reaction step. MS-ESI calculated for [M+H]⁺ 317, found 317.

### Step 3

Under nitrogen atmosphere, compound **21-7** (502 mg, 790 µmol), compound **87-3** (300 mg, 949 µmol), and sodium carbonate (251 mg, 2.37 mmol) were dissolved in dioxane (24.0 mL) and water (6.00 mL), and the reaction mixture was added with [1,1'-bis(diphenylphosphino)ferrocene]dichloropalladium(II) (57.9 mg, 79.1 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.37) to obtain compound **87-4.** MS-ESI calculated for [M+H]⁺ 618, found 618.

### Step 4

Compound **87-4** (320 mg, 518 µmol) was dissolved in ethyl acetate (10.0 mL), and hydrochloride ethyl acetate solution (4 mol/L, 1.30 mL) was added thereto, and the reaction mixture was stirred and reacted for 2 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 5% to 35%, 10 minutes) to obtain the hydrochloride of compound **87.** ¹H NMR (400MHz, CD₃OD) δ = 8.67 (s, 1H), 7.89 (s, 1H), 7.68 - 7.59 (m, 3H), 7.38 - 7.25 (m, 3H), 4.45 - 4.39 (m, 1H), 4.20 - 4.16 (m, 1H), 3.89 (s, 2H), 3.63 - 3.38 (m, 3H), 2.22 - 2.18 (m, 1H), 1.94 - 1.90 (m, 1H), 1.84 - 1.74 (m, 8H). MS-ESI calculated for [M+H]⁺ 518, found 518.

### Example 88

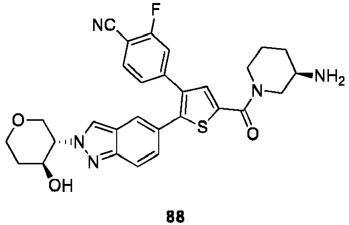

### Synthetic route:

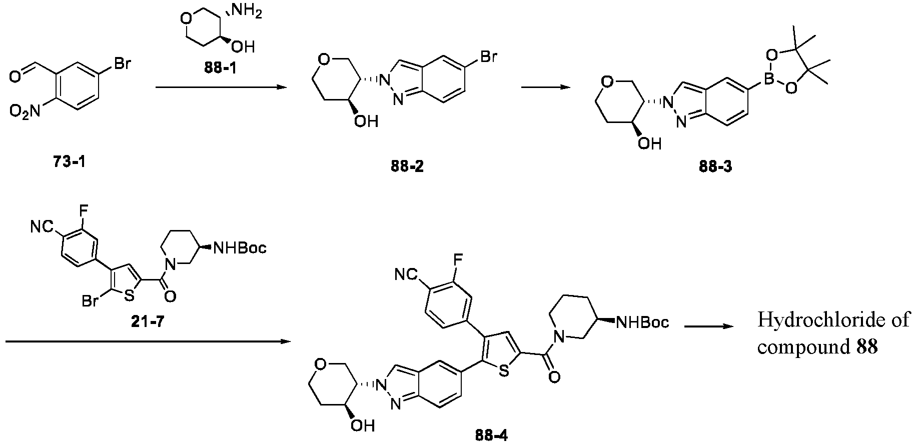

### Step 1

Compound **73-1** (1.50 g, 6.52 mmol) was dissolved in isopropanol (20.0 mL), then **88-1** (917 mg, 7.82 mmol) was added thereto, and the reaction mixture was stirred at 80°C for 4 hours, then cooled to 25°C, added dropwise with tributylphosphine (4.83 mL, 19.6 mmol), stirred, and reacted for 12 hours at 80°C. The reaction mixture was added with saturated ammonium chloride aqueous solution (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, Rf = 0.35) to obtain compound 88-2. ¹H NMR (400MHz, CD₃OD) δ = 8.28 (s, 1H), 7.91 - 7.86 (m, 1H), 7.54 (d, *J*=9.2 Hz, 1H), 7.35 (dd, *J*=2.0, 9.2 Hz, 1H), 4.41 - 4.23 (m, 2H), 4.16 - 4.09 (m, 1H), 4.07 - 4.01 (m, 1H), 3.80 - 3.75 (m, 1H), 3.67 - 3.55 (m, 1H), 2.12 - 2.08 (m, 1H), 1.85 - 1.70 (m, 1H). MS-ESI calculated for [M+H]⁺ 299, found 299.

### Step 2

Under nitrogen atmosphere, compound **88-2** (700 mg, 2.36 mmol), bis(pinacolato)diboron (1.79 g, 7.07 mmol), and potassium acetate (694 mg, 7.07 mmol) were dissolved in dioxane (20.0 mL), and the reaction mixture was added with [1,1'-bis(diphenylphosphino)ferrocene]dichloropalladium(II) (172 mg, 236 µmol). The reaction mixture was stirred and reacted at 100°C for 12 hours. The reaction mixture was concentrated under reduced pressure, and the residue was purified by silica gel column chromatography (1/1, petroleum ether/ethyl acetate, R_{f} = 0.21) to obtain compound **88-3.** MS-ESI calculated for [M+H]⁺ 345, found 345.

### Step 3

Under nitrogen atmosphere, compound **21-7** (369 mg, 726 µmol), compound **88-3** (300 mg, 872 µmol), and sodium carbonate (231 mg, 2.18 mmol) were dissolved in dioxane (24.0 mL) and water (6.00 mL), and the reaction mixture was added with [1,1'-bis(diphenylphosphino)ferrocene]dichloropalladium(II) (53.1 mg, 72.6 µmol), and the reaction mixture was stirred and reacted for 12 hours at 100°C. The reaction mixture was concentrated under reduced pressure, and the residue was purified by silica gel column chromatography (1/2, petroleum ether/ethyl acetate, R_{f} = 0.24) to obtain compound **88-4.** MS-ESI calculated for [M+H]⁺ 646, found 646.

### Step 4

Compound **88-4** (210 mg, 325 µmol) was dissolved in ethyl acetate (10.0 mL), and hydrochloride ethyl acetate solution (4 mol/L, 0.813 mL) was added thereto, and the reaction mixture was stirred and reacted for 2 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **88.** ¹H NMR (400MHz, CD₃OD) δ = 8.52 (s, 1H), 7.85 (s, 1H), 7.71 - 7.60 (m, 3H), 7.38 - 7.27 (m, 2H), 7.22 (dd, *J*=1.6, 8.8 Hz, 1H), 4.50 - 4.40 (m, 2H), 4.35 - 4.31 (m, 1H), 4.22 - 4.15 (m, 2H), 4.12 - 4.05 (m, 1H), 3.93 - 3.87 (m, 1H), 3.72 - 3.60 (m, 1H), 3.58 - 3.41 (m, 3H), 2.25 - 2.19 (m, 1H), 2.18 - 2.09 (m, 1H), 1.90 - 1.86 (m, 1H), 1.88 - 1.70 (m, 3H). MS-ESI calculated for [M+H]⁺ 546, found 546.

### Example 89

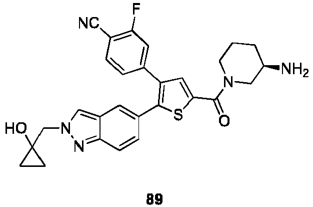

### Synthetic route:

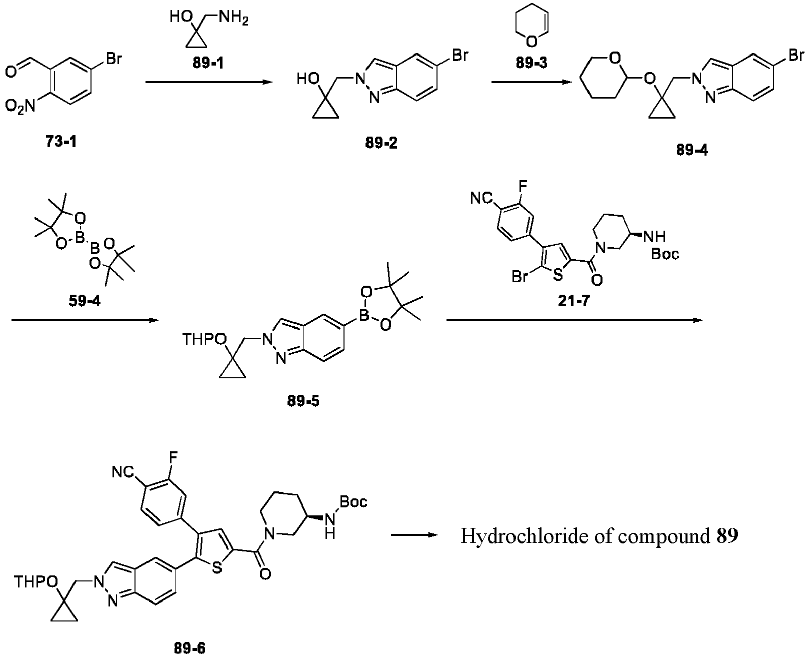

### Step 1

Under nitrogen atmosphere, compound **73-1** (2.0 g, 8.70 mmol) was dissolved in isopropanol (20.0 mL), and 1-(aminomethyl)-1-cyclopropanol (871 mg, 0.478 mmol) was added thereto, and the reaction mixture was stirred for 4 hours at 80°C, added with tributylphosphine (5.28 g, 26.1 mmol) at 0°C, stirred for 12 hours at 80°C, added with saturated ammonium chloride (30 mL), and extracted with ethyl acetate (30 mL × 1). The organic phase was washed with saturated brine (30 mL × 1), dried over anhydrous sodium sulfate, filtered, concentrated under reduced pressure, and the residue was purified by silica gel column chromatography (1/1, petroleum ether/ethyl acetate, R_{f} = 0.20) to obtain compound **89-2.** ¹H NMR (400MHz, CDCl₃) δ = 7.94 (s, 1H), 7.84 (s, 1H), 7.61 (d, *J*=9.2 Hz, 1H), 7.38 (dd, *J*=1.6, 9.2 Hz, 1H), 4.48 (s, 2H), 1.01 - 0.95 (m, 2H), 0.81 - 0.75 (m, 2H). MS-ESI calculated for [M+H]⁺ 267, found 267.

### Step 2

Under nitrogen atmosphere, compound **89-2** (100 mg, 374 µmol) was dissolved in anhydrous dichloromethane (10.0 mL), and then 2,3-dihydropyran (142 mg, 1.68 mmol) and pyridinium p-toluenesulfonate (18.8 mg, 74.9 µmol) were added thereto. The reaction mixture was stirred for 12 hours at 25°C, and concentrated under reduced pressure. The residue was purified by silica gel column chromatography (1/1, petroleum ether/ethyl acetate, Rf = 0.68) to obtain compound **89-4.** MS-ESI calculated for [M+H]⁺ 353, found 353.

### Step 3

Under nitrogen atmosphere, compound **89-4** (120 mg, 0.342 mmol), bis(pinacolato)diboron (260 mg, 1.02 mmol), and potassium acetate (168 mg, 1.71 mmol) were dissolved in dioxane (10.0 mL), and the reaction mixture was added with [1,1'-bis(diphenylphosphino)ferrocene]dichloropalladium(II) (50.0 mg, 68.3 µmol), and the reaction mixture was stirred and reacted for 12 hours at 95°C, and concentrated under reduced pressure. The residue was purified by silica gel column chromatography (1/1, petroleum ether/ethyl acetate, Rf = 0.60) to obtain compound **89-5.** ¹H NMR (400MHz, CDCl₃) δ = 8.30 - 8.26 (m, 2H), 7.68 - 7.64 (m, 2H), 5.04 (d, *J*=14.4 Hz, 1H), 4.74 - 4.69 (m, 1H), 4.31 (d, *J*=14.4 Hz, 1H), 3.85 - 3.75 (m, 1H), 3.51 - 3.42 (m, 1H), 1.78 - 1.68 (m, 1H), 1.64 - 1.55 (m, 1H), 1.52 - 1.42 (m, 4H), 1.37 (s, 12H), 1.21 - 1.15 (m, 1H), 1.02 - 0.91 (m, 2H), 0.85 - 0.79 (m, 1H). MS-ESI calculated for [M+H]⁺ 399, found 399.

### Step 4

Under nitrogen atmosphere, compound **21-7** (150 mg, 0.295 mmol), compound **89-5** (123 mg, 0.310 mmol), and sodium carbonate (93.8 mg, 0.885 mmol) were dissolved in dioxane (8.00 mL) and water (2.00 mL), and the reaction mixture was added with [1,1'-bis(diphenylphosphino)ferrocene]dichloropalladium(II) (21.6 mg, 25.9 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf = 0.30) to obtain compound **89-6.** ¹H NMR (400MHz, CDCl₃) δ = 8.32 (s, 1H), 7.75 (s, 1H), 7.70 - 7.52 (m, 2H), 7.46 - 7.36 (m, 1H), 7.34 - 7.27 (m, 1H), 7.20 - 6.95 (m, 2H), 5.15 - 5.03 (m, 1H), 4.79 - 4.57 (m, 3H), 4.54 - 4.37 (m, 1H), 4.23 (d, *J*=14.4 Hz, 1H), 3.93 - 3.83 (m, 1H), 3.75 - 3.59 (m, 1H), 3.56 - 3.45 (m, 1H), 3.07 - 2.82 (m, 2H), 2.12 - 2.06 (m, 1H), 1.95 - 1.82 (m, 2H), 1.83 - 1.75 (m, 1H), 1.69 - 1.59 (m, 2H), 1.54 - 1.49 (m, 3H), 1.47 (s, 9H), 1.21 - 1.11 (m, 1H), 1.04 - 0.91 (m, 2H), 0.88 - 0.80 (m, 1H). MS-ESI calculated for [M+H]⁺ 700, found 700.

### Step 5

Compound **89-6** (150 mg, 0.214 mmol) was dissolved in ethyl acetate (5.00 mL), and hydrochloride ethyl acetate solution (4 mol/L, 5 mL) was added dropwise thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **89.** ¹H NMR (400MHz, CD₃OD) δ = 8.88 (s, 1H), 8.04 (s, 1H), 7.73 (d, *J*=8.8 Hz, 1H), 7.69 - 7.64 (m, 2H), 7.49 (d, *J*=8.8 Hz, 1H), 7.36 (d, *J*=10.4 Hz, 1H), 7.30 (d, *J*=8.0 Hz, 1H), 4.72 (s, 2H), 4.51 - 4.39 (m, 1H), 4.25 - 4.11 (m, 1H), 3.60 - 3.41 (m, 3H), 2.27 - 2.18 (m, 1H), 2.00 - 1.89 (m, 1H), 1.87 - 1.69 (m, 2H), 1.02 - 0.96 (m, 2H), 0.96 - 0.89 (m, 2H). MS-ESI calculated for [M+H]⁺ 516, found 516.

### Example 90

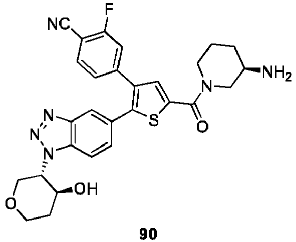

### Synthetic route:

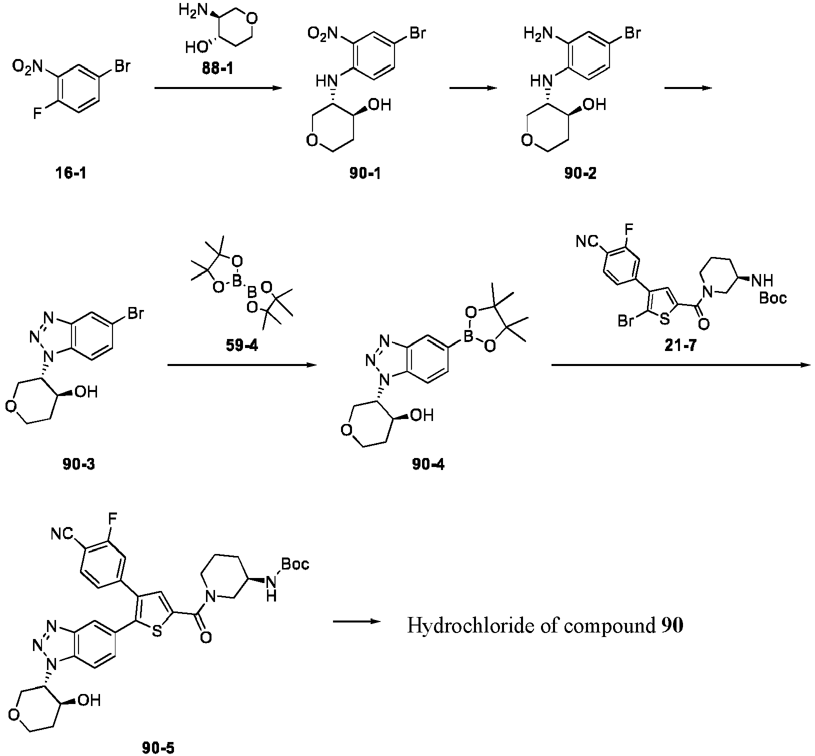

### Step 1

Under nitrogen atmosphere, compound **16-1** (2.00 g, 9.09 mmol) and (3*S*,4*S*)-3-aminotetrahydro-2H-pyran-4-ol (1.28 g, 10.9 mmol) were dissolved in *N,N-*dimethylformamide (10.0 mL), and *N*,*N*-diisopropylethylamine (2.35 g, 18.2 mmol) was added thereto, and the reaction mixture was stirred and reacted for 12 hours at 80°C, added with water (20 mL), extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.19) to obtain compound **90-1.** MS-ESI calculated for [M+H]⁺ 319, found 319.

### Step 2

Compound **90-1** (1.30 g, 4.10 mmol) was dissolved in concentrated hydrochloric acid (15.0 mL), and tin dichloride (5.55 g, 24.6 mmol) was added thereto, and the reaction mixture was stirred and reacted for 12 hours at 25°C. The reaction mixture was adjusted to pH = 13 with sodium hydroxide aqueous solution (10 mol/L), and extracted with ethyl acetate (50 mL × 1). The organic phase was washed with saturated brine (50 mL × 1), dried over anhydrous sodium sulfate, filtered, and concentrated under reduced pressure to obtain compound **90-2.** MS-ESI calculated for [M+H]⁺ 287, found 287.

### Step 3

Compound **90-2** (1.10 g, 3.83 mmol) was dissolved in acetic acid (16.0 mL), and a solution of sodium nitrite (396 mg, 5.75 mmol) in water (8.00 mL) was added dropwise thereto, and the reaction mixture was stirred for 1 hour at 25°C, added with water (30 mL), and extracted with ethyl acetate (50 mL × 2). The organic phase was sequentially washed with saturated sodium bicarbonate aqueous solution (100 mL × 3) and saturated brine (100 mL × 1), dried over anhydrous sodium sulfate, filtered, and concentrated under reduced pressure. The residue was purified by silica gel column chromatography (1/1, petroleum ether/ethyl acetate, Rf = 0.51) to obtain compound **90-3.** MS-ESI calculated for [M+H]⁺ 298, found 298.

### Step 4

Under nitrogen atmosphere, compound **90-3** (380 mg, 1.27 mmol), bis(pinacolato)diboron (1.02 g, 4.02 mmol), and potassium acetate (658 mg, 6.70 mmol) were dissolved in dioxane (10.0 mL), and the reaction mixture was added with [1,1'-bis(diphenylphosphino)ferrocene]dichloropalladium(II) (98.1 mg, 0.134 mmol), and the reaction mixture was stirred and reacted for 12 hours at 100°C, and concentrated under reduced pressure. The residue was purified by silica gel column chromatography (1/1, petroleum ether/ethyl acetate, R_{f} = 0.21) to obtain compound **90-4.** MS-ESI calculated for [M+H]⁺ 346, found 346.

### Step 5

Under nitrogen atmosphere, compound **21-7** (400 mg, 0.787 mmol), compound **90-4** (407 mg, 1.18 mmol), and sodium carbonate (250 mg, 2.36 mmol) were dissolved in dioxane (8.00 mL) and water (2.00 mL), and the reaction mixture was added with [1,1'-bis(di-tert-butylphosphino)ferrocene]dichloropalladium(II) (57.6 mg, 78.7 µmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf = 0.21) to obtain compound **90-5.** MS-ESI calculated for [M+H]⁺ 647, found 647.

### Step 6

Compound **90-5** (400 mg, 0.214 mmol) was dissolved in ethyl acetate (10.0 mL), and hydrochloride ethyl acetate solution (4 mol/L, 10.0 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 5 to 35%, 10 minutes) to obtain the hydrochloride of compound **90.** ¹H NMR (400MHz, CD₃OD) δ = 7.96 (s, 1H), 7.86 (d, *J*=8.8 Hz, 1H), 7.67 - 7.60 (m, 2H), 7.42 (d, *J*=9.2 Hz, 1H), 7.34 (d, *J*=10.0 Hz, 1H), 7.25 (d, *J*=8.8 Hz, 1H), 4.78 - 4.68 (m, 1H), 4.49 - 4.41 (m, 1H), 4.40 - 4.32 (m, 1H), 4.24 - 4.13 (m, 2H), 4.12 - 3.99 (m, 2H), 3.67 (m, 1H), 3.60 - 3.41 (m, 3H), 2.28 - 2.12 (m, 2H), 1.99 - 1.67 (m, 4H). MS-ESI calculated for [M+H]⁺ 547, found 547.

### Example 91

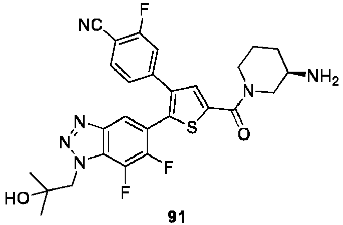

### Synthetic route:

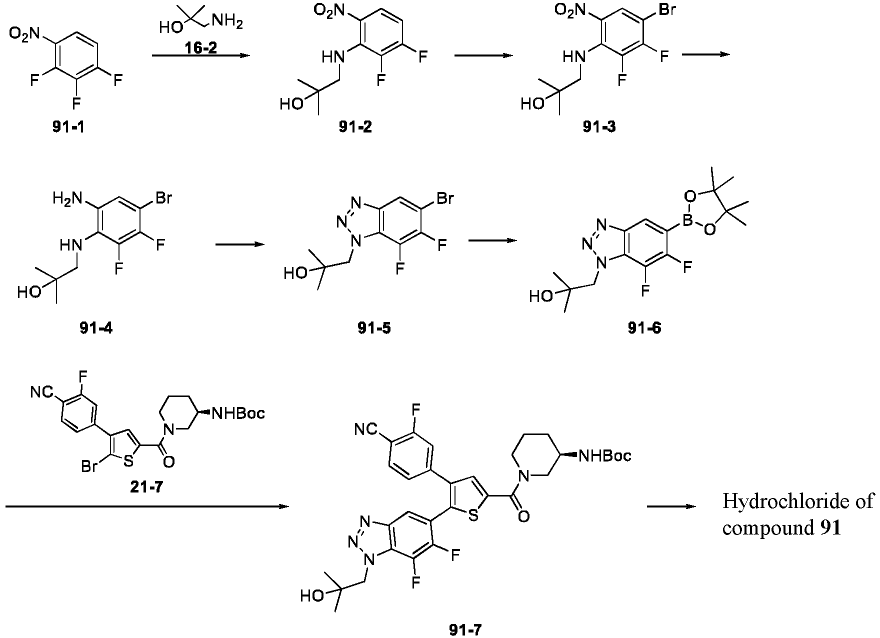

### Step 1

Compounds **91-1** (3.05 g, 17.2 mmol) and **16-2** (1.84 g, 20.7 mmol) were dissolved in *N*,*N*-dimethylformamide (20.0 mL), and *N,N*-*diisopropylethylamine* (6.68 g, 51.7 mmol) was added thereto, and the reaction mixture was stirred and reacted for 12 hours at 80°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (4/1, petroleum ether/ethyl acetate, R_{f}= 0.41) to obtain compound **91-2.** ¹H NMR (400MHz, CD₃OD) δ = 8.13-8.01 (m, 1H), 6.68 - 6.54 (m, 1H), 3.58 (d, *J*=3.2 Hz, 2H), 1.28 (s, 6H). MS-ESI calculated for [M+H]⁺ 247, found 247.

### Step 2

Compound **91-2** (1.50 g, 6.09 mmol) was dissolved in *N*,*N*-dimethylformamide (20.0 mL). *N*-Bromosuccinimide (1.41 g, 7.92 mmol) was added thereto, and the reaction mixture was stirred and reacted for 2 hours at 90°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (3/1, petroleum ether/ethyl acetate, Rf = 0.60) to obtain compound **91-3.** ¹H NMR (400MHz, CD₃OD) δ = 8.27 - 8.22 (m, 1H), 3.59 (d, *J*=3.6 Hz, 2H), 1.32 - 1.26 (m, 6H). MS-ESI calculated for [M+H]⁺ 325, found 325.

### Step 3

Compound **91-3** (1.70 g, 5.23 mmol) was dissolved in concentrated hydrochloric acid (20.0 mL), then tin dichloride (7.08 g, 31.4 mmol) was added thereto, and the reaction mixture was stirred and reacted for 4 hours at 25°C. The reaction mixture was adjusted to pH = 13 with sodium hydroxide aqueous solution (4 mol/L), and extracted with ethyl acetate (50 mL × 2). The organic phase was washed with saturated brine (50 mL × 1), dried over anhydrous sodium sulfate, filtered, and concentrated under reduced pressure to obtain crude compound **91-4,** which was used directly in the next reaction step. MS-ESI calculated for [M+H]⁺ 295, found 295.

### Step 4

Compound **91-4** (1.50 g, 5.08 mmol) was dissolved in acetic acid (20.0 mL), and a solution of sodium nitrite (526 mg, 7.62 mmol) in water (10.0 mL) was added dropwise thereto, and the reaction mixture was stirred for 2 hours at 25°C. The reaction mixture was added with water (50 mL), and extracted with ethyl acetate (50 mL × 2). The organic phase was washed with saturated ammonium chloride aqueous solution (50 mL), dried over anhydrous sodium sulfate, filtered, and concentrated under reduced pressure. The residue was purified by silica gel column chromatography (2/1, petroleum ether/ethyl acetate, R_{f} = 0.59) to obtain compound **91-5.** ¹H NMR (400MHz, CD₃OD) δ = 8.20 - 8.14 (m, 1H), 4.75 (s, 2H), 1.34 - 1.26 (m, 6H). MS-ESI calculated for [M+H]⁺ 306, found 306.

### Step 5

Under nitrogen atmosphere, compound **91-5** (600 mg, 1.96 mmol), bis(pinacolato)diboron (1.49 g, 5.88 mmol), and potassium acetate (577 mg, 5.88 mmol) were dissolved in dioxane (20.0 mL), and the reaction mixture was added with [1,1'-bis(diphenylphosphino)ferrocene]dichloropalladium(II) (143 mg, 196 µmol). The reaction mixture was stirred and reacted at 100°C for 12 hours. The reaction mixture was concentrated under reduced pressure, and the residue was purified by silica gel column chromatography (2/1, petroleum ether/ethyl acetate, Rf = 0.42) to obtain compound **91-6.** ¹H NMR (400MHz, CD₃OD) δ = 8.13 - 8.10 (m, 1H), 4.72 (s, 2H), 1.40 (s, 12H), 1.28 (s, 6H). MS-ESI calculated for [M+H]⁺ 354, found 354.

### Step 6

Under nitrogen atmosphere, compound **21-7** (300 mg, 590 µmol), compound **91-6** (250 mg, 707 µmol), and sodium carbonate (188 mg, 1.77 mmol) were dissolved in dioxane (16.0 mL) and water (4.00 mL), and the reaction mixture was added with [1,1'-bis(diphenylphosphino)ferrocene]dichloropalladium(II) (46.3 mg, 43.2 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.24) to obtain compound **91-7.** MS-ESI calculated for [M+H]⁺ 655, found 655.

### Step 7

Compound **91-7** (240 mg, 367 µmol) was dissolved in ethyl acetate (10.0 mL), and hydrochloride ethyl acetate solution (4 mol/L, 0.916 mL) was added thereto, and the reaction mixture was stirred and reacted for 2 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **91.** ¹H NMR (400MHz, CD₃OD) δ = 8.10 - 7.98 (m, 1H), 7.72 (s, 1H), 7.70 - 7.64 (m, 1H), 7.43 - 7.36 (m, 1H), 7.29 - 7.23 (m, 1H), 4.76 (s, 2H), 4.43 - 3.99 (m, 1H), 4.19 - 4.17 (m, 1H), 3.65 - 3.41 (m, 3H), 2.24 - 2.20 (m, 1H), 2.00 - 1.89 (m, 1H), 1.86 - 1.71 (m, 2H), 1.31 (s, 6H). MS-ESI calculated for [M+H]⁺ 555, found 555.

### Example 92

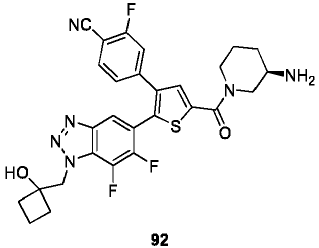

### Synthetic route:

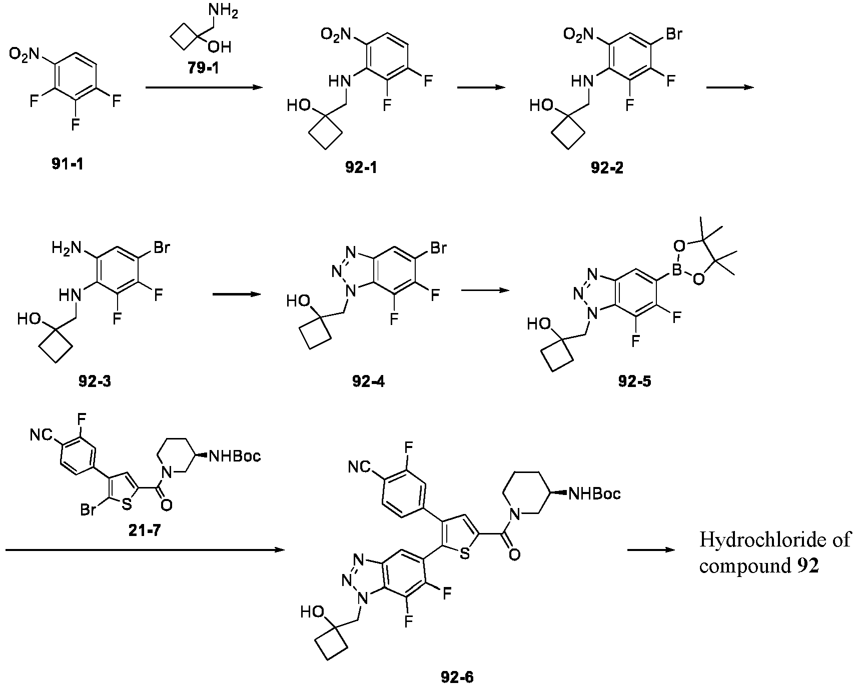

### Step 1

Compounds **91-1** (3.00 g, 16.9 mmol) and **79-1** (2.06 g, 20.3 mmol) were dissolved in *N*,*N*-dimethylformamide (20.0 mL), and *N*,*N*-diisopropylethylamine (6.57 g, 50.8 mmol) was added thereto, and the reaction mixture was stirred and reacted for 12 hours at 80°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (2/1, petroleum ether/ethyl acetate, Rf= 0.38) to obtain compound **92-1.** ¹H NMR (400MHz, CD₃OD) δ = 8.06 - 7.94 (m, 1H), 6.68 - 6.52 (m, 1H), 3.73 (d, *J*=3.2 Hz, 2H), 2.21 - 2.05 (m, 4H), 1.91 - 1.72 (m, 1H), 1.70 - 1.53 (m, 1H). MS-ESI calculated for [M+H]⁺ 259, found 259.

### Step 2

Compound **92-1** (2.00 g, 7.75 mmol) was dissolved in *N*,*N*-dimethylformamide (20.0 mL). *N*-Bromosuccinimide (1.79 g, 10.1 mmol) was added thereto, and the reaction mixture was stirred and reacted for 2 hours at 90°C. The reaction mixture was added with water (100 mL), and extracted with ethyl acetate (100 mL × 2). The organic phase was washed with saturated brine (100 mL × 1), dried over anhydrous sodium sulfate, filtered, and concentrated under reduced pressure. The residue was purified by silica gel column chromatography (3/1, petroleum ether/ethyl acetate, Rf = 0.64) to obtain compound **92-2.** ¹H NMR (400MHz, CD₃OD) δ = 8.24 - 8.20 (m, 1H), 3.72 (d, *J*=3.6 Hz, 2H), 2.16 - 2.06 (m, 4H), 1.86 - 1.74 (m, 1H), 1.68 - 1.55 (m, 1H). MS-ESI calculated for [M+H]⁺ 337 and 339, found 337 and 339.

### Step 3

Compound **92-2** (2.04 g, 6.05 mmol) was dissolved in concentrated hydrochloric acid (30.0 mL), then tin dichloride (8.19 g, 36.3 mmol) was added thereto, and the reaction mixture was stirred and reacted for 2 hours at 25°C. The reaction mixture was adjusted to pH = 13 with sodium hydroxide aqueous solution (4 mol/L), and extracted with ethyl acetate (50 mL × 2). The organic phase was washed with saturated brine (50 mL × 1), dried over anhydrous sodium sulfate, filtered, and concentrated under reduced pressure to obtain crude compound **92-3,** which was used directly in the next reaction step. MS-ESI calculated for [M+H]⁺ 307 and 309, found 307 and 309.

### Step 4

Compound **92-3** (1.66 g, 5.40 mmol) was dissolved in acetic acid (20.0 mL), and a solution of sodium nitrite (559 mg, 8.11 mmol) in water (10 mL) was added dropwise thereto, and the reaction mixture was stirred for 1 hour at 25°C. The reaction mixture was added with water (50 mL), and extracted with ethyl acetate (50 mL × 2). The organic phase was washed with saturated ammonium chloride aqueous solution (50 mL), dried over anhydrous sodium sulfate, filtered, and concentrated under reduced pressure. The residue was purified by silica gel column chromatography (2/1, petroleum ether/ethyl acetate, Rf= 0.59) to obtain compound **92-4.** ¹H NMR (400MHz, CD₃OD) δ = 8.15 - 8.11 (m, 1H), 4.87 (s, 2H), 2.3 - 2.30 (m, 2H), 2.12 - 1.99 (m, 2H), 1.93 - 1.67 (m, 2H). MS-ESI calculated for [M+H]⁺ 318 and 320, found 318 and 320.

### Step 5

Under nitrogen atmosphere, compound **92-4** (0.500 g, 1.57 mmol), bis(pinacolato)diboron (1.20 g, 4.72 mmol), and potassium acetate (463 mg, 4.72 mmol) were dissolved in dioxane (20.0 mL), and the reaction mixture was added with [1,1'-bis(diphenylphosphino)ferrocene]dichloropalladium(II) (115 mg, 157 µmol). The reaction mixture was stirred and reacted at 100°C for 12 hours. The reaction mixture was concentrated under reduced pressure, and the residue was purified by silica gel column chromatography (2/1, petroleum ether/ethyl acetate, R_{f} = 0.33) to obtain compound **92-5.** MS-ESI calculated for [M+H]⁺ 366, found 366.

### Step 6

Under nitrogen atmosphere, compound **21-7** (419 mg, 826 µmol), compound **92-5** (603 mg, 1.65 mmol), and sodium carbonate (262 mg, 2.48 mmol) were dissolved in dioxane (20.0 mL) and water (5.00 mL), and the reaction mixture was added with [1,1'-bis(diphenylphosphino)ferrocene]dichloropalladium(II) (60.4 mg, 82.6 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.31) to obtain compound **92-6.** MS-ESI calculated for [M+H]⁺ 667, found 667.

### Step 7

Compound **92-6** (200 mg, 300 µmol) was dissolved in ethyl acetate (10.0 mL), and hydrochloride ethyl acetate solution (4 mol/L, 0.750 mL) was added thereto, and the reaction mixture was stirred and reacted for 2 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **92.** ¹H NMR (400MHz, CD₃OD) δ = 7.98 - 7.94 (m, 1H), 7.70 (s, 1H), 7.67 - 7.62 (m, 1H), 7.38 (dd, *J*=1.2, 10.4 Hz, 1H), 7.23 (dd, *J*=1.6, 8.0 Hz, 1H), 4.86 (s, 2H), 4.44 - 4.36 (m, 1H), 4.20 - 4.12 (m, 1H), 3.69 - 3.39 (m, 3H), 2.41 - 2.29 (m, 2H), 2.24 - 2.20 (m, 1H), 2.13 - 2.00 (m, 2H), 1.98 - 1.67 (m, 5H). MS-ESI calculated for [M+H]⁺ 567, found 567.

### Example 93

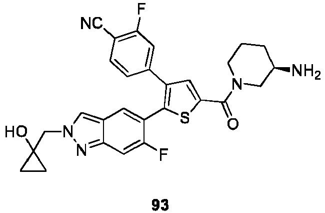

### Synthetic route:

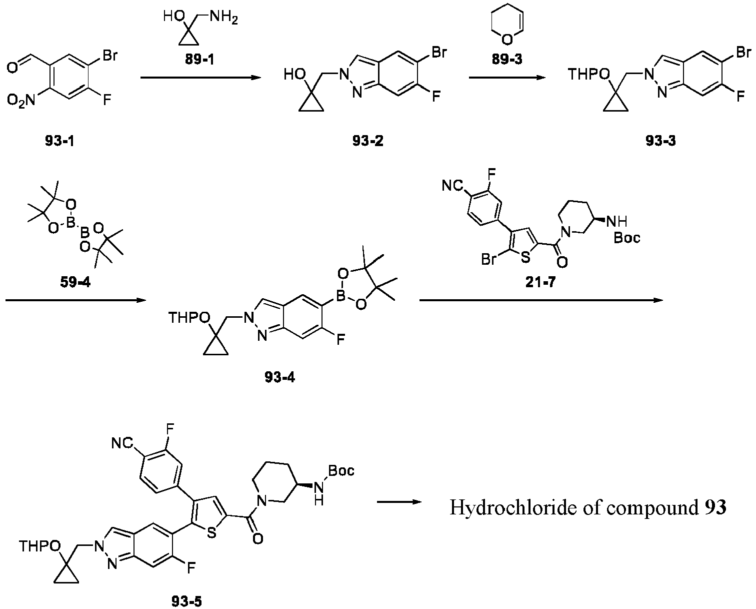

### Step 1

Under nitrogen atmosphere, compound **93-1** (1.50 g, 6.05 mmol) was dissolved in isopropanol (15.0 mL), and 1-(aminomethyl)-1-cyclopropanol (606 mg, 6.96 mmol) was added thereto, and the reaction mixture was stirred for 4 hours at 80°C, added with tributylphosphine (5.28 g, 26.1 mmol) at 0°C, stirred for 12 hours at 80°C, added with saturated ammonium chloride (30 mL), and extracted with ethyl acetate (30 mL × 1). The organic phase was washed with saturated brine (30 mL × 1), dried over anhydrous sodium sulfate, filtered, concentrated under reduced pressure, and the residue was purified by silica gel column chromatography (1/1, petroleum ether/ethyl acetate, R_{f} = 0.20) to obtain compound **93-2.** ¹H NMR (400MHz, CDCl₃) δ = 7.95 (s, 1H), 7.91 (d, *J*=6.4 Hz, 1H), 7.41 (d, *J*=9.6 Hz, 1H), 4.46 (s, 2H), 1.00 - 0.95 (m, 2H), 0.80 - 0.75 (m, 2H). MS-ESI calculated for [M+H]⁺ 287, found 287.

### Step 2

Under nitrogen atmosphere, compound **93-2** (1.40 g, 4.91 mmol) was dissolved in anhydrous dichloromethane (5.00 mL), and then 2,3-dihydropyran (1.86 g, 22.1 mmol) and pyridinium p-toluenesulfonate (247 mg, 0.982 mmol) were added thereto. The reaction mixture was stirred for 12 hours at 25°C, and concentrated under reduced pressure. The residue was separated by silica gel column chromatography (1/1, petroleum ether/ethyl acetate, Rf = 0.68) to obtain compound **93-3.** MS-ESI calculated for [M+H]⁺ 369, found 369.

### Step 3

Under nitrogen atmosphere, compound **93-3** (400 mg, 1.08 mmol), bis(pinacolato)diboron (825 mg, 3.25 mmol), and potassium acetate (319 mg, 3.25 mmol) were dissolved in dioxane (10.0 mL), and the reaction mixture was added with [1,1'-bis(diphenylphosphino)ferrocene]dichloropalladium(II) (79.3 mg, 0.108 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.60) to obtain compound **93-4.** MS-ESI calculated for [M+H]⁺ 417, found 417.

### Step 4

Under nitrogen atmosphere, compound **21-7** (310 mg, 0.610 mmol), compound **93-4** (381 mg, 0.915 mmol), and sodium carbonate (194 mg, 1.83 mmol) were dissolved in dioxane (8.00 mL) and water (2.00 mL), and the reaction mixture was added with [1,1'-bis(diphenylphosphino)ferrocene]dichloropalladium(II) (44.6 mg, 61.0 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was separated by silica gel column chromatography (1/1, petroleum ether/ethyl acetate, Rf= 0.30) to obtain compound **93-5.** MS-ESI calculated for [M+Na]⁺ 740, found 740.

### Step 5

Compound **93-5** (350 mg, 0.488 mmol) was dissolved in ethyl acetate (10.0 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 15 to 45%, 10 minutes) to obtain the hydrochloride of compound **93.** ¹H NMR (400MHz, CD₃OD) δ = 8.66 (s, 1H), 8.04 (d, *J*=7.2 Hz, 1H), 7.69 (s, 1H), 7.66 - 7.59 (m, 1H), 7.38 (d, *J*=10.4 Hz, 1H), 7.32 (dd, *J*=1.2, 10.4 Hz, 1H), 7.27 (dd, *J*=1.6, 8.0 Hz, 1H), 4.60 (s, 2H), 4.47 - 4.38 (m, 1H), 4.22 - 4.13 (m, 1H), 3.58 - 3.41 (m, 3H), 2.27 - 2.16 (m, 1H), 1.99 - 1.90 (m, 1H), 1.86 - 1.68 (m, 2H), 0.98 - 0.92 (m, 2H), 0.91 - 0.86 (m, 2H). MS-ESI calculated for [M+H]⁺ 534, found 534.

### Example 94

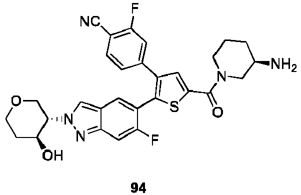

### Synthetic route:

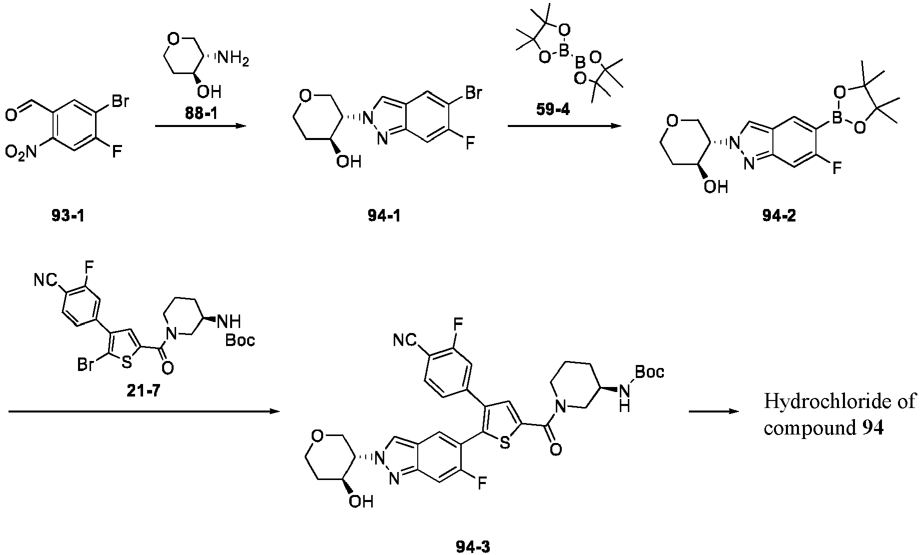

### Step 1

Under nitrogen atmosphere, compound **93-1** (500 mg, 2.02 mmol) was dissolved in isopropanol (15.0 mL), and compound **88-1** (272 mg, 2.32 mmol) was added thereto, and the reaction mixture was stirred for 4 hours at 80°C, added with tributylphosphine (1.22 g, 6.05 mmol) at 0°C, stirred for 12 hours at 80°C, and added with saturated ammonium chloride (30 mL), extracted with ethyl acetate (30 mL × 1). The organic phase was washed with saturated brine (30 mL × 1), dried over anhydrous sodium sulfate, filtered, and concentrated under reduced pressure. The residue was purified by silica gel column chromatography (1/1, petroleum ether/ethyl acetate, R_{f} = 0.20) to obtain compound **94-1.** MS-ESI calculated for [M+H]⁺ 315, found 315.

### Step 2

Under nitrogen atmosphere, compound **94-1** (460 mg, 1.46 mmol), bis(pinacolato)diboron (1.11 g, 4.38 mmol), and potassium acetate (430 mg, 4.38 mmol) were dissolved in dioxane (10.0 mL), and the reaction mixture was added with [1,1'-bis(diphenylphosphino)ferrocene]dichloropalladium(II) (107 mg, 0.146 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.60) to obtain compound **94-2.** MS-ESI calculated for [M+H]⁺ 363, found 363.

### Step 3

Under nitrogen atmosphere, compound **21-7** (320 mg, 0.629 mmol), compound **94-2** (402 mg, 0.944 mmol), and sodium carbonate (200 mg, 1.89 mmol) were dissolved in dioxane (8.00 mL) and water (2.00 mL), and the reaction mixture was added with [1,1'-bis(di-tert-butylphosphino)ferrocene]dichloropalladium(II) (46.1 mg, 63.0 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf = 0.30) to obtain compound **94-3.** MS-ESI calculated for [M+H]⁺ 664, found 664.

### Step 4

Compound **94-3** (230 mg, 0.347 mmol) was dissolved in ethyl acetate (10.0 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **94.** ¹H NMR (400MHz, CD₃OD) δ = 8.56 (s, 1H), 7.95 (d, *J*=6.8 Hz, 1H), 7.68 (s, 1H), 7.65 - 7.59 (m, 1H), 7.31 (d, *J*=10.8 Hz, 2H), 7.26 (dd, *J*=1.6, 8.0 Hz, 1H), 4.48 - 4.38 (m, 2H), 4.36 - 4.27 (m, 1H), 4.22 - 4.13 (m, 2H), 4.10 - 4.01 (m, 1H), 3.88 (t, *J*=10.8 Hz, 1H), 3.67 - 3.58 (m, 1H), 3.57 - 3.40 (m, 3H), 2.27 - 2.18 (m, 1H), 2.16 - 2.07 (m, 1H), 1.98 - 1.88 (m, 1H), 1.87 - 1.68 (m, 3H). MS-ESI calculated for [M+H]⁺ 564, found 564.

### Example 95

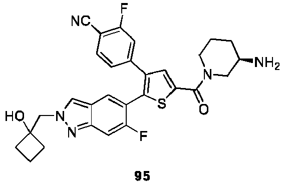

### Synthetic route:

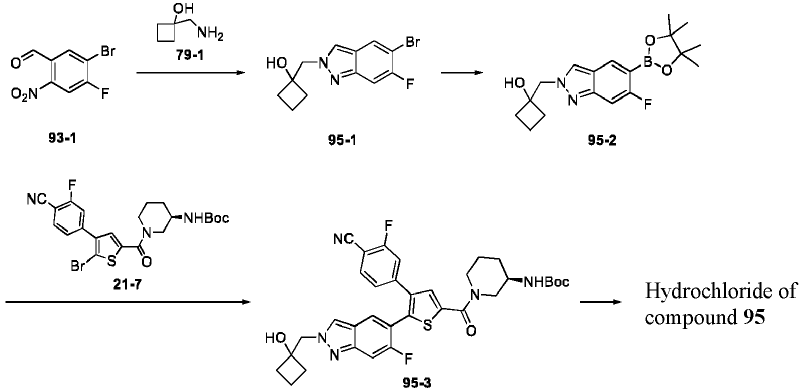

### Step 1

Compound **93-1** (1.50 g, 6.05 mmol) was dissolved in isopropanol (5.00 mL), then 79-1 (734 mg, 7.26 mmol) was added thereto, and the reaction mixture was stirred at 80°C for 4 hours, then cooled to 25°C, added dropwise with tributylphosphine (4.48 mL, 18.1 mmol), stirred, and reacted for 12 hours at 80°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, Rf = 0.43) to obtain compound **95-1.** ¹H NMR (400MHz, CD₃OD) δ = 8.29 (s, 1H), 8.05 (d, *J*=6.4 Hz, 1H), 7.39 (d, *J*=9.6 Hz, 1H), 4.53 (s, 2H), 2.27 - 2.17 (m, 2H), 2.13 - 2.01 (m, 2H), 1.88 - 1.63 (m, 2H). MS-ESI calculated for [M+H]⁺ 299, found 299.

### Step 2

Under nitrogen atmosphere, compound **95-1** (700 mg, 2.34 mmol), bis(pinacolato)diboron (1.78 g, 7.02 mmol), and potassium acetate (689 mg, 7.02 mmol) were dissolved in dioxane (15.0 mL), and the reaction mixture was added with [1,1'-bis(diphenylphosphino)ferrocene]dichloropalladium(II) (171 mg, 234 µmol). The reaction mixture was stirred and reacted at 100°C for 12 hours. The reaction mixture was concentrated under reduced pressure, and the residue was purified by silica gel column chromatography (1/1, petroleum ether/ethyl acetate, R_{f} = 0.31) to obtain compound **95-2.** MS-ESI calculated for [M+H]⁺ 347, found 347.

### Step 3

Under nitrogen atmosphere, compound **21-7** (330 mg, 650 µmol), compound **95-2** (450 mg, 1.30 mmol), and sodium carbonate (207 mg, 1.95 mmol) were dissolved in dioxane (24.0 mL) and water (6.00 mL), and the reaction mixture was added with [1,1'-bis(diphenylphosphino)ferrocene]dichloropalladium(II) (47.6 mg, 65.0 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.22) to obtain compound **95-3.** MS-ESI calculated for [M+H]⁺ 648, found 648.

### Step 4

Compound **95-3** (320 mg, 405 µmol) was dissolved in ethyl acetate (15.0 mL), and hydrochloride ethyl acetate solution (4 mol/L, 1.01 mL) was added thereto, and the reaction mixture was stirred and reacted for 1 hour at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **95.** ¹H NMR (400MHz, CD₃OD) δ = 8.61 (s, 1H), 8.02 (d, *J*=7.2 Hz, 1H), 7.69 (s, 1H), 7.65 - 7.60 (m, 1H), 7.39 - 7.30 (m, 2H), 7.26 (d, *J*=8.0 Hz, 1H), 4.63 (s, 2H), 4.46 - 4.40 (m, 1H), 4.20 - 4.10 (m, 1H), 3.62 - 3.38 (m, 3H), 2.28 - 2.17 (m, 3H), 2.16 - 2.05 (m, 2H), 1.98 - 1.88 (m, 1H), 1.86 - 1.68 (m, 4H). MS-ESI calculated for [M+H]⁺ 548, found 548.

### Example 96

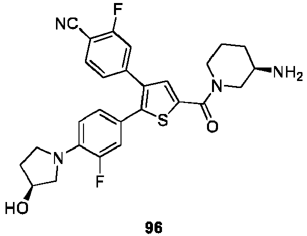

### Synthetic route:

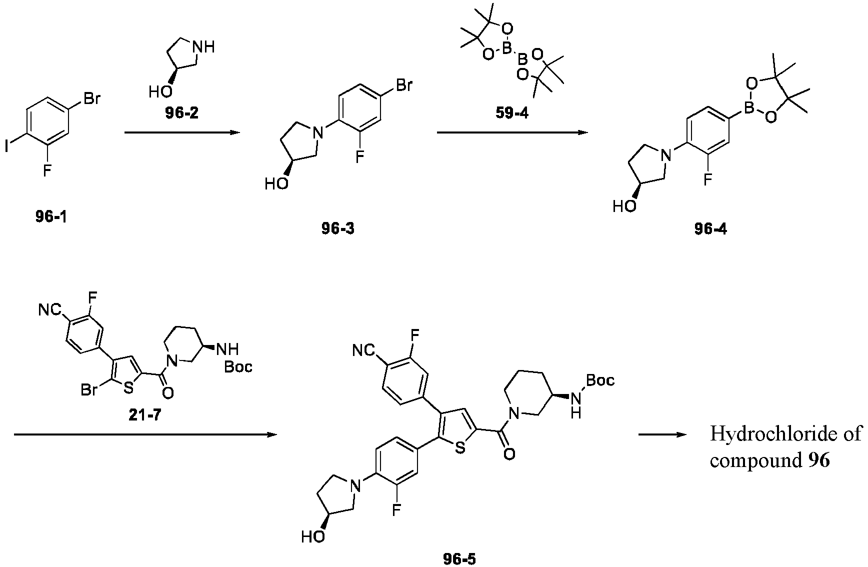

### Step 1

Under nitrogen atmosphere, compound **96-1** (2.6 g, 8.64 mmol) and compound **96-2** (1.51 g, 17.3 mmol) were dissolved in *N*,*N*-dimethylethanolamine (30.0 mL), and then potassium phosphate monohydrate (3.98 g, 17.3 mmol) and cuprous iodide (329 mg, 1.73 mmol) were added thereto, and the reaction mixture was stirred and reacted for 60 hours at 55°C. The reaction mixture was added with saturated ammonium chloride (50 mL), and extracted with dichloromethane (50 mL × 2). The organic phase was washed with saturated brine (50 mL), dried over anhydrous sodium sulfate, filtered, and concentrated under reduced pressure. The residue was purified by silica gel column chromatography (3/1, petroleum ether/ethyl acetate, R_{f} = 0.54) to obtain compound **96-3.** MS-ESI calculated for [M+H]⁺ 262, found 262.

### Step 2

Under nitrogen atmosphere, compound **96-3** (260 mg, 1.00 mmol), bis(pinacolato)diboron (762 mg, 3.00 mmol), and potassium acetate (294 mg, 3.00 mmol) were dissolved in dioxane (10.0 mL), and the reaction mixture was added with [1,1'-bis(diphenylphosphino)ferrocene]dichloropalladium(II) (73.0 mg, 0.100 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.61) to obtain compound **96-4.** MS-ESI calculated for [M+H]⁺ 308, found 308.

### Step 3

Under nitrogen atmosphere, compound **21-7** (280 mg, 0.551 mmol), compound **96-4** (254 mg, 0.826 mmol), and sodium carbonate (175 mg, 1.65 mmol) were dissolved in dioxane (8.00 mL) and water (2.00 mL), and the reaction mixture was added with [1,1'-bis(di-tert-butylphosphino)ferrocene]dichloropalladium(II) (35.9 mg, 55.1 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf = 0.36) to obtain compound **96-5.** MS-ESI calculated for [M+H]⁺ 609, found 609.

### Step 4

Compound **96-5** (300 mg, 0.493 mmol) was dissolved in ethyl acetate (10.0 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain compound **96.** ¹H NMR (400MHz, CD₃OD) δ = 7.70 (t, *J*=7.6 Hz, 1H), 7.55 (s, 1H), 7.37 - 7.27 (m, 2H), 7.08 - 6.94 (m, 3H), 4.60 - 4.52 (m, 1H), 4.47 - 4.36 (m, 1H), 4.21 - 4.10 (m, 1H), 3.80 - 3.67 (m, 2H), 3.64 - 3.55 (m, 1H), 3.53 - 3.38 (m, 4H), 2.25 - 2.13 (m, 2H), 2.11 - 2.01 (m, 1H), 1.97 - 1.87 (m, 1H), 1.85 - 1.66 (m, 2H). MS-ESI calculated for [M+H]⁺ 509, found 509.

### Example 97

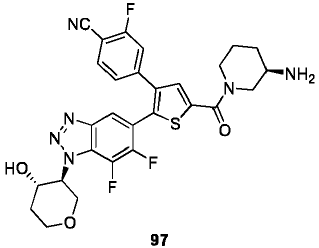

### Synthetic route:

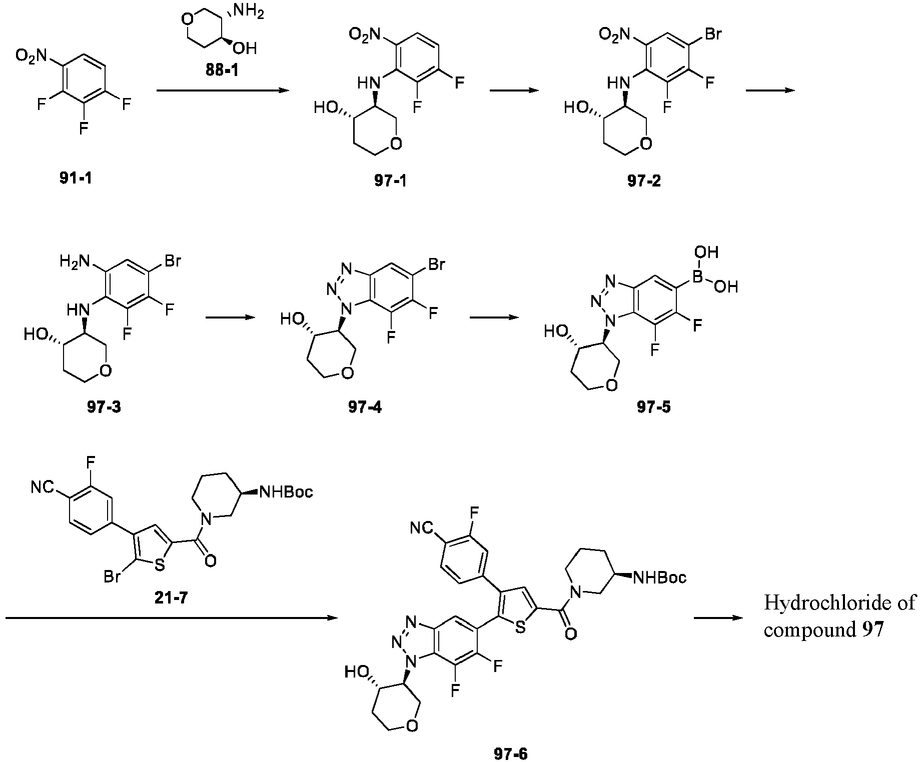

### Step 1

Compounds **91-1** (1.10 g, 6.21 mmol) and **88-1** (800 mg, 6.83 mmol) were dissolved in *N*,*N*-dimethylformamide (25.0 mL), and *N*,*N*-diisopropylethylamine (2.41 g, 18.6 mmol) was added thereto, and the reaction mixture was stirred and reacted for 12 hours at 80°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, Rf= 0.27) to obtain compound **97-1.** ¹H NMR (400MHz,) δ = 8.06 - 7.94 (m, 1H), 6.68 - 6.52 (m, 1H), 3.73 (d, *J*=3.2 Hz, 2H), 2.21 - 2.05 (m, 4H), 1.91 - 1.72 (m, 1H), 1.70 - 1.53 (m, 1H). MS-ESI calculated for [M+H]⁺ 275, found 275.

### Step 2

Compound **97-1** (1.20 g, 7.75 mmol) was dissolved in *N*,*N*-dimethylformamide (20.0 mL). *N*-Bromosuccinimide (857 mg, 4.81 mmol) was added thereto, and the reaction mixture was stirred and reacted for 1 hour at 90°C. The reaction mixture was added with water (100 mL), and extracted with ethyl acetate (100 mL × 2). The organic phase was washed with saturated brine (100 mL × 1), dried over anhydrous sodium sulfate, filtered, and concentrated under reduced pressure. The residue was purified by silica gel column chromatography (3/1, petroleum ether/ethyl acetate, Rf = 0.63) to obtain compound **97-2.** ¹H NMR (400MHz, CD₃OD) δ = 8.29 - 8.27 (m, 1H), 4.12 - 4.07 (m, 1H), 3.97 - 3.86 (m, 1H), 3.83 - 3.71 (m, 2H), 3.59 - 3.54 (m, 1H), 3.42 - 3.38 (m, 1H), 2.08 - 1.94 (m, 1H), 1.64 - 1.56 (m, 1H). MS-ESI calculated for [M+H]⁺ 355, found 355.

### Step 3

Compound **97-2** (650 mg, 1.84 mmol) was dissolved in concentrated hydrochloric acid (10.0 mL), then tin dichloride (2.49 g, 11.0 mmol) was added thereto, and the reaction mixture was stirred and reacted for 1 hour at 25°C. The reaction mixture was adjusted to pH = 11 with sodium hydroxide aqueous solution (4 mol/L), and extracted with ethyl acetate (50 mL × 2). The organic phase was washed with saturated brine (50 mL × 1), then dried over anhydrous sodium sulfate, filtered, and concentrated under reduced pressure to obtain crude compound **97-3,** which was used directly in the next reaction step. MS-ESI calculated for [M+H]⁺ 323, found 323.

### Step 4

Compound **97-3** (0.55 g, 1.70 mmol) was dissolved in acetic acid (10.0 mL), and a solution of sodium nitrite (176 mg, 2.55 mmol) in water (5.00 mL) was added dropwise thereto, and the reaction mixture was stirred for 1 hour at 25°C. The reaction mixture was added with water (50 mL), and extracted with ethyl acetate (50 mL × 2). The organic phase was washed with saturated ammonium chloride aqueous solution (50 mL × 3), dried over anhydrous sodium sulfate, filtered, and concentrated under reduced pressure. The residue was purified by silica gel column chromatography (1/1, petroleum ether/ethyl acetate, Rf = 0.59) to obtain compound **97-4.** MS-ESI calculated for [M+H]⁺ 334, found 334.

### Step 5

Under nitrogen atmosphere, compound **97-4** (0.45 g, 1.35 mmol), bis(pinacolato)diboron (1.03 g, 4.04 mmol), and potassium acetate (396 mg, 4.04 mmol) were dissolved in dioxane (20.0 mL), and the reaction mixture was added with [1,1'-bis(diphenylphosphino)ferrocene]dichloropalladium(II) (98.5 mg, 135 µmol). The reaction mixture was stirred and reacted at 100°C for 12 hours. The reaction mixture was concentrated under reduced pressure, and the residue was purified by silica gel column chromatography (2/1, petroleum ether/ethyl acetate, Rf = 0.38) to obtain compound **97-5.** ¹H NMR (400MHz, CD₃OD) δ = 8.16 - 8.14 (m, 1H), 4.80 - 4.76 (m, 1H), 4.45 - 4.33 (m, 1H), 4.30 - 4.25 (m, 1H), 4.16 - 4.10 (m, 1H), 4.06 - 3.99 (m, 1H), 3.78-3.60 (m, 1H), 2.23 - 2.14 (m, 1H), 1.95 - 1.81 (m, 1H). MS-ESI calculated for [M+H]⁺ 300, found 300.

### Step 6

Under nitrogen atmosphere, compound **21-7** (320 mg, 630 µmol), compound **97-5** (360 mg, 944 µmol), and sodium carbonate (133 mg, 1.26 mmol) were dissolved in dioxane (24.0 mL) and water (6.00 mL), and the reaction mixture was added with [1,1'-bis(diphenylphosphino)ferrocene]dichloropalladium(II) (46.1 mg, 63.0 µmol), and the reaction mixture was stirred and reacted for 12 hours at 100°C. The reaction mixture was concentrated under reduced pressure, and the residue was purified by silica gel column chromatography (2/1, petroleum ether/ethyl acetate, R_{f} = 0.22) to obtain compound **97-6.** MS-ESI calculated for [M+H-56]⁺ 627, found 627.

### Step 7

Compound **97-6** (270 mg, 178 µmol) was dissolved in ethyl acetate (10.0 mL), and hydrochloride ethyl acetate solution (4 mol/L, 0.44 mL) was added thereto, and the reaction mixture was stirred and reacted for 3 hours at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 10% to 40%, 10 minutes) to obtain the hydrochloride of compound **97.** ¹H NMR (400MHz, CD₃OD) δ = 8.01 (d, *J*=4.4 Hz, 1H), 7.72 (s, 1H), 7.70 - 7.64 (m, 1H), 7.41 (d, *J*=10.4 Hz, 1H), 7.26 (d, *J*=8.0 Hz, 1H), 4.84 - 4.73 (m, 1H), 4.48 - 4.27 (m, 3H), 4.23 - 3.99 (m, 3H), 3.74 - 3.64 (m, 1H), 3.62 - 3.41 (m, 3H), 2.22 - 2.15 (m, 2H), 2.01 - 1.69 (m, 4H). MS-ESI calculated for [M+H]⁺ 583, found 583.

### Example 98

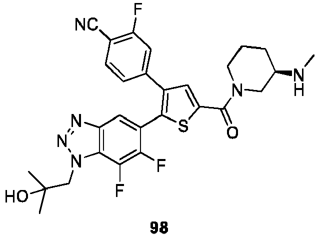

### Synthetic route:

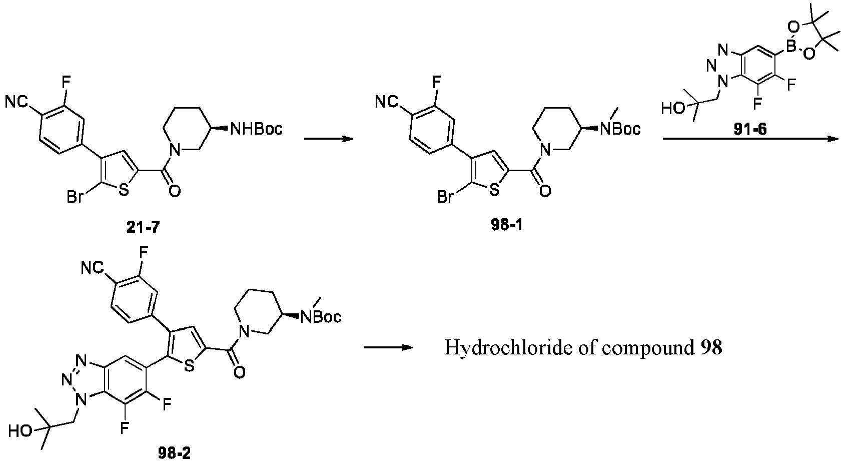

### Step 1

At 0°C, sodium hydride (118 mg, 6.52 mmol, purity: 60%) was dissolved in *N,N-*dimethylformamide (10.0 mL), and then the reaction mixture was added dropwise with a solution of **21-7** (500 mg, 983 µmol) in *N*,*N*-dimethylformamide (10.0 mL), and then added dropwise with iodomethane (306 µL, 4.92 mmol), stirred, and reacted for 12 hours at 25°C. The reaction mixture was added with saturated sodium bicarbonate aqueous solution (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (2/1, petroleum ether/ethyl acetate, R_{f} = 0.35) to obtain compound **98-1.** MS-ESI calculated for [M+Na]⁺ 544, found 544.

### Step 2

Under nitrogen atmosphere, compound **98-1** (300 mg, 574 µmol), compound **91-6** (243 mg, 689 µmol), and sodium carbonate (183 mg, 1.72 mmol) were dissolved in dioxane (24.0 mL) and water (6.00 mL), and the reaction mixture was added with [1,1'-bis(diphenylphosphino)ferrocene]dichloropalladium(II) (42.0 mg, 57.4 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.18) to obtain compound **98-2.** MS-ESI calculated for [M+H]⁺ 669, found 669.

### Step 3

Compound **98-2** (110 mg, 164 µmol) was dissolved in ethyl acetate (5.00 mL), and hydrochloride ethyl acetate solution (4 mol/L, 0.82 mL) was added thereto, and the reaction mixture was stirred and reacted for 1 hour at 25°C. After the reaction mixture was concentrated, the residue was purified by preparative high performance liquid chromatography (chromatographic column: Phenomenex Genimi NX C18 150 * 30 mm * 4 µm, mobile phase A: hydrochloric acid aqueous solution with a volume fraction of 0.05%; mobile phase B: acetonitrile; B%: 28% to 58%, 9 minutes) to obtain the hydrochloride of compound **98.** ¹H NMR (400MHz, CD₃OD) δ = 8.00 (d, *J*=4.8 Hz, 1H), 7.74 (s, 1H), 7.67 (t, *J*=7.6 Hz, 1H), 7.40 (d, *J*=10.4 Hz, 1H), 7.26 (d, *J*=7.6 Hz, 1H), 4.76 (s, 2H), 4.32 - 4.28 (m, 1H), 4.15 - 4.10 (m, 1H), 3.82 - 3.70 (m, 2H), 3.45 - 3.99 (m, 1H), 2.83 (s, 3H), 2.32 - 2.27 (m, 1H), 1.99 - 1.88 (m, 2H), 1.80 - 1.70 (m, 1H), 1.31 (s, 6H). MS-ESI calculated for [M+H]⁺ 569, found 569.

### Example 99

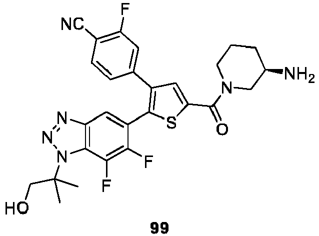

### Synthetic route:

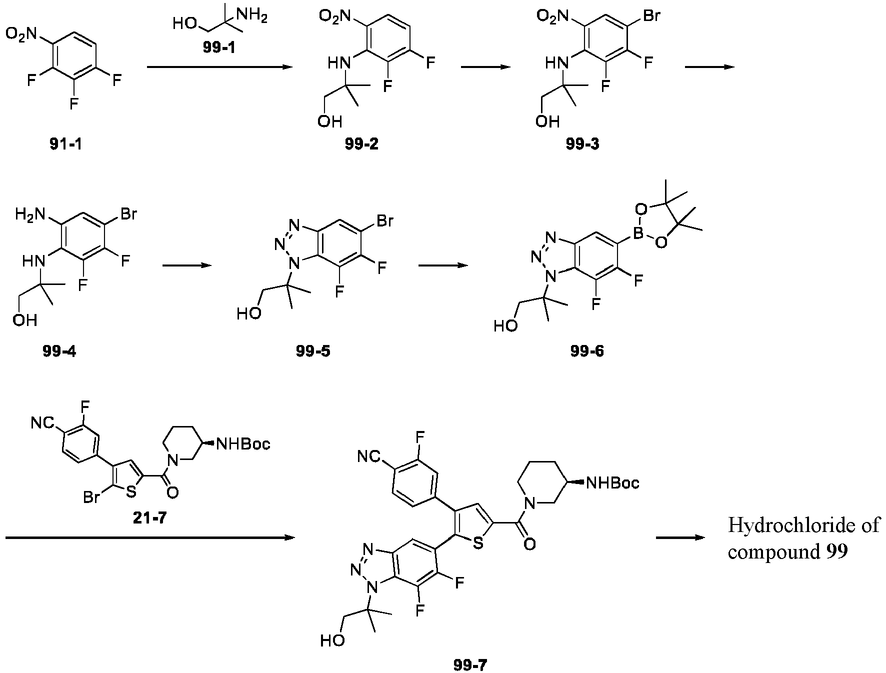

### Step 1

Compounds **91-1** (1.50 g, 8.47 mmol) and **99-1** (0.906 g, 10.2 mmol) were dissolved in *N*,*N*-dimethylformamide (25.0 mL), and *N*,*N*-diisopropylethylamine (3.28 g, 25.4 mmol) was added thereto, and the reaction mixture was stirred and reacted for 12 hours at 80°C. The reaction mixture was added with water (100 mL), and extracted with ethyl acetate (100 mL × 2). The organic phase was washed with saturated brine (100 mL × 1), dried over anhydrous sodium sulfate, filtered, and concentrated under reduced pressure. The residue was purified by silica gel column chromatography (2/1, petroleum ether/ethyl acetate, R_{f} = 0.41) to obtain compound **99-2.** ¹H NMR (400MHz, CD₃OD) δ = 8.04 - 8.01 (m, 1H), 6.85 - 6.68 (m, 1H), 3.55 (s, 2H), 1.35 (s, 3H), 1.34 (s, 3H). MS-ESI calculated for [M+H]⁺ 247, found 247.

### Step 2

Compound **99-2** (1.78 g, 7.23 mmol) was dissolved in *N*,*N*-dimethylformamide (30.0 mL). *N*-Bromosuccinimide (1.42 g, 7.95 mmol) was added thereto, and the reaction mixture was stirred and reacted for 1 hour at 90°C. The reaction mixture was added with water (50 mL), and extracted with ethyl acetate (50 mL × 2). The organic phase was washed with saturated brine (50 mL × 1), dried over anhydrous sodium sulfate, filtered, and concentrated under reduced pressure. The residue was purified by silica gel column chromatography (2/1, petroleum ether/ethyl acetate, Rf = 0.48) to obtain compound **99-3.** ¹H NMR (400MHz, CD₃OD) δ = 8.27 - 8.23 (m, 1H), 3.54 (s, 2H), 1.36 (s, 3H), 1.35 (s, 3H). MS-ESI calculated for [M+H]⁺ 325, found 325.

### Step 3

Compound **99-3** (2.00 g, 6.15 mmol) was dissolved in concentrated hydrochloric acid (20.0 mL), then tin dichloride (8.33 g, 36.9 mmol) was added thereto, and the reaction mixture was stirred and reacted for 1.5 hours at 25°C. The reaction mixture was adjusted to pH = 11 with sodium hydroxide aqueous solution (4 mol/L), and extracted with ethyl acetate (100 mL × 2). The organic phase was washed with saturated brine (100 mL × 1), dried over anhydrous sodium sulfate, filtered, and concentrated under reduced pressure to obtain crude compound **99-4,** which was used directly in the next reaction step. MS-ESI calculated for [M+H]⁺ 297, found 297.

### Step 4

Compound **99-4** (1.70 g, 5.76 mmol) was dissolved in acetic acid (20.0 mL), and a solution of sodium nitrite (596 mg, 8.64 mmol) in water (10.0 mL) was added dropwise thereto, and the reaction mixture was stirred for 2 hours at 25°C. The reaction mixture was added with water (100 mL), and extracted with ethyl acetate (100 mL × 2). The organic phase was washed with saturated ammonium chloride aqueous solution (100 mL), dried over anhydrous sodium sulfate, filtered, and concentrated under reduced pressure. The residue was purified by silica gel column chromatography (2/1, petroleum ether/ethyl acetate, R_{f} = 0.59) to obtain compound **99-5.** ¹H NMR (400MHz, CD₃OD) δ = 8.20 - 8.19 (m, 1H), 4.02 (s, 2H), 1.85 (s, 6H). MS-ESI calculated for [M+H]⁺ 306, found 306.

### Step 5

Under nitrogen atmosphere, compound **99-5** (1.30 g, 4.25 mmol), bis(pinacolato)diboron (3.24 g, 12.7 mmol), and potassium acetate (1.25 g, 12.7 mmol) were dissolved in dioxane (30.0 mL), and the reaction mixture was added with [1,1'-bis(diphenylphosphino)ferrocene]dichloropalladium(II) (311 mg, 425 µmol). The reaction mixture was stirred and reacted at 100°C for 12 hours. The reaction mixture was concentrated under reduced pressure, and the residue was purified by silica gel column chromatography (2/1, petroleum ether/ethyl acetate, R_{f} = 0.44) to obtain compound **99-6.** MS-ESI calculated for [M+H]⁺ 354 and 272, found 354 and 272.

### Step 6

Under nitrogen atmosphere, compound **21-7** (1.33 g, 2.61 mmol), compound **99-6** (1.66 g, 4.70 mmol), and sodium carbonate (830 mg, 7.83 mmol) were dissolved in dioxane (32.0 mL) and water (8.00 mL), and the reaction mixture was added with [1,1'-bis(diphenylphosphino)ferrocene]dichloropalladium(II) (191 mg, 261 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.14) to obtain compound **99-7.** MS-ESI calculated for [M+H]⁺ 655, found 655.

### Step 7

Compound **99-7** (400 mg, 397 µmol) was dissolved in ethyl acetate (10.0 mL), and hydrochloride ethyl acetate solution (4 mol/L, 0.99 mL) was added thereto, and the reaction mixture was stirred and reacted for 1 hour at 25°C. After the reaction mixture was concentrated, the residue was purified by preparative high performance liquid chromatography (chromatographic column: Phenomenex Genimi NX C18 150 * 40 mm * 5 µm, mobile phase A: hydrochloric acid aqueous solution with a volume fraction of 0.05%; mobile phase B: acetonitrile; B%: 12% to 42%, 10 minutes) to obtain the hydrochloride of compound **99.** ¹H NMR (400MHz, CD₃OD) δ = 8.02 (d, *J*=5.6 Hz, 1H), 7.72 (s, 1H), 7.70 - 7.65 (m, 1H), 7.43 (d, 10.4 Hz, 1H), 7.26 (d, *J*=8.4 Hz, 1H), 4.44 - 4.40 (m, 1H), 4.23 - 4.15 (m, 1H), 4.02 (s, 2H), 3.66 - 3.42 (m, 3H), 2.30 - 2.18 (m, 1H), 2.01 - 1.90 (m, 1H), 1.86 (s, 6H), 1.84 - 1.71 (m, 2H). MS-ESI calculated for [M+H]⁺ 555, found 555.

### Example 100

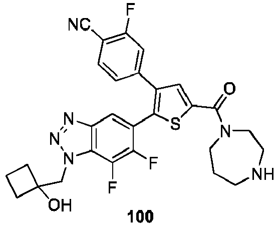

### Synthetic route:

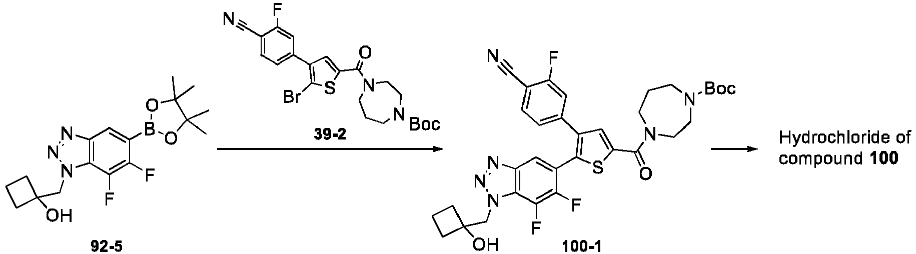

### Step 1

Under nitrogen atmosphere, compound **39-2** (300 mg, 0.590 mmol), compound **92-5** (259 mg, 0.708 mmol), and sodium carbonate (188 mg, 1.77 mmol) were dissolved in dioxane (8 mL) and water (2 mL), and the reaction mixture was added with [1,1'-bis(diphenylphosphino)ferrocene]dichloropalladium(II) (43.2 mg, 0.0590 mmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.30) to obtain compound **100-1.** MS-ESI calculated for [M-56+H]⁺ 611, found 611.

### Step 2

Compound **100-1** (100 mg, 0.150 mmol) was dissolved in ethyl acetate (3.5 mL), and hydrochloride ethyl acetate solution (4 mol/L, 3.5 mL) was added dropwise thereto. The reaction mixture was stirred for 1 hour at 25°C, concentrated under reduced pressure, and purified by preparative high performance liquid chromatography (chromatographic column: Phenomenex Synergi C18 150 * 30 mm * 4 µm; mobile phase: mobile phase A: hydrochloric acid aqueous solution with a volume fraction of 0.05%; mobile phase B: acetonitrile; B%: 29% to 59%, 9 minutes) to obtain the hydrochloride of compound **100.** ¹H NMR (400MHz, CD₃OD) δ = 7.95 (d, *J*=4.8 Hz, 1H), 7.74 (s, 1H), 7.64 (t, *J*=7.6 Hz, 1H), 7.37 (d, *J*=10.0 Hz, 1H), 7.22 (d, *J*=7.6 Hz, 1H), 4.89 (s, 2H), 4.17 - 3.90 (m, 4H), 3.54 - 3.44 (m, 2H), 3.39 - 3.40 (m, 2H), 2.39 - 2.31 (m, 2H), 2.30 - 2.20 (m, 2H), 2.13 - 2.02 (m, 2H), 1.92 - 1.81 (m, 1H), 1.80 - 1.70 (m, 1H). MS-ESI calculated for [M+H]⁺ 567, found 567.

### Example 101

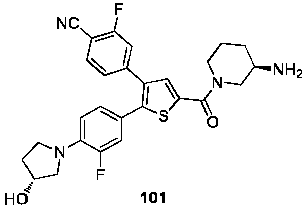

### Synthetic route:

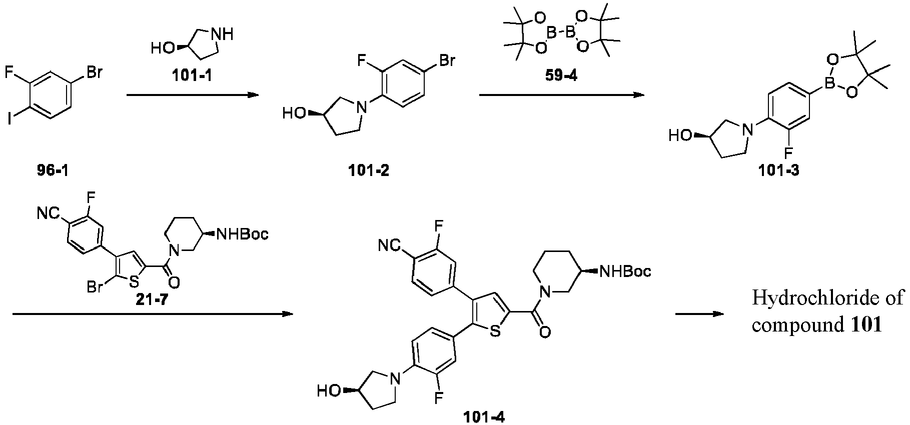

### Step 1

Compounds **96-1** (3.10 g, 10.30 mmol) and **101-1** (1.80 g, 20.61 mmol) were dissolved in *N,N-*dimethylethanolamine (20.0 mL), and then potassium phosphate monohydrate (4.74 g, 20.61 mmol) and cuprous iodide (392 mg, 2.06 mmol) were added thereto. The reaction mixture was stirred and reacted for 60 hours at 55°C. The reaction mixture was added with saturated ammonium chloride solution (80 mL), and extracted with dichloromethane (80 mL × 2). The organic phase was washed with saturated brine (80 mL × 1), dried over anhydrous sodium sulfate, filtered, and concentrated under reduced pressure. The residue was purified by silica gel column chromatography (3/1, petroleum ether/ethyl acetate, R_{f} = 0.36) to obtain compound **101-2.** MS-ESI calculated for [M+H]⁺ 260, found 260.

### Step 2

Under nitrogen atmosphere, compounds **101-2** (390 mg, 1.50 mmol), **59-4** (1.14 g, 4.50 mmol), and potassium acetate (441 mg, 4.50 mmol) were dissolved in dioxane (10.0 mL), and the reaction mixture was added with [1,1'-bis(diphenylphosphino)ferrocene]dichloropalladium(II) (109 mg, 150 µmol). The reaction mixture was stirred and reacted at 100°C for 12 hours. The reaction mixture was concentrated under reduced pressure, and the residue was purified by silica gel column chromatography (3/1, petroleum ether/ethyl acetate, R_{f} = 0.40) to obtain compound **101-3.** MS-ESI calculated for [M+H]⁺ 308, found 308.

### Step 3

Under nitrogen atmosphere, compound **21-7** (450 mg, 885 µmol), compound **101-3** (326 mg, 1.06 mmol), and sodium carbonate (187 mg, 1.77 mmol) were dissolved in dioxane (10.0 mL) and water (2.00 mL), and the reaction mixture was added with [1,1'-bis(diphenylphosphino)ferrocene]dichloropalladium(II) (65.0 mg, 89.0 µmol), and the reaction mixture was stirred and reacted for 12 hours at 100°C. The reaction mixture was concentrated under reduced pressure, and the residue was purified by silica gel column chromatography (1/1, petroleum ether/ethyl acetate, R_{f} = 0.18) to obtain compound **101-4.** MS-ESI calculated for [M+H]⁺ 609, found 609.

### Step 4

Compound **101-4** (323 mg, 530 µmol) was dissolved in ethyl acetate (4.00 mL), and hydrochloride ethyl acetate solution (4 mol/L, 2.65 mL) was added thereto, and the reaction mixture was stirred and reacted for 1 hour at 25°C. After the reaction mixture was concentrated, the residue was purified by preparative high performance liquid chromatography (chromatographic column: Phenomenex C18 150 * 40 mm * 5 µm, mobile phase A: hydrochloric acid aqueous solution with a volume fraction of 0.05%; mobile phase B: acetonitrile; B%: 10% to 50%, 10 minutes) to obtain the hydrochloride of compound **101.** ¹H NMR (400MHz, CD₃OD) δ = 7.71 (t, *J*=7.2 Hz, 1H), 7.56 (s, 1H), 7.39 - 7.28 (m, 2H), 7.00 (d, *J*=12.0 Hz, 2H), 6.93 - 6.81 (m, 1H), 4.62 - 4.50 (m, 1H), 4.42 (d, *J*=12.0 Hz, 1H), 4.17 (d, *J*=13.6 *H*z, 1H), 3.80 - 3.64 (m, 2H), 3.58 - 3.41 (m, 5H), 2.25 - 2.12 (m, 2H), 2.09 - 2.01 (m, 1H), 1.97 - 1.88 (m, 1H), 1.85 - 1.71 (m, 2H). MS-ESI calculated for [M+H]⁺ 509, found 509.

### Example 102

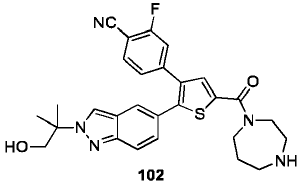

### Synthetic route:

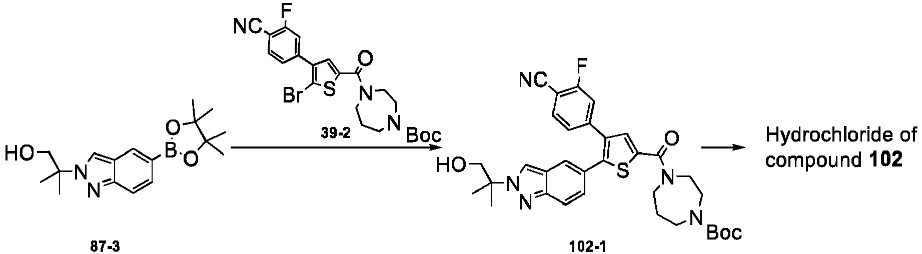

### Step 1

Under nitrogen atmosphere, compound **39-2** (500 mg, 0.983 mmol), compound **87-3** (373 mg, 1.18 mmol), and sodium carbonate (313 mg, 2.95 mmol) were dissolved in dioxane (12.0 mL) and water (3.00 mL), and the reaction mixture was added with [1,1'-bis(diphenylphosphino)ferrocene]dichloropalladium(II) (72.0 mg, 980 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf = 0.30) to obtain compound **102-1.** MS-ESI calculated for [M+H]⁺ 618, found 618.

### Step 2

Compound **102-1** (200 mg, 0.347 mmol) was dissolved in ethyl acetate (5.00 mL), and hydrochloride ethyl acetate solution (4 mol/L, 7.02 mL) was added thereto. The reaction mixture was stirred for 1 hour at 25°C, concentrated under reduced pressure, and purified by preparative high performance liquid chromatography (chromatographic column: Xtimate C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **102.** ¹H NMR (400MHz, CD₃OD) δ = 8.77 (s, 1H), 7.93 (s, 1H), 7.73 - 7.59 (m, 3H), 7.42 - 7.21 (m, 3H), 4.30 - 4.25(m, 4H), 3.89 (s, 2H), 3.56 - 3.44 (m, 2H), 3.42 - 3.36 (m, 2H), 2.36 - 2.18 (m, 2H), 1.78 (s, 6H). MS-ESI calculated for [M+H]⁺ 518, found 518.

### Example 103

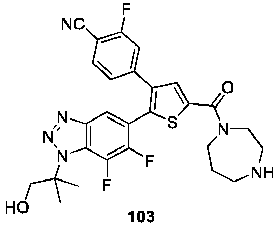

### Synthetic route:

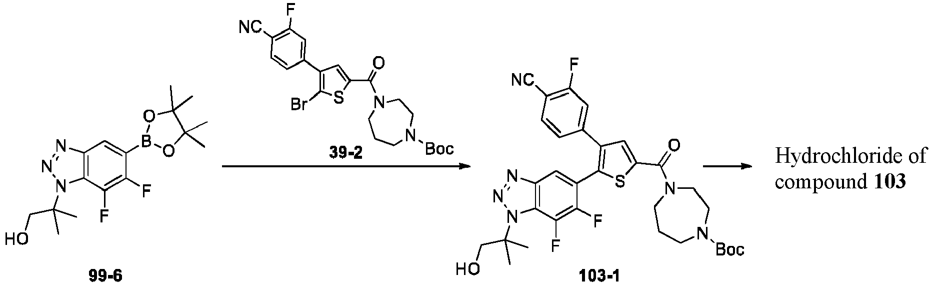

### Step 1

Under nitrogen atmosphere, compound **39-2** (380 mg, 0.747 mmol), compound **99-6** (317 mg, 0.897 mmol), and potassium phosphate (476 mg, 2.24 mmol) were dissolved in dioxane (12.0 mL) and water (3.00 mL), and the reaction mixture was added with [1,1'-bis(di-tert-butylphosphino)ferrocene]dichloropalladium(II) (49.0 mg, 74.7 µmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.30) to obtain compound **103-1.** MS-ESI calculated for [M+H]⁺ 655, found 655.

### Step 2

Compound **103-1** (160 mg, 244 µmol) was dissolved in ethyl acetate (3.00 mL), and hydrochloride ethyl acetate solution (4 mol/L, 15.0 mL) was added thereto. The reaction mixture was stirred for 1 hour at 25°C, concentrated under reduced pressure, and purified by preparative high performance liquid chromatography (chromatographic column: Phenomenex C18 150 * 40 mm * 5 µm; mobile phase: A: hydrochloric acid aqueous solution with a volume fraction of 0.05%; mobile phase B: acetonitrile 12% to 42%, 10 minutes) to obtain the hydrochloride of compound **103.** ¹H NMR (400MHz, CD₃OD) δ = 7.90 (d, *J*=4.4 Hz, 1H), 7.65 (s, 1H), 7.56 (t, *J*=7.6 Hz, 1H), 7.31 (d, *J*=10.0 Hz, 1H), 7.14 (d, *J*=7.6 Hz, 1H), 4.20 - 3.85 (m, 6H), 3.48 - 3.35 (m, 2H), 3.21 (s, 2H), 2.25 - 2.10 (m, 2H), 1.74 (s, 6H). MS-ESI calculated for [M+H]⁺ 555, found 555.

### Example 104

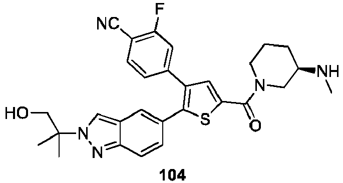

### Synthetic route:

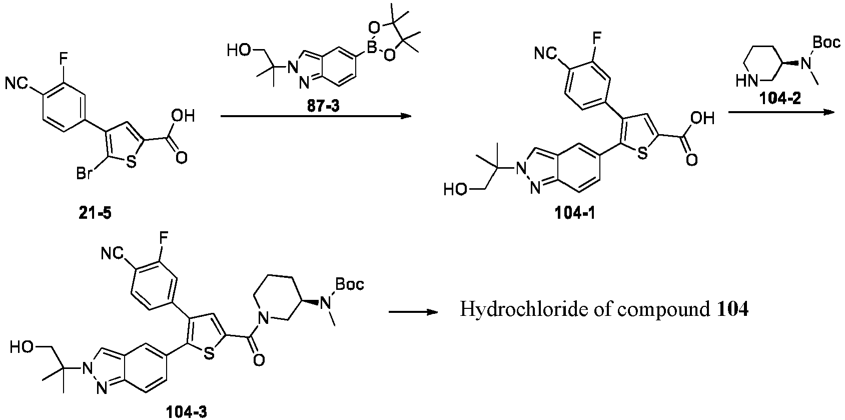

### Step 1

Under nitrogen atmosphere, compound **21-5** (4.69 g, 14.4 mmol), compound **87-3** (5.00 g, 15.8 mmol), and sodium carbonate (3.05 mg, 28.8 mmol) were dissolved in *N,N-*dimethylacetamide (40.0 mL) and water (10.0 mL), and the reaction mixture was added with [1,1'-bis(diphenylphosphino)ferrocene]dichloropalladium(II) (101 mg, 0.138 mmol), stirred, and reacted for 12 hours at 90°C, added with water (1000 mL), added with hydrochloric acid (1 mol/L) dropwise to adjust the pH to 3.0, and concentrated under reduced pressure to obtain compound **104-1.** MS-ESI calculated for [M+H]⁺ 436, found 436.

### Step 2

Under nitrogen atmosphere, compound **104-1** (500 mg, 1.15 mmol) was dissolved in anhydrous dichloromethane (10.0 mL), and then triethylamine (349 mg, 3.44 mmol), compound **104-2** (295 mg, 1.38 mmol), and 2-(7-azabenzotriazol-1-yl)-*N,N,N',N'-*tetramethyluronium hexafluorophosphate (655 mg, 1.72 mmol) were added thereto. The reaction mixture was stirred for 2 hours at 25°C. The reaction mixture was added with water (20 mL), and extracted with dichloromethane (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **104-3.** ¹H NMR (400MHz, CDCl₃) δ 8.15 - 8.05 (m, 1H), 7.75 -7.69 (m, 1H), 7.62 (d, *J*=8.8 Hz, 1H), 7.50 - 7.37 (m, 2H), 7.27 - 7.20 (m, 2H), 7.10 (d, *J*=8.4 Hz, 1H), 4.68 - 4.55 (m, 1H), 4.51 - 4.43 (m, 1H), 3.95 (s, 2H), 3.66 - 3.56 (m, 1H), 3.06 - 2.89 (m, 2H), 2.83 (s, 3H), 2.02 - 1.90 (m, 4H), 1.73 (s, 9H), 1.46 (s, 6H). MS-ESI calculated for [M+H]⁺ 632, found 632.

### Step 3

Compound **104-3** (600 mg, 0.950 mmol) was dissolved in ethyl acetate (10.0 mL), and hydrochloride ethyl acetate solution (4 mol/L, 5.00 mL) was added dropwise thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 15 to 45%, 10 minutes) to obtain the hydrochloride of compound **104.** ¹H NMR (400MHz, CD₃OD) δ 8.65 (s, 1H), 7.89 (s, 1H), 7.69 - 7.62 (m, 3H), 7.38 - 7.25 (m, 3H), 4.35 - 4.23 (m, 1H), 4.15 - 4.02 (m, 1H), 3.89 (s, 2H), 3.80 - 3.61 (m, 2H), 3.44 - 3.34 (m, 1H), 2.80 (s, 3H), 2.33-2.20 (m, 1H), 1.97 - 1.81 (m, 2H), 1.76 (s, 6H), 1.75 - 1.69 (m, 1H). MS-ESI calculated for [M+H]⁺ 532, found 532.

### Example 105

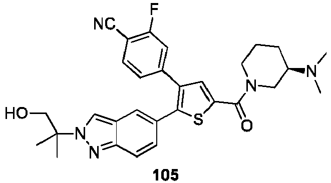

### Synthetic route:

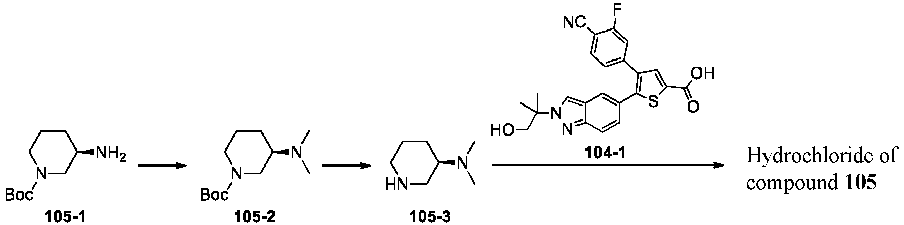

### Step 1

Under nitrogen atmosphere, compound **105-1** (1.0 g, 4.99 mmol) was dissolved in anhydrous dichloromethane (10.0 mL), and then 37% of formaldehyde aqueous solution (2.03 g, 25.0 mmol), acetic acid (30.0 mg, 0.499 mmol), and sodium triacetoxyborohydride (2.12 g, 9.99 mmol) were added thereto. The reaction mixture was stirred for 12 hours at 25°C, added with saturated sodium bicarbonate (20 mL), and extracted with dichloromethane (20 mL × 1). The organic phase was washed with saturated brine (20 mL × 1), dried over anhydrous sodium sulfate, filtered, and concentrated under reduced pressure to obtain compound **105-2.** ¹H NMR (400MHz, CDCl₃) δ 4.40 - 4.08 (m, 1H), 4.04 - 3.90 (m, 1H), 2.69 - 2.62 (m, 1H), 2.32 (s, 6H), 2.20 - 2.10 (m, 1H), 2.04 - 1.95 (m, 1H), 1.94 - 1.82 (m, 1H), 1.77 - 1.67 (m, 1H), 1.46 (s, 9H), 1.43 - 1.24 (m, 2H).

### Step 2

Compound **105-2** (1.1 g, 4.82 mmol) was dissolved in ethyl acetate (10 mL), and hydrochloride ethyl acetate solution (4 mol/L, 10 mL) was added dropwise thereto at 25°C. The reaction mixture was stirred for 1 hour at 25°C, and concentrated under reduced pressure to obtain the hydrochloride of compound **105-3.** ¹H NMR (400MHz, CD₃OD) δ 3.84 - 3.76 (m, 1H), 3.72 - 3.63 (m, 1H), 3.46 - 3.39 (m, 1H), 3.30 - 3.23 (m, 1H), 3.07 - 2.99 (m, 1H), 2.99 - 2.93 (m, 6H), 2.34 - 2.26 (m, 1H), 2.22 - 2.12 (m, 1H), 1.94 - 1.82 (m, 2H).

### Step 3

Under nitrogen atmosphere, compound **104-1** (350 mg, 1.15 mmol) was dissolved in anhydrous dichloromethane (10 mL), and then triethylamine (407 mg, 4.02 mmol), the hydrochloride of compound **105-3** (172 mg, 1.04 mmol), and 2-(7-azabenzotriazol-1-yl)-*N,N,N',N'*-tetramethyluronium hexafluorophosphate (458 mg, 1.21 mmol) were added thereto, and the reaction mixture was stirred for 2 hours at 25°C. The reaction mixture was added with water (20 mL) and extracted with dichloromethane (20 mL × 1). The organic phase was washed with saturated brine (20 mL × 1), dried over anhydrous sodium sulfate, filtered, and concentrated under reduced pressure. The residue was purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 15 to 45%, 10 minutes) to obtain the hydrochloride of compound **105.** ¹H NMR (400MHz, CD₃OD) δ 8.69 (s, 1H), 7.90 (s, 1H), 7.70 - 7.60 (m, 3H), 7.39 - 7.22 (m, 3H), 4.65 - 4.53 (m, 1H), 4.36 - 4.22 (m, 1H), 3.89 (s, 2H), 3.66 - 3.56 (m, 1H), 3.55 - 3.38 (m, 2H), 3.01 (s, 3H), 2.96 (s, 3H), 2.34 - 2.22 (m, 1H), 2.04 - 1.92 (m, 2H), 1.77 (s, 6H), 1.75 - 1.68 (m, 1H). MS-ESI calculated for [M+H]⁺ 546, found 546.

### Example 106

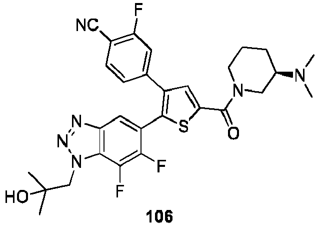

### Synthetic route:

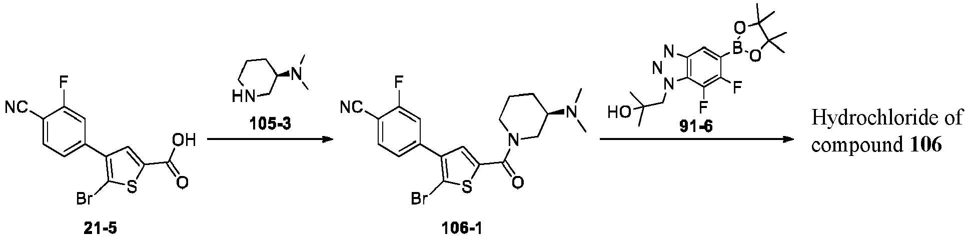

### Step 1

Compound **21-5** (600 mg, 1.84 mmol) and the hydrochloride of **105-3** (393 mg, 2.39 mmol) were dissolved in *N,N*-dimethylformamide (15.0 mL), and then 2-(7-azabenzotriazol-1-yl)-*N,N,N',N*'-tetramethyluronium hexafluorophosphate (1.05 g, 2.76 mmol) and *N,N-*diisopropylethylamine (713 mg, 5.52 mmol) were added thereto, and the reaction mixture was stirred and reacted for 2 hours at 25°C. The reaction mixture was added with water (100 mL), and extracted with ethyl acetate (50 mL × 2). The organic phase was washed with saturated brine (40 mL × 3), dried over anhydrous sodium sulfate, filtered, and concentrated under reduced pressure. The residue was purified by silica gel column chromatography (1/1, petroleum ether/ethyl acetate, R_{f} = 0.53) to obtain compound **106-1.** ¹H NMR (400MHz, CD₃OD) δ = 7.90 - 7.84 (m, 1H), 7.67 - 7.61 (m, 2H), 7.47 (s, 1H), 4.68 - 4.53 (m, 1H), 4.43 (d, *J*=11.6 Hz, 1H), 4.31 - 4.14 (m, 1H), 3.16 - 3.12 (m, 1H), 2.58 - 2.56 (m, 1H), 2.42 (s, 6H), 2.10 - 2.08 (m, 1H), 1.89 - 1.86 (m, 1H), 1.66 - 1.56 (m, 2H). MS-ESI calculated for [M+H]⁺ 436, found 436.

### Step 2

Under nitrogen atmosphere, compound **106-1** (1.10 g, 2.03 mmol), compound **91-6** (1.07 g, 2.77 mmol), and potassium phosphate (956 mg, 4.06 mmol) were dissolved in dioxane (16.0 mL) and water (4.00 mL), and the reaction mixture was added with [1,1'-bis(di-tert-butylphosphino)ferrocene]dichloropalladium(II) (132 mg, 202 µmol), stirred, and reacted for 12 hours at 90°C. The reaction mixture was concentrated under reduced pressure, and the residue was purified by silica gel column chromatography (1/1, petroleum ether/ethyl acetate, R_{f} = 0.43) and then purified by high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **106.** ¹H NMR (400MHz, CD₃OD) δ = 8.00 (d, *J*=4.8 Hz, 1H), 7.74 (s, 1H), 7.67 (t, *J*=7.6 Hz, 1H), 7.40 (d, *J*=9.6 Hz, 1H), 7.29 - 7.22 (m, 1H), 4.76 (s, 2H), 4.57 (d, *J*=12.4 Hz, 1H), 4.30 - 4.27 (m, 1H), 3.67-3.62 (m, 1H), 3.52 - 3.50 (m, 2H), 3.04 - 2.98 (m, 6H), 2.31-2.29 (m, 1H), 2.02-1.99 (m, 2H), 1.78-1.75 (m, 1H), 1.31 (s, 6H). MS-ESI calculated for [M+H]⁺ 583, found 583.

### Example 107

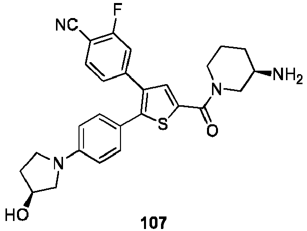

### Synthetic route:

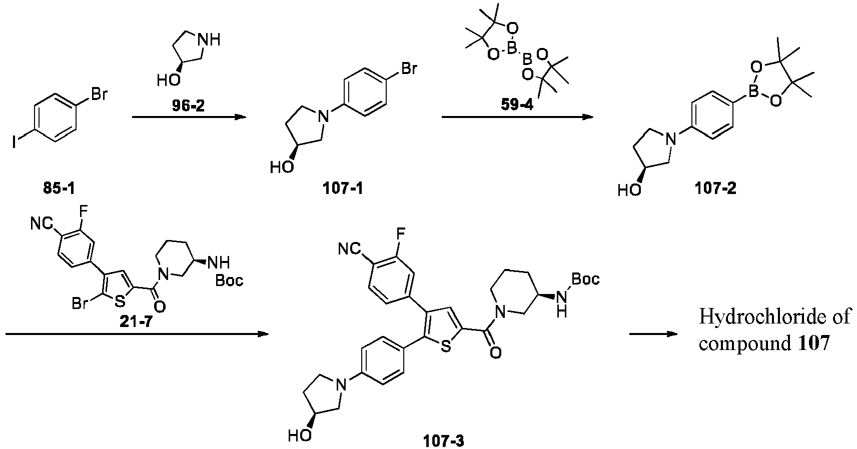

### Step 1

Under nitrogen atmosphere, compounds **85-1** (3.0 g, 10.6 mmol) and **96-2** (1.85 g, 21.2 mmol) were dissolved in *N,N-*dimethylethanolamine (30.00 mL), and then potassium phosphate monohydrate (4.88 g, 21.2 mmol) and cuprous iodide (404 mg, 2.12 mmol) were added thereto, and the reaction mixture was stirred and reacted for 12 hours at 55°C. The reaction mixture was added with saturated ammonium chloride (50 mL), and extracted with dichloromethane (50 mL × 1). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, R_{f} = 0.26) to obtain compound **107-1.** ¹H NMR (400MHz, DMSO-*d*₆) δ 7.31 - 7.22 (m, 2H), 6.50 - 6.41 (m, 2H), 4.95 (d, *J*=3.6 Hz, 1H), 4.43 - 4.34 (m, 1H), 3.39 - 3.33 (m, 1H), 3.30 - 3.26 (m, 1H), 3.25 - 3.19 (m, 1H), 3.05 - 2.99 (m, 1H), 2.08 - 1.97 (m, 1H), 1.93 - 1.83 (m, 1H). MS-ESI calculated for [M+H]⁺ 242, 244, found 242, 244.

### Step 2

Under nitrogen atmosphere, compounds **107-1** (870 mg, 3.59 mmol), **59-4** (2.74 g, 10.8 mmol), and potassium acetate (1.06 g, 10.8 mmol) were dissolved in dioxane (10 mL), and the reaction mixture was added with [1,1'-bis(diphenylphosphino)ferrocene]dichloropalladium(II) (526 mg, 0.719 mmol), and the reaction mixture was stirred and reacted for 2 hours at 95°C, and concentrated under reduced pressure. The residue was purified by silica gel column chromatography (1/1, petroleum ether/ethyl acetate, R_{f} = 0.25) to obtain compound **107-2.** ¹H NMR (400MHz, CDCl₃) δ 7.69 (d, *J*=8.4 Hz, 2H), 6.55 (d, *J*=8.4 Hz, 2H), 4.65 - 4.59 (m, 1H), 3.59 - 3.52 (m, 2H), 3.43 - 3.38 (m, 1H), 3.33 - 3.29 (m, 1H), 2.23 - 2.11 (m, 2H), 1.33 (s, 12H).

### Step 3

Under nitrogen atmosphere, compound **21-7** (700 mg, 1.38 mmol), compound **107-2** (439 mg, 1.52 mmol), and sodium carbonate (439 mg, 4.14 mmol) were dissolved in dioxane (8 mL) and water (2 mL), and the reaction mixture was added with [1,1'-bis(diphenylphosphino)ferrocene]dichloropalladium(II) (101 mg, 0.138 mmol). The reaction mixture was stirred and reacted for 12 hours at 100°C, added with water (20 mL), and extracted with ethyl acetate (20 mL × 1).The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **107-3.** MS-ESI calculated for [M+H]⁺ 591, found 591.

### Step 4

Compound **107-3** (580 mg, 0.982 mmol) was dissolved in ethyl acetate (10 mL), and hydrochloride ethyl acetate solution (4 mol/L, 10 mL) was added dropwise thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 15 to 45%, 10 minutes) to obtain the hydrochloride of compound **107.** ¹H NMR (400MHz, CD₃OD) δ 7.68 (t, *J*=7.2 Hz, 1H), 7.55 (s, 1H), 7.37 - 7.22 (m, 4H), 6.92 (d, *J*=8.4 Hz, 2H), 4.65 - 4.55 (m, 1H), 4.43 - 4.36 (m, 1H), 4.21 - 4.11 (m, 1H), 3.68 - 3.61 (m, 2H), 3.56 - 3.37 (m, 5H), 2.32 - 2.17 (m, 2H), 2.16 - 2.06 (m, 1H), 1.96 - 1.86 (m, 1H), 1.84 - 1.66 (m, 2H). MS-ESI calculated for [M+H]⁺ 491, found 491.

### Example 108

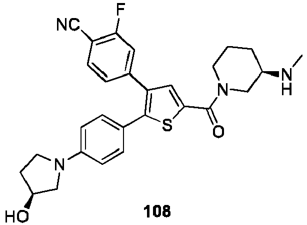

### Synthetic route:

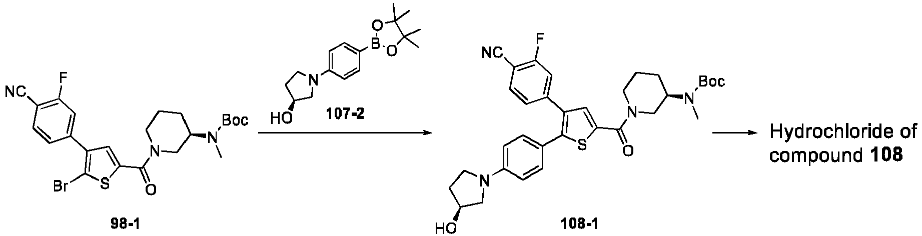

### Step 1

Under nitrogen atmosphere, compound **98-1** (700 mg, 1.34 mmol), compound **107-2** (426 mg, 1.47 mmol), and sodium carbonate (426 mg, 4.02 mmol) were dissolved in dioxane (8 mL) and water (2 mL), and the reaction mixture was added with [1,1'-bis(diphenylphosphino)ferrocene]dichloropalladium(II) (98.0 mg, 0.134 mmol). The reaction mixture was stirred and reacted for 12 hours at 95°C, added with water (20 mL), and extracted with ethyl acetate (20 mL × 1).The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **108-1.** MS-ESI calculated for [M+H]⁺ 605, found 605.

### Step 2

Compound **108-1** (400 mg, 0.661 mmol) was dissolved in ethyl acetate (10 mL), and hydrochloride ethyl acetate solution (4 mol/L, 10 mL) was added thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 12 to 42%, 10 minutes) to obtain the hydrochloride of compound **108.** ¹H NMR (400MHz, CD₃OD) δ 7.68 (t, *J*=7.2 Hz, 1H), 7.57 (s, 1H), 7.34 - 7.23 (m, 4H), 7.00 (d, *J*=8.4 Hz, 2H), 4.65 - 4.59 (m, 1H), 4.35 - 4.24 (m, 1H), 4.13 - 4.01 (m, 1H), 3.77 - 3.54 (m, 5H), 3.44 - 3.34 (m, 2H), 2.79 (s, 3H), 2.33 - 2.20 (m, 2H), 2.17 - 2.08 (m, 1H), 1.95 - 1.81 (m, 2H), 1.78 - 1.65 (m, 1H). MS-ESI calculated for [M+H]⁺ 505, found 505.

### Example 109

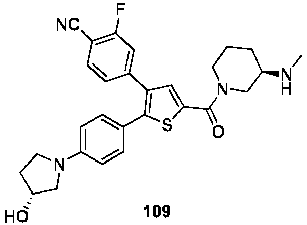

### Synthetic route:

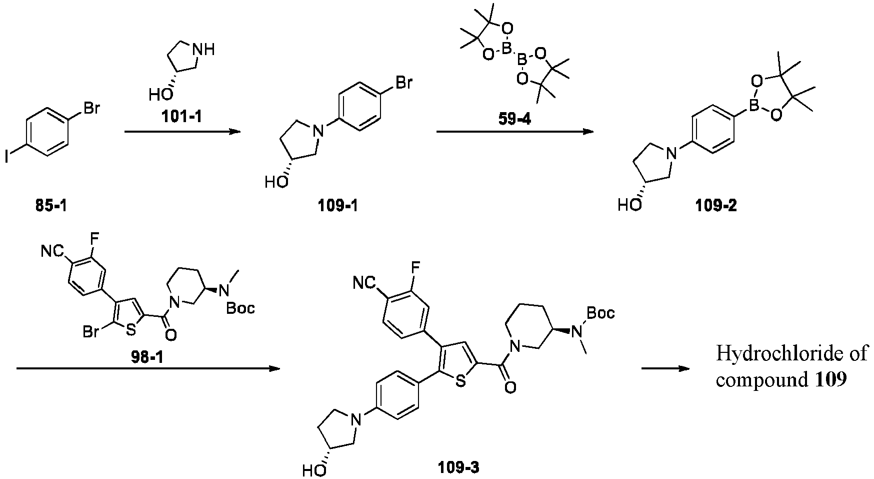

### Step 1

Under nitrogen atmosphere, compounds **85-1** (3.0 g, 10.6 mmol) and **101-1** (1.85 g, 21.2 mmol) were dissolved in *N,N-*dimethylethanolamine (30.0 mL), and then potassium phosphate monohydrate (4.88 g, 21.2 mmol) and cuprous iodide (404 mg, 2.12 mmol) were added thereto, and the reaction mixture was stirred and reacted for 12 hours at 55°C. The reaction mixture was added with saturated ammonium chloride (50 mL), and extracted with dichloromethane (50 mL × 1). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, R_{f} = 0.24) to obtain compound **109-1.** ¹H NMR (400MHz, DMSO-*d*₆) δ 7.30 - 7.22 (m, 2H), 6.49 - 6.41 (m, 2H), 4.96 (d, *J*=3.6 Hz, 1H), 4.43 - 4.35 (m, 1H), 3.37 (d, *J*=4.8 Hz, 1H), 3.32 - 3.26 (m, 1H), 3.26 - 3.21 (m, 1H), 3.05 - 2.99 (m, 1H), 2.08 - 1.96 (m, 1H), 1.92 - 1.82 (m, 1H). MS-ESI calculated for [M+H]⁺ 242, 244, found 242, 244.

### Step 2

Under nitrogen atmosphere, compounds **109-1** (900 mg, 3.72 mmol), **59-4** (1.89 g, 7.43 mmol), and potassium acetate (1.09 g, 11.1 mmol) were dissolved in dioxane (10.0 mL), and the reaction mixture was added with [1,1'-bis(diphenylphosphino)ferrocene]dichloropalladium(II) (272 mg, 0.372 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.25) to obtain compound **109-2.** ¹H NMR (400MHz, CDCl₃) δ 7.69 (d, *J*=8.4 Hz, 2H), 6.55 (d, *J*=8.8 Hz, 2H), 4.64 - 4.58 (m, 1H), 3.58 - 3.50 (m, 2H), 3.45 - 3.38 (m, 1H), 3.35 - 3.28 (m, 1H), 2.23 - 2.09 (m, 2H), 1.33 (s, 12H).

### Step 3

Under nitrogen atmosphere, compound **98-1** (550 mg, 1.05 mmol), compound **109-2** (335 mg, 1.16 mmol), and sodium carbonate (335 mg, 3.16 mmol) were dissolved in dioxane (8 mL) and water (2 mL), and the reaction mixture was added with [1,1'-bis(diphenylphosphino)ferrocene]dichloropalladium(II) (77.0 mg, 0.105 mmol). The reaction mixture was stirred and reacted for 12 hours at 95°C, added with water (20 mL), and extracted with ethyl acetate (20 mL × 1).The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **109-3.** MS-ESI calculated for [M+H]⁺ 605, found 605.

### Step 4

Compound **109-3** (360 mg, 0.446 mmol) was dissolved in ethyl acetate (10 mL), and hydrochloride ethyl acetate solution (4 mol/L, 10 mL) was added thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 16 to 46%, 10 minutes) to obtain the hydrochloride of compound **109.** ¹H NMR (400MHz, CD₃OD) δ 7.67 (t, *J*=7.6 Hz, 1H), 7.58 (s, 1H), 7.35 - 7.26 (m, 4H), 7.13 (d, *J*=9.2 Hz, 2H), 4.67 - 4.61 (m, 1H), 4.37 - 4.26 (m, 1H), 4.14 - 4.01 (m, 1H), 3.77 - 3.57 (m, 5H), 3.49 - 3.43 (m, 1H), 3.42 - 3.34 (m, 1H), 2.79 (s, 3H), 2.36 - 2.21 (m, 2H), 2.20 - 2.10 (m, 1H), 1.97 - 1.82 (m, 2H), 1.79 - 1.64 (m, 1H). MS-ESI calculated for [M+H]⁺ 505, found 505.

### Example 110

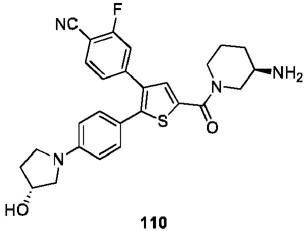

### Synthetic route:

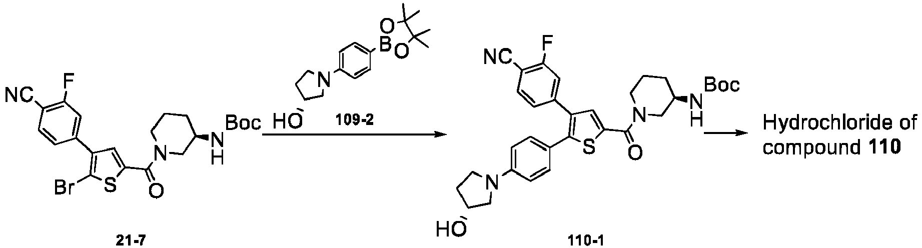

### Step 1

Under nitrogen atmosphere, compound **21-7** (600 mg, 1.18 mmol), compound **109-2** (375 mg, 1.30 mmol), and sodium carbonate (375 mg, 3.54 mmol) were dissolved in dioxane (8 mL) and water (2 mL), and the reaction mixture was added with [1,1'-bis(di-tert-butylphosphino)ferrocene]dichloropalladium(II) (76.9 mg, 0.118 mmol). The reaction mixture was stirred and reacted for 12 hours at 95°C, added with water (20 mL), and extracted with ethyl acetate (20 mL × 1).The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.21) to obtain compound **110-1.** MS-ESI calculated for [M+H]⁺ 591, found 591.

### Step 2

Compound **110-1** (300 mg, 0.508 mmol) was dissolved in ethyl acetate (10 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **110.** ¹H NMR (400MHz, CD₃OD) δ 7.68 (t, *J*=7.6 Hz, 1H), 7.54 (s, 1H), 7.34 - 7.27 (m, 2H), 7.21 (*J*=8.4 Hz, 2H), 6.81 (d, *J*=8.4 Hz, 2H), 4.63 - 4.53 (m, 1H), 4.46 - 4.34 (m, 1H), 4.22 - 4.10 (m, 1H), 3.64 - 3.55 (m, 2H), 3.54 - 3.33 (m, 5H), 2.28 - 2.16 (m, 2H), 2.15 - 2.05 (m, 1H), 1.97 - 1.85 (m, 1H), 1.83 - 1.66 (m, 2H). MS-ESI calculated for [M+H]⁺ 491, found 491.

### Example 111

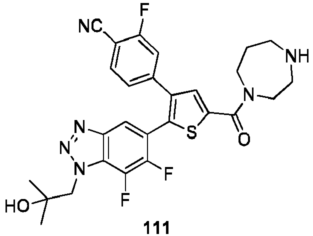

### Synthetic route:

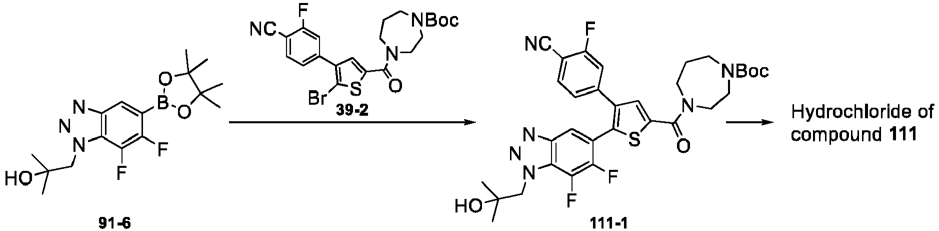

### Step 1

Under nitrogen atmosphere, compound **91-6** (625 mg, 1.77 mmol), compound **39-2** (600 mg, 1.18 mmol), and potassium phosphate (501 mg, 2.36 mmol) were dissolved in dioxane (10.0 mL) and water (2.5 mL), and the reaction mixture was added with [1,1'-bis(di-tert-butylphosphino)ferrocene]dichloropalladium(II) (77.0 mg, 118 µmol), stirred, and reacted for 2 hours at 90°C. The reaction mixture was concentrated under reduced pressure, and the residue was purified by silica gel column chromatography (1/1, petroleum ether/ethyl acetate, R_{f} = 0.33) to obtain compound **111-1.** MS-ESI calculated for [M-56+H]⁺ 599, found 599.

### Step 2

Compound **111-1** (565 mg, 863 µmol) was dissolved in ethyl acetate (2.00 mL), and hydrochloride ethyl acetate solution (4 mol/L, 10.0 mL) was added thereto, and the reaction mixture was stirred and reacted for 0.5 hours at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 10% to 45%, 10 minutes) to obtain the hydrochloride of compound **111.** ¹H NMR (400MHz, CD₃OD) δ = (d, *J*=5.2 Hz, 1H), 7.76 (s, 1H), 7.70 - 7.63 (m, 1H), 7.40 (d, *J*=10.4 Hz, 1H), 7.25 (d, *J*=8.4 Hz, 1H), 4.77 (s, 2H), 4.20 - 3.93 (m, 4H), 3.56 - 3.46 (m, 2H), 3.45 - 3.39 (m, 2H), 2.34 - 2.21 (m, 2H), 1.31 (s, 6H). MS-ESI calculated for [M+H]⁺ 555, found 555.

### Example 112

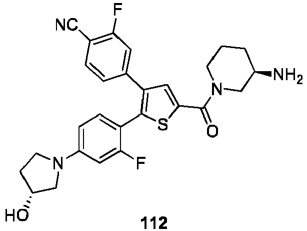

### Synthetic route:

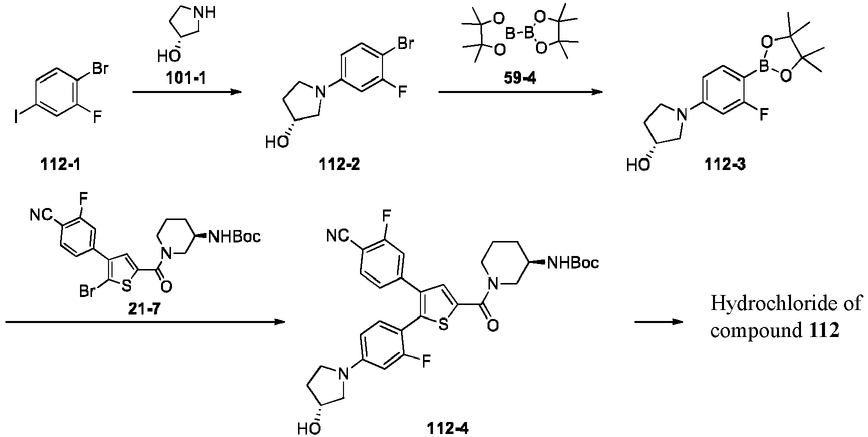

### Step 1

Compounds 112-1 (5.00 g, 16.6 mmol) and **101-1** (2.90 g, 33.23 mmol) were dissolved in *N,N-*dimethylethanolamine (17.0 mL), and then potassium phosphate (7.05 g, 33.23 mmol) and cuprous iodide (316 mg, 1.66 mmol) were added thereto. Under nitrogen atmosphere, the reaction mixture was stirred and reacted for 12 hours at 55°C. The reaction mixture was added with water (150 mL), and extracted with dichloromethane (150 mL × 2). The organic phase was washed with saturated brine (100 mL × 2), dried over anhydrous sodium sulfate, filtered, and concentrated under reduced pressure. The residue was purified by silica gel column chromatography (1/1, petroleum ether/ethyl acetate, R_{f} = 0.35) to obtain compound **112-2.** MS-ESI calculated for [M+H]⁺ 260, found 260.

### Step 2

Under nitrogen atmosphere, compounds **112-2** (1.80 g, 6.92 mmol), **59-4** (5.27 g, 20.76 mmol), and potassium acetate (2.04 g, 20.76 mmol) were dissolved in dioxane (60.0 mL), and the reaction mixture was added with [1,1'-bis(diphenylphosphino)ferrocene]dichloropalladium(II) (1.01 g, 1.38 mol). The reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.48) to obtain compound **112-3.** MS-ESI calculated for [M+H]⁺ 308, found 308.

### Step 3

Under nitrogen atmosphere, compound **21-7** (413 mg, 813 µmol), compound **112-3** (250 mg, 813 µmol), and sodium carbonate (172 mg, 1.63 mmol) were dissolved in dioxane (6.00 mL) and water (1.50 mL), and the reaction mixture was added with [1,1'-bis(diphenylphosphino)ferrocene]dichloropalladium(II) (59.0 mg, 81 µmol), and the reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.60) to obtain compound **112-4.** MS-ESI calculated for [M+H]⁺ 609, found 609.

### Step 4

Compound **112-4** (410 mg, 673 µmol) was dissolved in ethyl acetate (2.0 mL), and hydrochloride ethyl acetate solution (4 mol/L, 10.0 mL) was added thereto, and the reaction mixture was stirred and reacted for 20 minutes at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **112.** ¹H NMR (400MHz, CD₃OD) δ = 7.68 (d, *J*=8.4 Hz, 1H), 7.61 (s, 1H), 7.33 - 7.26 (m, 2H), 7.17 (t, *J*=8.4 Hz, 1H), 6.45 (d, *J*=8.4 Hz, 1H), 6.30 (d, *J*=13.2 Hz, 1H), 4.59 - 4.52 (m, 2H), 4.42 - 4.34 (m, 1H), 4.20 - 4.12 (m, 1H), 3.55 - 3.48 (m, 3H), 3.46 - 3.39 (m, 2H), 3.27 - 3.22 (m, 1H), 2.26 - 2.13 (m, 2H), 2.11 - 2.02 (m, 1H), 1.93 (m, 1H), 1.76 (m, 2H). MS-ESI calculated for [M+H]⁺ 509, found 509.

### Example 113

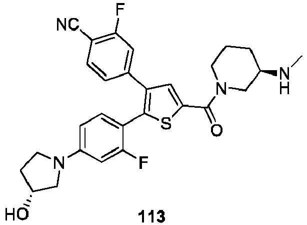

### Synthetic route:

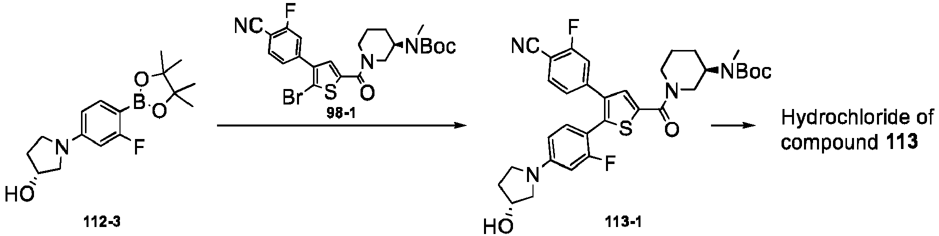

### Step 1

Under nitrogen atmosphere, compound **98-1** (425 mg, 813 µmol), compound **112-3** (250 mg, 813 µmol), and sodium carbonate (172 mg, 1.63 mmol) were dissolved in dioxane (6.00 mL) and water (1.50 mL), and the reaction mixture was added with [1,1'-bis(diphenylphosphino)ferrocene]dichloropalladium(II) (59.0 mg, 81.0 µmol), and the reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.52) to obtain compound **113-1.** MS-ESI calculated for [M+H]⁺ 623, found 623.

### Step 2

Compound **113-1** (400 mg, 642 µmol) was dissolved in ethyl acetate (2.00 mL), and hydrochloride ethyl acetate solution (4 mol/L, 9.80 mL) was added thereto, and the reaction mixture was stirred and reacted for 20 minutes at 25°C. After the reaction mixture was concentrated, the residue was purified by preparative high performance liquid chromatography (chromatographic column: Phenomenex C18 150 * 40 mm * 5 µm, mobile phase A: hydrochloric acid aqueous solution with a volume fraction of 0.05%; mobile phase B: acetonitrile; B%: 12% to 42%, 10 minutes) to obtain the hydrochloride of compound **113.** ¹H NMR (400MHz, CD₃OD) δ = 7.70 - 7.65 (m, 1H), 7.62 (s, 1H), 7.32 - 7.25 (m, 2H), 7.16 (t, *J*=8.8 Hz, 1H), 6.45 (d, *J*=8.4 Hz, 1H), 6.31 (d, *J*=13.6 Hz, 1H), 4.59 - 4.50 (m, 1H), 4.32 - 4.20 (m, 1H), 4.14 - 4.04 (m, 1H), 3.80 - 3.67 (m, 2H), 3.55 - 3.45 (m, 2H), 3.43 - 3.36 (m, 2H), 3.28 - 3.21 (m, 1H), 2.81 (s, 3H), 2.30 - 2.14 (m, 2H), 2.11 - 2.02 (m, 1H), 1.96 - 1.81 (m, 2H), 1.80 - 1.69 (m, 1H). MS-ESI calculated for [M+H]⁺ 523, found 523.

### Example 114

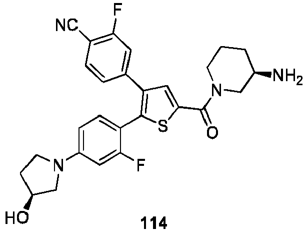

### Synthetic route:

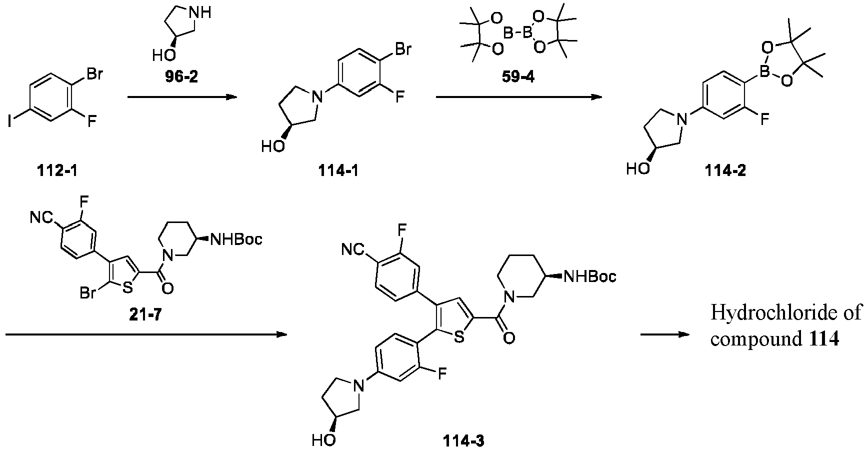

### Step 1

Compounds **112-1** (4.70 g, 15.6 mmol) and **96-2** (2.72 g, 31.24 mmol) were dissolved in *N,N*-dimethylethanolamine (16.0 mL), and then potassium phosphate (6.63 g, 31.24 mmol) and cuprous iodide (297 mg, 1.56 mmol) were added thereto, and the reaction mixture was stirred and reacted for 12 hours at 55°C, added with water (200 mL), extracted with dichloromethane (100 mL × 2). The organic phase was washed with saturated brine (100 mL × 1), dried over anhydrous sodium sulfate, filtered, and concentrated under reduced pressure. The residue was purified by silica gel column chromatography (1/1, petroleum ether/ethyl acetate, R_{f} = 0.32) to obtain compound **114-1.** ¹H NMR (400MHz, CD₃OD) δ = 7.29 (t, *J*=8.4 Hz, 1H), 6.41 - 6.26 (m, 2H), 4.57 - 4.49 (m, 1H), 3.49 - 3.38 (m, 2H), 3.32 - 3.28 (m, 1H), 3.21 - 3.15 (m, 1H), 2.23 - 2.10 (m, 1H), 2.09 - 1.98 (m, 1H). MS-ESI calculated for [M+H]⁺ 260, found 260.

### Step 2

Under nitrogen atmosphere, compounds **114-1** (2.56 g, 9.84 mmol), **59-4** (7.50 g, 29.53 mmol), and potassium acetate (2.90 g, 29.5 mmol) were dissolved in dioxane (60.0 mL), and the reaction mixture was added with [1,1'-bis(diphenylphosphino)ferrocene]dichloropalladium(II) (720 mg, 984 µmol). The reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.68) to obtain compound **114-2.** MS-ESI calculated for [M+H]⁺ 308, found 308.

### Step 3

Under nitrogen atmosphere, compound **21-7** (496 mg, 976 µmol), compound **114-2** (300 mg, 976 µmol), and sodium carbonate (207 mg, 1.95 mmol) were dissolved in dioxane (6.00 mL) and water (1.50 mL), and the reaction mixture was added with [1,1'-bis(diphenylphosphino)ferrocene]dichloropalladium(II) (71.0 mg, 97.0 µmol), and the reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.51) to obtain compound **114-3.** MS-ESI calculated for [M+H]⁺ 609, found 609.

### Step 4

Compound **114-3** (450 mg, 739 µmol) was dissolved in ethyl acetate (2.00 mL), and hydrochloride ethyl acetate solution (4 mol/L, 11.2 mL) was added thereto, and the reaction mixture was stirred and reacted for 20 minutes at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 15% to 45%, 10 minutes) to obtain the hydrochloride of compound **114.** ¹H NMR (400MHz, CD₃OD) δ =7.70 - 7.65 (m, 1H), 7.61 (s, 1H), 7.33 - 7.26 (m, 2H), 7.17 (t, *J*=8.4 Hz, 1H), 6.45 (dd, *J*=2.0, 8.8 Hz, 1H), 6.31 (dd, *J*=2.4, 13.6 Hz, 1H), 4.59 - 4.53 (m, 1H), 4.43 - 4.35 (m, 1H), 4.21 - 4.12 (m, 1H), 3.58 - 3.37 (m, 6H), 3.28 - 3.22 (m, 1H), 2.27 - 2.13 (m, 2H), 2.11 - 2.02 (m, 1H), 1.97 - 1.87 (m, 1H), 1.85 - 1.69 (m, 2H). MS-ESI calculated for [M+H]⁺ 509, found 509.

### Example 115

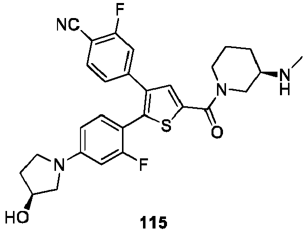

### Synthetic route:

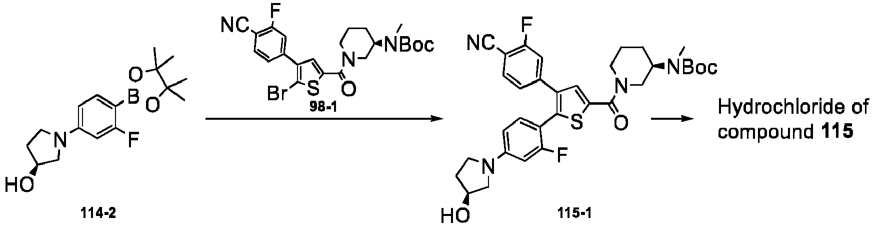

### Step 1

Under nitrogen atmosphere, compound **98-1** (679 mg, 1.30 mmol), compound **114-2** (400 mg, 1.30 mmol), and sodium carbonate (275 mg, 2.60 mmol) were dissolved in dioxane (6.00 mL) and water (1.50 mL), and the reaction mixture was added with [1,1'-bis(diphenylphosphino)ferrocene]dichloropalladium(II) (95.0 mg, 130 µmol), and the reaction mixture was stirred and reacted for 12 hours at 90°C. The reaction mixture was concentrated under reduced pressure, and the residue was purified by silica gel column chromatography (1/1, petroleum ether/ethyl acetate, R_{f} = 0.52) to obtain compound **115-1.** MS-ESI calculated for [M+H]⁺ 623, found 623.

### Step 2

Compound **115-1** (550 mg, 883 µmol) was dissolved in ethyl acetate (2.00 mL), and hydrochloride ethyl acetate solution (4 mol/L, 10.0 mL) was added thereto, and the reaction mixture was stirred and reacted for 20 minutes at 25°C. After the reaction mixture was concentrated, the residue was purified by preparative high performance liquid chromatography (chromatographic column: Xtimate C18 150 * 40 mm * 5 µm, mobile phase A: hydrochloric acid aqueous solution with a volume fraction of 0.05%; mobile phase B: acetonitrile; B%: 14% to 44%, 10 minutes) to obtain the hydrochloride of compound **115.** ¹H NMR (400MHz, CD₃OD) δ = 7.70 - 7.65 (m, 1H), 7.63 (s, 1H), 7.32 - 7.29 (m, 1H), 7.28 (s, 1H), 7.17 (t, *J*=8.4 Hz, 1H), 6.46 (dd, *J*=2.4, 8.4 Hz, 1H), 6.32 (dd, *J*=2.0, 13.6 Hz, 1H), 4.61 - 4.50 (m, 1H), 4.35 - 4.21 (m, 1H), 4.15 - 4.03 (m, 1H), 3.80 - 3.62 (m, 2H), 3.57 - 3.46 (m, 2H), 3.44 - 3.36 (m, 2H), 3.29 - 3.21 (m, 1H), 2.81 (s, 3H), 2.32 - 2.02 (m, 3H), 1.97 - 1.82 (m, 2H), 1.81 - 1.67 (m, 1H). MS-ESI calculated for [M+H]⁺ 523, found 523.

### Example 116

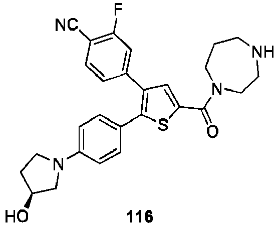

### Synthetic route:

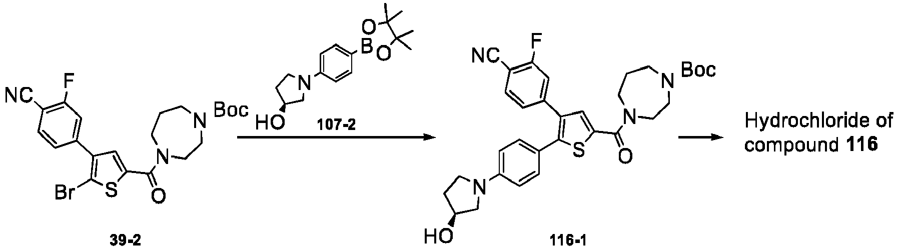

### Step 1

Under nitrogen atmosphere, compound **39-2** (600 mg, 1.18 mmol), compound **107-2** (375 mg, 1.30 mmol), and sodium carbonate (375 mg, 3.54 mmol) were dissolved in dioxane (8.00 mL) and water (2.00 mL), and the reaction mixture was added with [1,1'-bis(di-tert-butylphosphino)ferrocene]dichloropalladium(II) (76.9 mg, 0.118 mmol). The reaction mixture was stirred and reacted for 12 hours at 95°C, added with water (20 mL), and extracted with ethyl acetate (20 mL × 1).The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.21) to obtain compound **116-1.** MS-ESI calculated for [M+H]⁺ 591, found 591.

### Step 2

Compound **116-1** (700 mg, 0.782 mmol) was dissolved in ethyl acetate (10 mL), and hydrochloride ethyl acetate solution (4 mol/L, 10 mL) was added dropwise thereto at 25°C. The reaction mixture was stirred for 1 hour at 25°C, concentrated under reduced pressure, and separated by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **116.** ¹H NMR (400MHz, CD₃OD) δ 7.71 - 7.64 (m, 1H), 7.59 (s, 1H), 7.33 - 7.26 (m, 2H), 7.22 (d, *J*=8.4 Hz, 2H), 6.86 (d, *J*=8.4 Hz, 2H), 4.62 - 4.56 (m, 1H), 4.15 - 3.91 (m, 4H), 3.66 - 3.57 (m, 2H), 3.55 - 3.44 (m, 3H), 3.41 - 3.34 (m, 3H), 2.30 - 2.19 (m, 3H), 2.15 - 2.05 (m, 1H). MS-ESI calculated for [M+H]⁺ 491, found 491.

### Example 117

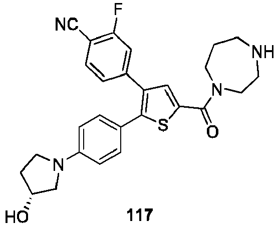

### Synthetic route:

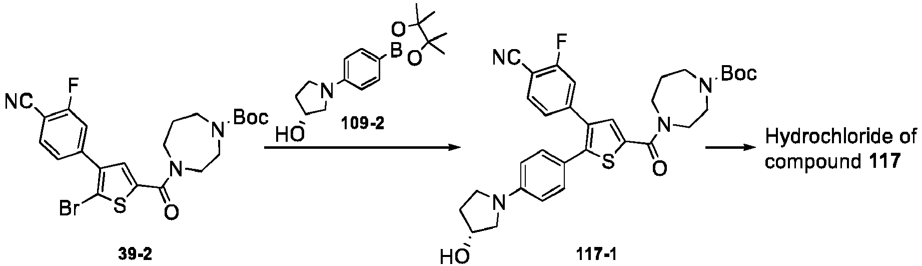

### Step 1

Under nitrogen atmosphere, compound **39-2** (700 mg, 1.38 mmol), compound **109-2** (438 mg, 1.51 mmol), and sodium carbonate (438 mg, 4.13 mmol) were dissolved in dioxane (8.00 mL) and water (2.00 mL), and the reaction mixture was added with [1,1'-bis(di-tert-butylphosphino)ferrocene]dichloropalladium(II) (90.0 mg, 0.118 mmol). The reaction mixture was stirred and reacted for 12 hours at 95°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.21) to obtain compound **117-1.** MS-ESI calculated for [M+H]⁺ 591, found 591.

### Step 2

Compound **117-1** (600 mg, 0.741 mmol) was dissolved in ethyl acetate (10.0 mL), and hydrochloride ethyl acetate solution (4 mol/L, 10.0 mL) was added dropwise thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 12 to 42%, 10 minutes) to obtain the hydrochloride of compound **117.** ¹H NMR (400MHz, CD₃OD) δ 7.68 (t, *J*=7.2 Hz, 1H), 7.60 (s, 1H), 7.33 - 7.24 (m, 4H), 6.97 (d, *J*=8.4 Hz, 2H), 4.65 - 4.58 (m, 1H), 4.14 - 3.88 (m, 4H), 3.70 - 3.61 (m, 2H), 3.61 - 3.53 (m, 1H), 3.52 - 3.43 (m, 2H), 3.42 - 3.35 (m, 3H), 2.34 - 2.18 (m, 3H), 2.17 - 2.08 (m, 1H). MS-ESI calculated for [M+H]⁺ 491, found 491.

### Example 118

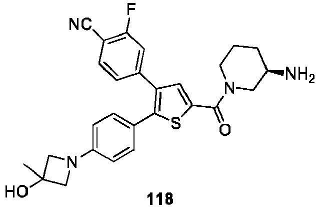

### Synthetic route:

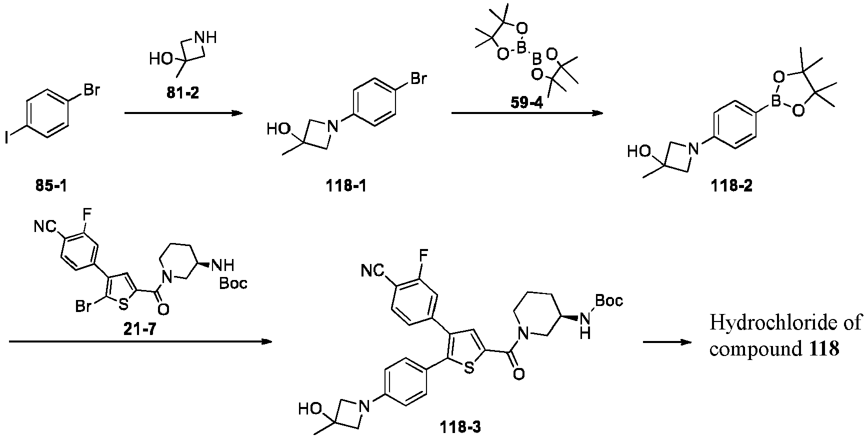

### Step 1

Under nitrogen atmosphere, compounds **85-1** (2.5 g, 8.84 mmol) and **81-2** (2.18 g, 17.7 mmol) were dissolved in *N,N-*dimethylethanolamine (20.0 mL), and then potassium phosphate monohydrate (8.14 g, 35.4 mmol) and cuprous iodide (337 mg, 1.77 mmol) were added thereto, and the reaction mixture was stirred and reacted for 12 hours at 55°C. The reaction mixture was added with saturated ammonium chloride (50 mL), and extracted with dichloromethane (50 mL × 1). The organic phase was washed with saturated brine (50 mL × 1), dried over anhydrous sodium sulfate, filtered, and concentrated under reduced pressure. The residue was purified by silica gel column chromatography (1/1, petroleum ether/ethyl acetate, R_{f} = 0.24) to obtain compound **118-1.** ¹H NMR (400MHz, DMSO-*d*₆) δ 7.30 - 7.25 (m, 2H), 6.40 - 6.34 (m, 2H), 5.53 (s, 1H), 3.72 (d, *J*=7.6 Hz, 2H), 3.57 (d, *J*=7.6 Hz, 2H), 1.43 (s, 3H).

### Step 2 ES18490 - 238

Under nitrogen atmosphere, compounds **118-1** (1.43 g, 5.91 mmol), **59-4** (3.00 g, 11.8 mmol), and potassium acetate (1.74 g, 17.7 mmol) were dissolved in dioxane (10.0 mL), and the reaction mixture was added with [1,1'-bis(diphenylphosphino)ferrocene]dichloropalladium(II) (432 mg, 0.591 mmol), and the reaction mixture was stirred and reacted for 12 hours at 95°C, and concentrated under reduced pressure. The residue was purified by silica gel column chromatography (1/1, petroleum ether/ethyl acetate, R_{f} = 0.25) to obtain compound **118-2.** ¹H NMR (400MHz, CDCl₃) δ 7.68 (d, *J*=8.4 Hz, 2H), 6.44 (d, *J*=8.4 Hz, 2H), 3.91 - 3.86 (m, 2H), 3.82 - 3.77 (m, 2H), 1.60 (s, 3H), 1.33 (s, 12H). MS-ESI calculated for [M+H]⁺ 290, found 290.

### Step 3

Under nitrogen atmosphere, compound **21-7** (800 mg, 1.57 mmol), compound **118-2** (501 mg, 1.73 mmol), and sodium carbonate (500 mg, 4.72 mmol) were dissolved in dioxane (8.00 mL) and water (2.00 mL), and the reaction mixture was added with [1,1'-bis(di-tert-butylphosphino)ferrocene]dichloropalladium(II) (103 mg, 0.157 mmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.21) to obtain compound **118-3.** MS-ESI calculated for [M+H]⁺ 591, found 591.

### Step 4

Compound **118-3** (780 mg, 1.32 mmol) was dissolved in ethyl acetate (10.0 mL), and hydrochloride ethyl acetate solution (4 mol/L, 10.0 mL) was added dropwise thereto at 25°C. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 12 to 42%, 10 minutes) to obtain the hydrochloride of compound **118.** ¹H NMR (400MHz, CD₃OD) δ 7.72 - 7.66 (m, 1H), 7.57 (s, 1H), 7.35 - 7.25 (m, 4H), 7.12 - 7.07 (m, 2H), 4.45 - 4.35 (m, 1H), 4.22 - 4.10 (m, 1H), 3.66 - 3.56 (m, 2H), 3.53 - 3.34 (m, 5H), 2.25 - 2.16 (m, 1H), 1.96 - 1.86 (m, 1H), 1.83 - 1.66 (m, 2H), 1.39 (s, 3H). MS-ESI calculated for [M+H]⁺ 491, found 491.

### Example 119

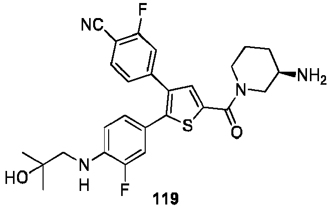

### Synthetic route:

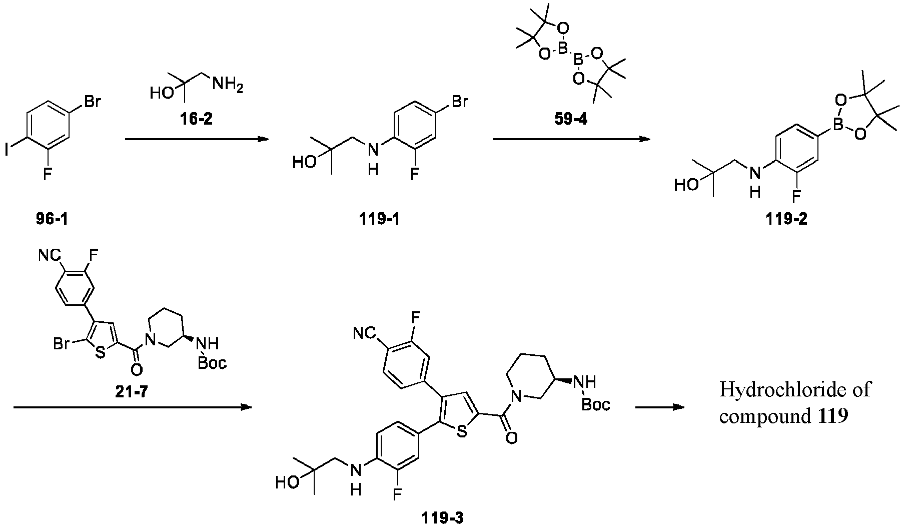

### Step 1

Compound 96-1 (7.00 g, 23.3 mmol) and compound **16-2** (4.15 g, 46.5 mmol) were dissolved in *N,N-*dimethylethanolamine (50.0 mL), and the reaction mixture was added with cuprous iodide (886 mg, 4.65 mmol) and potassium phosphate (9.88 g, 46.5 mmol). The reaction mixture was stirred and reacted for 12 hours at 55°C, added with saturated ammonium chloride solution (50 mL), and extracted with ethyl acetate (100 mL × 1). The organic phase was washed with saturated brine (100 mL × 1), dried over anhydrous sodium sulfate, filtered, and concentrated under reduced pressure. The residue was purified by silica gel column chromatography (6/1, petroleum ether/ethyl acetate, R_{f} = 0.86) to obtain compound **119-1.** MS-ESI calculated for [M+H]⁺ 264, found 264.

### Step 2

Under nitrogen atmosphere, compound **119-1** (1.00 g, 3.82 mmol), compound **59-4** (1.94 g, 7.63 mmol), and potassium acetate (749 mg, 7.63 mmol) were dissolved in dioxane (30 mL), and the reaction mixture was added with [1,1'-bis(diphenylphosphino)ferrocene]dichloropalladium(II) (279 mg, 0.381 mmol). The reaction mixture was stirred and reacted for 3 hours at 95°C, added with water (50 mL), and extracted with ethyl acetate (100 mL × 1).The organic phase was washed with saturated brine (100 mL × 1), dried over anhydrous sodium sulfate, filtered, and concentrated under reduced pressure. The residue was purified by silica gel column chromatography (1/1, petroleum ether/ethyl acetate, Rf= 0.30) to obtain compound **119-2.** MS-ESI calculated for [M+H]⁺ 310, found 310.

### Step 3

Under nitrogen atmosphere, compound **119-2** (1.00 g, 3.23 mmol), compound **21-7** (1.57 g, 3.08 mmol), and potassium acetate (605 mg, 6.16 mmol) were dissolved in dioxane (24.0 mL) and water (6.00 mL), and the reaction mixture was added with [1,1'-bis(diphenylphosphino)ferrocene]dichloropalladium(II) (225 mg, 0.308 mmol). The reaction mixture was stirred and reacted for 12 hours at 90°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, Rf= 0.24) to obtain compound **119-3.** MS-ESI calculated for [M+H]⁺ 611, found 611.

### Step 4

Compound **119-3** (1.2 g, 1.96 mmol) was dissolved in ethyl acetate (5.00 mL), and hydrochloride ethyl acetate solution (4 mol/L, 20 mL) was added thereto. The reaction mixture was stirred for 1 hour at 25°C, concentrated under reduced pressure, and purified by preparative high performance liquid chromatography (chromatographic column: Phenomenex C18 150 * 40 mm * 5 µm; mobile phase: A: hydrochloric acid aqueous solution with a volume fraction of 0.05%; mobile phase B: acetonitrile 12% to 42%, 10 minutes) to obtain the hydrochloride of compound **119.** ¹H NMR (400MHz, CD₃OD) δ = 7.74 - 7.66 (m, 1H), 7.55 (s, 1H), 7.37 - 7.27 (m, 2H), 7.07 - 6.99 (m, 3H), 4.42 (d, *J*=12.0 Hz, 1H), 4.23 - 4.08 (m, 1H), 3.56 - 3.36 (m, 3H), 3.22 (s, 2H), 2.30 - 2.14 (m, 1H), 1.90 - 1.94 (m, 1H), 1.86 - 1.64 (m, 2H), 1.30 (s, 6H). MS-ESI calculated for [M+H]⁺ 511, found 511.

### Example 120

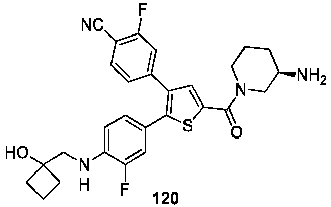

### Synthetic route:

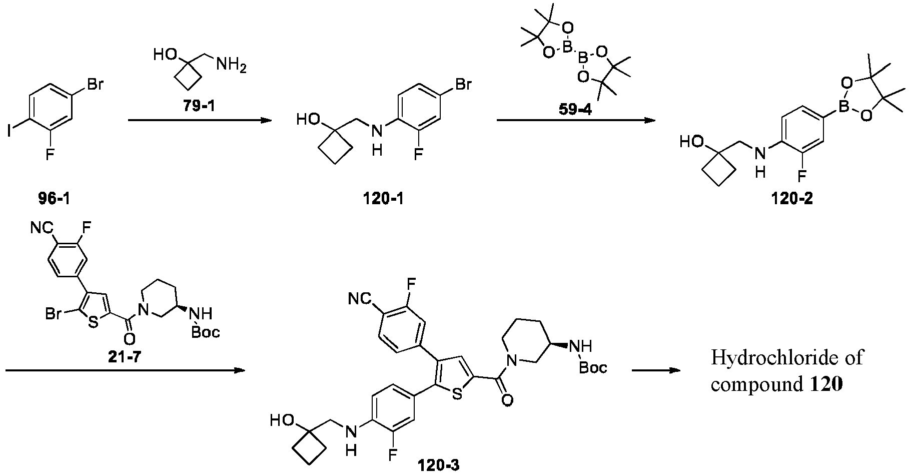

### Step 1

Compound **96-1** (7.00 g, 23.3 mmol) and compound **79-1** (4.71 g, 46.5 mmol) were dissolved in *N,N-*dimethylethanolamine (50.0 mL), and the reaction mixture was added with cuprous iodide (886 mg, 4.65 mmol) and potassium phosphate (9.88 g, 46.5 mmol). The reaction mixture was stirred and reacted for 12 hours at 55°C, added with saturated ammonium chloride solution (50 mL), and extracted with ethyl acetate (100 mL × 1). The organic phase was washed with saturated brine (100 mL × 1), dried over anhydrous sodium sulfate, filtered, and concentrated under reduced pressure. The residue was purified by silica gel column chromatography (6/1, petroleum ether/ethyl acetate, R_{f} = 0.90) to obtain compound **120-1.** MS-ESI calculated for [M+H]⁺ 276, found 276.

### Step 2

Under nitrogen atmosphere, compound **120-1** (1.00 g, 3.65 mmol), compound **59-4** (1.85 g, 7.30 mmol), and potassium acetate (1.07 mg, 10.9 mmol) were dissolved in dioxane (30.0 mL), and the reaction mixture was added with [1,1'-bis(diphenylphosphino)ferrocene]dichloropalladium(II) (263 mg, 0.365 mmol). The reaction mixture was stirred and reacted for 3 hours at 95°C, added with saturated ammonium chloride solution (50 mL), and extracted with ethyl acetate (100 mL × 1). The organic phase was washed with saturated brine (100 mL × 1), dried over anhydrous sodium sulfate, filtered, and concentrated under reduced pressure. The residue was purified by silica gel column chromatography (1/1, petroleum ether/ethyl acetate, R_{f} = 0.70) to obtain compound **120-2.** MS-ESI calculated for [M+H]⁺ 310, found 310.

### Step 3

Under nitrogen atmosphere, compound **120-2** (1.00 g, 3.11 mmol), compound **21-7** (1.51 g, 3.11 mmol), and potassium acetate (582 mg, 5.93 mmol) were dissolved in dioxane (24.0 mL) and water (6.00 mL), and the reaction mixture was added with [1,1'-bis(diphenylphosphino)ferrocene]dichloropalladium(II) (217 mg, 0.297 mmol). The reaction mixture was stirred and reacted for 12 hours at 95°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.24) to obtain compound **120-3.** MS-ESI calculated for [M+H]⁺ 623, found 623.

### Step 4

Compound **120-3** (1.20 g, 1.96 mmol) was dissolved in ethyl acetate (5.00 mL), and hydrochloride ethyl acetate solution (4 mol/L, 20.0 mL) was added thereto. The reaction mixture was stirred for 2 hours at 25°C, concentrated under reduced pressure, and purified by preparative high performance liquid chromatography (chromatographic column: Phenomenex C18 150 * 40 mm * 5 µm; mobile phase: A: hydrochloric acid aqueous solution with a volume fraction of 0.05%; mobile phase B: acetonitrile 12% to 42%, 10 minutes) to obtain the hydrochloride of compound **120.** 1H NMR (400MHz, CD3OD) δ = 7.75 - 7.67 (m, 1H), 7.56 (s, 1H), 7.37 - 7.28 (m, 2H), 7.13 - 7.03 (m, 3H), 4.45 - 4.35 (m, 1H), 4.22 - 4.08 (m, 1H), 3.55 - 3.39 (m, 3H), 3.38 (s, 2H), 2.26 - 2.17 (m, 1H), 2.17 - 2.09 (m, 4H), 1.96 - 1.60 (m, 5H). MS-ESI calculated for [M+H]⁺ 523, found 523.

### Example 121

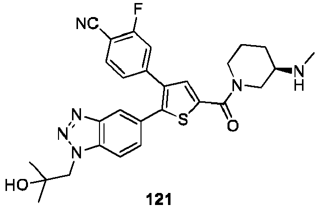

### Synthetic route:

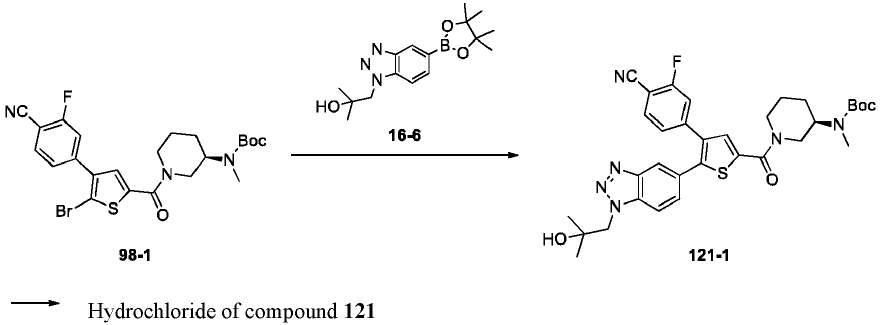

### Step 1

Under nitrogen atmosphere, compound **98-1** (800 mg, 1.53 mmol), compound **16-6** (583 mg, 1.84 mmol), and sodium carbonate (487 mg, 4.59 mmol) were dissolved in dioxane (8.00 mL) and water (2.00 mL), and the reaction mixture was added with [1,1'-bis(diphenylphosphino)ferrocene]dichloropalladium(II) (112 mg, 0.153 mmol). The reaction mixture was stirred and reacted for 2 hours at 80°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **121-1.** MS-ESI calculated for [M+H]⁺ 633, found 633.

### Step 2

Compound **121-1** (850 mg, 1.34 mmol) was dissolved in ethyl acetate (10.0 mL), and hydrochloride ethyl acetate solution (4 mol/L, 21.8 mL) was added thereto. The reaction mixture was stirred for 1 hour at 25°C, concentrated under reduced pressure, and purified by preparative high performance liquid chromatography (chromatographic column: Phenomenex Genimi NX C18 150 * 40 mm * 5 µm; mobile phase: mobile phase A: hydrochloric acid aqueous solution with a volume fraction of 0.05%; mobile phase B: acetonitrile; B%: 10 to 40%, 10 minutes) to obtain the hydrochloride of compound **121.** ¹H NMR (400MHz, CD₃OD) δ 7.97 (s, 1H), 7.86 (d, *J*=8.8 Hz, 1H), 7.68 - 7.62 (m, 2H), 7.41 (dd, *J*=1.6, 8.8 Hz, 1H), 7.38 - 7.33 (m, 1H), 7.26 (dd, *J*=1.6, 8.8 Hz, 1H), 4.69 (s, 2H), 4.37 - 4.26 (m, 1H), 4.16 - 4.03 (m, 1H), 3.84 - 3.60 (m, 2H), 3.48-3.35 (m, 1H), 2.81 (s, 3H), 2.33 - 2.19 (m, 1H), 2.00 - 1.84 (m, 2H), 1.81 - 1.67 (m, 1H), 1.28 (s, 6H). MS-ESI calculated for [M+H]⁺ 533, found 533.

### Example 122

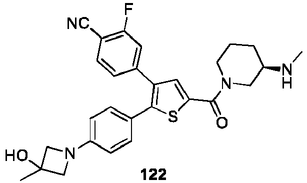

### Synthetic route:

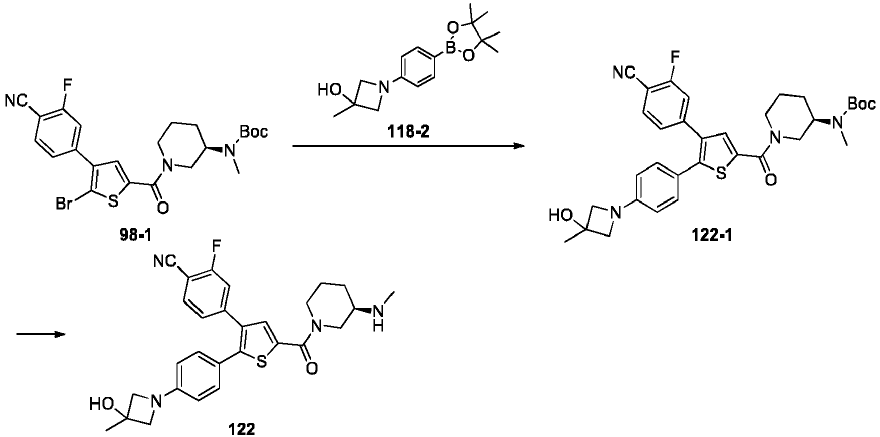

### Step 1

Under nitrogen atmosphere, compound **98-1** (780 mg, 1.34 mmol), compound **118-2** (475 mg, 1.64 mmol), and sodium carbonate (475 mg, 4.48 mmol) were dissolved in dioxane (8 mL) and water (2 mL), and the reaction mixture was added with [1,1'-bis(di-tert-butylphosphino)ferrocene]dichloropalladium(II) (97.3 mg, 0.149 mmol). The reaction mixture was stirred and reacted for 12 hours at 95°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.21) to obtain compound **122-1.** MS-ESI calculated for [M+H]⁺ 605, found 605.

### Step 2

Compound **122-1** (700 mg, 1.16 mmol) was dissolved in ethyl acetate (10.0 mL), and hydrochloride ethyl acetate solution (4 mol/L, 10.0 mL) was added dropwise thereto at 25°C. The reaction mixture was stirred for 1 hour at 25°C, concentrated under reduced pressure, and purified by preparative high performance liquid chromatography (chromatographic column: Phenomenex C18 80 * 40 mm * 3 µm; mobile phase: mobile phase A: ammonia aqueous solution with a volume fraction of 0.05%; mobile phase B: acetonitrile; B%: 44 to 47%, 8 minutes) to obtain compound **122.** ¹H NMR (400MHz, CD₃OD) δ 7.65 (t, *J*=7.6 Hz, 1H), 7.48 (s, 1H), 7.31 - 7.21 (m, 2H), 7.10 (d, *J*=8.4 Hz, 2H), 6.45 (d, *J*=8.4 Hz, 2H), 4.44 - 4.34 (m, 1H), 4.28 - 4.12 (m, 1H), 3.84 (d, *J*=7.6 Hz, 2H), 3.71 (d, *J*=7.6 Hz, 2H), 3.36 - 3.34 (m, 1H), 3.10 - 2.98 (m, 1H), 2.67 - 2.56 (m, 1H), 2.40 (s, 3H), 2.15 - 2.06 (m, 1H), 1.91 - 1.80 (m, 1H), 1.65 - 1.57 (m, 1H), 1.55 (s, 3H), 1.51 - 1.38 (m, 1H). MS-ESI calculated for [M+H]⁺ 505, found 505.

### Example 123

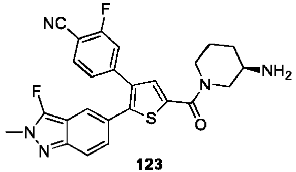

### Synthetic route:

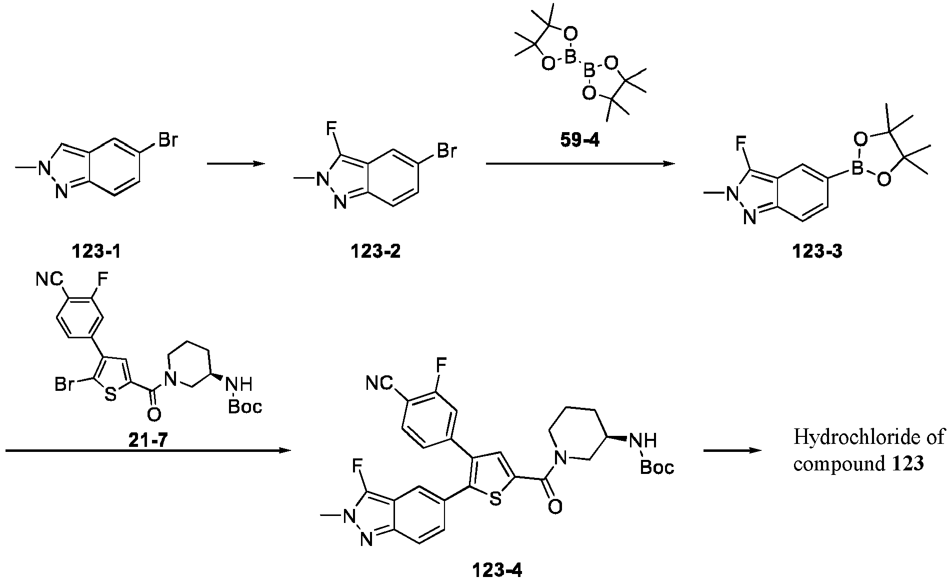

### Step 1

Compound **123-1** (3.00 g, 14.2 mmol) was dissolved in acetonitrile (100 mL), and the reaction mixture was added with 1-chloromethyl-4-fluoro-1,4-diazoniabicyclo[2.2.2]octane bis(tetrafluoroborate) (5.54 mg, 15.64 mmol). The reaction mixture was stirred and reacted for 12 hours at 25°C, added with water (50 mL), and extracted with ethyl acetate (100 mL × 1). The organic phase was washed with saturated brine (100 mL × 1), dried over anhydrous sodium sulfate, filtered, and concentrated under reduced pressure. The residue was purified by silica gel column chromatography (1/1, petroleum ether/ethyl acetate, R_{f} = 0.70) to obtain compound **123-2.** MS-ESI calculated for [M+H]⁺ 231, found 231. ¹H NMR (400MHz, CD₃OD) δ = 7.76 (d, *J*=1.2 Hz, 1H), 7.42 - 7.37 (m, 1H), 7.37 - 7.33 (m, 1H), 4.05 (d, *J*=2.0 Hz, 3H).

### Step 2

Under nitrogen atmosphere, compound **123-2** (625 mg, 2.73 mmol), compound **59-4** (1.39 g, 5.46 mmol), and potassium acetate (803 mg, 8.19 mmol) were dissolved in dioxane (30.0 mL), and the reaction mixture was added with [1,1'-bis(diphenylphosphino)ferrocene]dichloropalladium(II) (200 mg, 0.273 mmol). The reaction mixture was stirred and reacted for 12 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.60) to obtain compound **123-3.** MS-ESI calculated for [M+H]⁺ 277, found 277.

### Step 3

Under nitrogen atmosphere, compound **123-3** (600 mg, 2.14 mmol), compound **21-7** (1.00 g, 1.82 mmol), and potassium acetate (388 mg, 3.95 mmol) were dissolved in dioxane (12.0 mL) and water (3.00 mL), and the reaction mixture was added with [1,1'-bis(diphenylphosphino)ferrocene]dichloropalladium(II) (144 mg, 0.190 mmol). The reaction mixture was stirred and reacted for 3 hours at 100°C, added with water (20 mL), and extracted with ethyl acetate (20 mL × 1). The organic phase was washed with saturated brine (20 mL × 1), dried over anhydrous sodium sulfate, filtered, and concentrated under reduced pressure. The residue was purified by silica gel column chromatography (1/1, petroleum ether/ethyl acetate, R_{f} = 0.36) to obtain compound **123-4.** MS-ESI calculated for [M+H]⁺ 623, found 623.

### Step 4

Compound **123-4** (900 mg, 1.56 mmol) was dissolved in ethyl acetate (10.0 mL), and hydrochloride ethyl acetate solution (4 mol/L, 18.6 mL) was added dropwise thereto at 25°C. The reaction mixture was stirred for 1 hour at 25°C, concentrated under reduced pressure, and purified by preparative high performance liquid chromatography (chromatographic column: Xtimate C18 150 * 40 mm * 5 µm; mobile phase: A: hydrochloric acid aqueous solution with a volume fraction of 0.05%; mobile phase B: acetonitrile 18% to 48%, 10 minutes) to obtain the hydrochloride of compound **123.** ¹H NMR (400MHz, CD₃OD) δ = 7.70 - 7.60 (m, 3H), 7.44 (d, *J*=9.2 Hz, 1H), 7.33 (d, *J*=10.4 Hz, 1H), 7.28 (d, *J*=8.0 Hz, 1H), 7.12 (d, *J*=9.2 Hz, 1H), 4.50 - 4.37 (m, 1H), 4.20 - 4.14 (m, 1H), 4.08 (s, 3H), 3.56 - 3.40 (m, 3H), 2.28 - 2.16 (m, 1H), 2.00 - 1.67 (m, 3H). MS-ESI calculated for [M+H]⁺ 478, found 478.

**Biochemical assays:**

### Experiment 1A: Evaluation of enzyme activity

### Testing unit: Wuhan Heyan Biomedical Technology Co., Ltd.

The purpose of the assay was to test the *in vitro* inhibitory activity of the compounds against LSD1. The enzyme used in the assay was human LSD1, and the standard substrate was histone H3K4me peptide (20 µM). The activity of the compounds was determined by the enzyme-coupling fluorescent method by the combined detection of H₂O₂ generated after the reaction of LSD1 by horseradish peroxidase (HRP) and the fluorescent reagent Amplex Red. Starting from 10 µM, the compounds were 3-fold diluted to detect the IC₅₀ values of the compounds at 10 concentrations. The enzyme and substrate were incubated for 30 minutes before the compound was added to the substrate to start the reaction. Fluorescence detector: EnVision, excitation wavelength: Ex/Em = 530/590 nM.

The compounds were tested for LSD1 inhibitory activity, and the results are shown in Table 1.

**Table 1: In vitro enzyme activity screening test results of compounds of the present disclosure**

| Compound No. | IC₅₀ (nM) | Compound No. | IC₅₀ (nM) |
|---|---|---|---|
| Hydrochloride of compound 1 | 81.96 | Hydrochloride of compound 8 | 120.5 |
| Hydrochloride of compound 2 | 122.4 | Hydrochloride of compound 9 | 117.6 |
| Hydrochloride of compound 4 | 109.8 | Hydrochloride of compound 10 | 192.3 |
| Hydrochloride of compound 5 | 344.9 | Hydrochloride of compound 12 | 393.2 |
| Hydrochloride of compound 6 | 111.9 | Hydrochloride of compound 15 | 310.3 |
| Hydrochloride of compound 7 | 120.7 | | |

### Experiment 1B: Evaluation of enzyme activity

### Testing unit: Reaction Biology Corp., USA.

The purpose of the assay was to test the *in vitro* inhibitory activity of the compounds against LSD1. The enzyme used in the assay was human LSD1, and the standard substrate was histone H3(1-21)K4me2 peptide (10 µM). The activity of the compounds was determined by the enzyme-coupling fluorescent method by the combined detection of H₂O₂ generated after the reaction of LSD1 by horseradish peroxidase (HRP) and the fluorescent reagent Amplex Red. Starting from 10 µM, the compounds were 3-fold diluted to detect the IC₅₀ values of the compounds at 10 concentrations. The enzyme and substrate were incubated for 30 minutes before the compound was added to the substrate to start the reaction. Fluorescence detector: EnVision, excitation wavelength: Ex/Em = 535/590 nM.

The compounds were tested for LSD1 inhibitory activity, and the results are shown in Table 2.

**Table 2: In vitro enzyme activity screening test results of compounds of the present disclosure**

| Compound No. | IC₅₀ (nM) | Compound No. | IC₅₀ (nM) |
|---|---|---|---|
| Compound 14 | 5.37 | Compound 78 | 47.4 |
| Hydrochloride of compound 16 | 7.51 | Hydrochloride of compound 79 | 13.6 |
| Hydrochloride of compound 18 | 6.60 | Hydrochloride of compound 80 | 20.2 |
| Hydrochloride of compound 19 | 64.8 | Hydrochloride of compound 82 | 3.01 |
| Hydrochloride of compound 20 | 19.4 | Compound 84 | 13.2 |
| Hydrochloride of compound 21 | 19.4 | Hydrochloride of compound 85 | 14.2 |
| Hydrochloride of compound 23 | 76.8 | Hydrochloride of compound 86 | 16.0 |
| Hydrochloride of compound 24 | 70.9 | Hydrochloride of compound 87 | 24.8 |
| Hydrochloride of compound 25 | 63.9 | Hydrochloride of compound 88 | 87.6 |
| Hydrochloride of compound 26 | 19.6 | Hydrochloride of compound 89 | 15.2 |
| Hydrochloride of compound 27 | 48.2 | Hydrochloride of compound 90 | 37.3 |
| Hydrochloride of compound 29 | 29.7 | Hydrochloride of compound 91 | 13.0 |
| Hydrochloride of compound 30 | 122 | Hydrochloride of compound 92 | 9.81 |
| Hydrochloride of compound 31 | 26.7 | Hydrochloride of compound 93 | 18.0 |
| Hydrochloride of compound 33 | 38.0 | Hydrochloride of compound 94 | 98.4 |
| Hydrochloride of compound 34 | 36.5 | Hydrochloride of compound 95 | 10.1 |
| Hydrochloride of compound 35 | 24.6 | Hydrochloride of compound 96 | 20.6 |
| Hydrochloride of compound 36 | 3.44 | Hydrochloride of compound 97 | 25.3 |
| Hydrochloride of compound 37 | 1.73 | Hydrochloride of compound 98 | 12.6 |
| Compound 40 | 95.6 | Hydrochloride of compound 99 | 17.4 |
| Hydrochloride of compound 44 | 70.6 | Hydrochloride of compound 100 | 14.1 |
| Hydrochloride of compound 46 | 81.3 | Hydrochloride of compound 101 | 28.7 |
| Hydrochloride of compound 47 | 48.8 | Hydrochloride of compound 102 | 56.9 |
| Hydrochloride of compound 49 | 101 | Hydrochloride of compound 103 | 21.6 |
| Compound 50 | 28.3 | Hydrochloride of compound 104 | 16.6 |
| Hydrochloride of compound 52 | 251 | Hydrochloride of compound 107 | 15.1 |
| Hydrochloride of compound 54 | 49.9 | Hydrochloride of compound 109 | 10.7 |
| Hydrochloride of compound 56 | 109 | Hydrochloride of compound 110 | 9.84 |
| Compound 57 | 10.4 | Hydrochloride of compound 111 | 17.6 |
| Compound 58 | 20.4 | Hydrochloride of compound 112 | 5.84 |
| Compound 59 | 43.1 | Hydrochloride of compound 116 | 9.27 |
| Compound 60 | 18.4 | Hydrochloride of compound 117 | 19.0 |
| Hydrochloride of compound 62 | 30.1 | Hydrochloride of compound 118 | 13.8 |
| Hydrochloride of compound 64 | 29.6 | Hydrochloride of compound 119 | 17.7 |
| Hydrochloride of compound 66 | 73.4 | Hydrochloride of compound 120 | 9.4 |
| Hydrochloride of compound 72 | 8.56 | Hydrochloride of compound 121 | 14.3 |
| Hydrochloride of compound 73 | 11.8 | Compound 122 | 13.1 |
| Compound 74 | 33.2 | Hydrochloride of compound 123 | 26.7 |
| Compound 75 | 28.9 | | |

Conclusion: The compounds of the present disclosure exhibit significant inhibitory activity against LSD1.

### Experiment 2: Evaluation of inhibitory activity on NCI-H1417 cell proliferation

Experimental purpose: To test the inhibitory activity of test compounds on NCI-H1417 cell proliferation.

Experimental materials: RPMI 1640 medium, fetal bovine serum, Promega CellTiter-Glo reagent. The NCI-H1417 cell line was purchased from ATCC. Envision multilabel plate reader (PerkinElmer).

Experimental method: The compounds were dissolved to 10 mM and 5-fold diluted with DMSO in the compound plates, starting with a concentration of 2 mM. 3-fold serial dilution was performed with Bravo to 10 concentrations. Then 250 nL of the compound solution was transferred to the upper and lower double duplicate wells of a blank 384 cell plate with Echo. To each well containing the transferred 250 nL of DMSO/compound was added a cell suspension of 1000 cells/50 µL per well, and the compound was diluted 200-fold, i.e., resulting in an initial working concentration of 10 µM. The cell plate was incubated in a carbon dioxide incubator for 10 days. To the cell plate was added 25 µL of Promega CellTiter-Glo reagent per well and the mixture was shaken for 10 minutes at room temperature to stabilize the luminescence signal. Readings were performed on a PerkinElmer Envision multilabel plate reader.

Data analysis: Raw data were converted into inhibition rate using the formula: (Max - Ratio)/(Max - Min) * 100%. The IC₅₀ values could then be derived from four-parameter curve fitting. (derived from 205 mode in XLFIT5, iDBS)

The compounds were tested for inhibitory activity of compounds on NCI-H1417 cell proliferation, and the results are shown in Table 3.

**Table 3: Results of the inhibition assay of compounds of the present disclosure on NCI-H1417 cell proliferation**

| Compound No. | IC₅₀ (nM) | Compound No. | IC₅₀ (nM) |
|---|---|---|---|
| Hydrochloride of compound 1 | 6.88 | Hydrochloride of compound 79 | 3.32 |
| Hydrochloride of compound 2 | 1.75 | Hydrochloride of compound 80 | 16.14 |
| Hydrochloride of compound 4 | 30.15 | Hydrochloride of compound 82 | 1.73 |
| Hydrochloride of compound 6 | 37.89 | Compound 84 | 25.93 |
| Hydrochloride of compound 7 | 20.41 | Hydrochloride of compound 85 | 2.32 |
| Hydrochloride of compound 8 | 8.73 | Hydrochloride of compound 86 | 10.29 |
| Hydrochloride of compound 9 | 15.82 | Hydrochloride of compound 87 | 5.11 |
| Hydrochloride of compound 10 | 22.41 | Hydrochloride of compound 88 | 39.35 |
| Compound 14 | 0.54 | Hydrochloride of compound 89 | 1.61 |
| Hydrochloride of compound 16 | 0.17 | Hydrochloride of compound 90 | 37.57 |
| Hydrochloride of compound 18 | 18.16 | Hydrochloride of compound 91 | 0.83 |
| Hydrochloride of compound 20 | 32.31 | Hydrochloride of compound 92 | 0.43 |
| Hydrochloride of compound 21 | 6.83 | Hydrochloride of compound 93 | 4.11 |
| Hydrochloride of compound 24 | 30.62 | Hydrochloride of compound 94 | 32.78 |
| Hydrochloride of compound 26 | 6.87 | Hydrochloride of compound 95 | 2.51 |
| Hydrochloride of compound 29 | 17.17 | Hydrochloride of compound 96 | 4.79 |
| Hydrochloride of compound 31 | 11.27 | Hydrochloride of compound 97 | 9.60 |
| Hydrochloride of compound 33 | 16.21 | Hydrochloride of compound 98 | 0.07 |
| Hydrochloride of compound 34 | 9.92 | Hydrochloride of compound 99 | 4.26 |
| Hydrochloride of compound 35 | 8.84 | Hydrochloride of compound 100 | 0.37 |
| Hydrochloride of compound 36 | 1.17 | Hydrochloride of compound 101 | 3.35 |
| Hydrochloride of compound 37 | 0.93 | Hydrochloride of compound 102 | 31.41 |
| Compound 40 | 18.68 | Hydrochloride of compound 103 | 13.66 |
| Hydrochloride of compound 44 | 8.87 | Hydrochloride of compound 104 | 4.75 |
| Hydrochloride of compound 46 | 19.40 | Hydrochloride of compound 105 | 6.14 |
| Hydrochloride of compound 47 | 10.19 | Hydrochloride of compound 106 | 0.19 |
| Compound 50 | 9.19 | Hydrochloride of compound 107 | 3.31 |
| Hydrochloride of compound 54 | 5.11 | Hydrochloride of compound 108 | 2.11 |
| Hydrochloride of compound 56 | 56.79 | Hydrochloride of compound 109 | 2.03 |
| Compound 57 | 1.53 | Hydrochloride of compound 110 | 3.47 |
| Compound 58 | 3.56 | Hydrochloride of compound 111 | 2.12 |
| Compound 59 | 13.2 | Hydrochloride of compound 112 | 4.99 |
| Compound 60 | 2.36 | Hydrochloride of compound 113 | 3.98 |
| Hydrochloride of compound 62 | 11.18 | Hydrochloride of compound 114 | 5.18 |
| Hydrochloride of compound 63 | 28.57 | Hydrochloride of compound 115 | 1.94 |
| Hydrochloride of compound 64 | 16.16 | Hydrochloride of compound 117 | 40.17 |
| Hydrochloride of compound 65 | 11.05 | Hydrochloride of compound 118 | 31.58 |
| Compound 70 | 9.88 | Hydrochloride of compound 119 | 9.17 |
| Hydrochloride of compound 71 | 0.64 | Hydrochloride of compound 120 | 17.46 |
| Hydrochloride of compound 72 | 4.47 | Hydrochloride of compound 121 | 7.7 |
| Hydrochloride of compound 73 | 14.46 | Compound 122 | 9.83 |
| Compound 75 | 28.0 | Hydrochloride of compound 123 | 6.25 |

Conclusion: The compounds of the present disclosure have significant inhibitory activity on NCI-H1417 cell proliferation.

### Experiment 3: Pharmacokinetic evaluation of compounds

### Experimental purpose: To test the pharmacokinetics of compounds in CD-1 mice in vivo

### Experimental materials:

### CD-1 mice (male/female, 7 to 9 weeks old, Shanghai SLAC)

### Experimental operation:

The pharmacokinetic characteristics of the compounds in rodents after intravenous injection and oral administration were tested according to the standard protocol. In the experiment, the candidate compound was formulated into clear solutions, and a single intravenous injection and oral administration were given to mice. The vehicle for intravenous injection and oral administration was a mixed vehicle composed of 10% dimethyl sulfoxide and 90% hydroxypropyl β-cyclodextrin with a content of 10%. In this project, four male/female CD-1 mice were used. Two mice were injected intravenously at a dose of 1 mg/kg, and plasma samples were collected at 0.083, 0.25, 0.5, 1, 2, 4, 8, and 24 hours after administration; the other two were administered orally by gavage at a dose of 2 mg/kg, and plasma samples were collected at 0.25, 0.5, 1, 2, 4, 8, and 24 hours after administration. The samples were centrifuged at 3200 g for 10 minutes at 4°C, and plasma samples were obtained by separating the supernatant. A methanol solution containing internal standard with 20 times the volume of the sample was added thereto to precipitate the protein, and the mixture was centrifuged at 12000 g for 15 minutes at 4°C. 50 µL of the supernatant was taken and transferred to a 96-well plate and centrifuged again. The supernatant was taken for sampling. The plasma concentration was quantitatively analyzed using LC-MS/MS analysis method, and pharmacokinetic parameters, such as peak concentration (Cₘₐₓ), clearance rate (CL), half-life (T_{1/2}), volume of distribution at steady state (Vdss), area under drug-time curve (AUC₀₋ₗₐₛₜ), bioavailability (F).

The experimental results are shown in Table 4 and Table 5:

**Table 4: Pharmacokinetic test results in female CD-1 mice**

| Compound | Peak concentration Cₘₐₓ (nM) | Clearance rate CL (mL/min/kg) | Volume of distribution at steady state Vdss (L/kg) | Half life T_{1/2} (IV, h) | Area under drug-time curve AUC₀₋ₗₐₛₜ PO (nM. hr) | Bioavailability F (%) |
|---|---|---|---|---|---|---|
| Hydrochloride of compound 1* | 245 | 17.8 | 7.66 | 5.59 | 1562 | 47.2 |
| Hydrochloride of compound 2* | 209 | 19.6 | 7.90 | 5.23 | 1428 | 47.7 |
| Hydrochloride of compound 6 | 222 | 18.0 | 7.76 | 5.37 | 2519 | 64.0 |
| Hydrochloride of compound 7 | 176 | 20.9 | 6.19 | 3.74 | 1886 | 52.4 |
| Hydrochloride of compound 36 | 224 | 37.3 | 4.71 | 2.01 | 971 | 49.6 |

| | | | | | | |
|---|---|---|---|---|---|---|
| **[1305]** *Note: The sampling time points for intravenous injection of the hydrochloride of compound 1 and the hydrochloride of compound 2 are 0.033, 0.083, 0.25, 0.5, 1, 2, 4, 8, and 12 hours, and the sampling time points for oral gavage are 0.083, 0.25, 0.5, 1, 2, 4, 8, and 12 hours. The sampling time points of administration of other compounds were performed according to the above experimental protocol. | | | | | | |

**Table 5: Pharmacokinetic test results in male CD-1 mice**

| Compound | Peak concentration Cₘₐₓ (nM) 3 | Clearance rate CL (mL/min/kg) | Volume of distribution at steady state Vdss (L/kg) | Half life T_{1/2} (IV, h) | Area under drug-time curve AUC₀₋ₗₐₛₜ PO (nM. hr) | Bioavailability F (%) |
|---|---|---|---|---|---|---|
| Hydrochloride of compound 1 | 176 | 16.7 | 8.33 | 6.13 | 2241 | 53.9 |
| Hydrochloride of compound 36 | 148 | 31.5 | 4.47 | 2.1 | 742 | 33.9 |
| Hydrochloride of compound 80 | 109 | 33.7 | 4.89 | 1.93 | 576 | 31.9 |
| Hydrochloride of compound 87 | 267 | 25.7 | 3.13 | 1.54 | 1012 | 41.1 |
| Hydrochloride of compound 98 | 160 | 45.3 | 6.94 | 2.28 | 708 | 58.6 |
| Hydrochloride of compound 99 | 145 | 42.0 | 7.23 | 2.48 | 625 | 47.7 |

Conclusion: The compounds of the present disclosure have good pharmacokinetic properties, including good oral bioavailability, oral exposure, half-life, clearance rate, etc.

### Experiment 4: hERG potassium ion channel inhibition assay

Experimental purpose: To test the effects of the test examples on hERG potassium ion channels using an automated patch clamp method.

### Experimental methods

### 4.1 Cell preparation

4.1.1 CHO-hERG cells were cultured in a 175 cm² culture flask. When the cell density grew from 60% to 80%, the culture medium was removed. The cells were washed once with 7 mL of PBS (Phosphate Buffered Saline), and then 3 mL of Detachin cell dissociation reagent was added thereto for digestion.

4.1.2 After the digestion was completed, 7 mL of culture medium was added thereto for neutralization, then the mixture was centrifuged, the supernatant was aspirated, and then 5 mL of culture medium was added thereto for resuspension to ensure a cell density of 2 × 10⁶/mL to 5 × 10⁶/mL.

4.2 Solution preparation, and the composition of intracellular fluid and extracellular fluid are shown in Table 6.

**Table 6: Composition of intracellular fluid and extracellular fluid**

| Reagents | Extracellular fluid (mM) | Intracellular fluid (mM) |
|---|---|---|
| CaCl₂ | 1 | 1 |
| MgCl₂ | 1.25 | 1 |
| KCl | 5 | 140 |
| NaCl | 140 | 0 |
| Glucose | 10 | 0 |
| HEPES | 10 | 10 |
| EGTA | 0 | 10 |
| pH | Adjust pH to 7.4 with NaOH | Adjust pH to 7.2 with KOH |
| Osmotic pressure | 305 mOsm | 290 mOsm |

### 4.3 Electrophysiological recording process

The single-cell high-resistance sealing and whole-cell modeling formation processes were all automatically completed by the Qpatch instrument of the Shanghai Institute of Materia Medica, Chinese Academy of Sciences. After obtaining the whole-cell recording modeling, the cells were clamped at -80 mV, and before a 5-second +40 mV depolarizing stimulus was given, a 50-millisecond -50 mV prepulse was first given, then the voltage was repolarized to - 50 mV and holden for 5 seconds, and finally returned to -80 mV. This voltage stimulus was applied every 15 seconds, recorded for 2 minutes, and then the extracellular fluid was recorded for 5 minutes, and then the administration process was initiated, with compound concentrations starting at the lowest test concentration. The voltage stimulus was given for 2.5 minutes for each test concentration, and after all concentrations had been continuously given, 3 µM the positive control compound, Cisapride, was given. At least 3 cells were tested per concentration (n ≥ 3).

### 4.4 Compound preparation

4.4.1 The compound stock solution was diluted with DMSO, and 20 µL of DMSO solution was added with 10 µL of the compound stock solution, and serially diluted 3-fold to 6 DMSO concentrations.

4.4.2 4 µL of 6 DMSO concentrations of compounds were taken respectively and added to 396 µL of extracellular fluid, and the mixture was diluted 100-fold to 6 intermediate concentrations, then 80 µL of 6 intermediate concentrations of compounds was taken and added to 320 µL of extracellular fluid, and the mixture was diluted 5-fold to the final test concentration.

4.4.3 The highest test concentration was 40 µM, and the concentrations were respectively 40, 13.3, 4.4, 1.48, 0.494, and 0.165 µM for a total of six concentrations.

4.4.4 The DMSO content in the final test concentration did not exceed 0.2%, and this concentration of DMSO did not affect the hERG potassium channel.

4.4.5 The preparation of the compound was completed by the Bravo instrument throughout the dilution process.

### 4.5 Data analysis

Experimental data were analyzed by GraphPad Prism 5.0 software.

### 4.6 The test results are shown in Table 7.

**Table 7: Results of hERG IC₅₀ values for compounds of the present disclosure**

| Compound No. | hERG IC₅₀ (µM) | Compound No. | hERG IC₅₀ (µM) |
|---|---|---|---|
| Hydrochloride of compound 16 | > 40 | Compound 77 | > 40 |
| Hydrochloride of compound 30 | > 40 | Compound 81 | > 40 |
| Hydrochloride of compound 32 | > 40 | Hydrochloride of compound 82 | 14.9 |
| Hydrochloride of compound 45 | 32.6 | Hydrochloride of compound 86 | 21 |
| Hydrochloride of compound 46 | > 40 | Hydrochloride of compound 87 | 13.4 |
| Hydrochloride of compound 54 | > 40 | Hydrochloride of compound 88 | 21.9 |
| Hydrochloride of compound 56 | > 40 | Hydrochloride of compound 89 | 22.7 |
| Compound 59 | > 40 | Hydrochloride of compound 90 | > 40 |
| Compound 61 | > 40 | Hydrochloride of compound 91 | > 40 |
| Hydrochloride of compound 62 | > 40 | Hydrochloride of compound 94 | > 40 |
| Compound 67 | > 40 | Hydrochloride of compound 98 | 18.6 |
| Compound 70 | 18.7 | Hydrochloride of compound 99 | 27 |
| Hydrochloride of compound 73 | > 40 | Hydrochloride of compound 111 | 21.5 |
| Compound 76 | > 40 | Hydrochloride of compound 121 | 20.5 |

Conclusion: The compounds of the present disclosure exhibit no significant inhibitory effect on hERG potassium ion channels.

### Experiment 5: In vivo pharmacodynamic study of compounds on CB17-SCID mouse model of human small cell lung cancer NCI-H1417 cell subcutaneous xenograft tumor

### 5.1 Experimental purpose:

The purpose of this experiment was to evaluate the *in vivo* efficacy of the compounds of the present disclosure on CB17-SCID mouse model of human small cell lung cancer NCI-H1417 cell subcutaneous xenograft tumor.

### 5.2 Experimental animals:

Species: mice
Strain: CB17-SCID mice
Week age and weight: 6-8 weeks old, 18 to 22 grams
Gender: Female
Supplier: Beijing Vital River Laboratory Animal Technology Co., Ltd.

### 5.3 Experimental methods and steps

### 5.3.1 Cell culture

Human lung cancer cells NCI-H1417 were cultured *in vitro* in suspension in RPMI 1640 medium with 10% fetal bovine serum, 100 U/mL penicillin, and 100 µg/mL streptomycin at 37°C in a 5% CO₂ cell culture incubator. Routine digestion and passage treatment were performed twice a week. When the number of cells reached the required number, the cells were counted and resuspended in PBS at a density of 10 × 10⁶ cells/200 µL.

### 5.3.2 Tumor cell inoculation

0.2 mL of NCI-H1417 tumor cells (containing 10 × 10⁶ cells) suspension (PBS : matrix gel = 1:1) was subcutaneously inoculated on the right back of mice, and in pharmacodynamic experiments, on the 16th day after cell inoculation, when the average tumor volume reached 86 mm³, the subjects were randomly grouped and the administration started.

### 5.3.3 Preparation of test substance

The experimental vehicle was 5% dimethyl sulfoxide/ 95% (10% Solutol) solution. Preparation method: 3.8 mL of PEG400 was taken and added to 34.2 mL of double-distilled water, 2 mL of dimethyl sulfoxide was added thereto, and the mixture was mixed well. The test substance was dissolved with the vehicle, prepared as a homogeneous solution of a certain concentration, and stored at 4°C.

### 5.3.4 Tumor measurement and experimental indicators

The experimental indicator was to examine whether tumor growth was inhibited, delayed, or cured. The tumor diameter was measured twice a week using vernier calipers. The calculation formula of tumor volume is V = 0.5*a* × *b*², wherein *a* and *b* represent the long and short diameters of the tumor respectively.

The tumor inhibitory efficacy of compounds was evaluated by TGI (%). TGI(%) reflects the tumor growth inhibition rate. Calculation of TGI (%): TGI (%) = [1 - (Average tumor volume at the end of administration in a certain treatment group - Average tumor volume at the beginning of administration in this treatment group) / (Average tumor volume at the end of treatment in the vehicle control group - Average tumor volume at the beginning of treatment in the vehicle control group )] × 100%.

5.4 The experimental results are shown in Table 8.

**Table 8: Evaluation of tumor inhibitory efficacy of compounds on human small cell lung cancer NCI-H1417 cell subcutaneous xenograft tumor model (calculated based on tumor volume at day 28 after administration)**

| **Group** | **Mean tumor volume ± SEM (mm³) (Dav 28)** | **TGI (%)** |
|---|---|---|
| Vehicle group 5% DMSO / 95% (10% polyoxyl 15 hydroxystearate aqueous solution) | 446 ± 50 | / |
| Hydrochloride of compound 36 (10 mg/kg, p.o., QD) | 87 ± 11 | 99.65 |
| Hydrochloride of compound 36 (10 mg/kg, p.o., BID) | 53 ± 3 | 109.13 |
| Hydrochloride of compound 87 (30 mg/kg, p.o., BID) | 67 ± 5 | 105.17 |
| Hydrochloride of compound 98 (5 mg/kg, | 44 ± 2 | 111.58 |
| p.o., QD) | | |
| Hydrochloride of compound 98 (5 mg/kg, p.o., BID) | 34 ± 1 | 114.20 |
| Hydrochloride of compound 98 (10 mg/kg, p.o., QD) | 38 ± 3 | 113.35 |
| Hydrochloride of compound 121 (10 mg/kg, p.o., QD) | 75 ± 4 | 103.19 |

Conclusion: The compounds of the present disclosure have excellent tumor inhibitory effect on the human small cell lung cancer NCI-H1417 xenograft tumor model.

## Claims

1. A compound of formula (II) or a pharmaceutically acceptable salt thereof, 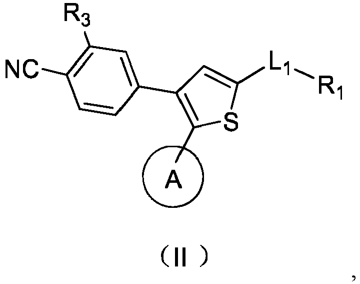
wherein Li is -C(=O)- or -C(=O)-NH-;
R₁ is R₁ₐ or 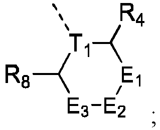
ring A is 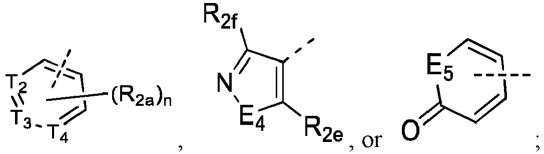
T₁ is CH or N;
T₂ is CR_{2c} or N;
T₃ is CR₂ or N;
T₄ is CR_{2b} or N;
E₁ is a single bond, -C(R₅)₂-, or -C(R₅)₂C(R₅)₂-;
E₂ is -NR₆- or -C(R₆₁)₂-;
E₃ is a single bond, -C(R₇)₂-, or -C(R₇)₂C(R₇)₂-;
E₄ is O, S, CH₂, or NR_{2g};
E₅ is O, CH₂, or NR_{2g};
R₁ₐ is C₁₋₃ alkyl substituted by 1, 2, or 3 Rₐ;
R₂ₐ is H or halogen;
R₂ and R_{2b} are each independently H, halogen, NH₂, C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, or -NHC₁₋₃ alkyl-C₃₋₆ cycloalkyl, wherein the C₁₋₃ alkyl, C₁₋₃ alkoxy, - S(=O)₂C₁₋₃ alkyl, -NHC₁₋₃ alkyl, 4- to 6-membered heterocycloalkyl, -C(=O)-4- to 6-membered heterocycloalkyl, and -NHC₁₋₃ alkyl-C₃₋₆ cycloalkyl are each independently and optionally substituted by 1, 2, or 3 R_{b};
R_{2c} is H;
or, R₂ and R_{2c}, together with the C atom to which they are attached, make the structural moiety 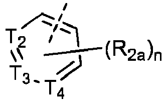 into 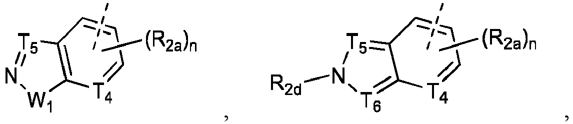 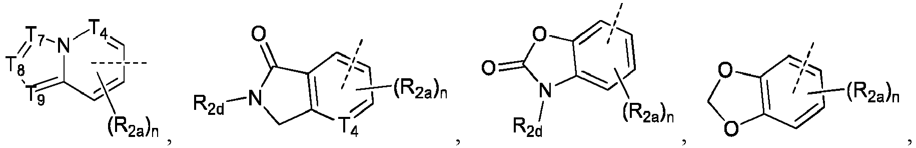 or 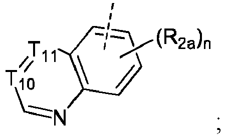
T₅, T₆, T₇, T₈, T₉, T₁₀, and T₁₁ are each independently N or CRₜ;
Wi is O or NR_{2d};
R_{2d} is H, C₁₋₄ alkyl, C₃₋₆ cycloalkyl, 4- to 6-membered heterocycloalkyl, -C₁₋₃ alkyl-C₃₋₆ cycloalkyl, or -C₁₋₃ alkyl-4- to 6-membered heterocycloalkyl, wherein the C₁₋₄ alkyl, C₃₋₆ cycloalkyl, 4- to 6-membered heterocycloalkyl, -C₁₋₃ alkyl-C₃₋₆ cycloalkyl, and -C₁₋₃ alkyl-4-to 6-membered heterocycloalkyl are each independently and optionally substituted by 1, 2, or 3 R_{c};
R₂ₑ, R_{2f}, and R_{2g} are each independently H or C₁₋₃ alkyl;
each R₃ is independently H, halogen, or C₁₋₃ alkoxy;
R₄ and R₈ are each independently H or C₁₋₃ alkyl;
each R₅ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{d};
R₆ is H or C₁₋₃ alkyl, wherein the C₁₋₃ alkyl is optionally substituted by 1, 2, or 3 Rₑ; each R₆₁ is independently H, halogen, OH, NH₂, or C₁₋₃ alkyl, wherein the C₁₋₃ alkyl is optionally substituted by 1, 2, or 3 R_{f};
each R₇ is independently H, halogen, OH, NH₂, C₁₋₃ alkyl, -NHC₁₋₃ alkyl, or -N(C₁₋₃ alkyl)₂, wherein the C₁₋₃ alkyl, -NHC₁₋₃ alkyl, and -N(C₁₋₃ alkyl)₂ are each independently and optionally substituted by 1, 2, or 3 R_{g};
or, when E₁ is -C(R₅)₂-, E₂ is -C(R₆₁)₂-, and E₃ is a single bond, R₅ and R₆₁, together with the C atom to which they are attached, make the structural moiety 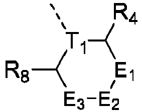 into 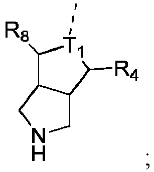
or, when E₂ is -C(R₆₁)₂-, two R₆₁, together with the C atom to which they are attached, make the structural moiety 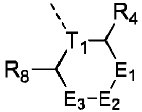 into 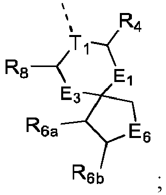
or, when E₃ is a single bond, R₄ and R₈, together with the C atom to which they are attached, make the structural moiety 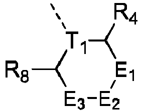 into 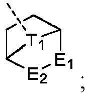
or, when T₁ is CH and E₁ is -C(R₅)₂-, R₅ and R₈, together with the C atom to which they are attached, make the structural moiety 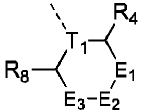 into 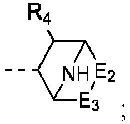
E₆ is NR_{6c} or O;
R₆ₐ and R_{6b} are each independently H, halogen, OH, =O, NH₂, or C₁₋₃ alkyl;
R_{6c} is H or C₁₋₃ alkyl;
Rₐ, R_{b}, R_{c}, R_{d}, Rₑ, R_{f}, and R_{g} are each independently F, Cl, Br, OH, CN, COOH, NH₂, C₁₋₃ alkyl, or C₁₋₃ alkoxy;
each Rₜ is independently H, F, Cl, Br, OH, CN, COOH, NH₂, C₁₋₃ alkyl, or C₁₋₃ alkoxy;
n is 1 or 2;
the 4- to 6-membered heterocycloalkyl contains 1, 2, 3, or 4 heteroatoms or heteroatom groups independently selected from -O-, -NH-, -S-, and N.

2. The compound or the pharmaceutically acceptable salt thereof according to claim 1, wherein the compound is 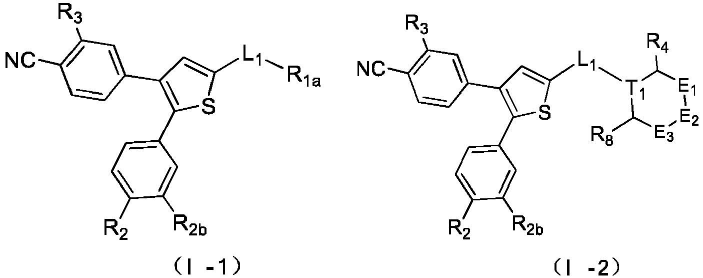 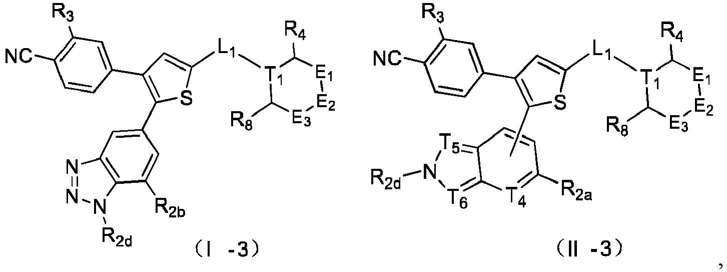 wherein
R₂ is H, halogen, NH₂, C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, or -NHC₁₋₃ alkyl-C₃₋₆ cycloalkyl, wherein the C₁₋₃ alkyl, C₁₋₃ alkoxy, -S(=O)₂C₁₋₃ alkyl, -NHC₁₋₃ alkyl, 4- to 6-membered heterocycloalkyl, -C(=O)-4- to 6-membered heterocycloalkyl, and -NHC₁₋₃ alkyl-C₃₋₆ cycloalkyl are each independently and optionally substituted by 1, 2, or 3 R_{b};
R_{b}, R₁ₐ, R₂ₐ, R_{2b}, R_{2d}, R₃, R₄, R₈, L₁, T₁, T₄, T₅, T₆, E₁, E₂, and E₃ are as defined in claim 1.

3. The compound or the pharmaceutically acceptable salt thereof according to claim 1 or 2, wherein Rₐ, R_{b}, R_{c}, R_{d}, Rₑ, R_{f}, and R_{g} are each independently F, Cl, Br, OH, CN, COOH, NH₂, CH₃, or OCH₃.

4. The compound or the pharmaceutically acceptable salt thereof according to claim 1 or 2, wherein each Rₜ is independently H, F, Cl, or CH₃.

5. The compound or the pharmaceutically acceptable salt thereof according to claim 1 or 2, wherein R₁ₐ is 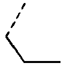 substituted by 1, 2, or 3 Rₐ.

6. The compound or the pharmaceutically acceptable salt thereof according to claim 5, wherein R₁ₐ is

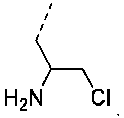

7. The compound or the pharmaceutically acceptable salt thereof according to claim 1 or 2, wherein R₂ₐ is H or F.

8. The compound or the pharmaceutically acceptable salt thereof according to claim 1 or 2, wherein R₂ and R_{2b} are each independently H, F, Cl, NH₂, -OCH₃, 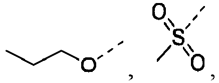 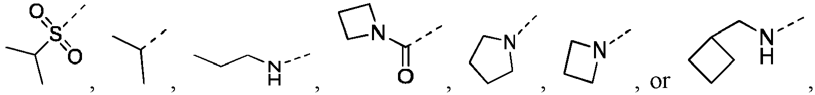 wherein the 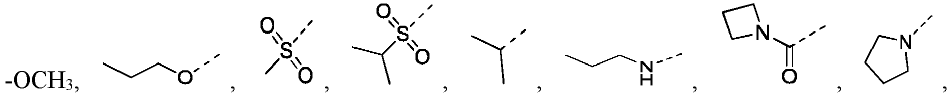 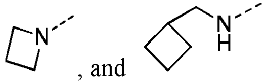 are each independently and optionally substituted by 1, 2, or 3 R_{b}.

9. The compound or the pharmaceutically acceptable salt thereof according to claim 8, wherein R₂ and R_{2b} are each independently H, F, Cl, NH₂, -OCH₃,

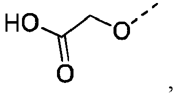

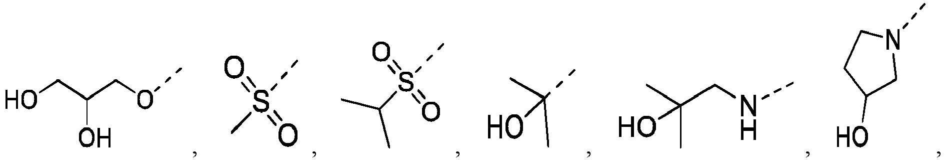

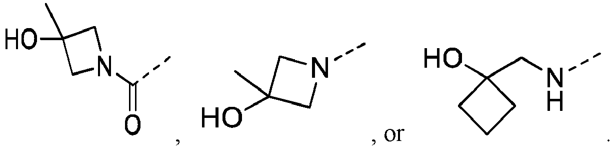

10. The compound or the pharmaceutically acceptable salt thereof according to claim 1 or 2, wherein R_{2d} is H, CH₃, 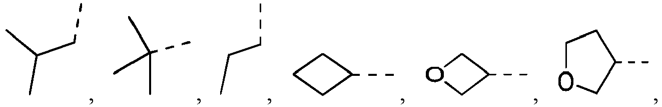 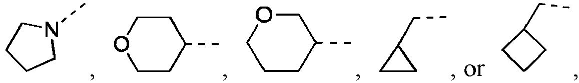 wherein the CH₃, 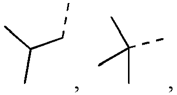 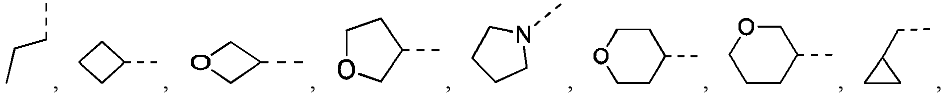 and 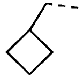 are optionally substituted by 1, 2, or 3 R_{c}.

11. The compound or the pharmaceutically acceptable salt thereof according to claim 1 or 2, wherein R_{2d} is H, CH₃, 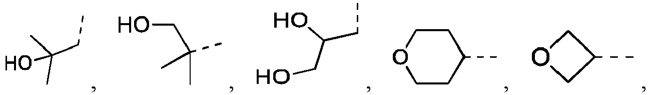 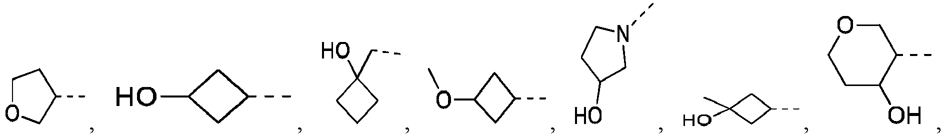 or

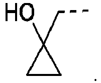

12. The compound or the pharmaceutically acceptable salt thereof according to claim 1, wherein R₂ₑ, R_{2f}, and R_{2g} are each independently H or CH₃.

13. The compound or the pharmaceutically acceptable salt thereof according to claim 1 or 2, wherein each R₃ is independently F or OCH₃.

14. The compound or the pharmaceutically acceptable salt thereof according to claim 1 or 2, wherein R₄ and R₈ are each independently H or CH₃.

15. The compound or the pharmaceutically acceptable salt thereof according to claim 1, wherein each R₅ is independently H, F, Cl, Br, I, OH, NH₂, CH₃, -NH-CH₃, or 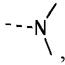 wherein the CH₃, -NH-CH₃, and 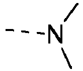 are each independently and optionally substituted by 1, 2, or 3 R_{d}.

16. The compound or the pharmaceutically acceptable salt thereof according to claim 15, wherein each R₅ is independently H, OH, NH₂, CH₃, or

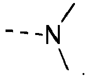

17. The compound or the pharmaceutically acceptable salt thereof according to claim 1, wherein R₆ is H, CH₃, or CH₂-CH₃, wherein the CH₃ and CH₂-CH₃ are each independently and optionally substituted by 1, 2, or 3 Rₑ.

18. The compound or the pharmaceutically acceptable salt thereof according to claim 17, wherein R₆ is H, CH₂-CN, or CH₂-CF₃.

19. The compound or the pharmaceutically acceptable salt thereof according to claim 1, wherein each R₆₁ is independently H, F, Cl, Br, I, OH, NH₂, or CH₃, and the CH₃ is optionally substituted by 1, 2, or 3 R_{f}.

20. The compound or the pharmaceutically acceptable salt thereof according to claim 19, wherein each R₆₁ is independently H, F, OH, or NH₂.

21. The compound or the pharmaceutically acceptable salt thereof according to claim 1, wherein R₆ₐ and R_{6b} are each independently H, OH, =O, NH₂, or CH₃.

22. The compound or the pharmaceutically acceptable salt thereof according to claim 1, wherein R_{6c} is H or CH₃.

23. The compound or the pharmaceutically acceptable salt thereof according to claim 1, wherein each R₇ is independently H, F, Cl, Br, I, OH, NH₂, CH₃, -NH-CH₃, or 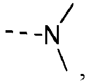 and the CH₃, -NH-CH₃, and 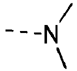 are optionally substituted by 1, 2, or 3 R_{g}.

24. The compound or the pharmaceutically acceptable salt thereof according to claim 23, wherein each R₇ is independently H, OH, NH₂, CH₃, or

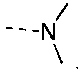

25. The compound or the pharmaceutically acceptable salt thereof according to claim 1, wherein the structural moiety

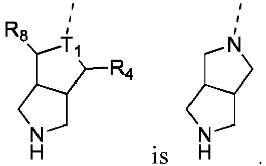

26. The compound or the pharmaceutically acceptable salt thereof according to claim 1, wherein the structural moiety

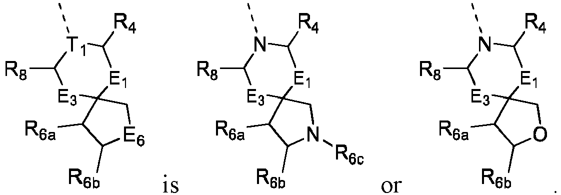

27. The compound or the pharmaceutically acceptable salt thereof according to claim 26, wherein the structural moiety

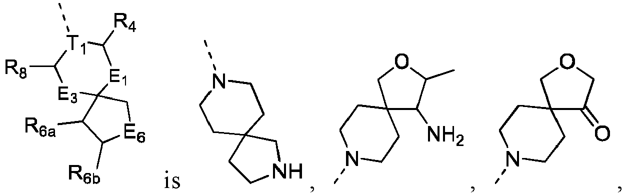

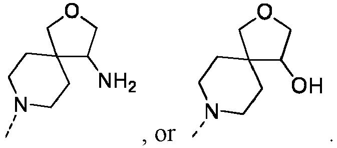

28. The compound or the pharmaceutically acceptable salt thereof according to claim 1, wherein the structural moiety 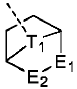 is

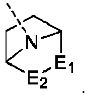

29. The compound or the pharmaceutically acceptable salt thereof according to claim 28, wherein the structural moiety 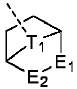 is

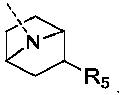

30. The compound or the pharmaceutically acceptable salt thereof according to claim 29, wherein the structural moiety 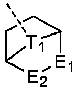 is

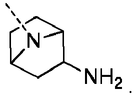

31. The compound or the pharmaceutically acceptable salt thereof according to claim 1, wherein the structural moiety 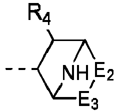 is

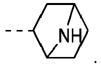

32. The compound or the pharmaceutically acceptable salt thereof according to claim 1, wherein R ₁ is

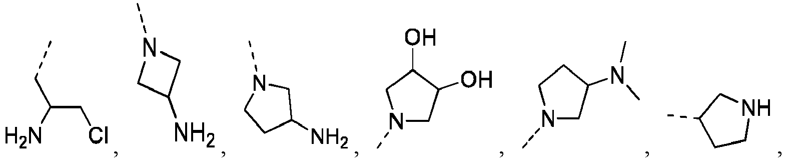

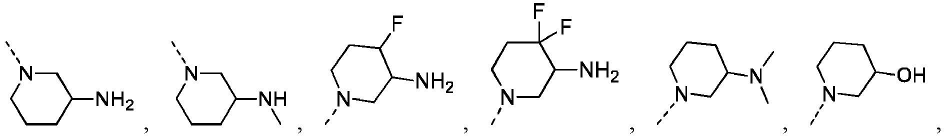

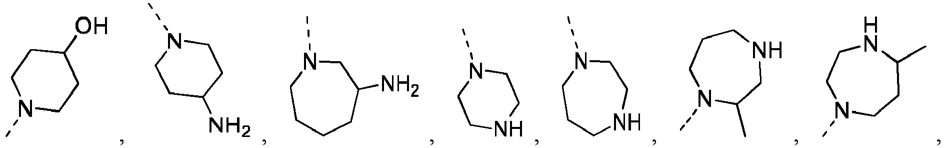

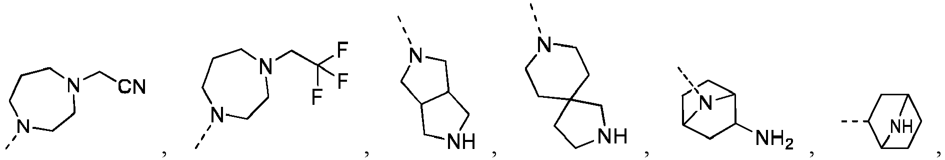

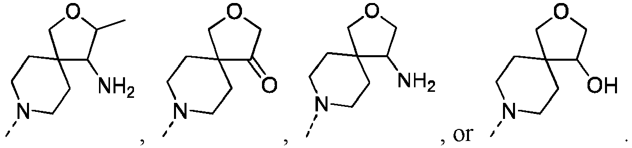

33. The compound or the pharmaceutically acceptable salt thereof according to claim 1, wherein ring A is

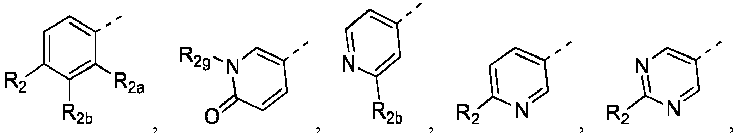

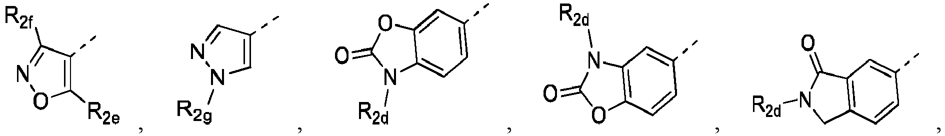

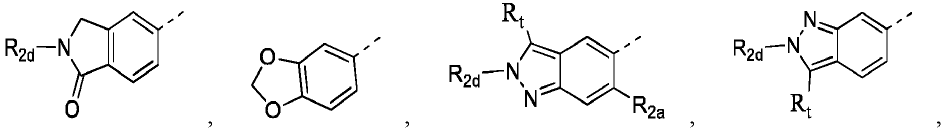

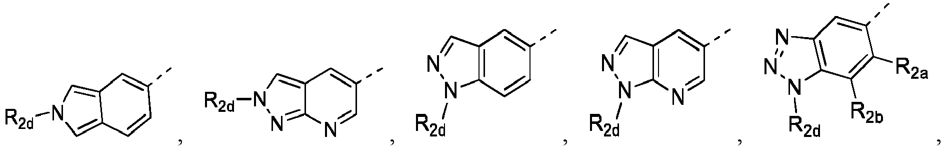

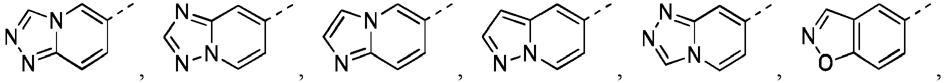

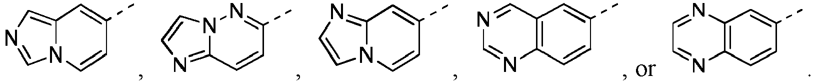

34. The compound or the pharmaceutically acceptable salt thereof according to claim 33, wherein ring A is

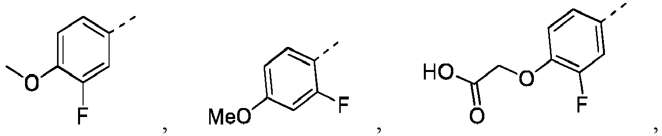

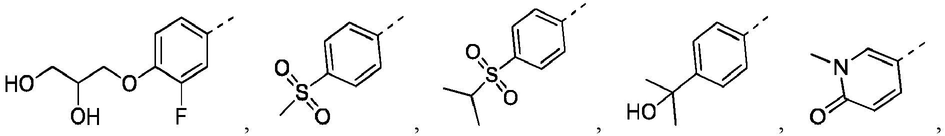

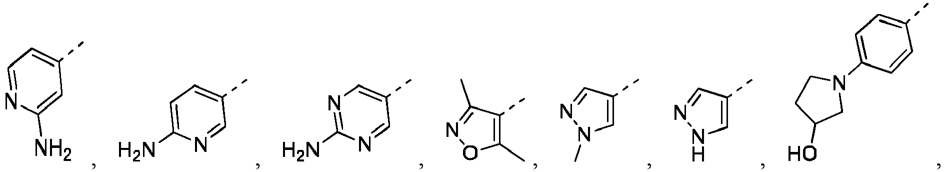

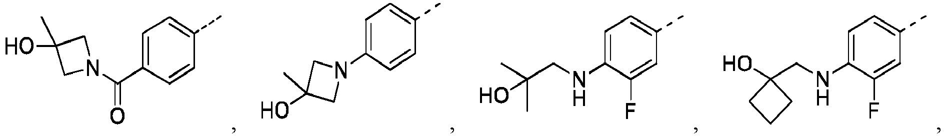

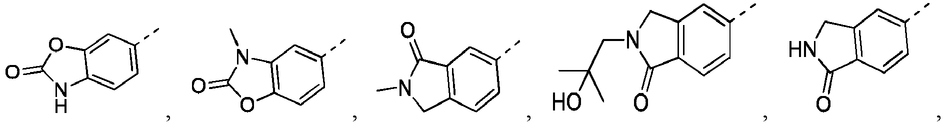

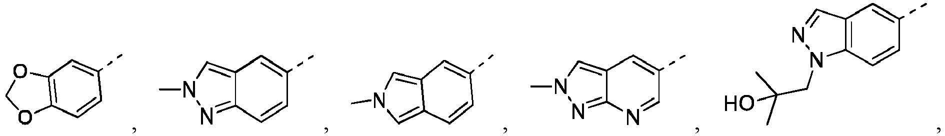

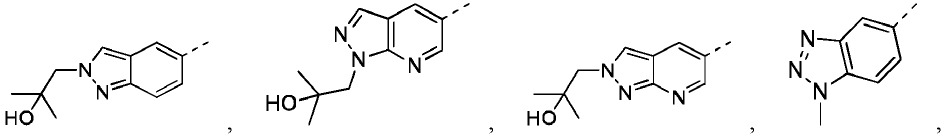

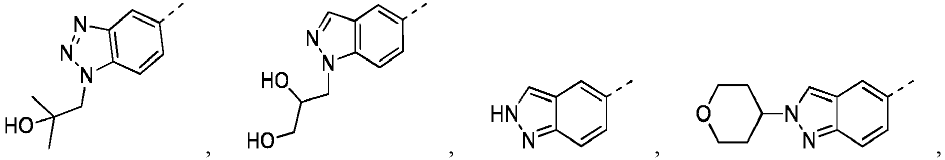

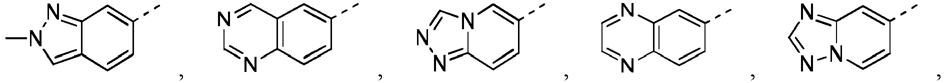

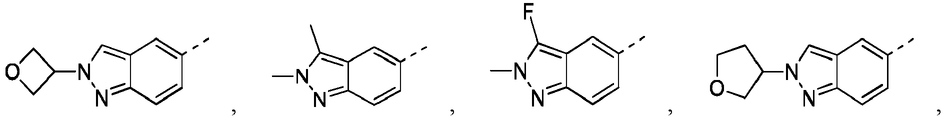

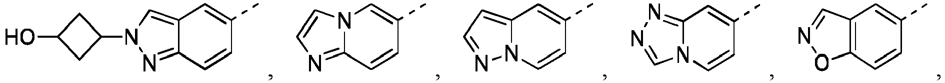

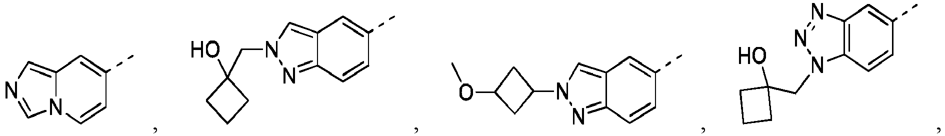

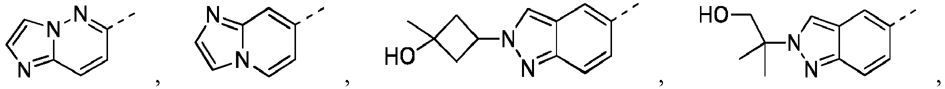

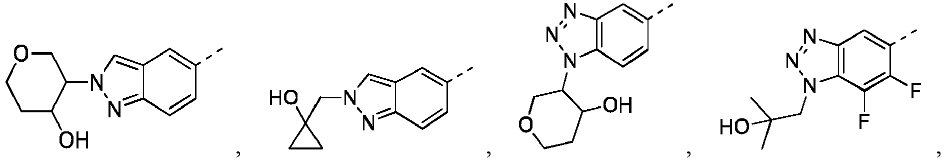

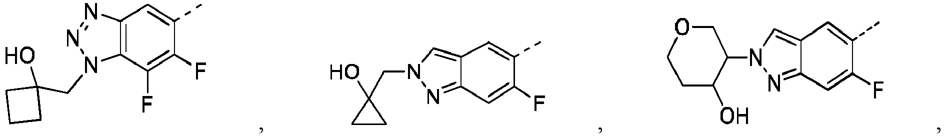

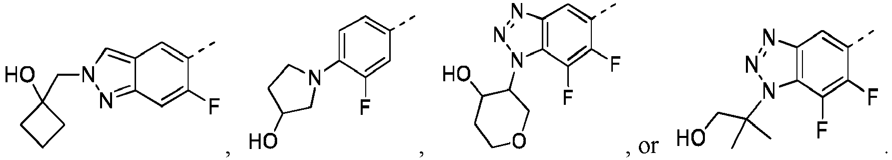

35. The compound or the pharmaceutically acceptable salt thereof according to claim 1, wherein the compound is 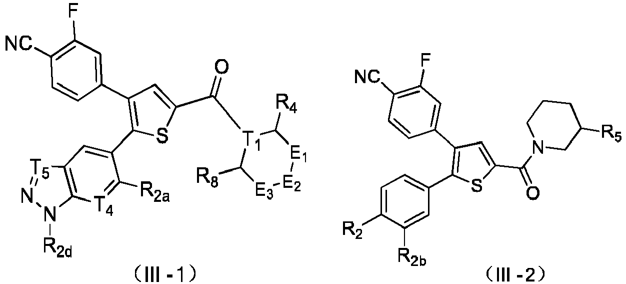 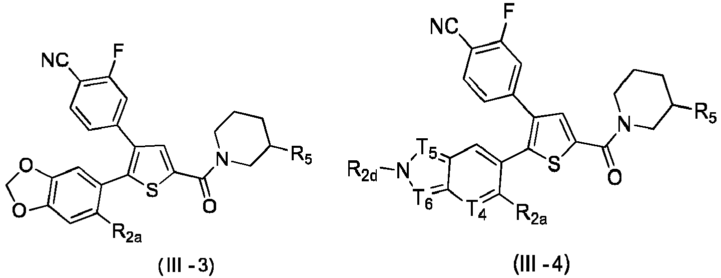 wherein
R₂ and R_{2b} are each independently H, halogen, NH₂, C₁₋₃ alkyl, C₁₋₃ alkoxy, -S(=O)₂C₁₋₃ alkyl, 4- to 6-membered heterocycloalkyl, or -C(=O)-4- to 6-membered heterocycloalkyl, wherein the C₁₋₃ alkyl, C₁₋₃ alkoxy, -S(=O)₂C₁₋₃ alkyl, 4- to 6-membered heterocycloalkyl, and -C(=O)-4- to 6-membered heterocycloalkyl are each independently and optionally substituted by 1, 2, or 3 R_{b};
R_{b}, R₂ₐ, R_{2d}, R₄, R₅, R₈, T₁, T₄, T₅, T₆, E₁, E₂, and E₃ are as defined in claim 1.

36. The compound or the pharmaceutically acceptable salt thereof according to claim 35, wherein the compound is 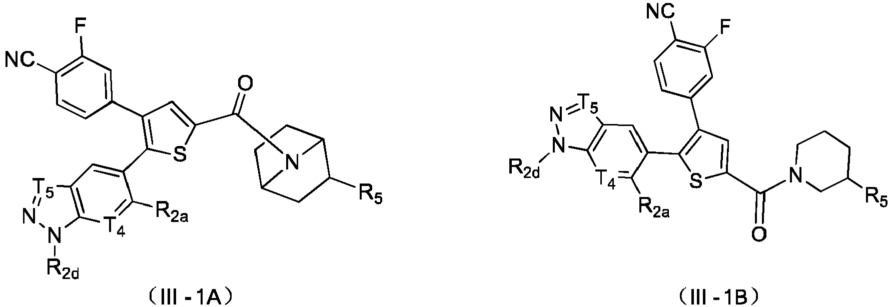 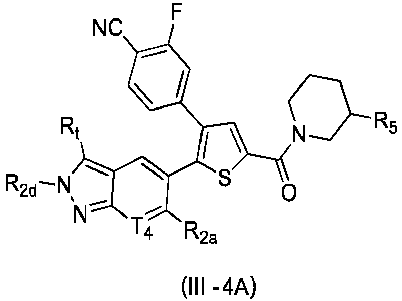 wherein R₂ₐ, R_{2d}, Rₜ, R₅, T₄, and T₅ are as defined in claim 35.

37. The compound or the pharmaceutically acceptable salt thereof according to claim 36, wherein the compound is 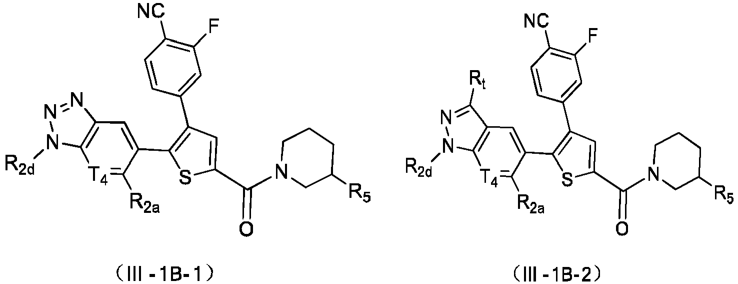 wherein R₂ₐ, R_{2d}, Rₜ, T₄, and R₅ are as defined in claim 36.

38. A compound of the following formula or a pharmaceutically acceptable salt thereof,

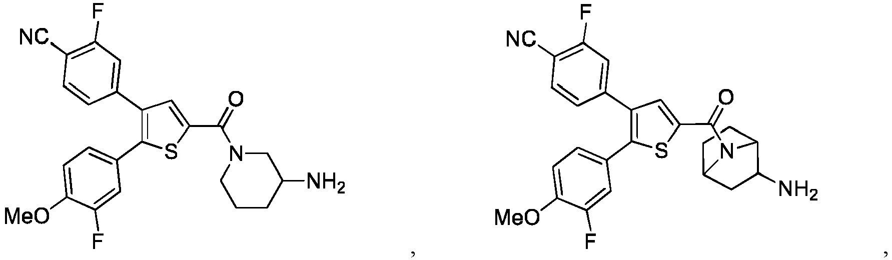

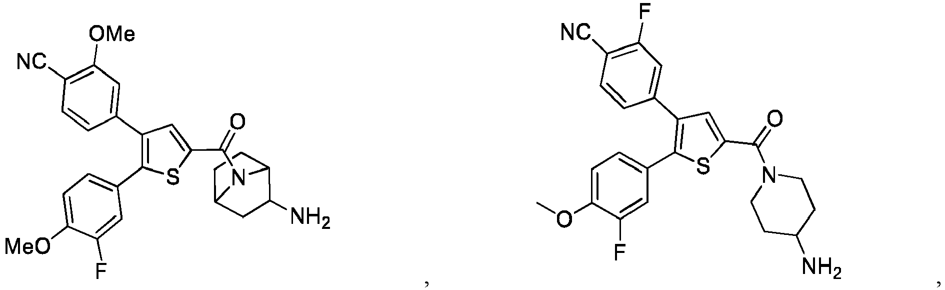

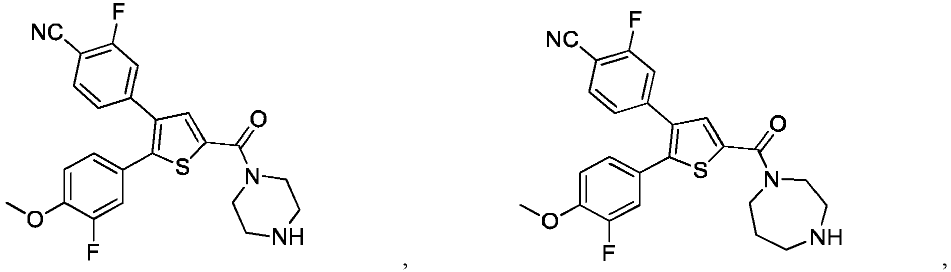

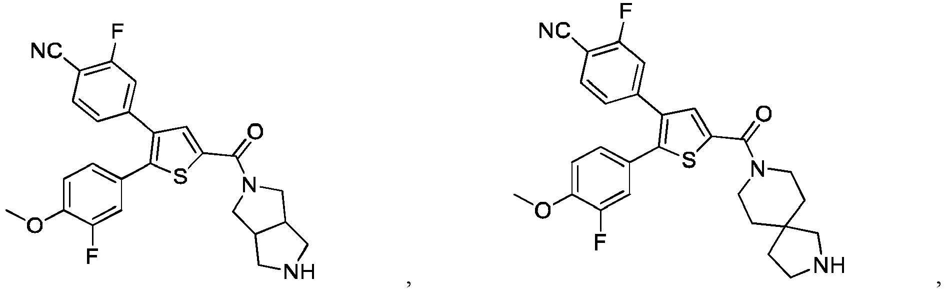

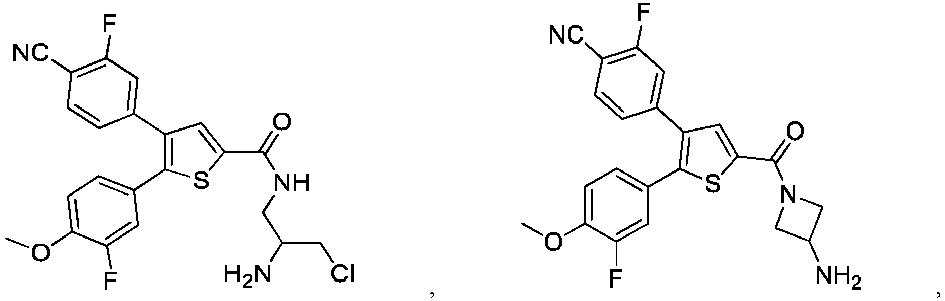

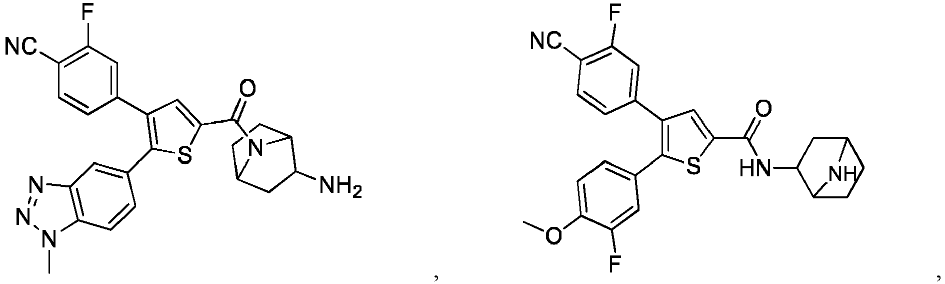

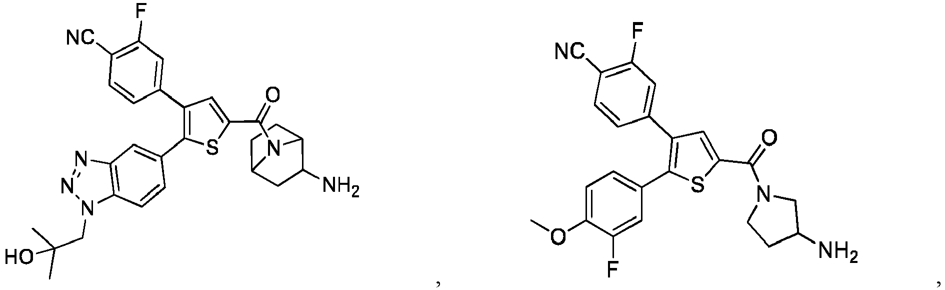

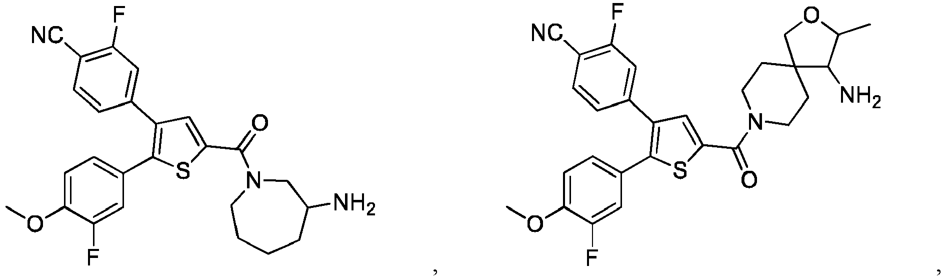

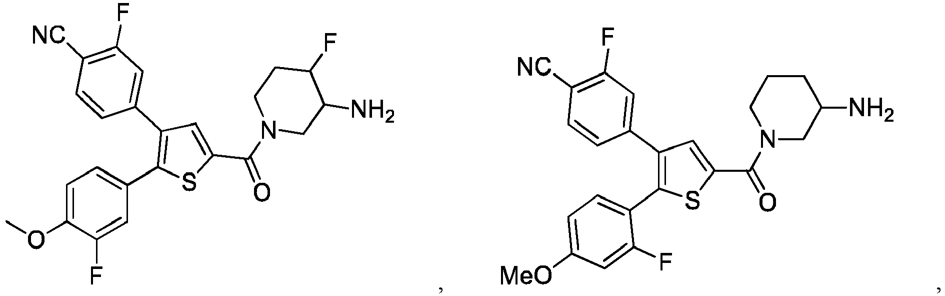

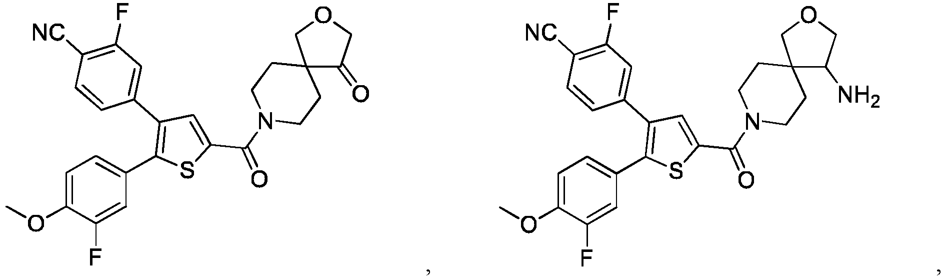

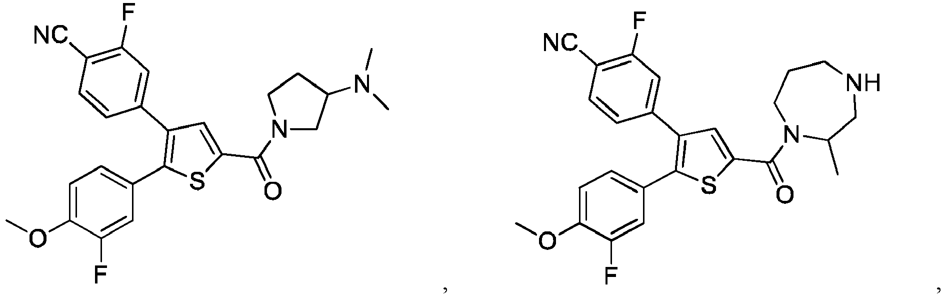

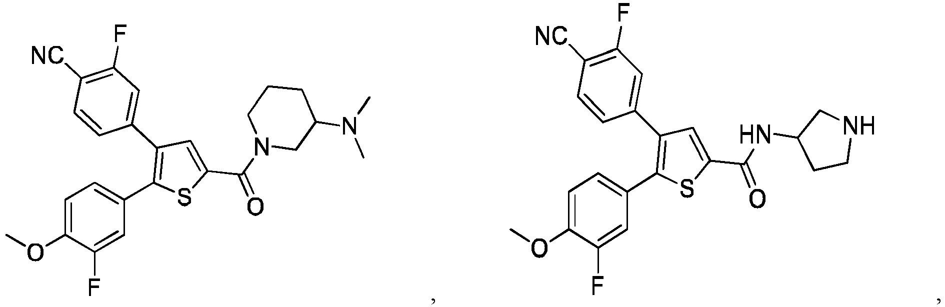

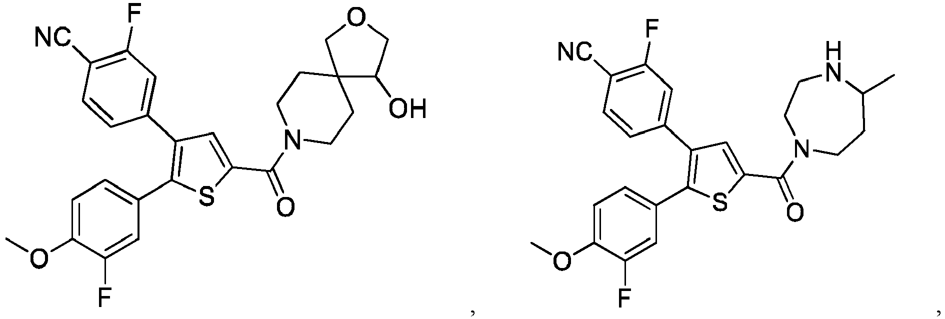

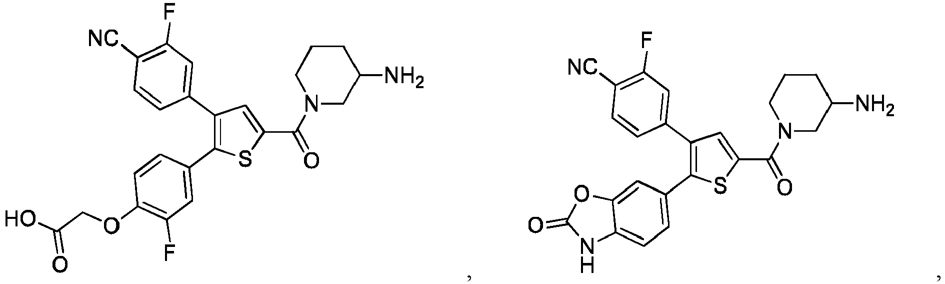

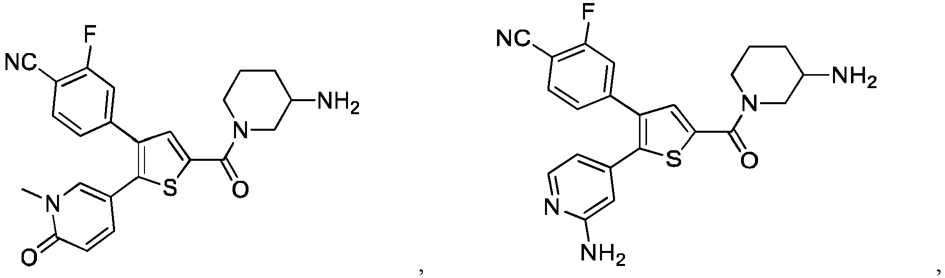

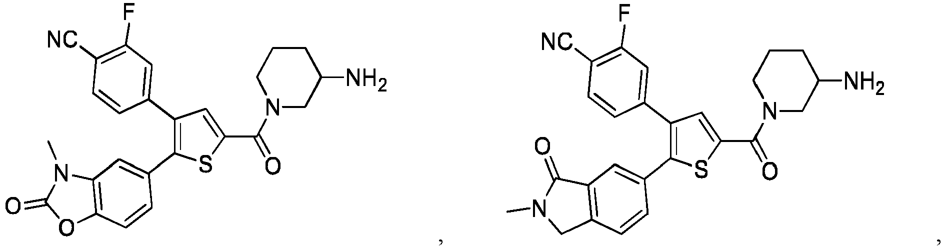

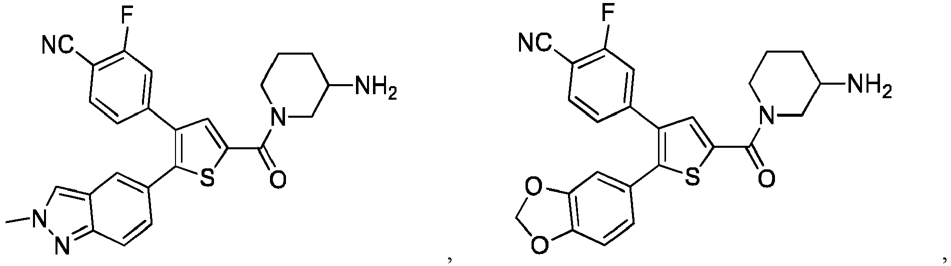

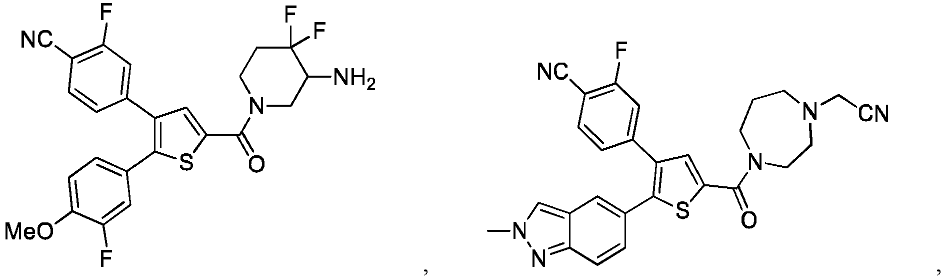

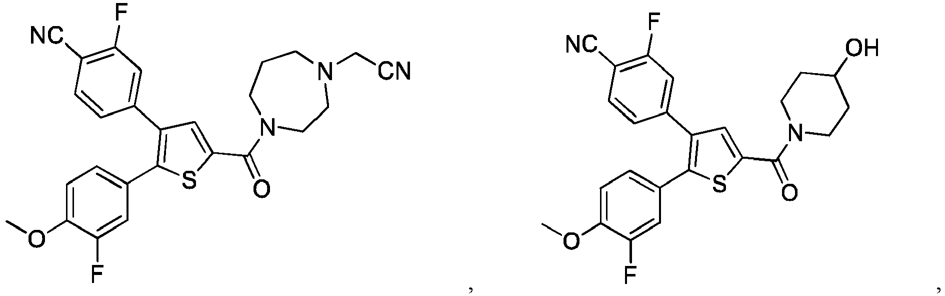

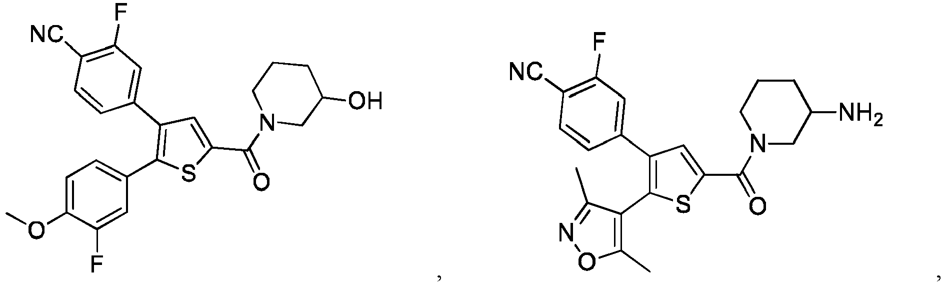

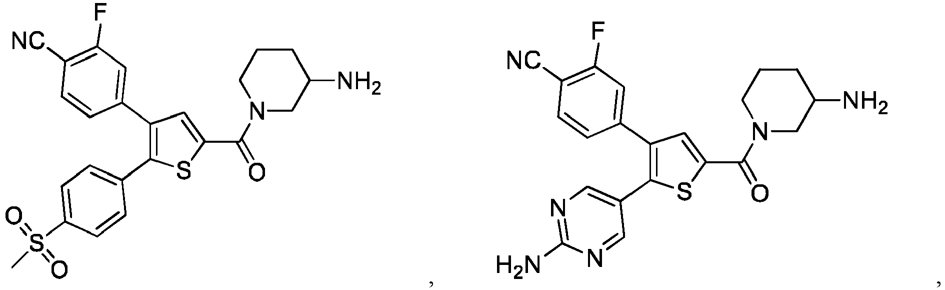

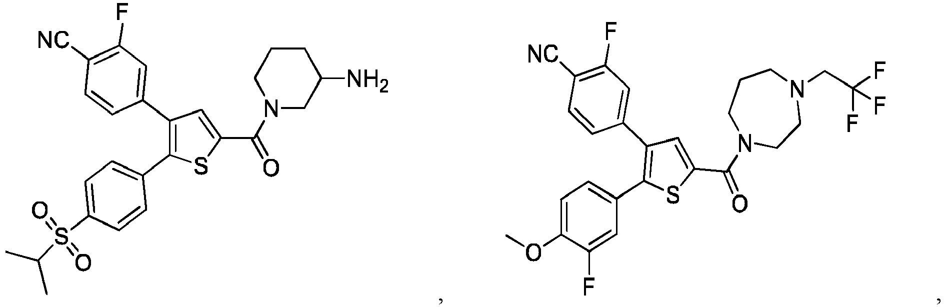

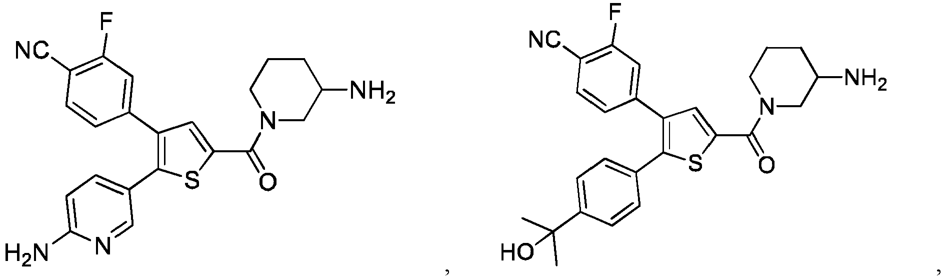

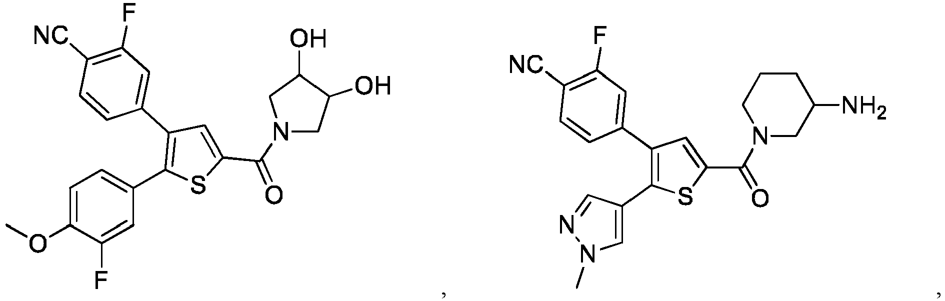

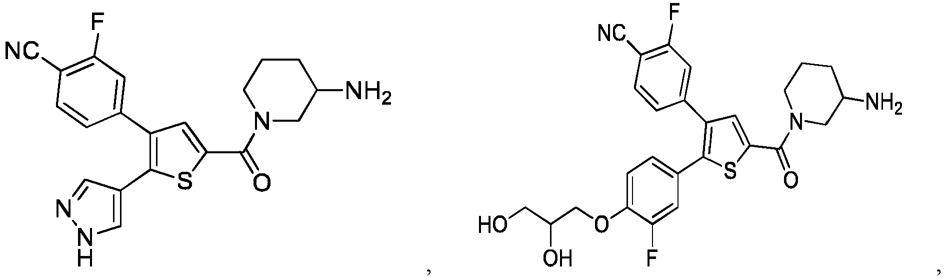

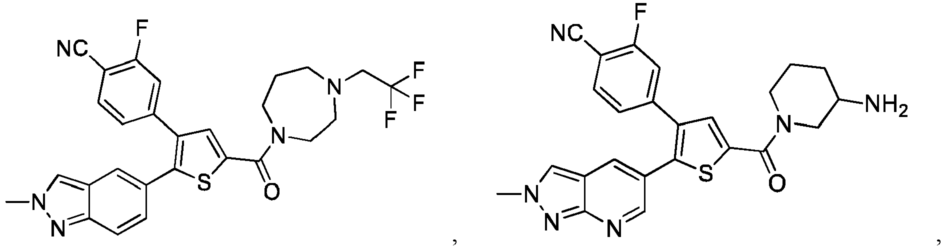

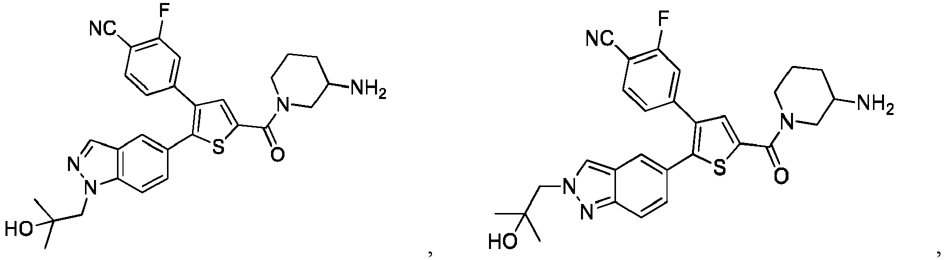

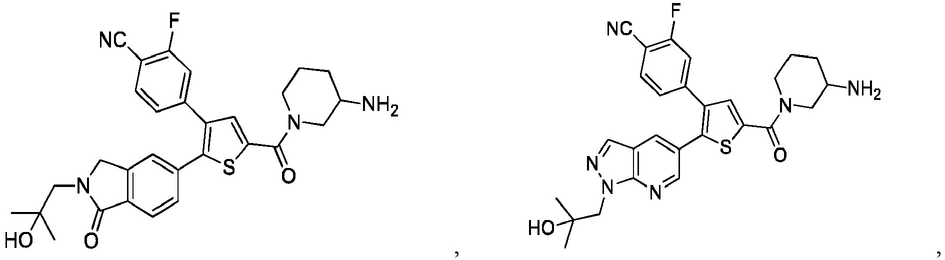

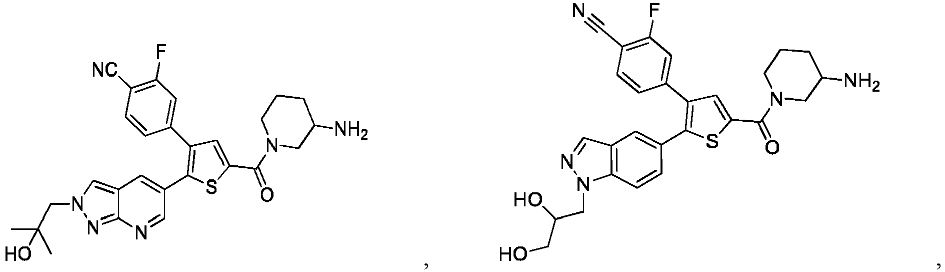

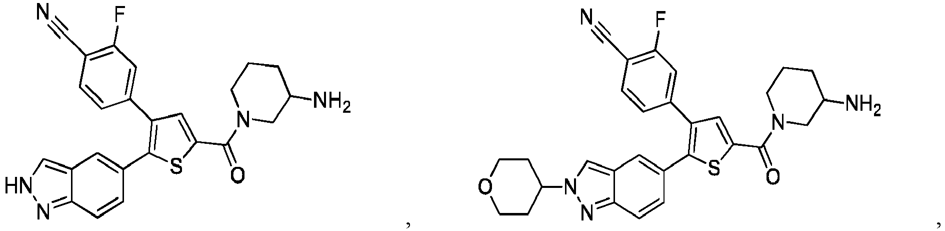

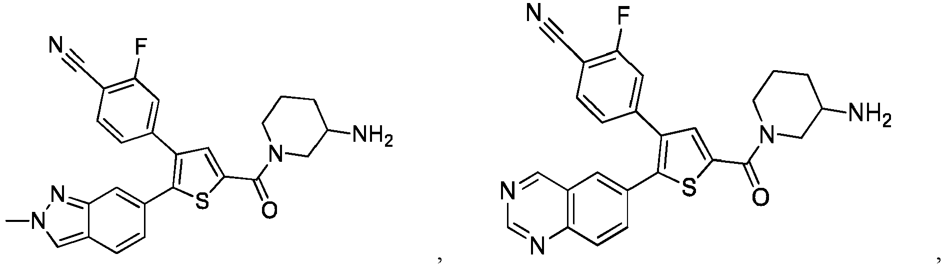

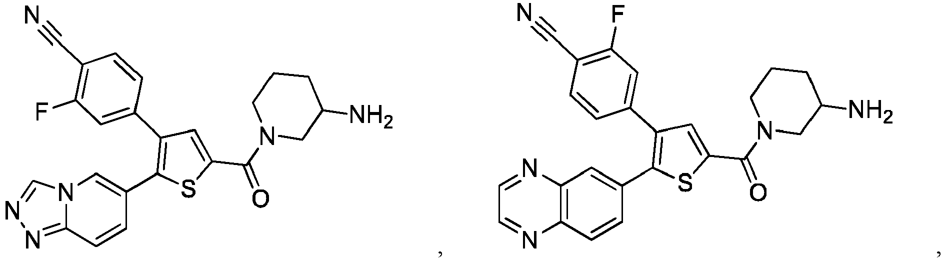

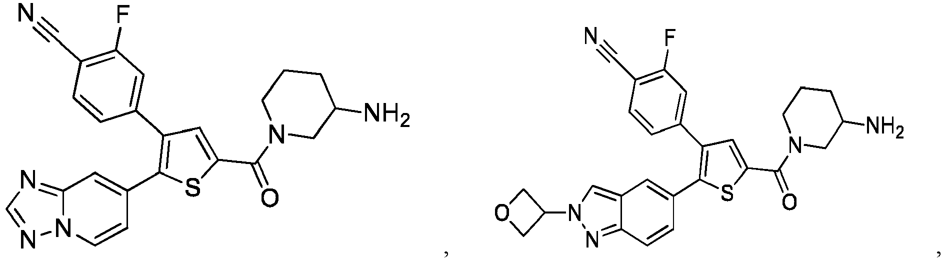

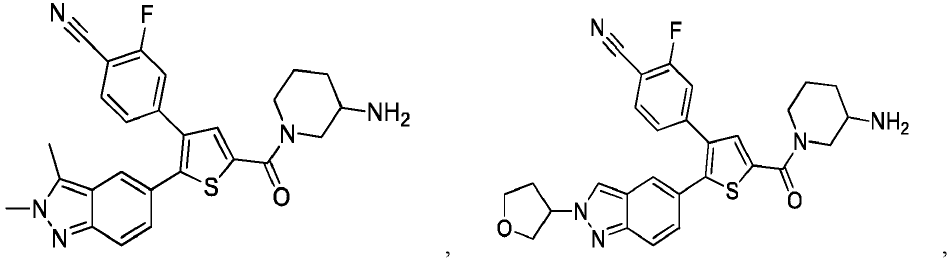

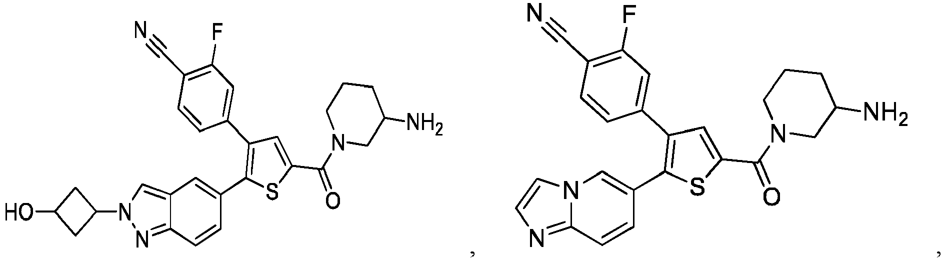

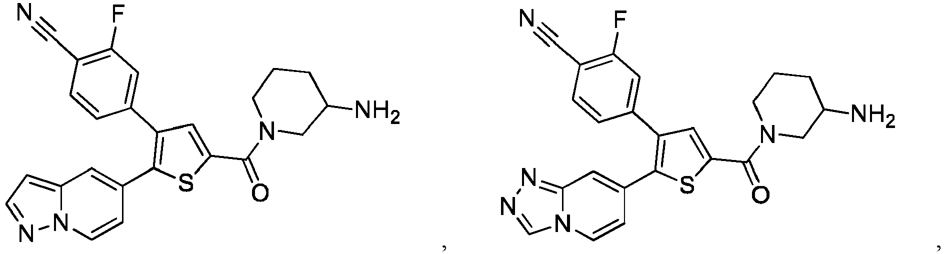

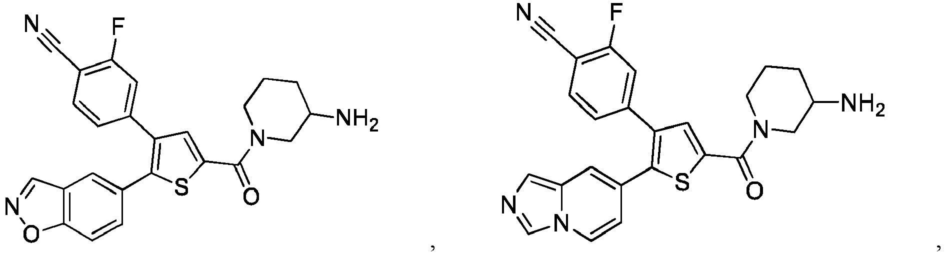

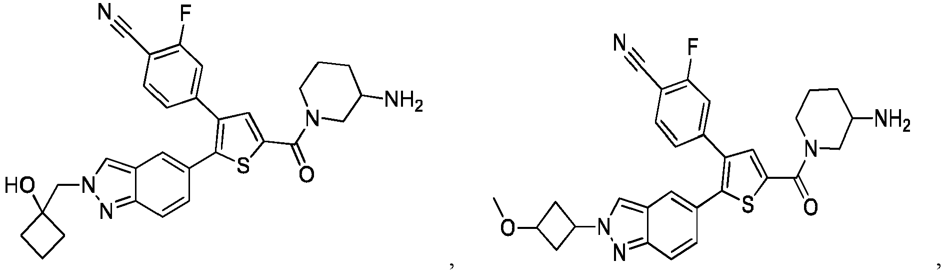

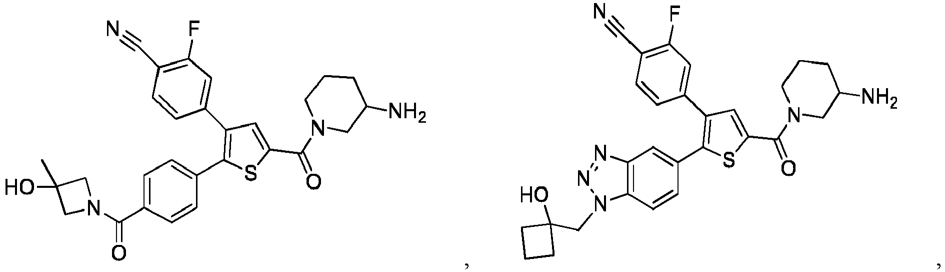

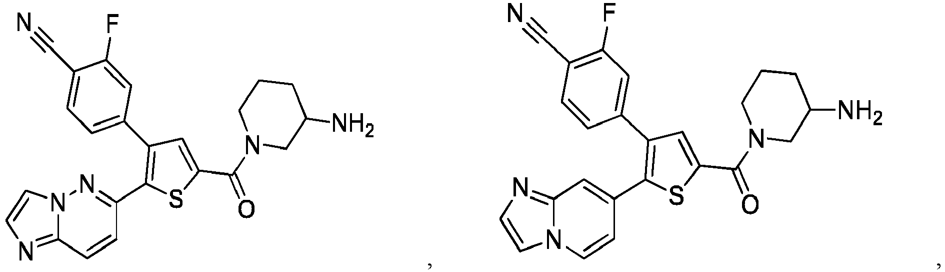

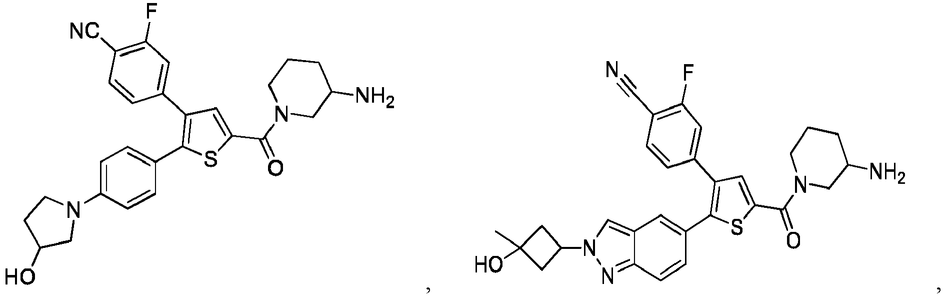

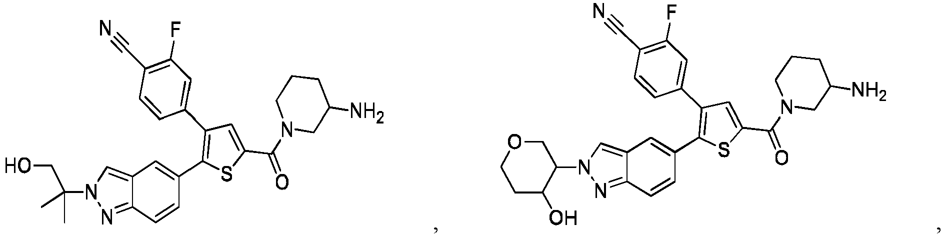

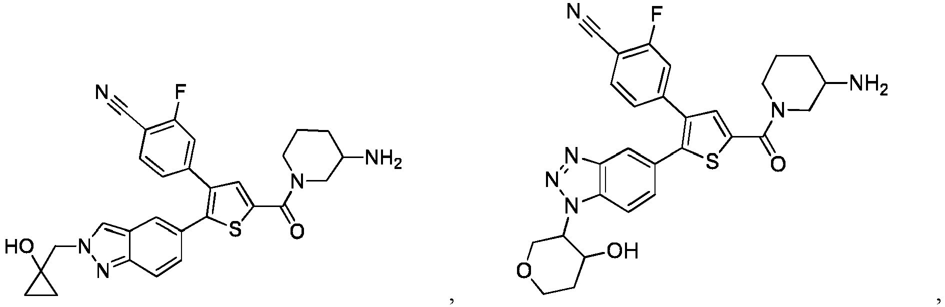

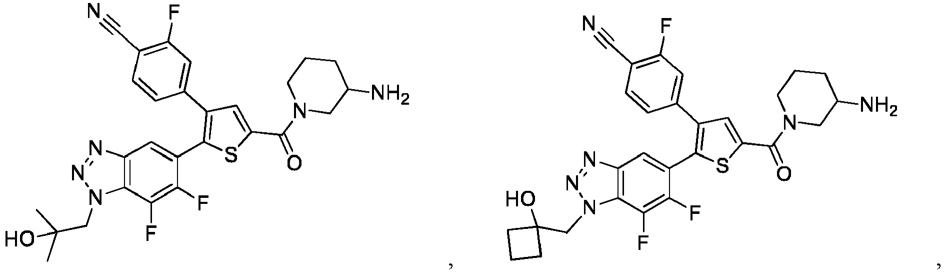

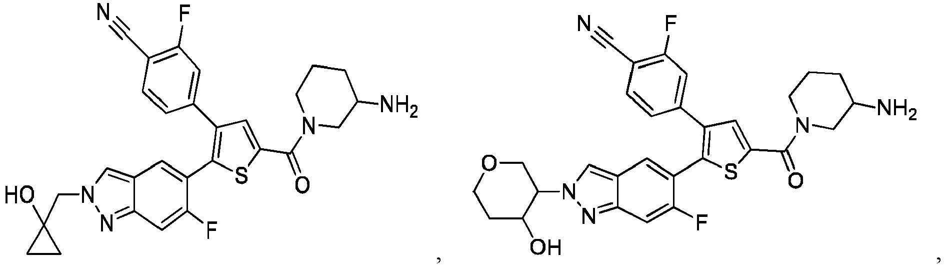

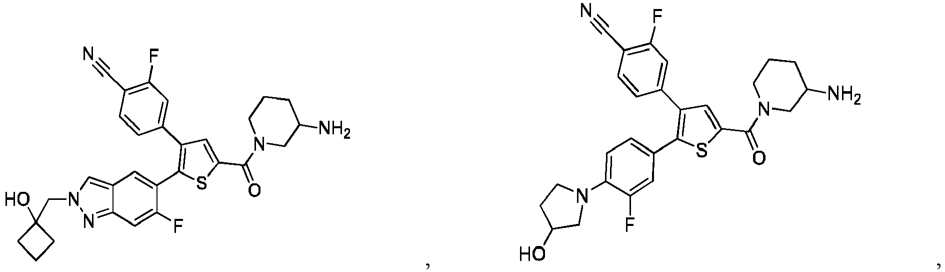

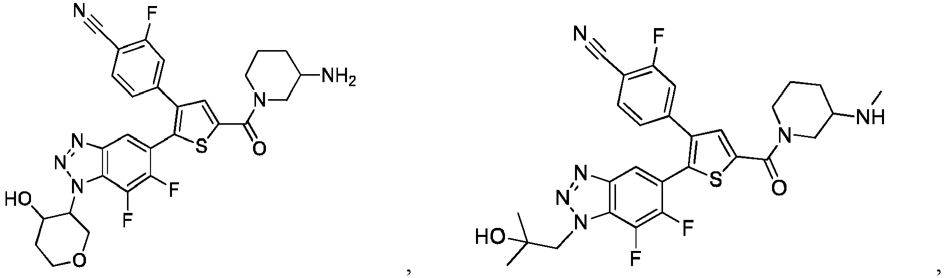

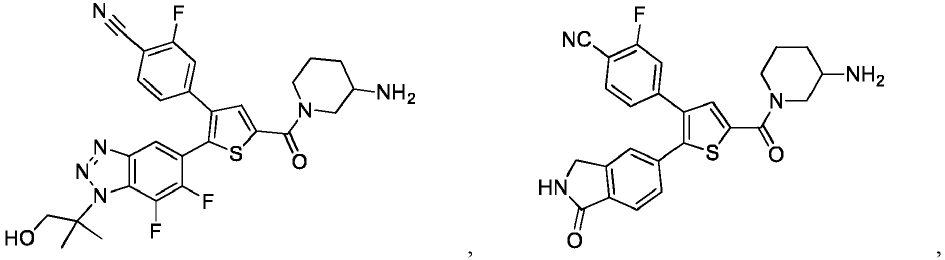

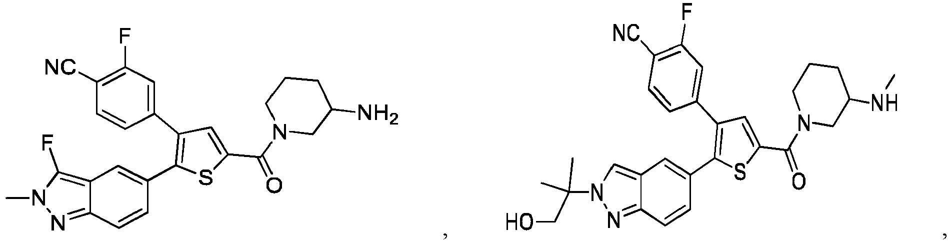

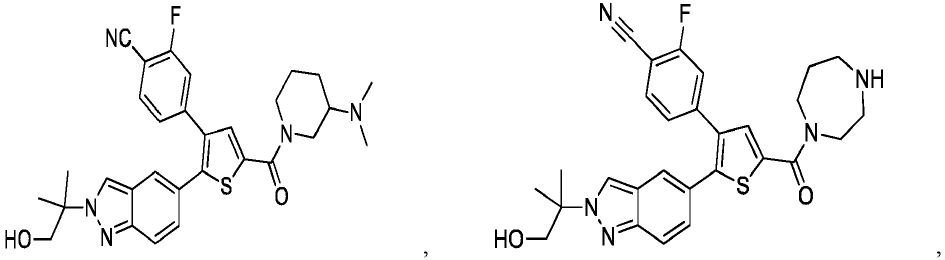

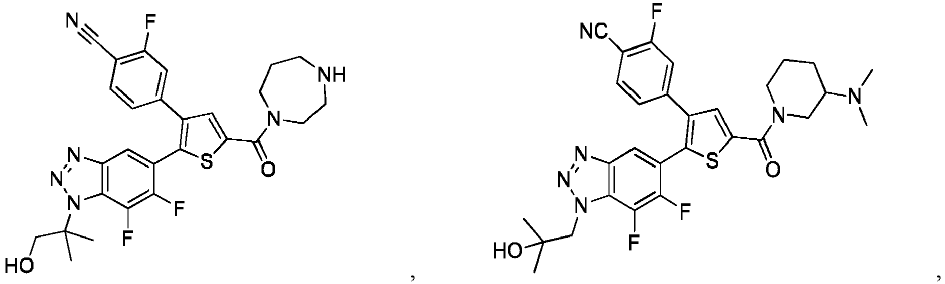

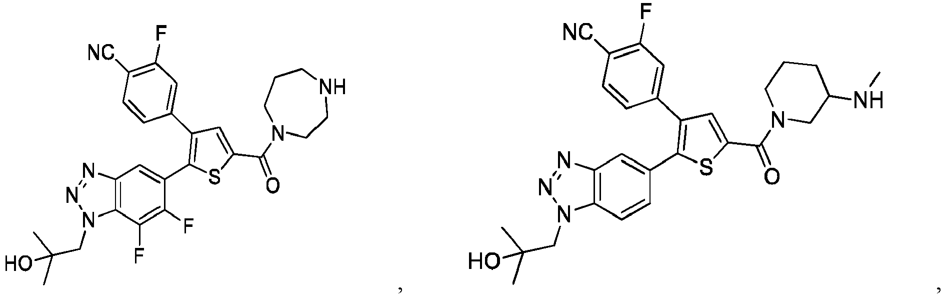

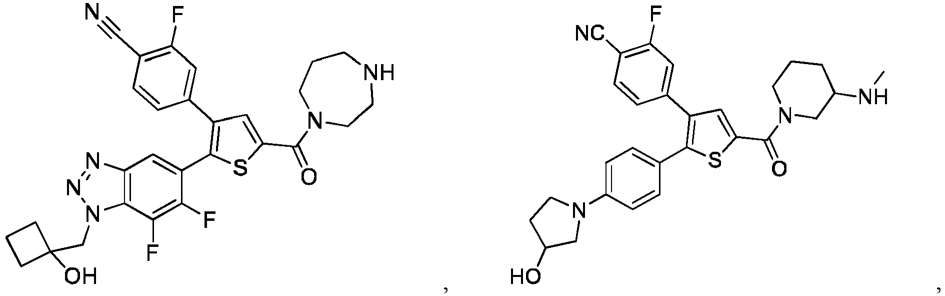

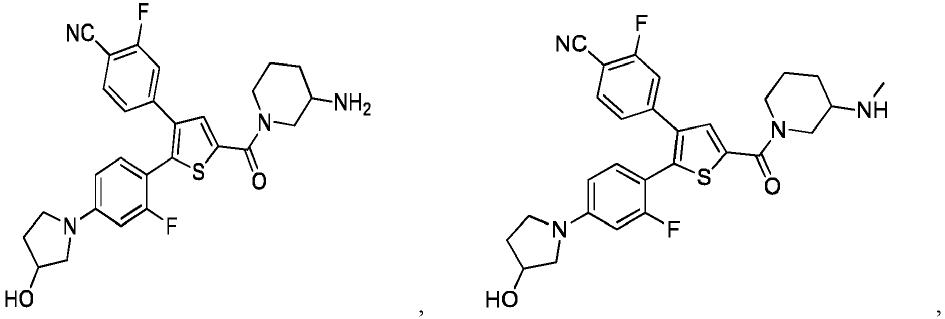

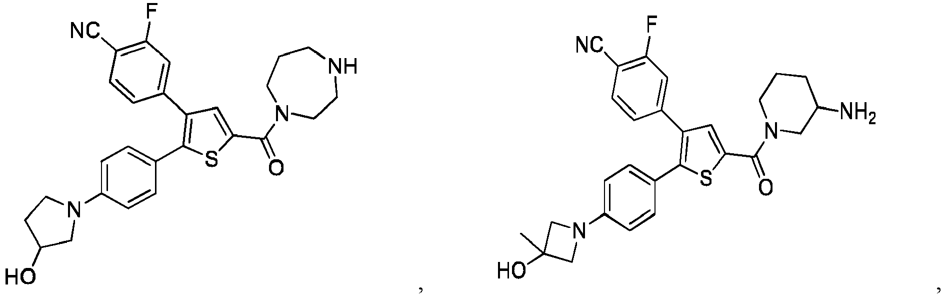

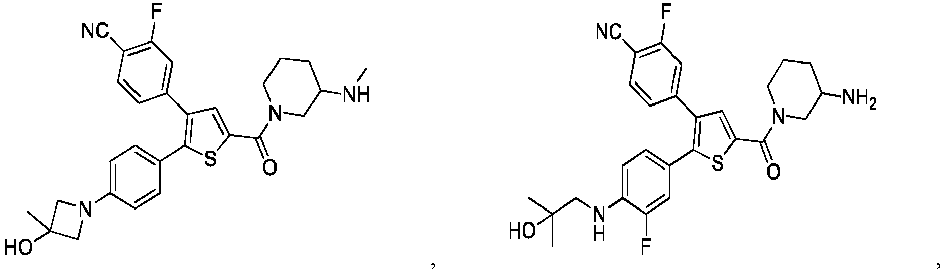

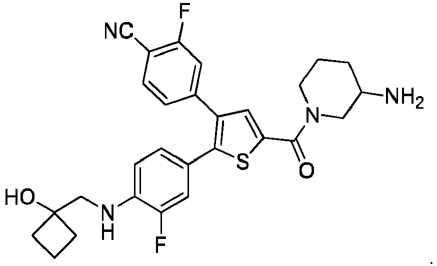

39. The compound or the pharmaceutically acceptable salt thereof according to claim 38, selected from,

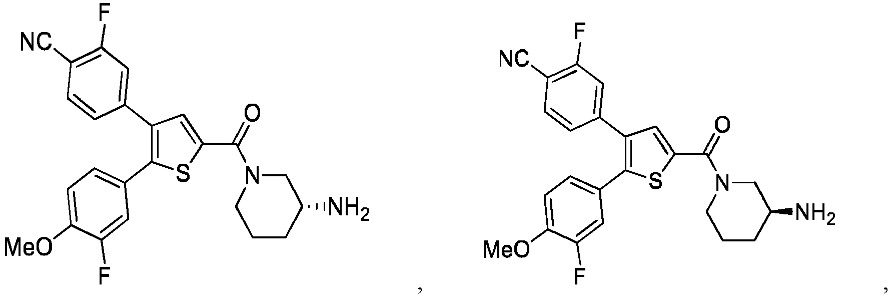

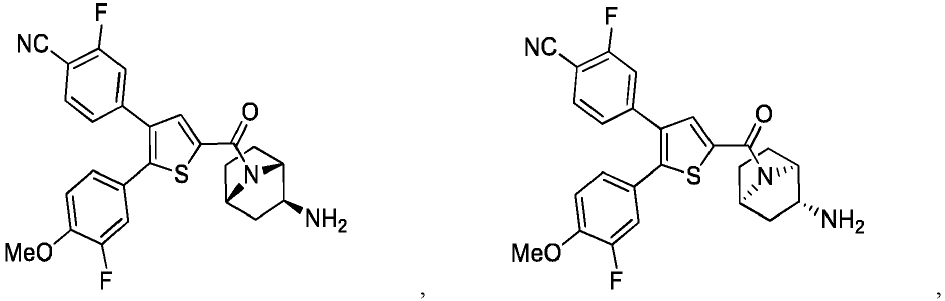

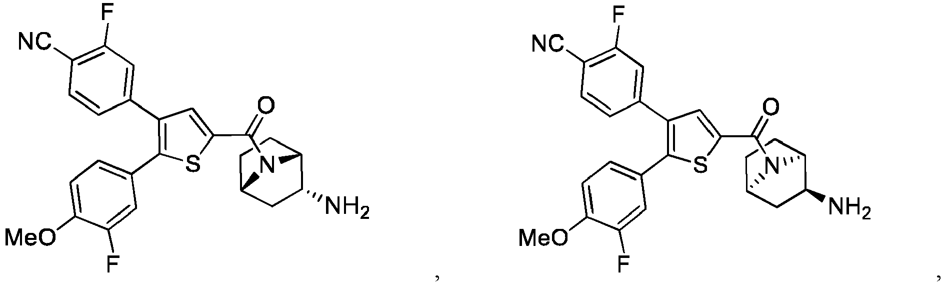

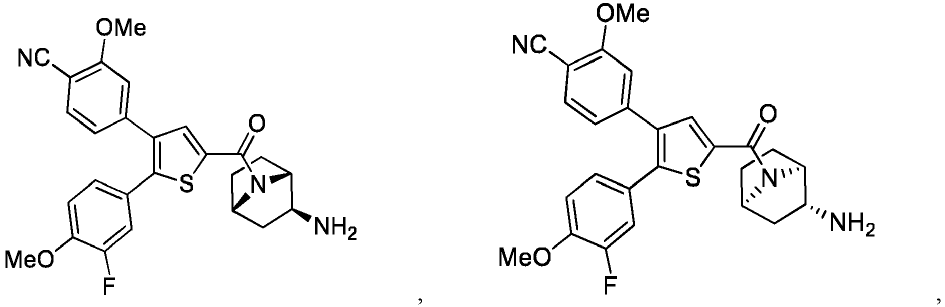

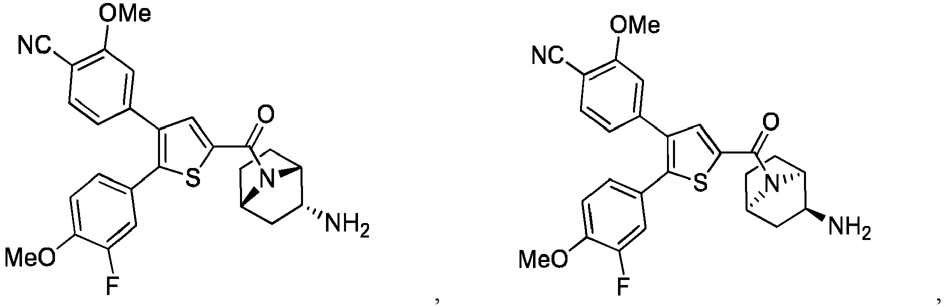

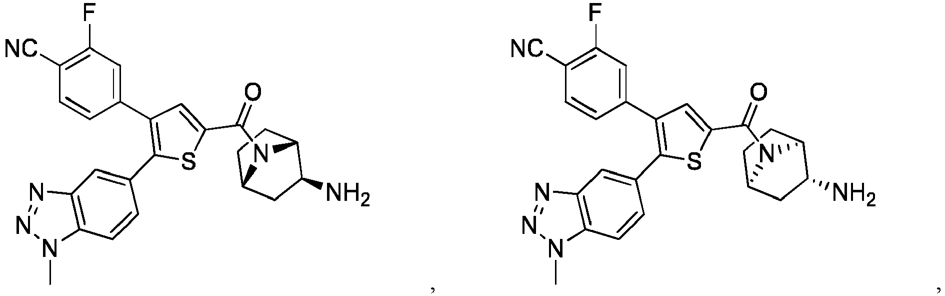

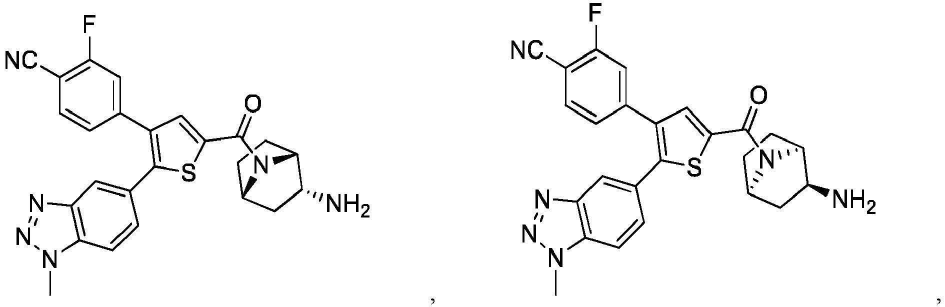

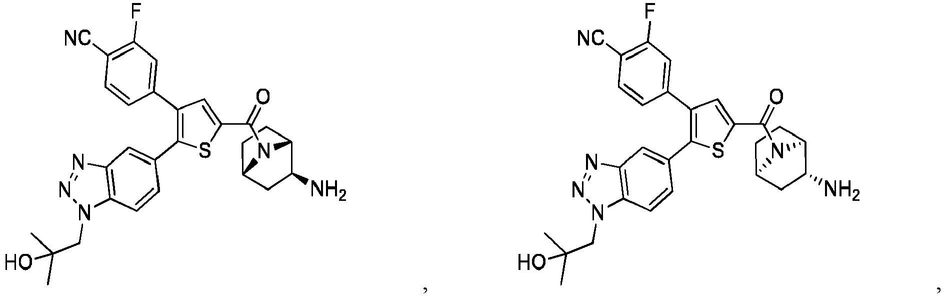

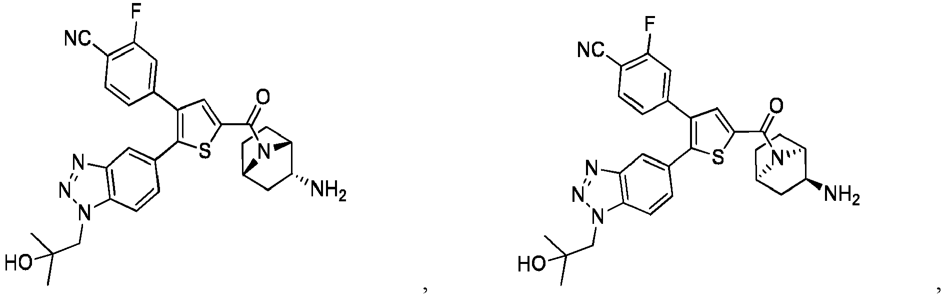

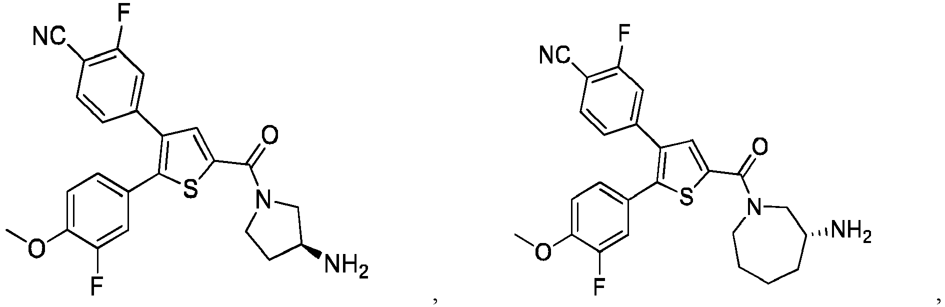

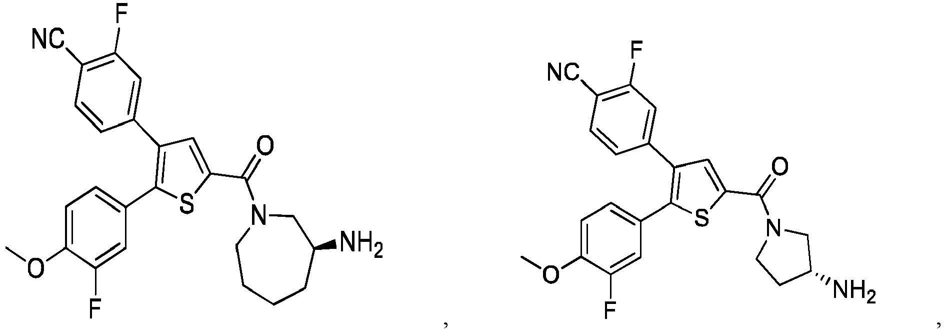

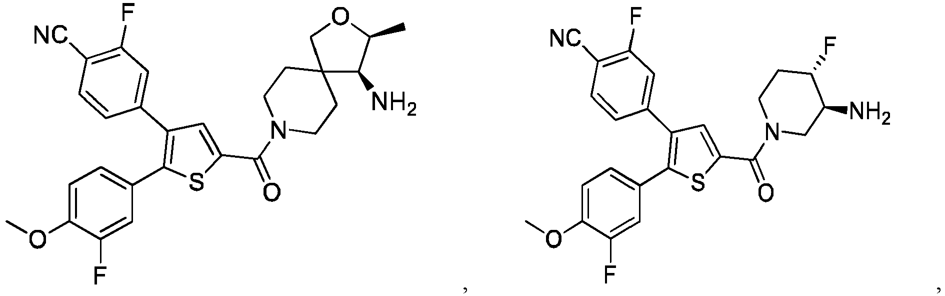

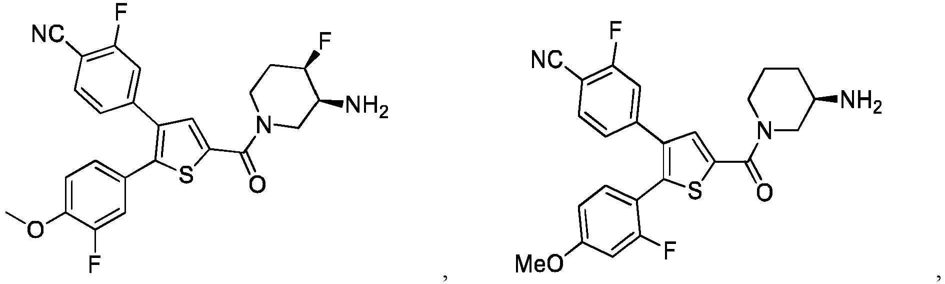

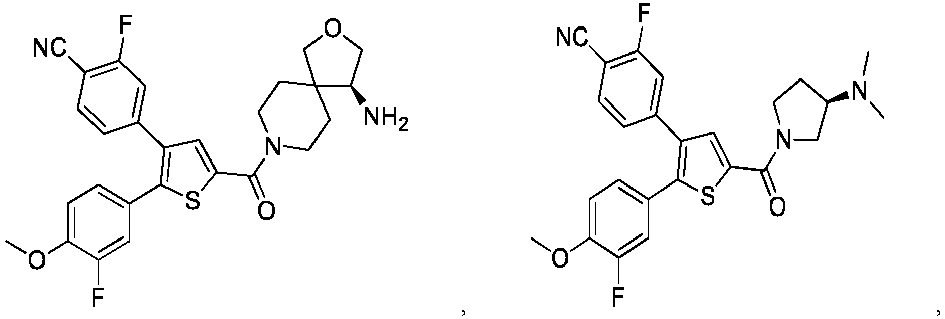

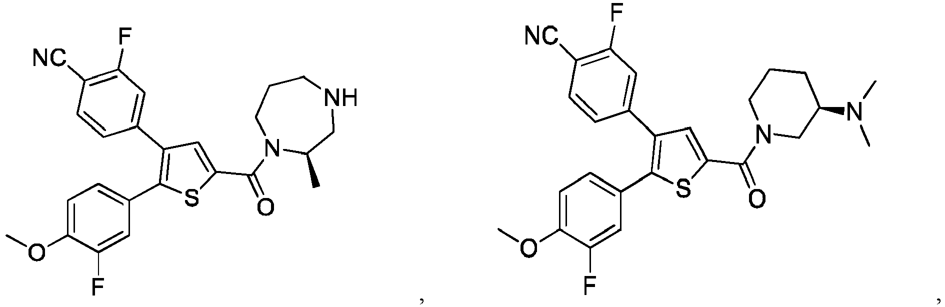

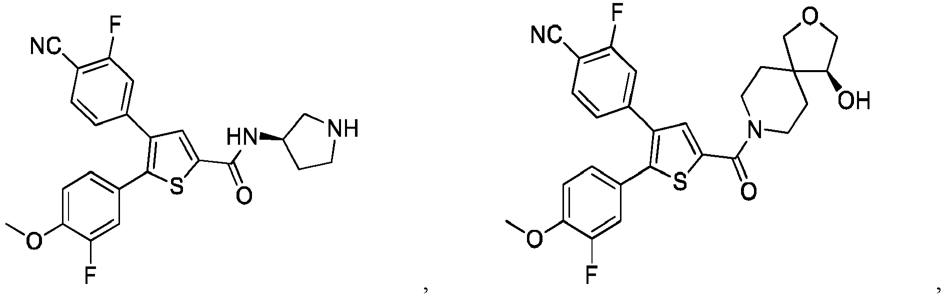

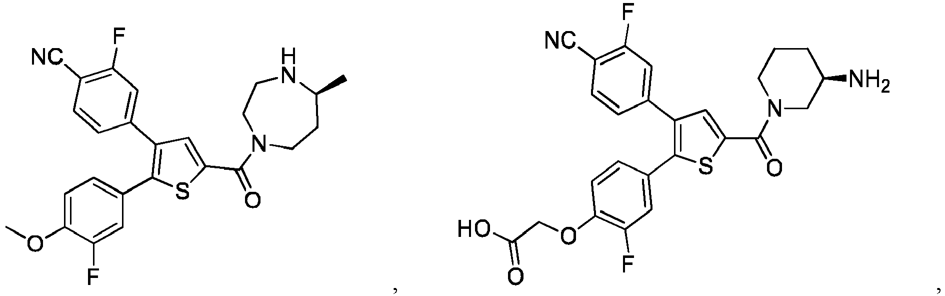

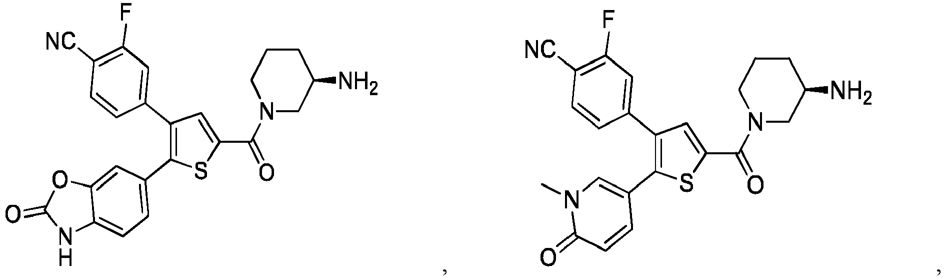

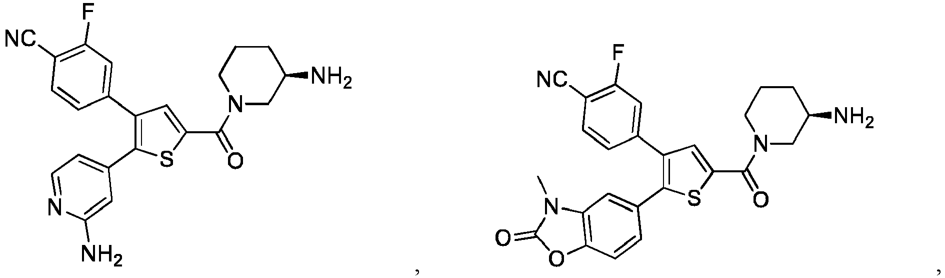

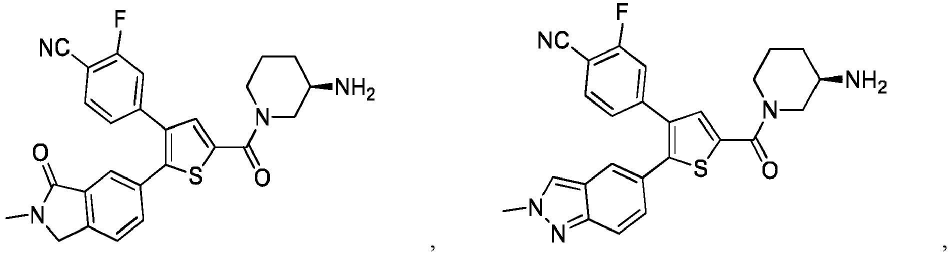

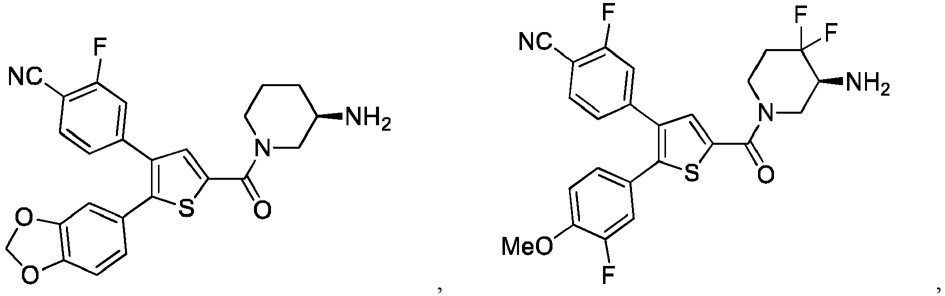

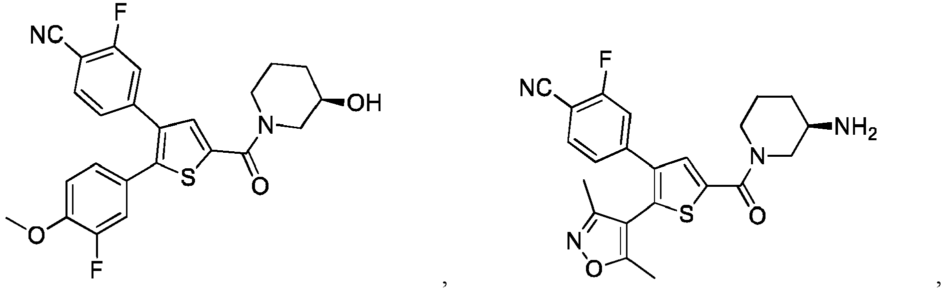

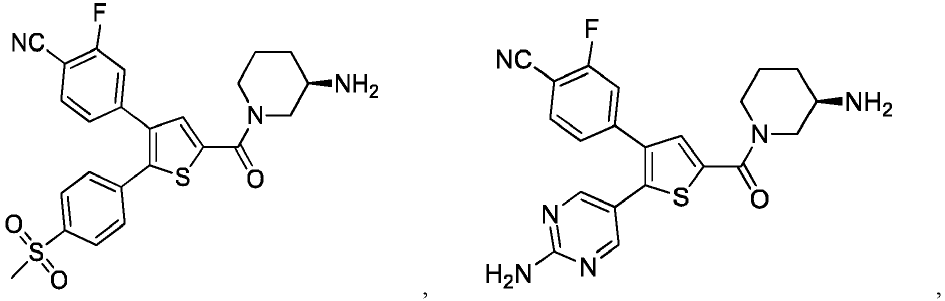

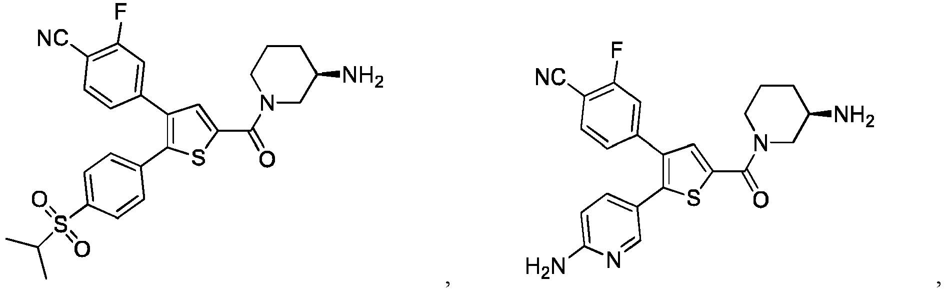

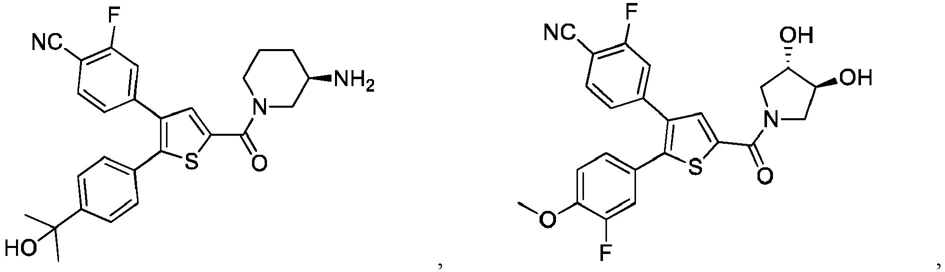

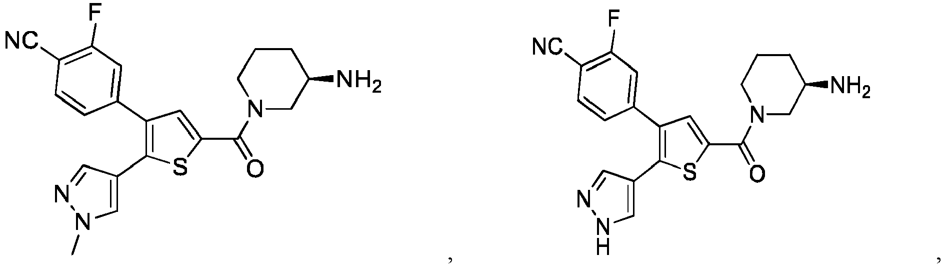

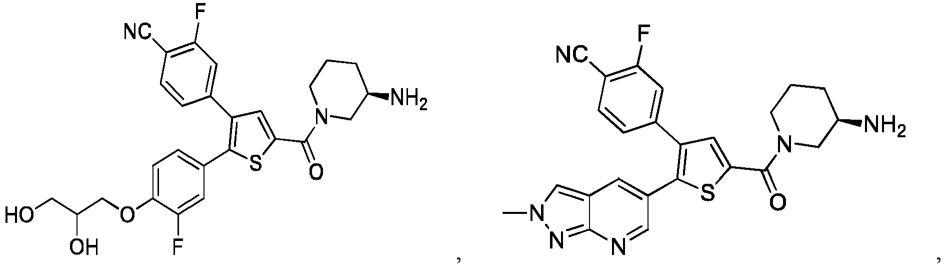

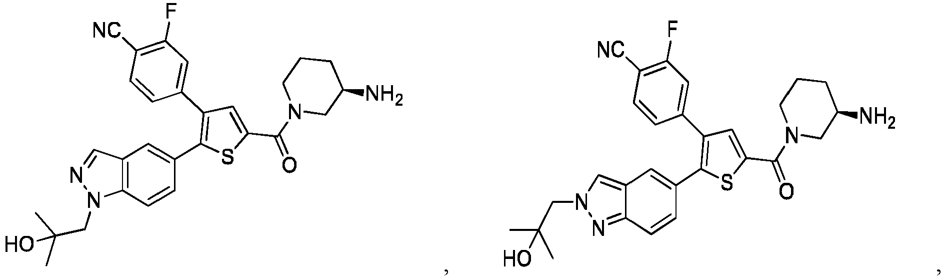

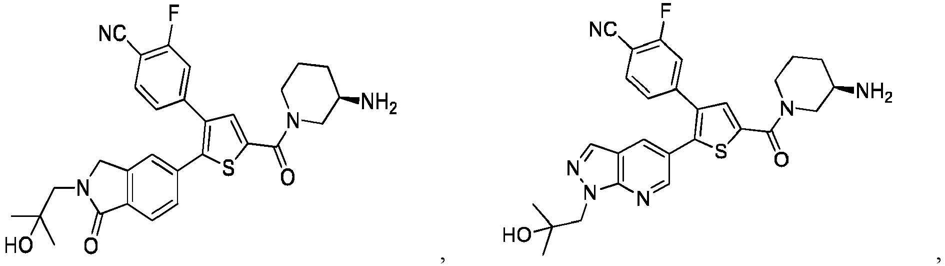

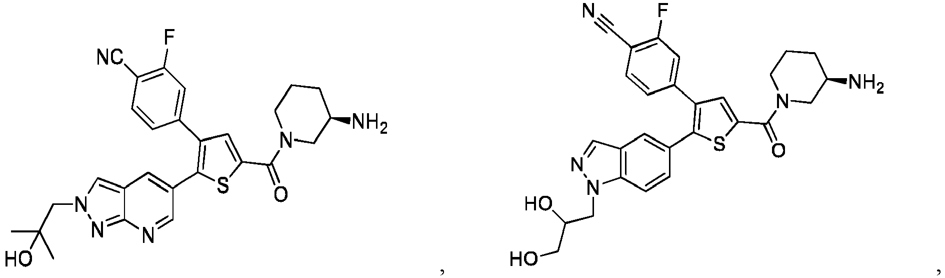

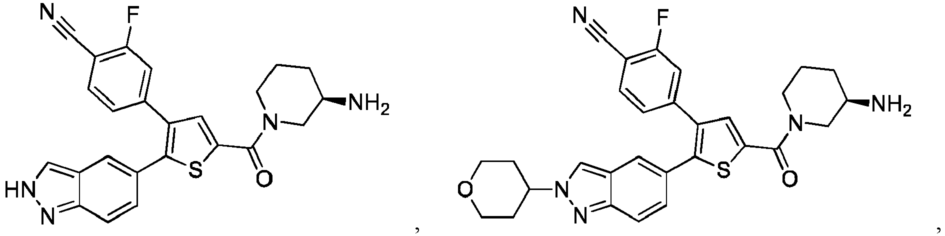

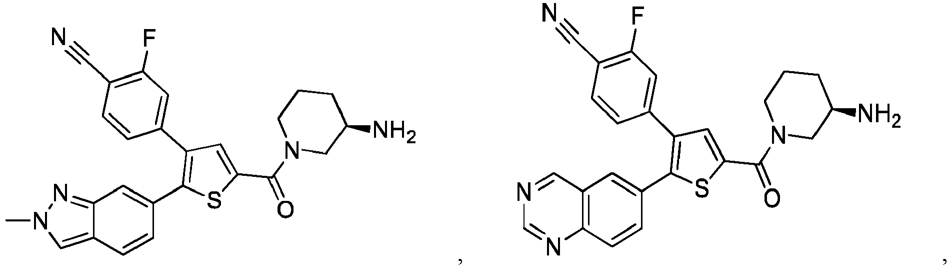

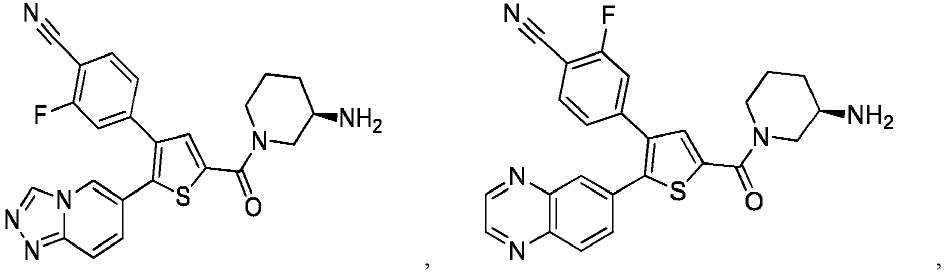

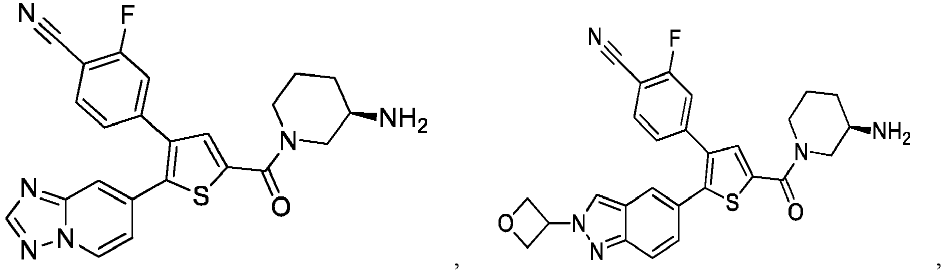

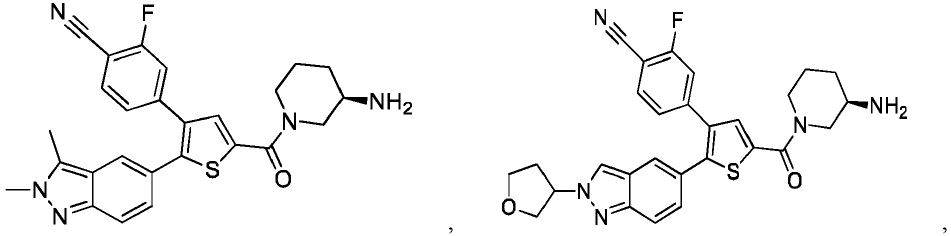

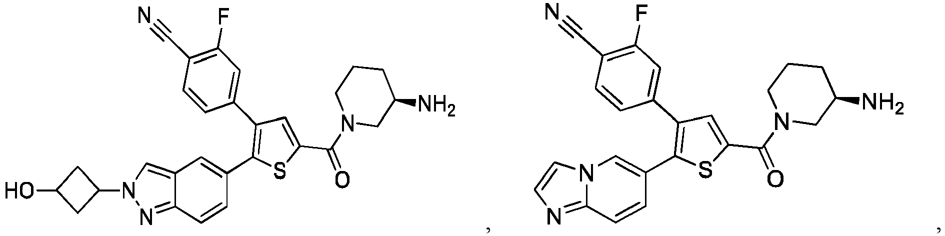

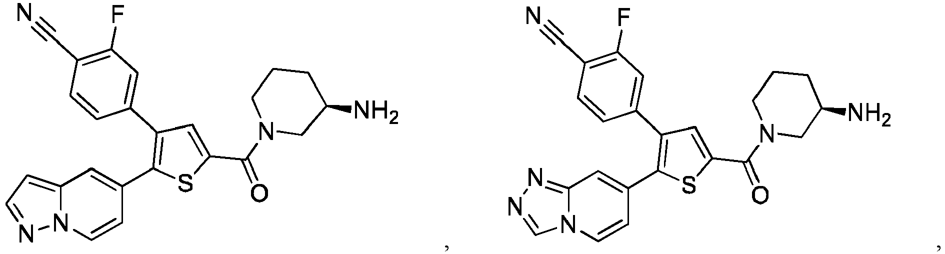

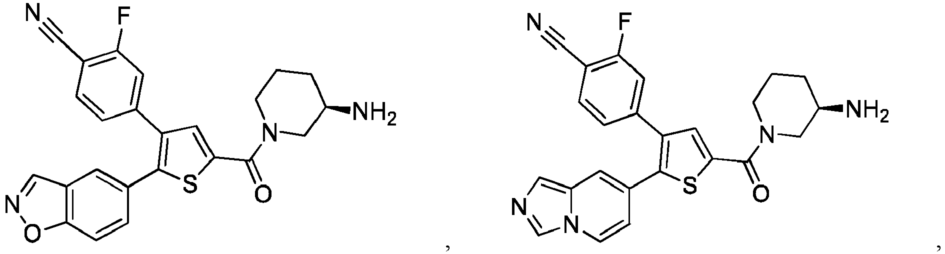

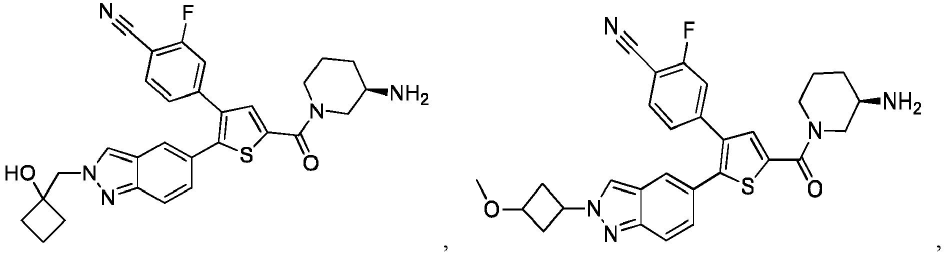

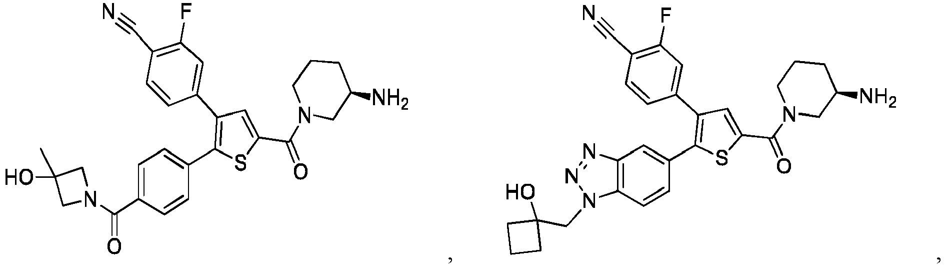

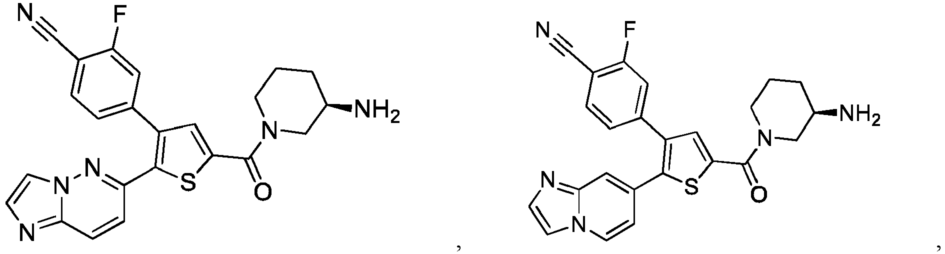

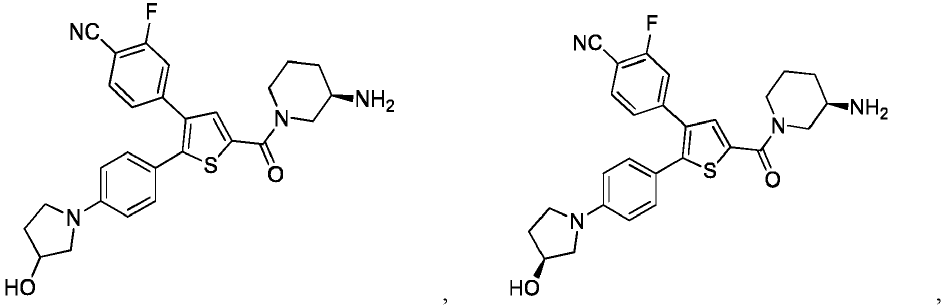

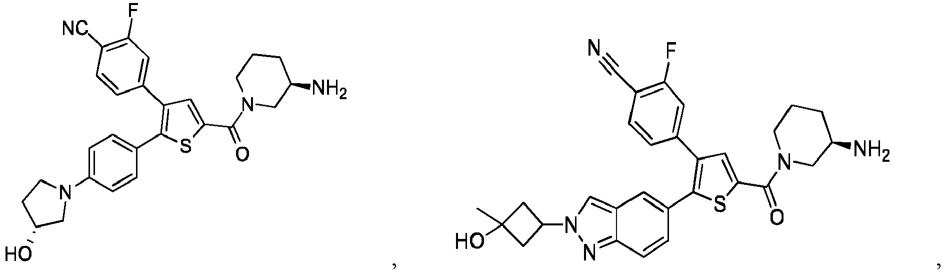

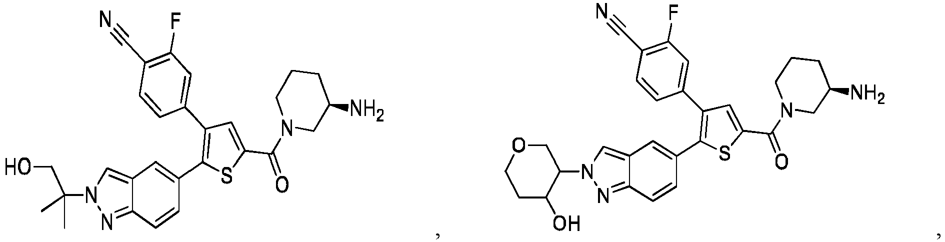

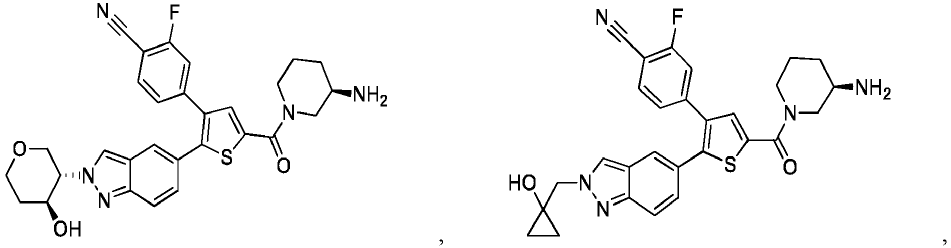

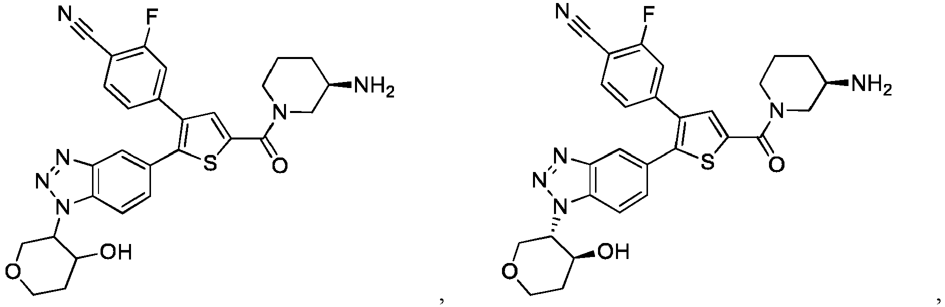

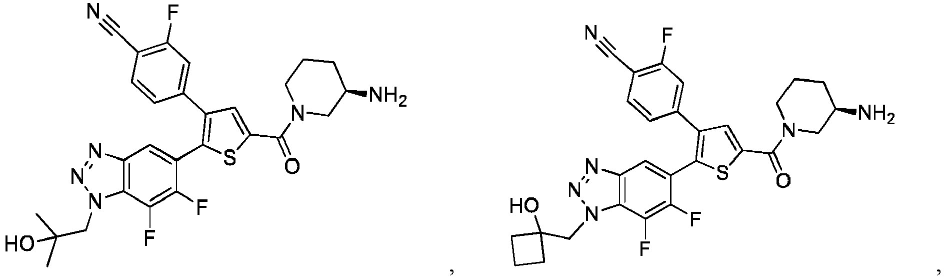

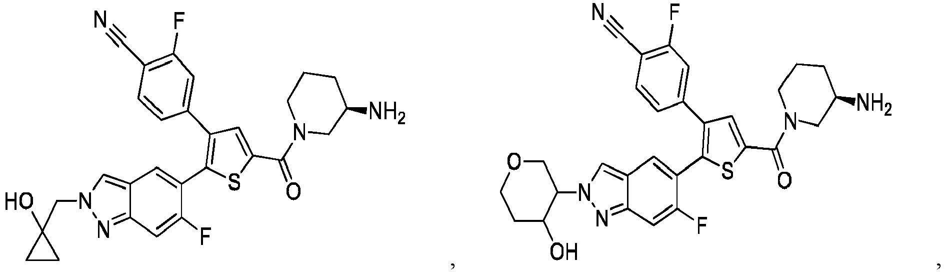

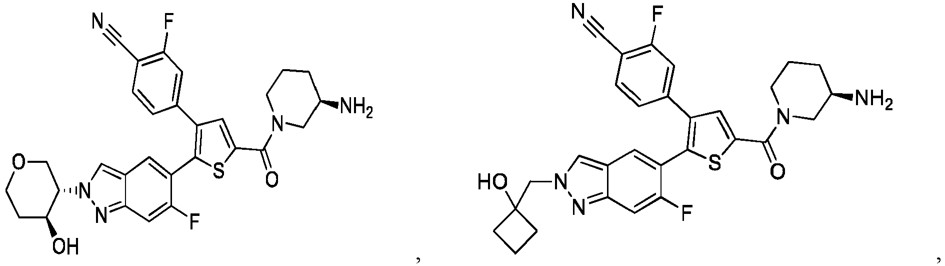

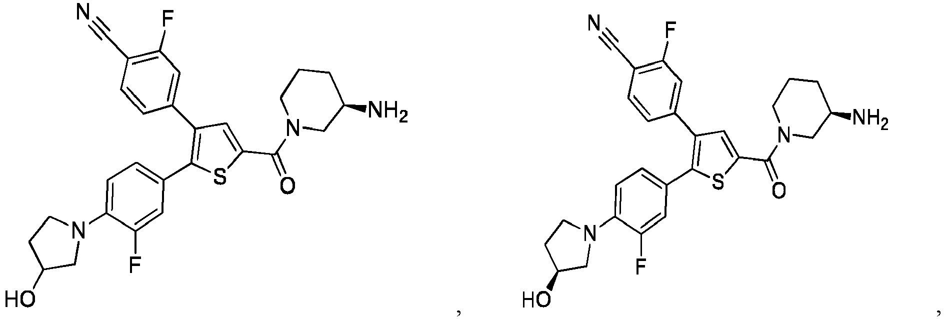

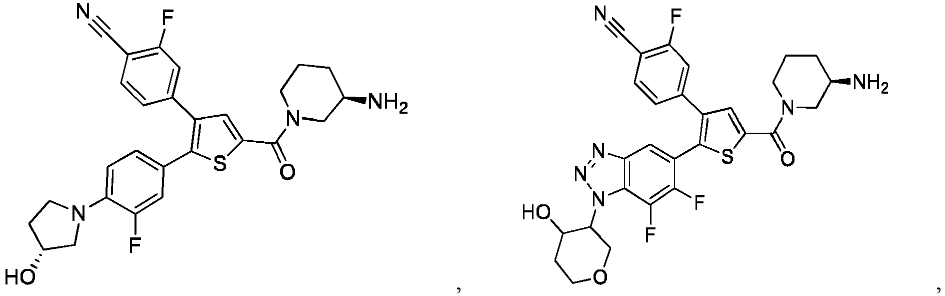

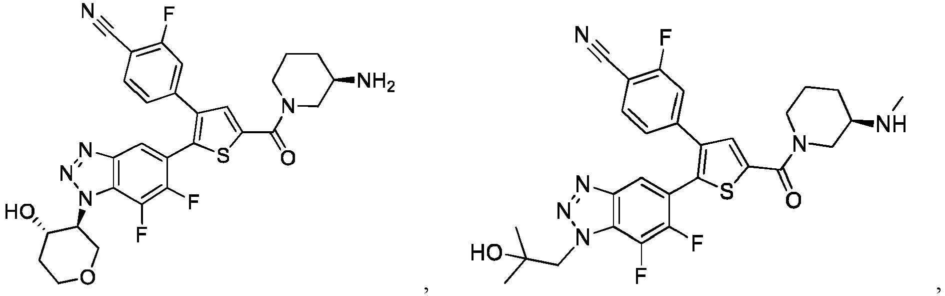

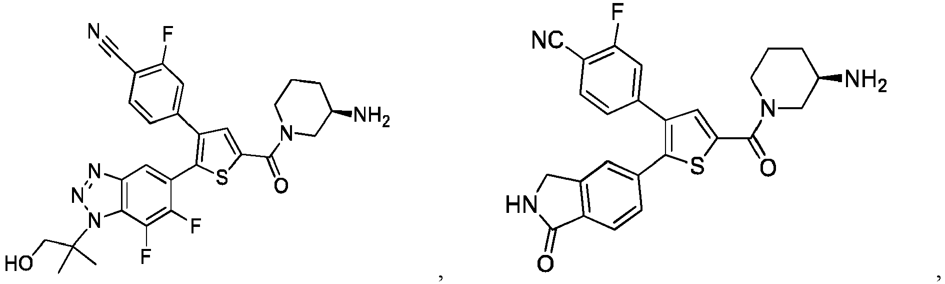

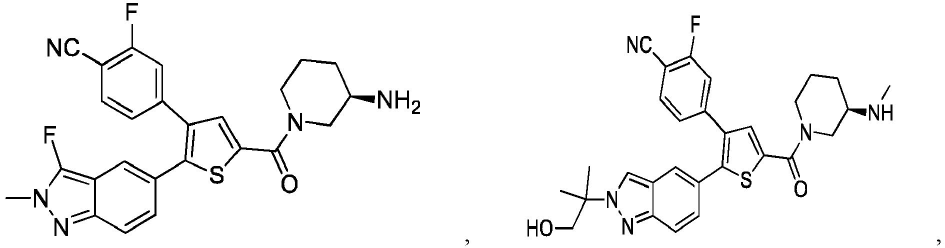

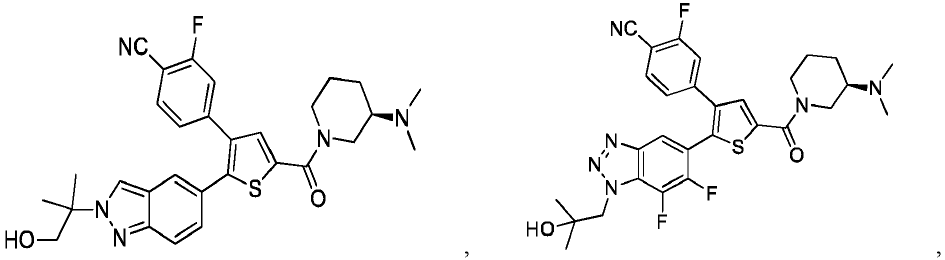

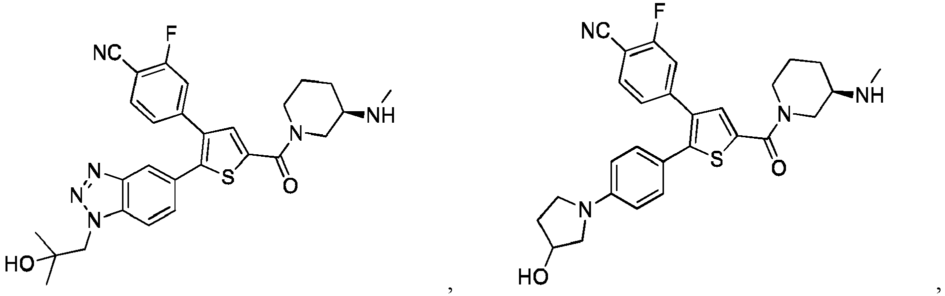

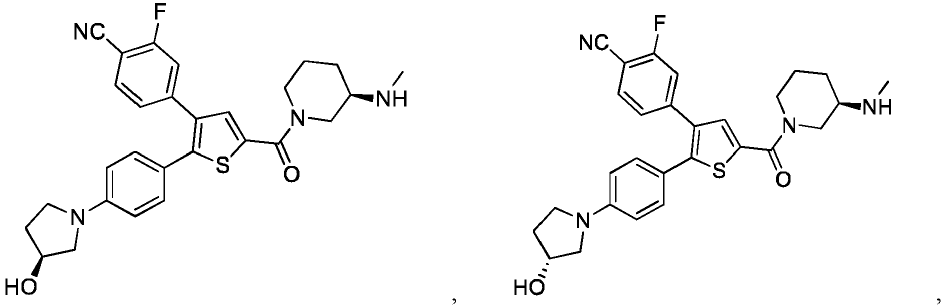

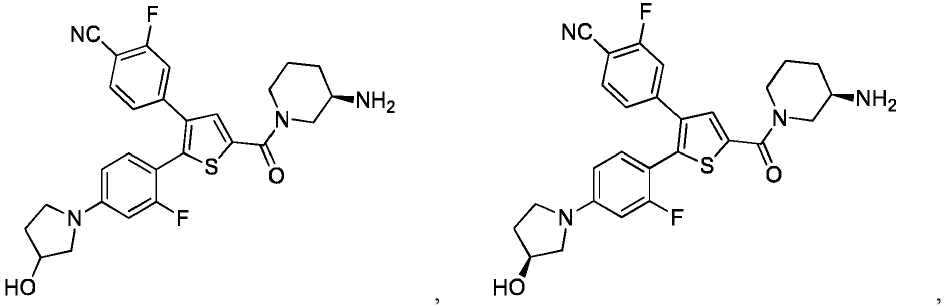

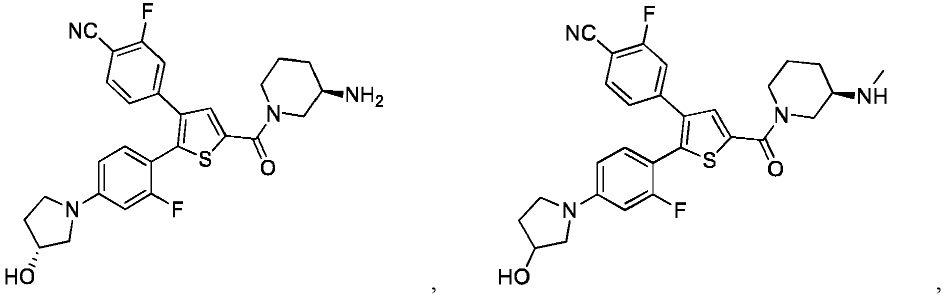

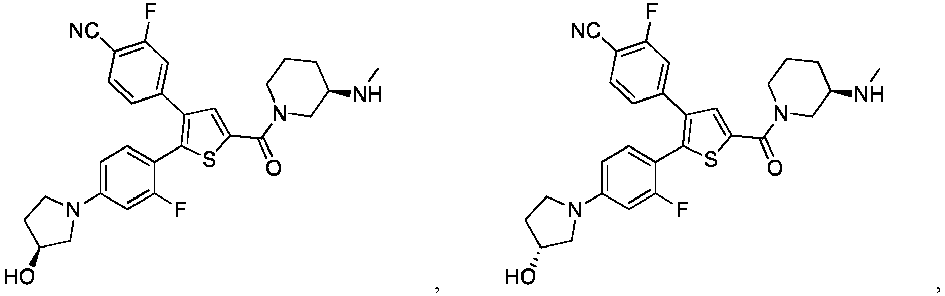

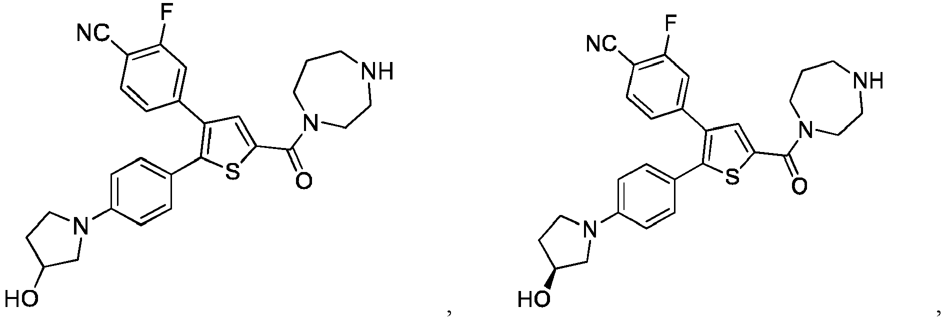

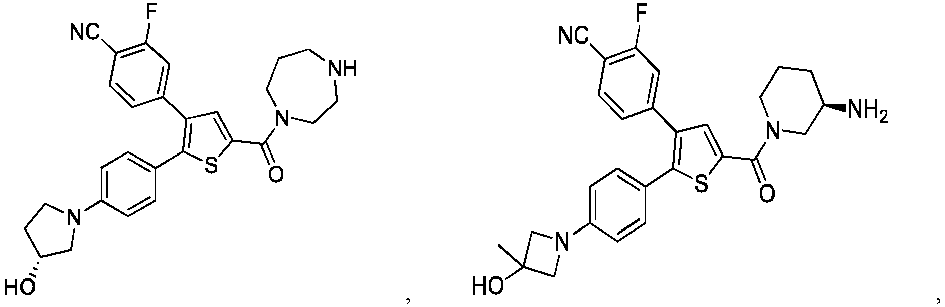

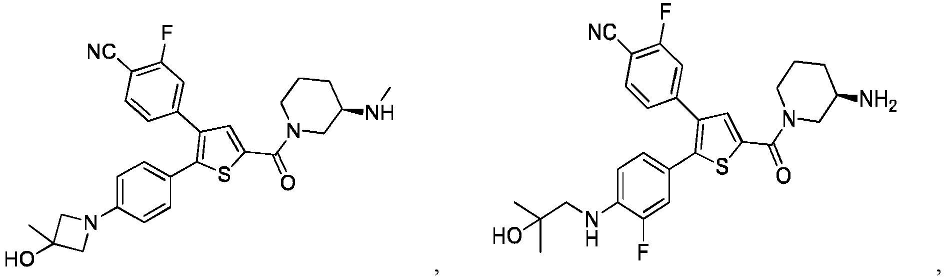

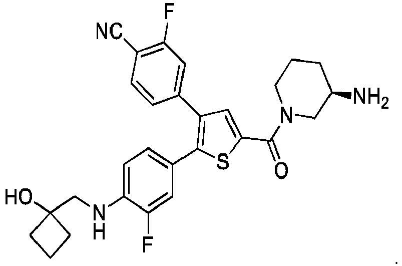

40. The compound or the pharmaceutically acceptable salt thereof according to any one of claims 1 to 39, wherein the salt is hydrochloride.

41. A use of the compound or the pharmaceutically acceptable salt thereof according to any one of claims 1 to 40 in the manufacture of a medicament for treating diseases related to LSD1.
